# EUROPEAN PATENT APPLICATION

(11) **EP 3 308 691 A1**
(43) Date of publication of application: **18.04.2018**
(21) Application number: 16807252.8
(22) Date of filing: 16.05.2016
(51) Int. Cl.: A61B 1/12

(54) **ENDOSCOPY WORK ASSISTANCE SYSTEM**

(30) Priority: 09.06.2015 JP 2015116969; 17.06.2015 JP 2015122297; 17.06.2015 JP 2015122298
(71) Applicant: Olympus Corporation, Hachioji-shi, Tokyo 192-8507 (JP)
(72) Inventor: HOSOI Takaharu, Hachioji-shi Tokyo 192-8507 (JP); NISHIMURA Hirokazu, Hachioji-shi Tokyo 192-8507 (JP); YADA Kohei, Hachioji-shi Tokyo 192-8507 (JP); ISHIBASHI Katsuyoshi, Hachioji-shi Tokyo 192-8507 (JP); BABA Toshiro, Hachioji-shi Tokyo 192-8507 (JP); HORI Yasuyuki, Hachioji-shi Tokyo 192-8507 (JP); AKIMOTO Hiromi, Hachioji-shi Tokyo 192-8507 (JP)
(74) Representative: Gunzelmann, Rainer
(86) International application number: PCT/JP2016/064441
(87) International publication number: WO 2016/199545

(57) **Abstract**

An examination schedule management unit manages an examination schedule of a plurality of endoscopic examinations, including an examination room where an endoscopic examination is to be performed, information on scheduled examination start time, that on scheduled examination end time, and examination type information on an examination content of an endoscopic examination. A cleaning schedule management unit manages a cleaning schedule of a plurality of endoscopes, including a cleaning machine, information on scheduled cleaning start time, and that on scheduled cleaning end time. A situation information acquisition unit acquires situation information on a situation of an examination, and a rescheduling processing unit determines based on the situation information that it is necessary to change the examination schedule and/or the cleaning schedule, and instructs at least one of the examination schedule management unit and the cleaning schedule management unit to change an element included in the examination schedule and/or the cleaning schedule.

## Description

### BACKGROUND OF THE INVENTION

### 1. Field of the Invention

The present invention relates to a technique for supporting endoscopic examination work in which scheduling of endoscopes to be used in endoscopic examinations is performed.

### 2. Description of the Related Art

The order of an endoscopic examination (hereinafter, also referred to as an "examination order") is generated in an in-hospital information system such as, for example, an ordering system, and is issued to an endoscopy department system. The examination order includes order information on an endoscopic examination, such as examination identification information (examination ID), scheduled examination start time and examination end time, patient identification information (patient ID), examination type, primary doctor of examination, and examination room.

An examination schedule for one day in an endoscopy department is generated by a plurality of examination orders, but cleaning processing of an endoscope to be used (hereinafter, also referred to as an "endoscope" or simply as a "scope") and a used endoscope is not included in an examination order. It is left to the determination at a work site which endoscope is to be used in an examination or which cleaning machine is to be used for the cleaning of a used endoscope. For example, a doctor instructs, immediately before an examination, a person preparing for examination, such as a technician or a nurse, to bring an endoscope to be used into an examination room by orally giving him/her information on the model of the endoscope. Alternatively, a person preparing for examination voluntarily brings an endoscope into an examination room by confirming an examination to be performed from now from an examination schedule table. Used endoscopes are brought into a cleaning room by persons preparing for examination, and ideally a person preparing for examination cleans the endoscopes in descending order of priority in consideration of the subsequent examination schedule.

Patent Document 1 discloses a technique for determining whether endoscopes to be used in each examination runs short according to examination start time specified by an examination schedule and scheduled cleaning end times specified by a cleaning schedule.

### Related Art Document

### Patent Document

[PATENT DOCUMENT 1] Japanese Patent Application Publication No. 2010-39560

### SUMMARY OF THE INVENTION

A medical facility where numerous endoscopic examinations are performed possesses various endoscopes and possesses multiple individuals of a frequently used model. In a medical facility where there are many endoscopes, as described above, a person preparing for examination cannot easily understand which endoscope should be brought into an examination room, and hence it takes time for preparation or determination. When a person preparing for examination brings an endoscope under an instruction from a doctor, the work is initiated after the instruction is received, and hence it takes extra time.

Because such a medical facility has a plurality of endoscope cleaning machines, it is also necessary to confirm the state of each cleaning machine each time. In a large hospital or an endoscopy center, a preparation time between examinations is only about several minutes, and hence there is a challenge for improving work efficiency or labor saving, but in reality there is an actual situation where it is difficult to efficiently perform work.

Additionally, a situation is not preferable, in which models that a primary doctor intends to use in an examination are all being used in other examinations or being cleaned. For example, when a primary doctor intends to use a small-diameter endoscope in a routine examination and when no small-diameter endoscope is available, it is inevitable to use, as a substitute, an endoscope for precise examination that is not small in diameter. For this reason, it is preferable that: an endoscope suitable for examination is used by avoiding, as much as possible, a situation where a small-diameter endoscope is used in an examination where it is not essential to use a small-diameter endoscope.

Additionally, an endoscope that has become more worn and aged is more likely to cause functional deterioration or a malfunction. An endoscope, which has been used extremely more times, used for a longer time, or used in the larger number of times of biopsies than other similar observation endoscopes in a medical facility, or which has been used in the extremely larger number of times of various treatments and procedures, used for a longer time, or used in the larger number of times of various treatment tools than other similar treatment endoscopes, is to be exceptionally worn out and aged. If the orientation of an angle changes due to extension of a wire, or if perforation of a forceps channel occurs due to insertion of a forceps or a cleaning brush, such an endoscope needs to be repaired. Usually, endoscopes are managed to be inspected regularly, but an endoscope that has been particularly worn out is put out of service for unscheduled repair, which is not preferable because the number of the endoscopes that can be used becomes small in a medical facility. Therefore, there is a demand for avoiding the use of the endoscopes that may be exceptionally worn out.

When a medical facility possesses endoscope cleaning machines of multiple types, a person preparing for examination is required to determine which cleaning machine is used for the cleaning of an endoscope that was used in an examination, but demanding always suitable determinations in the intervals among busy endoscopic examinations puts a burden on a person preparing for examination. Also, medicinal solutions to be used for the cleaning are generally different depending on the models of cleaning machines, but there are medicinal solutions that can have an undesirable influence, such as deterioration of an endoscope member. This is also referred to as an attack property of a medicinal solution, and when a specific individual of endoscopes is cleaned many times by a cleaning machine using a medical solution having a strong attack property, there is the possibility that the deterioration of the individual may be accelerated, which is not preferable.

Therefore, it is preferable to set an appropriate examination schedule and a cleaning schedule with respect to each individual of endoscopes such that a doctor and a person preparing for examination perform an examination and preparation work according to the respective schedules.

However, even when an examination schedule and a cleaning schedule are set in advance, there may be the possibility that examination work may not proceed as scheduled when the examination work actually starts. For example, if an examination takes a longer time than scheduled, the subsequent schedules are naturally deviated. Also, various cases where examination work does not proceed as scheduled are assumed, the cases including: the one where an examination pretreatment is not completed before the scheduled examination start time because outpatient arrival at a medical facility has been delayed; the one where an examination itself is cancelled because a patient cannot visit a medical facility in the first place; and the like. Therefore, even when an examination schedule and a cleaning schedule are set, it is preferable to appropriately perform a review of the examination schedule and the cleaning schedule by executing rescheduling processing in accordance with the actual examination situation.

Originally, when an examination schedule is set, a nurse must prepare an endoscope assigned to an examination, but at a busy work site an endoscope different from the scheduled one may be erroneously prepared. There are also the cases: where an endoscope scheduled to be used cannot be used because a trouble occurs in the endoscope after an examination schedule and a cleaning schedule are set; and where an unscheduled endoscope is additionally used during an examination. Therefore, even when an examination schedule and a cleaning schedule are set, it is preferable to appropriately perform a review of the examination schedule and the cleaning schedule by executing rescheduling processing depending on the actual situation of an endoscope.

In addition, when endoscope cleaning is not completed within a scheduled time due to occurrence of a trouble in a cleaning machine, the examination scheduled to use the endoscope and the subsequent cleaning schedules may be delayed. In addition, an examination schedule may be corrected because a primary doctor can no longer be in charge of an examination for a sudden reason. Therefore, even when an examination schedule and a cleaning schedule are set, it is preferable to appropriately perform a review of the examination schedule and the cleaning schedule by executing rescheduling processing depending on the situation of cleaning processing in a cleaning machine, the situation of a doctor, and the like.

The present invention has been made in view of these situations, and a purpose of the invention is to provide a technique for appropriately reconstructing a schedule in endoscopic examination work.

In order to solve the above problems, an endoscopic examination work support system according to an embodiment of the present invention comprises: an examination schedule management unit that manages an examination schedule of a plurality of endoscopic examinations, including an examination room where an endoscopic examination is to be performed, information on scheduled examination start time, that on scheduled examination end time, and examination type information on an examination content of an endoscopic examination; a first assignment processing unit that assigns, from a plurality of endoscopes, an endoscope to be used to each endoscopic examination managed by the examination schedule management unit; a second assignment processing unit that assigns, from a plurality of cleaning machines, a cleaning machine for cleaning an endoscope to be used in the each endoscopic examination; and a cleaning schedule management unit that manages a cleaning schedule of a plurality of endoscopes, including a cleaning machine, information on scheduled cleaning start time, and that on scheduled cleaning end time. The endoscopic examination work support system further comprises: a situation information acquisition unit that acquires situation information on the situation of an examination; and a rescheduling processing unit that determines based on the situation information that it is necessary to change the examination schedule and/or the cleaning schedule. The rescheduling processing unit instructs at least one of the examination schedule management unit and the cleaning schedule management unit to change an element included in the examination schedule and/or the cleaning schedule.

An endoscopic examination work support system according to another embodiment of the present invention comprises: an examination schedule management unit that manages an examination schedule of a plurality of endoscopic examinations, including an examination room where an endoscopic examination is to be performed, information on scheduled examination start time, that on scheduled examination end time, and examination type information on an examination content of an endoscopic examination; a first assignment processing unit that assigns, from a plurality of endoscopes, an endoscope to be used to each endoscopic examination managed by the examination schedule management unit; a second assignment processing unit that assigns, from a plurality of cleaning machines, a cleaning machine for cleaning an endoscope to be used in the each endoscopic examination; and a cleaning schedule management unit that manages a cleaning schedule of a plurality of endoscopes, including a cleaning machine, information on scheduled cleaning start time, and that on scheduled cleaning end time. The endoscopic examination work support system further comprises: a situation information acquisition unit that acquires situation information on the situation of an endoscope; and a rescheduling processing unit that determines based the situation information that it is necessary to change the examination schedule and/or the cleaning schedule. The rescheduling processing unit instructs at least one of the examination schedule management unit and the cleaning schedule management unit to change an element included in the examination schedule and/or the cleaning schedule.

An endoscopic examination work support system according to still another embodiment of the present invention comprises: an examination schedule management unit that manages an examination schedule of a plurality of endoscopic examinations, including an examination room where an endoscopic examination is to be performed, information on scheduled examination start time, that on scheduled examination end time, and examination type information on an examination content of an endoscopic examination; a first assignment processing unit that assigns, from a plurality of endoscopes, an endoscope to be used to each endoscopic examination managed by the examination schedule management unit; a second assignment processing unit that assigns, from a plurality of cleaning machines, a cleaning machine for cleaning an endoscope to be used in the each endoscopic examination; and a cleaning schedule management unit that manages a cleaning schedule of a plurality of endoscopes, including a cleaning machine, information on scheduled cleaning start time, and that on scheduled cleaning end time. The endoscopic examination work support system further comprises: a situation information acquisition unit that acquires situation information on the situation of a cleaning machine; and a rescheduling processing unit that determines based on the situation information that it is necessary to change the cleaning schedule and/or the examination schedule. The rescheduling processing unit instructs at least one of the cleaning schedule management unit and the examination schedule management unit to change an element included in the cleaning schedule and/or the examination schedule.

An endoscopic examination work support system according to still another embodiment of the present invention comprises: an examination schedule management unit that manages an examination schedule of a plurality of endoscopic examinations, including an examination room where an endoscopic examination is to be performed, information on scheduled examination start time, that on scheduled examination end time, and examination type information on an examination content of an endoscopic examination; a first assignment processing unit that assigns, from a plurality of endoscopes, an endoscope to be used to each endoscopic examination managed by the examination schedule management unit; a second assignment processing unit that assigns, from a plurality of cleaning machines, a cleaning machine for cleaning an endoscope to be used in the each endoscopic examination; and a cleaning schedule management unit that manages a cleaning schedule of a plurality of endoscopes, including a cleaning machine, information on scheduled cleaning start time, and that on scheduled cleaning end time. The first assignment processing unit has the function of assigning a doctor to an endoscopic examination. The endoscopic examination work support system further comprises: a situation information acquisition unit that acquires situation information on the situation of a doctor; and a rescheduling processing unit that determines based on the situation information that it is necessary to change the examination schedule and/or the cleaning schedule. The rescheduling processing unit instructs at least one of the examination schedule management unit and the cleaning schedule management unit to change an element included in the examination schedule and/or the cleaning schedule.

Arbitrary combinations of the above constituting elements and implementations of the invention in the form of methods, apparatuses, systems, recording media, computer programs and so forth may also be effective as additional modes of the present invention.

### Advantage of the Invention

According to the present invention, a technique for appropriately reconstructing a schedule in endoscopic examination work can be provided.

### BRIEF DESCRIPTION OF THE DRAWINGS

Embodiments will now be described, by way of example only, with reference to the accompanying drawings which are meant to be exemplary, not limiting, and wherein like elements are numbered alike in several Figures, in which:
Fig. 1 is a view illustrating a configuration of an endoscopic examination work support system according to an embodiment of the present invention;
Fig. 2 is a view for explaining a virtual status of an endoscope set during the generation of schedule information;
Fig. 3 is a view illustrating a configuration of an information management device for generating schedule information on an endoscope;
Fig. 4 is a view illustrating one example of the generated examination schedule;
Fig. 5 is a view illustrating one example of an examination type master table;
Fig. 6 is a view illustrating one example of a possessed endoscope master table;
Fig. 7 is a view illustrating a basic flowchart in which schedule information on an endoscope is generated;
Fig. 8 is a view illustrating a detailed flowchart of the assignment target examination extraction processing illustrated in S16 of the basic flowchart;
Fig. 9 is a view illustrating a detailed flowchart of the endoscope assignment processing illustrated in S18 of the basic flowchart;
Fig. 10 is a view illustrating a detailed flowchart of the status specification processing of S50;
Fig. 11 is a view illustrating a detailed flowchart of the endoscope retrieval processing of S52;
Fig. 12 is a view illustrating an examination schedule updated by an examination schedule management unit;
Fig. 13 is a view illustrating a detailed flowchart of the cleaning machine assignment processing illustrated in S20 of the basic flowchart;
Fig. 14 is a view illustrating a cleaning schedule generated by a cleaning schedule management unit;
Fig. 15 is a view illustrating schedule information on the individuals of endoscopes;
Fig. 16 is a view illustrating an examination schedule generated by the examination schedule management unit and a cleaning schedule generated by the cleaning schedule management unit;
Fig. 17 is a view illustrating an examination schedule generated by the examination schedule management unit and a cleaning schedule generated by the cleaning schedule management unit;
Fig. 18 is a view illustrating an examination schedule generated by the examination schedule management unit and a cleaning schedule generated by the cleaning schedule management unit;
Fig. 19 is a view illustrating the individual schedules of endoscopes;
Fig. 20 is a view illustrating an examination schedule generated by the examination schedule management unit and a cleaning schedule generated by the cleaning schedule management unit;
Fig. 21 is a view illustrating individual schedules of endoscopes for one day;
Fig. 22 is a view illustrating an endoscope order table held in an endoscope order holding unit;
Fig. 23 is a view illustrating a detailed flowchart of the endoscope retrieval processing in Example 1;
Fig. 24 is a view illustrating a usage condition table stored in a usage condition storage unit;
Fig. 25 is a view illustrating a detailed flowchart of S56 of the endoscope assignment processing illustrated in Fig. 9;
Fig. 26 is a view illustrating a cleaning machine order table held in a cleaning machine order holding unit;
Fig. 27 is a view illustrating a detailed flowchart of S110 of the cleaning machine assignment processing illustrated in Fig. 13;
Fig. 28 is a view illustrating a detailed flowchart of S114 of the cleaning machine assignment processing illustrated in Fig. 13;
Fig. 29 is a view illustrating a cleaning schedule generated by the cleaning schedule management unit in Example 3;
Fig. 30 is a view illustrating a preferential endoscope table stored in an assigned endoscope information holding unit;
Fig. 31 is a view illustrating a detailed flowchart of S56 of the endoscope assignment processing illustrated in Fig. 9;
Fig. 32 is a view illustrating an examination schedule updated by the examination schedule management unit;
Fig. 33 is a view illustrating one example of usage history information displayed on a terminal device;
Fig. 34 is a view illustrating one example of usage history information displayed on a terminal device;
Fig. 35 is a view illustrating a preferential person-in-charge table stored in an assigned person-in-charge information holding unit;
Fig. 36 is a view illustrating a flowchart of person-in-charge assignment processing;
Fig. 37 is a view illustrating a cleaning schedule generated by the cleaning schedule management unit;
Fig. 38 is a view illustrating a cleaning schedule updated by the cleaning schedule management unit;
Fig. 39 is a view illustrating one example of cleaning history information displayed on a terminal device;
Fig. 40 is a view illustrating one example of cleaning history information displayed on a terminal device;
Fig. 41 is a view illustrating examples of an examination schedule and a cleaning schedule;
Fig. 42 is a view illustrating one example of a possessed endoscope master table;
Fig. 43 is a view illustrating the configuration of a processing unit having the function of executing rescheduling processing;
Fig. 44 is a view illustrating an example in which an actual examination is delayed than scheduled;
Fig. 45 is a view illustrating a state where the scheduled examination start time and scheduled examination end time of an examination are delayed;
Fig. 46 is a view illustrating a state where the scheduled examination start time and scheduled examination end time of an examination are delayed;
Fig. 47 is a view illustrating a state where the scheduled cleaning start time and scheduled cleaning end time of cleaning are delayed;
Fig. 48 is a view illustrating an examination schedule table and a cleaning schedule table to be displayed on a display;
Fig. 49 is a view illustrating a state where a user designates scheduled examination start time;
Fig. 50 is a view illustrating an examination schedule and a cleaning schedule on each of which rescheduling processing is performed;
Fig. 51 is a view illustrating a state where an examination E22 is deleted from the examination schedule;
Fig. 52 is a view illustrating a flowchart of the reservation advancing processing in Example 8;
Fig. 53 is a view illustrating a detailed flowchart of the candidate examination extraction processing of S302;
Fig. 54 is a view illustrating a detailed flowchart of the advanced examination specification processing of S306;
Fig. 55 is a view illustrating a state where an examination E24 is moved to a free time frame;
Fig. 56 is a view illustrating a state where an examination E26 is moved to a free time frame;
Fig. 57 is a view illustrating a state where an examination E28 is moved to a free time frame;
Fig. 58 is a view illustrating a reconstructed examination schedule;
Fig. 59 is a view illustrating a reconstructed cleaning schedule;
Fig. 60 is a view illustrating examples of an examination schedule and a cleaning schedule;
Fig. 61 is a view illustrating one example of a possessed endoscope master table;
Fig. 62 is a view illustrating the configuration of a processing unit having the function of executing rescheduling processing;
Fig. 63 is a view illustrating an example in which another endoscope different from a scheduled one is used;
Fig. 64 is a view illustrating a flowchart of the schedule element change processing in Example 9;
Fig. 65 is a view illustrating an example in which the assignment of an endoscope in an examination is changed;
Fig. 66 is a view illustrating an example in which replacement processing of endoscopes has been performed;
Fig. 67 is a view illustrating an example in which an additional endoscope is used in addition to the scheduled endoscopes;
Fig. 68 is a view illustrating a flowchart of the schedule element change processing in Example 10;
Fig. 69 is a view illustrating an example in which cleaning processing of an endoscope is inserted into a cleaning schedule;
Fig. 70 is a view illustrating the relationship between an examination E32 and cleaning W29;
Fig. 71 is a view illustrating a state where the time frame of the examination E32 is changed;
Fig. 72 is a view illustrated the result of rescheduling a cleaning schedule;
Fig. 73 is a view illustrating an example in which an examination E40 is moved to the end of an examination schedule;
Fig. 74 is a view illustrating an example in which a malfunction of an endoscope is detected during cleaning;
Fig. 75 is a view illustrating a state where both the endoscope assignment information in an examination schedule and a cleaning schedule are deleted;
Fig. 76 is a view illustrating examples of an examination schedule and a cleaning schedule;
Fig. 77 is a view illustrating one example of a possessed endoscope master table;
Fig. 78 is a view illustrating the configuration of a processing unit having the function of executing rescheduling processing;
Fig. 79 is a view illustrating an example in which cleaning processing is stopped halfway due to occurrence of a trouble in the cleaning processing;
Fig. 80 is a view illustrating a state where the scheduled cleaning start time and the scheduled cleaning end time are delayed by 15 minutes, respectively;
Fig. 81 is a view illustrating a state where the time frame of an examination is reset;
Fig. 82 is a view illustrating one example of a doctor skill table;
Fig. 83 is a view illustrating the configuration of a doctor assignment unit;
Fig. 84 is a view illustrating a state where a doctor assigned to an examination is deleted;
Fig. 85 is a view illustrating a state where a doctor is assigned to an examination;
Fig. 86 is a view illustrating a state where a doctor is assigned to an examination;
Fig. 87 is a view illustrating the result of rescheduling an examination schedule and a cleaning schedule;
Fig. 88 is a view illustrating the relationship between an examination schedule and the examination impossible time zone of a doctor;
Fig. 89 is a view illustrating a state where the endoscope information assigned to an examination is deleted;
Fig. 90 is a view illustrating another example of the doctor skill table; and
Fig. 91 is a view illustrating the result of performing doctor rescheduling processing.

### DETAILED DESCRIPTION OF THE INVENTION

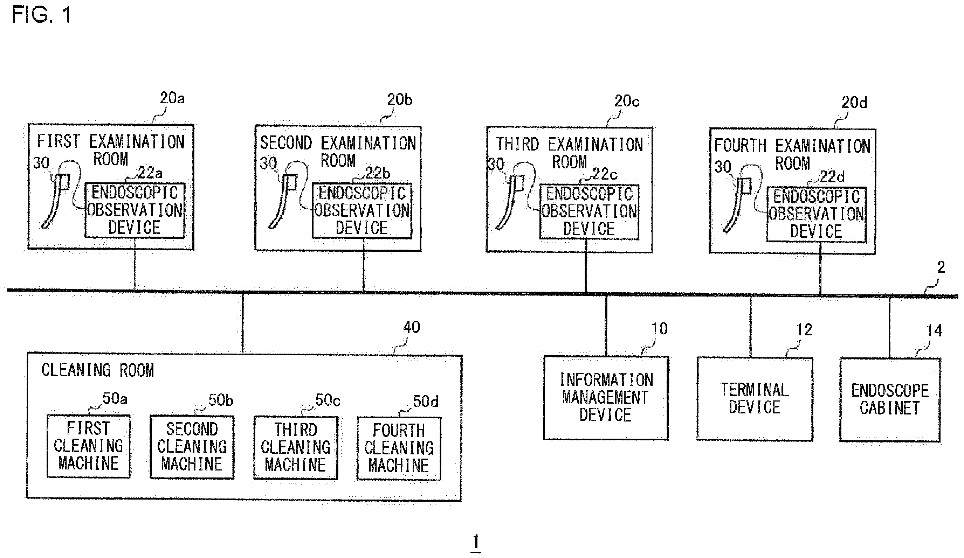

The invention will now be described by reference to the preferred embodiments. This does not intend to limit the scope of the invention, but to exemplify the invention. Fig. 1 is a view illustrating the configuration of an endoscopic examination work support system 1 according to an embodiment of the present invention. The endoscopic examination work support system 1 is a system for supporting endoscopic examination work, and achieves the functions of appropriately scheduling the use schedule and the cleaning schedule of the individuals of endoscopes (hereinafter, also and simply referred to as a "scope") 30 and of appropriately rescheduling the scheduled schedule. The endoscopic examination work support system 1 comprises: an information management device 10, a terminal device 12, an endoscope cabinet 14, endoscopic observation devices 22a to 22d, and a first cleaning machine 50a to a fourth cleaning machine 50d, which are interconnected to each other by a network 2, such as LAN (local area network) .

The endoscopic observation device is installed in each of a plurality of examination rooms. In this embodiment, the endoscopic observation device 22a is installed in a first examination room 20a, the endoscopic observation device 22b in a second examination room 20b, the endoscopic observation device 22c in a third examination room 20c, and the endoscopic observation device 22d in a fourth examination room 20d. In a medical facility, examination rooms are frequently divided into rooms for upper examinations and rooms for lower examinations. In the embodiment illustrated in Fig. 1, the first examination room 20a, the second examination room 20b, and the third examination room 20c are used for upper examinations, and the fourth examination room 20d is used for lower examinations. Hereinafter, when the first examination room 20a to the fourth examination room 20d are not particularly distinguished, each of them may be referred to as an "examination room 20", and when the endoscopic observation devices 22a to 22d are not particularly distinguished, each of them may be referred to as an "endoscopic observation device 22." The endoscope 30 is connected to the endoscopic observation device 22 such that an endoscopic examination is performed by a doctor.

A large hospital and a medical facility such as an endoscopy center possess various endoscopes (scopes) and possess a plurality of individuals of a model that is frequently used in order to perform a large number of endoscopic examinations a day. For example, the models of an endoscope for upper examination include an upper routine model to be used in a routine examination, an upper high image quality model that can provide a high resolution image, an upper nasal model to be inserted through a nostril, an upper expansion model that allows observation of the morphology of fine blood vessels on a mucosal surface and a structural pattern by the ducts of a gland, etc., an upper treatment model having a treatment function, and the like. On the other hand, the models of an endoscope for lower examination include a lower routine model to be used in a routine examination, a lower expansion model that allows observation of the morphology of fine blood vessels on a mucosal surface and a structure pattern by the ducts of a gland, etc., a lower treatment model having a treatment function, and the like. The endoscopes possessed by a medical facility are managed by being registered in a database.

A plurality of cleaning machines are installed in a cleaning room 40, and in this embodiment the first cleaning machine 50a, the second cleaning machine 50b, the third cleaning machine 50c, and the fourth cleaning machine 50d are provided. Hereinafter, when the first cleaning machine 50a to the fourth cleaning machine 50d are not particularly distinguished, each of them may also be referred to as a "cleaning machine 50." Although four cleaning machines 50 are installed in the single cleaning room 40 in this embodiment, they may be dispersedly installed in multiple cleaning rooms.

Medicinal solutions to be used for the cleaning are generally different depending on the models of the cleaning machine 50. For example, examples of the medicinal solutions to be used for the cleaning typically include peracetic acid, phtharal, strongly acidic electrolyzed water, and the like, and the cleaning machine 50 is designed to use only a predetermined medicinal solution. That is, the model of the cleaning machine 50 and a medicinal solution to be used are associated in one-to-one correspondence, and it is not recommended that the cleaning machine 50 uses a medicinal solution other than the determined medicinal solution. Additionally, the cleaning time may be different depending on the model of the cleaning machine 50, and thus the cleaning machine 50 has a characteristic peculiar to the model.

The endoscope cabinet 14 stores the endoscope 30. Before endoscopic examination work for one day is started, all the endoscopes 30 are stored in the endoscope cabinet 14, and a person preparing for examination, such as a technician, takes out the endoscope 30 from the endoscope cabinet 14, and brings it into the examination room 20 to connect to the endoscopic observation device 22. When an examination by a doctor ends, a person preparing for examination brings the used endoscope 30 into the cleaning room 40 to clean it by putting into the cleaning tank of the cleaning machine 50, and the cleaned endoscope 30 is brought into an examination room such that a doctor reuses it in a new examination.

In a medical facility, it is common that an individual name is given to an individual of the endoscope 30 in order to distinguish from other individuals. For example, the endoscopes 30 of the same type have the same shape, and hence each of them is managed by being provided with an individual name. A seal, or the like, on which an individual name is printed, is attached to the endoscope 30 in order to be distinguished by the individual name, whereby a doctor and a person preparing for examination can distinguish the respective individuals from each other. Additionally, RFID tags, or the like, have recently been embedded in the main bodies of endoscopes, so that each individual can be electronically distinguished by reading it when the endoscopic observation device 22 is connected to a camera control unit (CCU) or with the use of a tag reading means. Such an endoscope 30 can be distinguished similarly to the case where a seal is used, by acquiring the individual identification information in an RFID tag when connected to an CCU or before or after the cleaning in a cleaning machine.

The endoscopic examination work support system 1 of the embodiment sets, for each individual of the endoscope 30, schedule information in which it is determined: which examination uses the individual; which cleaning machine cleans the individual; and the like. Thereby, a person preparing for examination can understand which examination room 20 the individual should be brought into and which cleaning machine 50 the individual should be cleaned by, by seeing the schedule information. At the time, the person preparing for examination can transfer the endoscope 30 properly and clean it according to the schedule information in accordance with the individual name printed on the seal attached to the endoscope 30.

The schedule information on the endoscope 30 is generated by the information management device 10. The timing when the schedule information is generated is before endoscopic examination work for one day starts, and a person preparing for examination can determine the handling of the endoscope 30 by seeing the schedule information displayed on the screen of the terminal device 12. Although the terminal device 12 may be a stationary personal computer, it may be a terminal device such as a portable PDA (Personal Digital Assistant) or a tablet.

When generating the schedule information, the information management device 10 determines which endoscope 30 is to be assigned to an examination that is scheduled to start at a certain timing, but the endoscope 30 scheduled to be used at the timing or that scheduled to be cleaned at the timing cannot naturally be assigned to the examination. Therefore, the information management device 10 sets, when performing scheduling processing, a virtual status for each endoscope 30, thereby allowing the status of each endoscope 30 at an arbitrary timing to be confirmed.

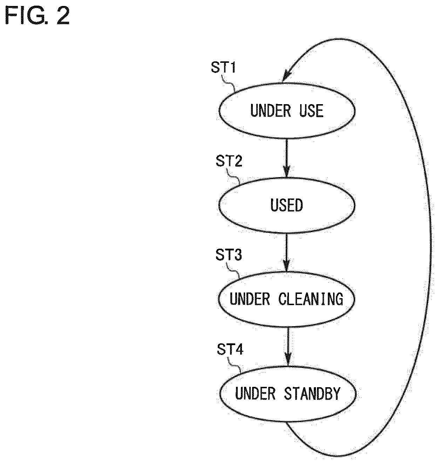

Fig. 2 is a view for explaining the virtual status of the endoscope 30 that is set during the generation of the schedule information. The endoscope 30 takes any one of the statuses including "under use" (ST1), "used" (ST2), "under cleaning" (ST3) and "under standby" (ST4). The arrows illustrated in Fig. 2 indicate the transition direction of the statuses. By grasping the statuses of all the endoscopes 30 at an arbitrary timing, the information management device 10 assigns the endoscope 30 proper at the timing to an examination.

In the four statuses illustrated in Fig. 2, the endoscope 30 that can be assigned to an examination is the endoscope whose status is "under standby", and the endoscopes 30 that are in other statuses cannot be assigned to an examination. The statuses of the endoscopes 30 stored in the endoscope cabinet 14 are "under standby", and therefore when the processing for generating the schedule information is started, it is assumed that the statuses of all the endoscopes 30 are "under standby."

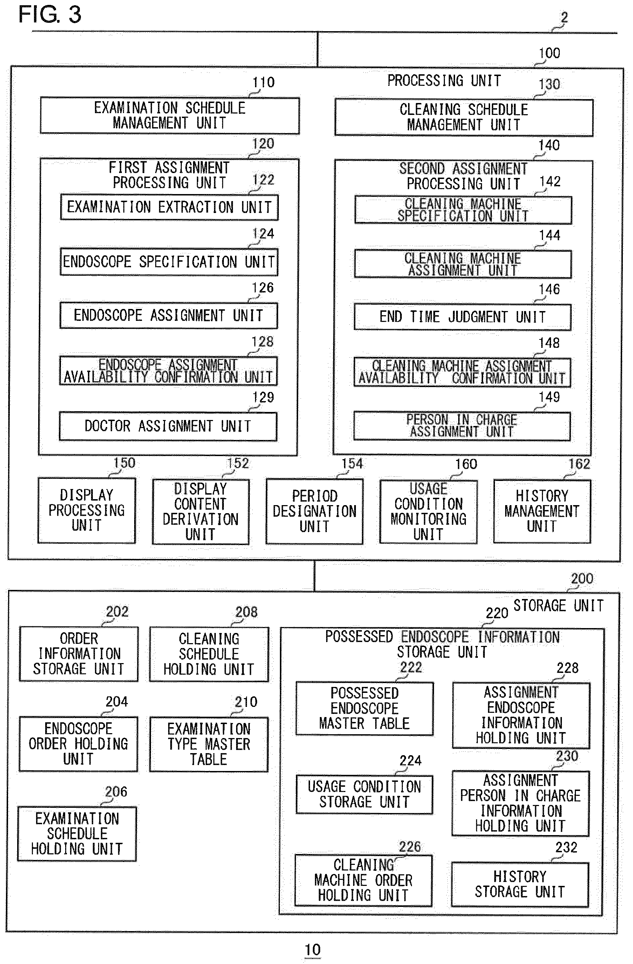

Fig. 3 illustrates the configuration of the information management device 10 that generates the schedule information on the endoscope 30. The information management device 10 comprises a processing unit 100 and a storage unit 200, and the processing unit 100 includes an examination schedule management unit 110, a first assignment processing unit 120, a cleaning schedule management unit 130, a second assignment processing unit 140, a display processing unit 150, a display content derivation unit 152, a period designation unit 154, and a usage condition monitoring unit 160.

Each component of the information management device 10 can be realized by a CPU, memory, or other LSIs of an arbitrary computer in terms of hardware, and realized by a program or the like loaded in a memory in terms of software, but herein functional blocks realized by the cooperation of hardware and software are depicted. Therefore, it is to be understood by those skilled in the art that these functional blocks can be realized in various forms, namely, solely in hardware, solely in software, or through a combination of hardware and software.

An examination order is generated in an in-hospital information system such as, for example, an ordering system, and is issued to an endoscopy department system. Before endoscopic examination work for one day starts, the information management device 10 acquires the examination orders for the one day that have been generated in the in-hospital information system, so that the use schedule and cleaning schedule of each individual of the endoscopes 30 possessed in a medical facility are scheduled. The acquired examination orders for the one day are stored in an order information storage unit 202. For example, the timing of the scheduling may be before the first examination on the examination day is performed or after the examination work on the previous day is ended, and in any case the scheduling only has to be performed at a timing when the examination orders for one day are fixed.

The examination order includes order information on an endoscopic examination, such as examination identification information (examination ID), information on scheduled examination start time, that on scheduled examination end time, patient identification information (patient ID), examination type information, primary doctor of the examination, and examination room. In the endoscopic examination work support system 1 illustrated in Fig. 1, it is determined that: the first examination room 20a, the second examination room 20b, and the third examination room 20c are used for upper examinations; and the fourth examination room 20d is used for lower examinations, and therefore any one of the first examination room 20a, the second examination room 20b, and the third examination room 20c is assigned, as an examination room, to an upper examination order, and the fourth examination room 20d is assigned to a lower examination order.

When the scheduling processing is started, the examination schedule management unit 110 first acquires a plurality of order information for one day from the order information storage unit 202 to generate an examination schedule. Specifically, the examination schedule management unit 110 generates and manages an examination schedule of a plurality of endoscopic examinations, including an examination room where the endoscopic examination specified by the examination ID is to be performed, information on scheduled examination start time, that on scheduled examination end time, examination type information on the examination contents of the endoscopic examination, and a primary doctor. The examination schedule management unit 110 stores the generated examination schedule in an examination schedule holding unit 206. Thereafter, the examination schedule management unit 110 updates the examination schedule by registering information on the endoscope 30 assigned to each examination by the first assignment processing unit 120, as described from now on.

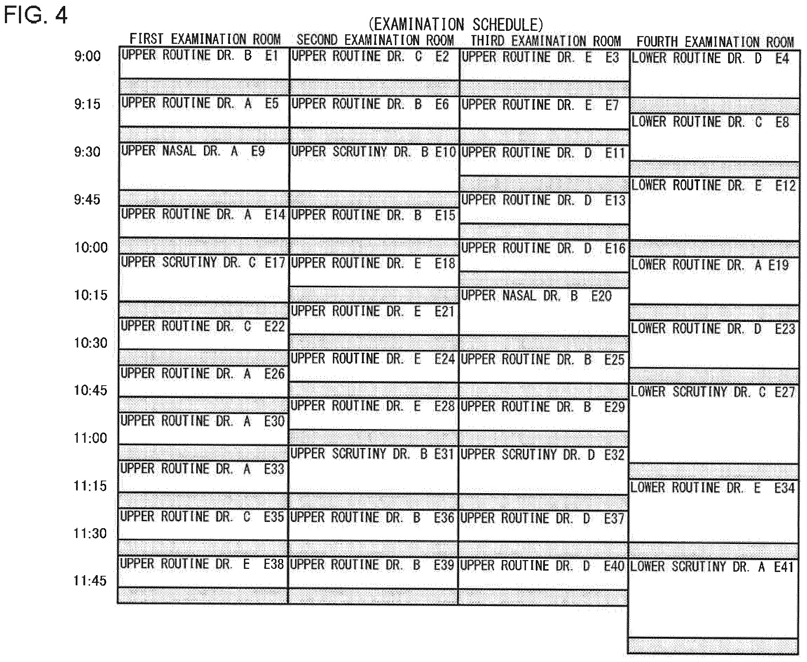

Fig. 4 illustrates one example of the generated examination schedule. When acquiring the order information from the order information storage unit 202, the examination schedule management unit 110 sets examination Nos. in the order starting from the earliest scheduled examination start time. In Fig. 4, the number of examinations for one day is 41, and examination Nos. E1 to E41 are set to the respective examinations. Herein, the examination schedule with examination No. E1 is indicated as follows: the examination room is the first examination room 20a; the scheduled examination start time is 9:00; the scheduled examination end time is 9:10; the examination type is an "upper routine examination"; and the primary doctor is a "doctor B." The examination ID is assigned to an examination, and this examination ID is different from an examination number, but the examination number also uniquely distinguishes an examination in an examination schedule, and hence in the following description, an examination number may be used as the information for specifying an examination.

In the present embodiment, the examination schedule illustrated in Fig. 4 is automatically derived from a plurality of examination orders for one day, but when information on scheduled examination start time, that on scheduled examination end time, that on a primary doctor, and that on an examination room are not included in the order information, the examination schedule management unit 110 may generate an examination schedule. The examination schedule management unit 110 also has the functions of reconstructing the generated examination schedule and of performing rescheduling processing in accordance with the situation of an examination.

For example, the storage unit 200 stores: an examination type master table in which the scheduled examination time of each examination type is stored; a primary doctor master table in which primary doctors are stored; and the condition of an examination to be performed in an examination room (i.e., information for specifying whether it is an upper examination or a lower examination). The examination order includes patient identification information (patient ID) and examination type information, and when acquiring the examination order for one day, the examination schedule management unit 110 generates an examination schedule by referring to the examination type master table, the primary doctor master table, and the examination condition.

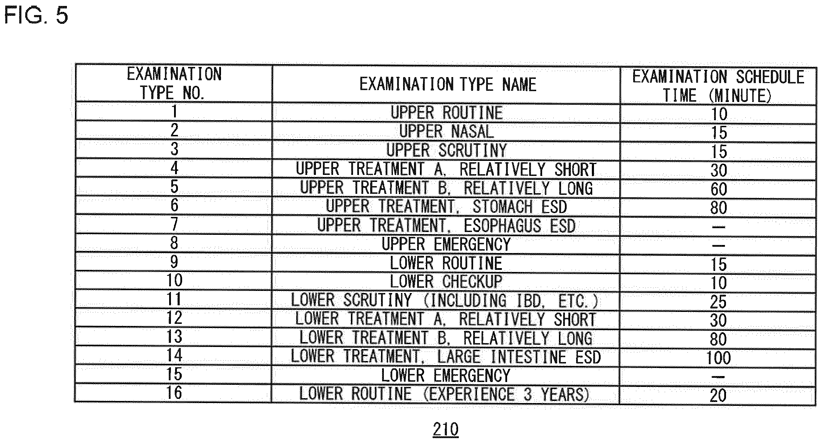

Fig. 5 illustrates one example of an examination type master table 210. In the examination type master table 210, the scheduled examination time of each examination type is recorded. When patient identification information (patient ID) and examination type information are included in the examination order, the examination schedule management unit 110 first assigns one examination to each examination room by referring to the examination type information on each examination included in the examination order. When the examination type information designates an upper examination, the examination is assigned to any one of the first examination room 20a, the second examination room 20b, and the third examination room 20c by referring to the examination condition for an examination room, on the other hand, when the examination type information designates a lower examination, the examination is assigned to the fourth examination room 20d. Further, the examination schedule management unit 110 sets a predetermined preparation time (e.g., 5 minutes) as an interval between examinations.

In the examination type master table 210, it is recorded, for example, that: the scheduled examination time of the "upper routine examination" with examination type No. 1 is 10 minutes; that of the "upper nasal examination" with examination type No. 2 is 15 minutes; and the like. The scheduled examination time of the "lower routine examination (experience 3 years)" with examination type No. 16 is set to be 5 minutes longer than that of the "lower routine examination" with examination type No. 9, but this means that: it is incorporated in advance as scheduled time that when a doctor (young doctor) with less than 3-year experience performs an examination, it takes about 5 minutes longer than a doctor (veteran doctor) with 3-year or more experience. Alternatively, it may be set in the primary doctor master table that a young doctor needs more time than a veteran doctor. The examination schedule management unit 110 assigns one examination to each examination room 20 and sets scheduled examination start time and scheduled examination end time, according to the examination type master table 210.

Next, the doctor assignment unit 129 in the first assignment processing unit 120 assigns a doctor to an examination in each examination room 20. At this time, the doctor assignment unit 129 assigns primary doctors to examinations such that the same primary doctor does not overlap in the same time zone. As described above, the examination schedule management unit 110 assigns one examination to each examination room 20, and the doctor assignment unit 129 assigns a doctor to the assigned examination, so that an examination schedule is generated. When the doctor assignment unit 129 assigns a doctor to an examination, the examination schedule management unit 110 again assigns one examination to each examination room by referring to the examination type information on each examination included in an unprocessed examination order, and the doctor assignment unit 129 assigns a doctor to the assigned examination. The examination schedule illustrated in Fig. 4 is generated by repeating this.

In the examination schedule illustrated in Fig. 4, the scheduled examination time of the lower routine examination indicated by examination No. E12 is set to be 20 minutes. This is because when a doctor E is assigned to the lower routine examination indicated by examination No. E12 by the doctor assignment unit 129, the scheduled time of the examination indicated by the examination No. E12 is set to be 5 minutes longer than the scheduled examination time (15 minutes) of a normal lower routine examination since the doctor E is a young doctor with less than 3-year experience. When the examination schedule management unit 110 assigns the lower routine examination indicated by the examination No. E12 to the fourth examination room 20d, the examination schedule management unit 110 sets the scheduled examination time to be 15 minutes as usual; on the other hand, when the doctor assignment unit 129 assigns the doctor E to the examination, the examination schedule management unit 110 resets the scheduled examination end time by lengthening the scheduled examination times by 5 minutes with reference to the scheduled examination time of the examination type No. 16 illustrated in Fig. 5.

As described above, when examination room information, information on scheduled examination start time, that on scheduled examination end time, primary doctor information, and the like are not included in the order information, the processing unit 100 may have the function of automatically generating an examination schedule, which is achieved in the following way: the examination schedule management unit 110 sets, as the premise of assigning the endoscope 30 to an examination, an examination room where the examination is to be performed, scheduled examination start time, and scheduled examination end time by referring to the examination type master table 210, etc., as described above; and the doctor assignment unit 129 assigns a doctor to the examination.

A possessed endoscope information storage unit 220 stores information and data on the endoscopes 30 that a medical facility possesses, and includes a possessed endoscope master table 222, a usage condition storage unit 224, a cleaning machine order holding unit 226, an assigned endoscope information holding unit 228, an assigned person-in-charge information holding unit 230, and a history recording unit 232. The possessed endoscope master table 222 is a database for managing the endoscopes 30 that a medical facility possesses, and the information on all the endoscopes 30 that the medical facility possesses are registered.

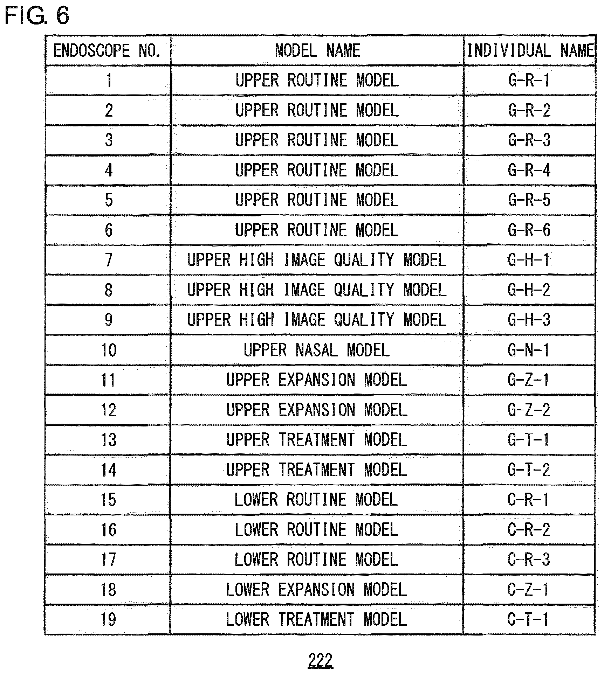

Fig. 6 illustrates one example of the possessed endoscope master table 222. The possessed endoscope master table 222 registers the endoscope Nos. set in the medical facility, model names, and individual names in the medical facility, by associating them with each other. Herein, as endoscope models for upper examination, an upper routine model to be used in a routine examination, an upper high image quality model that can provide a high resolution image, an upper nasal model to be inserted through a nostril, an upper expansion model that allows observation of the morphology of fine blood vessels on a mucosal surface and a structural pattern by the ducts of a gland, etc., and an upper treatment model having a treatment function are registered.

Six upper routine models are possessed in the medical facility, which are provided with individual names of G-R-1, G-R-2, G-R-3, G-R-4, G-R-5, and G-R-6, respectively. Three upper high image quality models are possessed, which are provided with individual names of G-H-1, G-H-2, and G-H-3, respectively; one upper nasal model is possessed, which is provided with an individual name of G-N-1; two upper expansion models are possessed, which are provided with individual names of G-Z-1 and G-Z-2, respectively; and two upper treatment models are possessed, which are provided with individual names of G-T-1 and G-T-2, respectively.

On the other hand, as endoscope models for lower examinations, a routine lower model to be used in a routine examination, a lower expansion model that allows observation of the morphology of fine blood vessels on a mucosal surface and a structure pattern by the ducts of a gland, etc., and a lower treatment model having a treatment function are registered. Three lower routine models are possessed, which are provided with individual names of C-R-1, C-R-2, and C-R-3, respectively; one lower expansion model is possessed, which is provided with an individual name of C-Z-1; and one lower treatment model is possessed, which is provided with an individual name of C-T-1.

Each endoscope 30 is attached with a tape or the like on which each individual name is printed so that a doctor or a person preparing for examination can visually specify individuals. A means for distinguishing and displaying individuals is not limited to tapes, but in particular, when there are a plurality of endoscopes 30 of the same model, it is preferable that a means, which allows individuals to be visually specified so that they can be distinguished within the same models, is provided.

Hereinafter, the processing for generating the schedule information on the endoscope 30 will be described. Generating the schedule information on the endoscope 30 means assigning the endoscopes 30 to examinations and assigning the endoscopes 30 that were used in examinations to cleaning machines, and as a result, an examination schedule and a cleaning schedule are generated, and a schedule of the individuals of the endoscopes 30 is also generated.

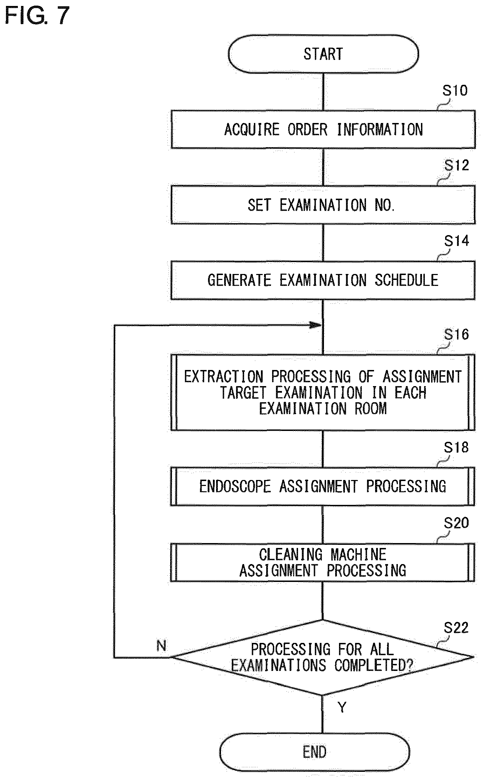

Fig. 7 illustrates a basic flowchart for generating the schedule information on the endoscope 30. The examination schedule management unit 110 acquires a plurality of order information for one day from the order information storage unit 202 (S10). With reference to the scheduled examination start times in a plurality of order information, the examination schedule management unit 110 sets examination Nos. in the order starting from the earliest scheduled examination start time (S12), whereby generates an examination schedule (S14). For a plurality of examinations whose scheduled examination start times are the same, examination Nos. may be set in ascending order of examination room Nos. The examination room Nos. are set to be "1" for the first examination room 20a, "2" for the second examination room 20b, "3" for the third examination room 20c, and "4" for the fourth examination room 20d. The examination schedule generated in S14 is the one illustrated in Fig. 4, in which by taking time axis as the vertical axis and examination rooms as the horizontal axis, an examination order is assigned within a time frame designated by the information on scheduled examination start time and that on scheduled examination end time.

The information on scheduled examination start time and that on scheduled examination end time may be information on time itself indicating hour and minute, but may be ones indicating time zones in 5-minute increments. For example, when the scheduling is performed with 5 minutes as one unit in an endoscopy department, the information on scheduled examination start time and that on scheduled examination end time may designate a scheduled examination start time and a scheduled examination end time, respectively, based on a frame with 5 minutes as one unit.

The schedule information on the endoscope 30 is generated by assigning the endoscope 30 to an examination in the examination schedule and assigning a cleaning machine, which performs cleaning after the scheduled examination end time, to the assigned endoscope 30. Therefore, processing for extracting an examination, to which the endoscope 30 is to be assigned in each examination room, is first executed (S16). When the examination in each examination room is extracted by the assignment target examination extraction processing, processing for assigning the endoscope 30 to the extracted examination is executed by the first assignment processing unit 120 (S18). When the endoscope 30 is assigned to the examination by the endoscope assignment processing, the information on the assigned endoscope 30 is registered in the examination schedule, and subsequently processing for assigning the cleaning machine 50 for cleaning to the assigned endoscope 30 is executed by the second assignment processing unit 140 (S20). The information on the cleaning machine 50 assigned to the endoscope 30 is registered in the cleaning schedule. The steps of S16 to S20 are repeated until all the examinations are completed (S22/N), and when the assignment processing is completed for all the examinations (S22/Y), scheduling processing of the endoscope 30 ends.

When the steps of S16 to S20 are executed, the endoscope 30 is assigned to an examination in each examination room 20, and the cleaning machine 50 is assigned to the assigned endoscope 30. When the use schedule and cleaning schedule of the endoscope 30 are thus determined, the second assignment processing unit 140, which assigned the cleaning machine 50, sets a processed flag for the examination No. of the examination to which the endoscope 30 was assigned. The first assignment processing unit 120 that assigns the endoscope 30 to an examination refers to the flag of each examination No. and executes the step of S16 until the flags of all the examination Nos. are processed (S22/N), and the flags of all the examination Nos. are processed (S22/Y), the scheduling processing of the endoscope 30 ends without returning to the step of S16.

Returning to Fig. 3, the first assignment processing unit 120 performs processing for assigning the endoscope 30 to an examination in the examination schedule. Specifically, the first assignment processing unit 120 has the function of executing the steps of S16 and S18 of the basic flowchart, and includes an examination extraction unit 122, an endoscope specification unit 124, an endoscope assignment unit 126, an endoscope assignment availability confirmation unit 128, and a doctor assignment unit 129. As described above, the doctor assignment unit 129 takes charge of processing for assigning a doctor to an examination in generating the examination schedule.

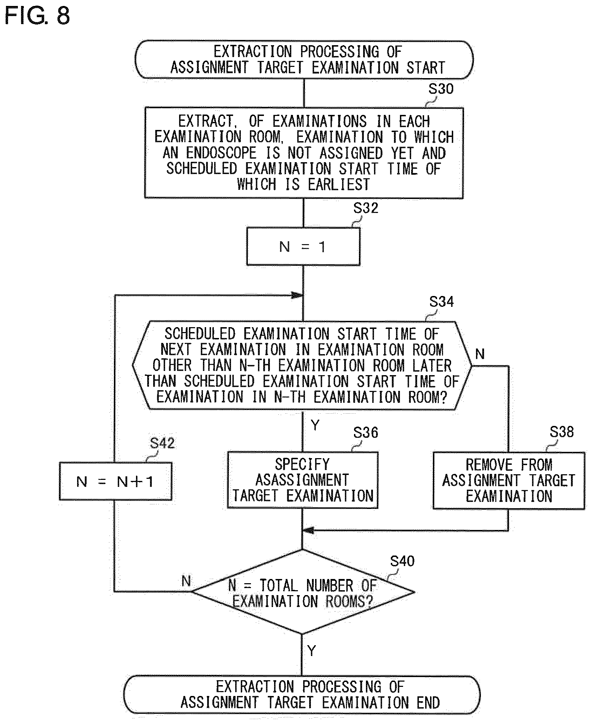

Fig. 8 illustrates a detailed flowchart of the assignment target examination extraction processing illustrated in S16 of the basic flowchart. In the first assignment processing unit 120, the examination extraction unit 122 extracts, of the examinations in each examination room 20 in the examination schedule, an examination that is not yet assigned with the endoscope 30 and the scheduled examination start time of which is earliest (S30). In the examination schedule illustrated in Fig. 4, the endoscope 30 is not yet assigned to any examination, so that the examination extraction unit 122 extracts the respective examinations the scheduled examination start time of each of which is earliest in each examination room 20. Herein, the examination with examination No. E1, the examination with examination No. E2, the examination with examination No. E3, and the examination with examination No. E4 are extracted from the first examination room 20a, the second examination room 20b, the third examination room 20c, and the fourth examination room 20d, respectively. Hereinafter, for convenience of description, the examination with examination No. E1 may be referred to as an "examination E1", and the examination with examination No. E2 as an "examination E2", etc.

Subsequently, the examination extraction unit 122 sets "N = 1" (S32), and determines whether the scheduled examination start time of the examination next to the examination extracted from an examination room other than the N-th examination room is later than the scheduled examination start time of the examination extracted from the N-th examination room (S34). Herein, by comparing the scheduled examination start time of the examination E1 extracted from the first examination room 20a with the scheduled examination start times of the examinations next to the examinations E2, E3, and E4 respectively extracted from the second examination room 20b, the third examination room 20c, and the fourth examination room 20d, that is, with the scheduled examination start times of the examinations E6, E7, and E8, the examination extraction unit 122 determines whether all of the scheduled examination start times of the examinations E6, E7, and E8 are later than the scheduled examination start time of the examination E1. In the examination schedule illustrated in Fig. 4, all of the scheduled examination start times of the examinations E6, E7, and E8 are later than the scheduled examination start time of the examination E1 (S34/Y), and hence the examination extraction unit 122 specifies the examination E1 as an examination to which the endoscope 30 is to be assigned (S36). If even any one of the scheduled examination start times of the examinations E6, E7, and E8 is earlier than the scheduled examination start time of the examination E1 (S34/N), the examination extraction unit 122 excludes the examination E1 from the examinations to which the endoscope 30 is to be assigned (S 38).

Subsequently, it is determined whether N is equal to the total number of examination rooms (in this embodiment, the total number thereof = 4) (S40), and when N does not reach the total number of examination rooms (S40/N), N is incremented by 1 (S42) and the processing returns to S34.

In S34, by comparing the scheduled examination start time of the examination E2 extracted from the second examination room 20b with the scheduled examination start times of the examinations next to the examinations E1, E3, and E4 respectively extracted from the first examination room 20a, the third examination room 20c, and the fourth examination room 20d, that is, with the scheduled examination start times of the examinations E5, E7, and E8, the examination extraction unit 122 determines whether all of the scheduled examination start times of the examinations E5, E7, and E8 are later than the scheduled examination start time of the examination E1. In the examination schedule illustrated in Fig. 4, all of the scheduled examination start times of the examinations E5, E7, and E8 are later than the scheduled examination start time of the examination E2 (S34/Y), and hence the examination extraction unit 122 specifies the examination E2 as an examination to which the endoscope 30 is to be assigned (S36).

As described above, the determination processing of S34 is executed for all the examinations extracted from each examination room 20 in S30. Herein, all the examinations with examination Nos. E1, E2, E3, and E4 extracted from each examination room 20 in S 30 are specified as examinations to which the endoscope 30 is to be assigned (S36), and because N reaches the total number of examination rooms at that time (S40/Y), the assignment target examination extraction processing ends. With reference to the basic flowchart illustrated in Fig. 7, when the assignment target examination extraction processing of S16 ends, the endoscope assignment processing of S18 is started.

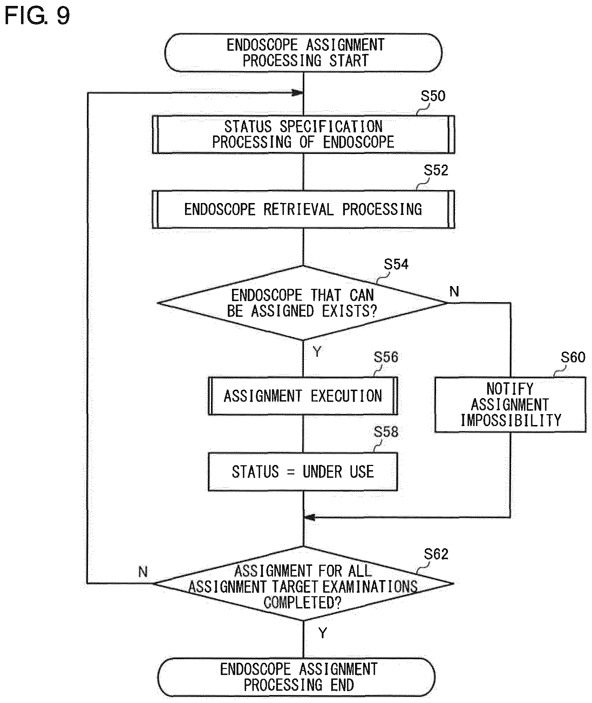

Fig. 9 illustrates a detailed flowchart of the endoscope assignment processing illustrated in S18 of the basic flowchart. In the first assignment processing unit 120, the endoscope assignment unit 126 performs processing for assigning, of a plurality of possessed endoscopes 30, an endoscope to be used to each endoscopic examination whose schedule is managed by the examination schedule management unit 110.

As a premise for performing the endoscope assignment processing, the endoscope specification unit 124 first specifies the statuses of all the endoscopes 30 at the scheduled start time of the examination extracted as an endoscope assignment target (S50). As described with reference to Fig. 2, the status of the endoscope 30 is specified by any one of ST1 to ST4.

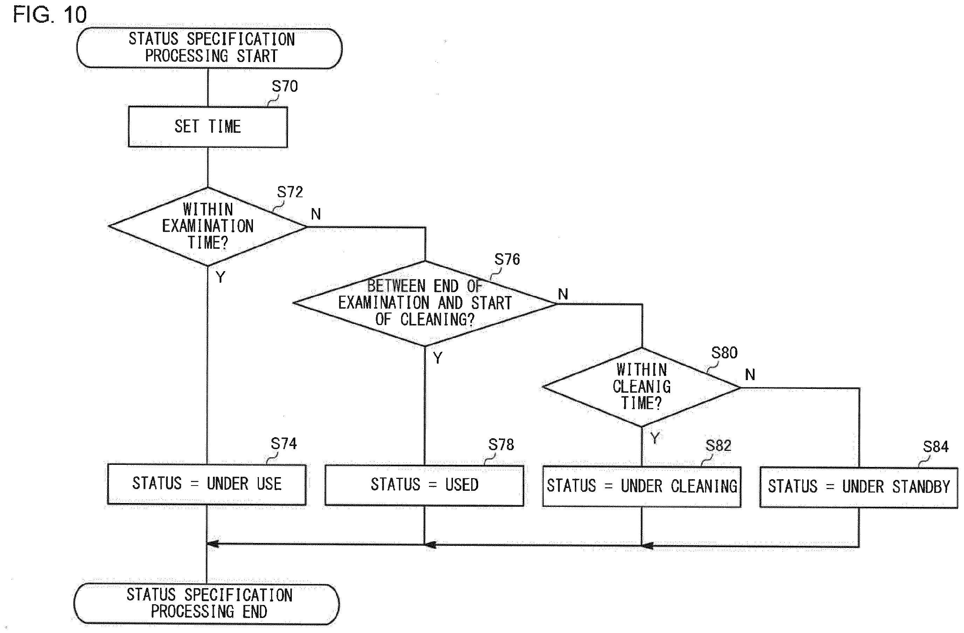

Fig. 10 illustrates a detailed flowchart of the status specification processing of S50. The endoscope specification unit 124 first sets the scheduled examination start time of an examination to be an assignment target (S70). Because the scheduled examination start times of the examinations E1 to E4 are all 9:00, time is set to 9:00 herein. The endoscope specification unit 124 specifies the statuses, at the set time, of all the endoscopes with endoscope Nos. 1 to 19 recorded in the possessed endoscope master table 222.

With reference to the schedule information on the endoscope 30, if the set time (9:00) is within the time period of the assigned examination (S72/Y), the status of the endoscope 30 is specified as "under use" (S74). Also, if the set time is outside the time period of the examination (S72/N) and is after the end of the assigned examination and before the start of cleaning (S76/Y), the status of the endoscope 30 is specified as "used" (S78). Also, if the set time is after the end of the examination and is not before the start of cleaning (S76/N) but within a cleaning time (S80/Y), the status of the endoscope 30 is specified as "under cleaning" (S82). Also, if the set time is not even within a cleaning time (S80/N), the status of the endoscope 30 is specified as "under standby" (S84). In this way, the endoscope specification unit 124 can grasp which endoscope is "under standby" at a scheduled examination start time, that is, which endoscope can be assigned, by specifying the statuses, at the set time, of all the endoscopes.

Returning to Fig. 9, the endoscope specification unit 124 executes retrieval processing on the possessed endoscopes to specify an available endoscope 30 (S52). Herein, the endoscope specification unit 124 executes the retrieval processing by narrowing down to the endoscopes specified as "under standby" in S50. Because the endoscope 30, the status of which is other than "under standby", that is, "under use", "used", or "under cleaning", cannot be assigned to an examination at that time, retrieval efficiency can be improved by excluding it from the retrieval targets.

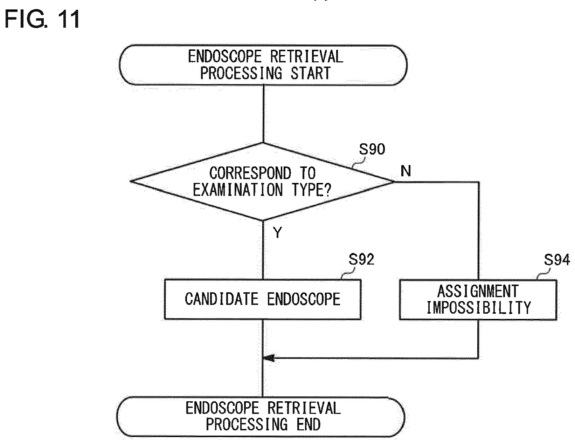

Fig. 11 illustrates a detailed flowchart of the endoscope retrieval processing in S52. The endoscope specification unit 124 executes retrieval processing on all the endoscopes 30 whose statuses are "under standby." Herein, the endoscope specification unit 124 specifies the examination type of an examination to be an assignment target. Herein, the scheduled examination start times of all the examinations E1 to E4 that are assignment targets are 9:00, and it is specified in S50 that the statuses of all the endoscopes 30 are "under standby" at 9:00. Therefore, the endoscope specification unit 124 determines whether the endoscope 30 whose status is "under standby" corresponds to the examination type of each of the examinations E1, E2, E3, and E4 (S90).

In the embodiment, the endoscope specification unit 124 specifies an available endoscope 30 based on the examination type information on an endoscopic examination to be an assignment target. In the embodiment, the examination type is distinguished by whether it is an upper examination or a lower examination. Therefore, when the examination type information on an examination indicates an upper examination, it is determined in S90 that an endoscope for upper examination corresponds and an endoscope for lower examination does not correspond. Similarly, when the examination type information on an examination indicates a lower examination, it is determined that an endoscope for lower examination corresponds and an endoscope for upper examination does not correspond.

With reference to the possessed endoscope master table 222 illustrated in Fig. 6 and the examination schedule illustrated in Fig. 4, the endoscope specification unit 124 determines the endoscopes with endoscope Nos. 1 to 14, which are endoscopes for upper examination, as candidate endoscopes for the upper examinations with examination Nos. E1, E2, and E3 (S92); on the other hand, determines the endoscopes with endoscope Nos. 15 to 19, which are endoscopes for lower examination, as unassignable for the examinations with examination Nos. E1, E2, and E3 (S94). Additionally, the endoscope specification unit 124 determines the endoscopes with endoscope Nos. 15 to 19, which are endoscopes for lower examination, as candidate endoscopes for the lower examination with examination No. E4 (S92); on the other hand, determines the endoscopes with endoscope Nos. 1 to 14, which are endoscopes for upper examination, as unassignable for the examination with examination No. E4 (S94). The endoscope specification unit 124 notifies the endoscope assignment unit 126 of the correspondence between the specified candidate endoscopes and the examination Nos.

Returning to Fig. 9, the endoscope assignment unit 126 assigns the endoscope 30 to be used to each examination managed by the examination schedule management unit 110 based on the candidate endoscopes specified by the endoscope specification unit 124. Specifically, the endoscope assignment unit 126 assigns one of the candidate endoscopes specified by the endoscope specification unit 124 to an endoscopic examination. In the following example, of the plurality of candidate endoscopes, an endoscope with a smaller endoscope No., set in the possessed endoscope master table 222 illustrated in Fig. 6, is sequentially assigned to an examination, but it is not intended to limit to this order.

The endoscope assignment unit 126 first determines that there is an endoscope assignable to the examination E1 (S54/Y). Herein, it is notified by the endoscope specification unit 124 that the endoscopes with endoscope Nos. 1 to 14 can be assigned to the examinations E1, E2, and E3, and therefore the endoscope assignment unit 126 assigns the endoscope G-R-1 with endoscope No. 1 to the examination E 1 (S56). The endoscope assignment unit 126 sets the status of the endoscope G-R-1 to be "under use" such that the same endoscope G-R-1 is not assigned to another examination (S 58). When the status is set to be "under use", the endoscope G-R-1 is excluded from the candidate endoscopes in the next assignment by the endoscope assignment unit 126.

Next, the endoscope assignment unit 126 determines that there is an endoscope that can be assigned to the examination E 2 (S54/Y), and assigns the endoscope G-R-2 with endoscope No. 2 to the examination E2 (S56), and sets the status of the endoscope G-R-2 to be "under use" (S58). Similarly, the endoscope assignment unit 126 assigns the endoscope G-R-3 with endoscope No. 3 to the examination E3 (S56), and sets the status of the endoscope G-R-3 to be "under use" (S58).

Next, the endoscope assignment unit 126 determines that there is an endoscope assignable to an examination E4 (S54/Y). Herein, it is notified by the endoscope specification unit 124 that the endoscopes with endoscope Nos. 15 to 19 can be assigned to the examination E4, and therefore the endoscope assignment unit 126 assigns an endoscope C-R-1 with endoscope No. 15 to the examination E4 (S56), and sets the status of the endoscope C-R-1 to be "under use" (S58).

In S54, when there is no endoscope that can be assigned to an examination (S54/N), the endoscope assignment unit 126 notifies a user that assignment cannot be performed (S60). The timing of this notification may be after the assignment processing of the endoscope 30 is completed for all the examinations. Before endoscopic examination work for one day starts, at least the user needs to recognize that there is an examination to which the endoscope 30 is not assigned.

The assignment processing by the endoscope assignment unit 126 is repeated until the assignment of the endoscopes 30 to all the extracted examinations to be assignment targets is completed (S62/N), and when the endoscopes 30 are assigned to all the examinations (herein, E1 to E4) (S62/Y), this endoscope assignment processing ends. The results of the assignment by the endoscope assignment unit 126 is notified to the examination schedule management unit 110.

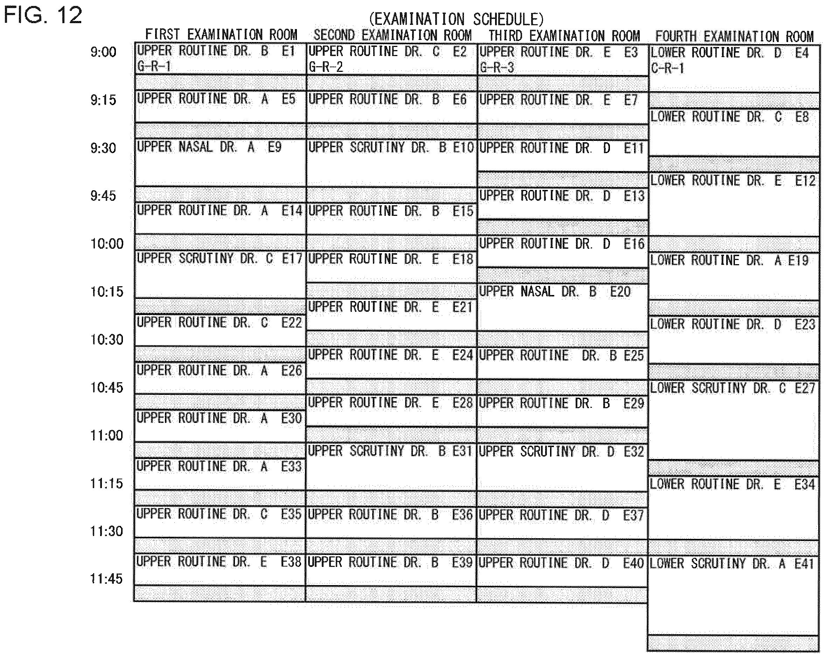

Fig. 12 illustrates an examination schedule updated by the examination schedule management unit 110. When the results of the assignment is notified from the endoscope assignment unit 126, the examination schedule management unit 110 registers the assigned endoscope 30 in the corresponding examination. Herein, it is registered that: the endoscope G-R-1 is used in the examination E1; the endoscope G-R-2 in the examination E2; the endoscope G-R-3 in the examination E3; and the endoscope C-R-1 in the examination E4. The examination schedule management unit 110 records the updated examination schedules in the examination schedule holding unit 206. In this way, the schedule information on the endoscopes G-R-1, G-R-2, G-R-3, and C-R-1 are generated.

Returning to Fig. 7, when the endoscope assignment processing of S18 ends, the cleaning machine assignment processing of S20 is started. In Fig. 3, the cleaning schedule management unit 130 manages a cleaning schedule of a plurality of endoscopes, including the cleaning machines 50, information on scheduled cleaning start time, and that on scheduled cleaning end time. The second assignment processing unit 140 assigns, of a plurality of the cleaning machines 50, a cleaning machine 50 for cleaning the endoscope 30 to be used in each endoscopic examination. The cleaning schedule management unit 130 generates a cleaning schedule of the endoscopes 30 based on the cleaning machines 50 assigned to the endoscopes 30 by the second assignment processing unit 140, and records it in the cleaning schedule holding unit 208.

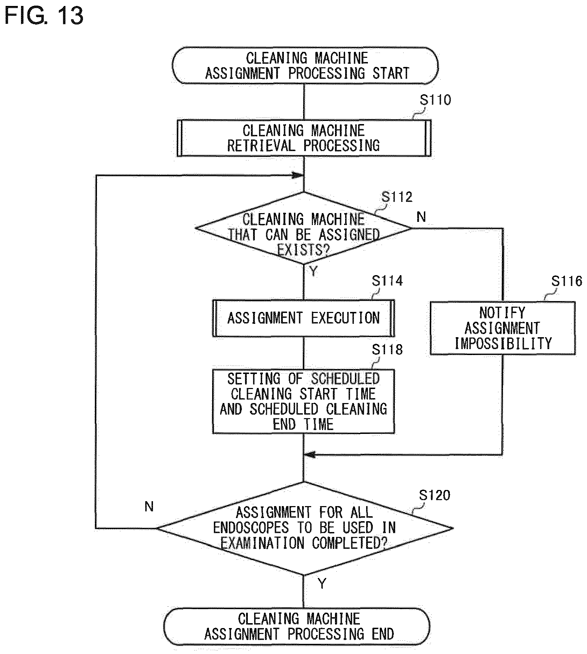

Fig. 13 illustrates a detailed flowchart of the cleaning machine assignment processing indicated by S20 of the basic flowchart. The second assignment processing unit 140 includes a cleaning machine specification unit 142, a cleaning machine assignment unit 144, an end time determination unit 146, a cleaning machine assignment availability confirmation unit 148, and a person-in-charge assignment unit 149.

The cleaning machine specification unit 142 specifies available cleaning machines 50 by executing retrieval processing on the possessed cleaning machines 50 for the respective endoscopes 30 assigned in S18 (S110). When the use of the cleaning machine 50 is not restricted, that is, when the use of all the cleaning machines 50 is permitted to the endoscopes 30, the cleaning machine specification unit 142 specifies that all of the first cleaning machine 50a to the fourth cleaning machine 50d are available. At this time, the cleaning machine specification unit 142 acquires an available time zone for each cleaning machine 50 by referring to the cleaning schedule held in the cleaning schedule holding unit 208. The available time zone means a time zone during which cleaning is not scheduled. When the second assignment processing unit 140 initially executes the cleaning machine assignment processing, the cleaning schedule is blank, that is, no cleaning schedule is registered with any of the cleaning machines 50, and therefore all the time zones are available. The available cleaning machines 50 and their available time zones are notified to the cleaning machine assignment unit 144.

The cleaning machine assignment unit 144 assigns, of a plurality of available cleaning machines 50, a cleaning machine 50 for cleaning the endoscope 30 to be used in each endoscopic examination. Herein, the cleaning machine assignment unit 144 assigns the cleaning machine 50 for cleaning an endoscope 30 assigned to an endoscopic examination by the endoscope assignment unit 126, so that a time after the scheduled examination end time of the endoscope 30 becomes a scheduled cleaning start time. In the present embodiment, it is made possible from the viewpoint of improving work efficiency to set the same time as the scheduled examination end time to be the scheduled cleaning start time, but an interval of a predetermined period of time may be provided between the scheduled examination end time and the scheduled cleaning start time.

The cleaning machine assignment unit 144 assigns the cleaning machine 50 to, of a plurality of the endoscopes 30 to which the cleaning machine 50 is to be assigned, that is, of a plurality of the endoscopes 30 assigned to examinations in S18, the endoscopes 30 in the order starting from the earliest scheduled examination end time. Herein, the scheduled examination end times of the examinations E1 to E3 are 9:10 and that of the examination E4 is 9:15, and hence the cleaning machine assignment unit 144 assigns the cleaning machines 50 to the endoscopes to be used, in the order of the examinations E1, E2, E3, and E4. Alternatively, the cleaning machine assignment unit 144 may assign the cleaning machines 50 in the order of assigning the endoscopes 30 to examinations. In the present embodiment, it is assumed that the cleaning machine 50, which can be assigned to the endoscope 30, always exists (S112/Y), but when the assignable cleaning machine 50 does not exist (S112/N), it is notified that the cleaning machine 50 cannot be assigned to the endoscope 30 (S116).

The cleaning machine assignment unit 144 assigns the first cleaning machine 50a to the endoscope G-R-1 to be used in the examination E1 (S114). In the embodiment, the scheduled cleaning times of all the cleaning machines 50 are set to be 20 minutes, but the scheduled cleaning time may be different for each cleaning machine 50, and that may also be different depending on cleaning modes in the cleaning machine 50. The cleaning machine assignment unit 144 sets the scheduled end time of the examination E1 to be a scheduled cleaning start time, and sets the time 20 minutes after that to be a scheduled cleaning end time (S118). As a result of this assignment, the status of the first cleaning machine 50a between 9:10 and 9:30 is set to be "under use." The status of the cleaning machine 50 becomes either "under use" or "under standby", and when the processing for generating schedule information is started, it is assumed that the statuses of all the cleaning machines 50 are "under standby." The status of the endoscope G-R-1 between 9:10 and 9:30 is "under cleaning."

Next, the cleaning machine assignment unit 144 assigns the second cleaning machine 50b to the endoscope G-R-2 to be used in the examination E2 (S114). The cleaning machine assignment unit 144 sets the scheduled end time of the examination E2 to be a scheduled cleaning start time (9:10), and sets the time 20 minutes after that (9:30) to be a scheduled cleaning end time (S118). As a result of this assignment, the status of the second cleaning machine 50b between 9:10 and 9:30 is set to be "under use."

Next, the cleaning machine assignment unit 144 assigns the third cleaning machine 50c to the endoscope G-R-3 to be used in the examination E3 (S114). The cleaning machine assignment unit 144 sets the scheduled end time of the examination E3 to be a scheduled cleaning start time (9:10), and sets the time 20 minutes after that (9:30) to be a scheduled cleaning end time (S118). As a result of this assignment, the status of the third cleaning machine 50c between 9:10 and 9:30 is set to be "under use."

Finally, the cleaning machine assignment unit 144 assigns the fourth cleaning machine 50d to the endoscope C-R-1 to be used in the examination E4 (S114). The cleaning machine assignment unit 144 sets the scheduled end time of the examination E4 to be a scheduled cleaning start time (9:15), and sets the time 20 minutes after that (9:35) to be a scheduled cleaning end time (S118). As a result of this assignment, the status of the fourth cleaning machine 50d between 9:15 and 9:35 is set to be "under use."

As described above, the cleaning machine assignment processing is repeated until the cleaning machines 50 are assigned to all the endoscopes 30 to be used in examinations (S120/N). When the cleaning machines 50 are assigned to all the endoscopes 30 to be used in examinations (S120/Y), the cleaning machine assigning processing ends. The results of the assignment by the cleaning machine assignment unit 144 is notified to the cleaning schedule management unit 130.

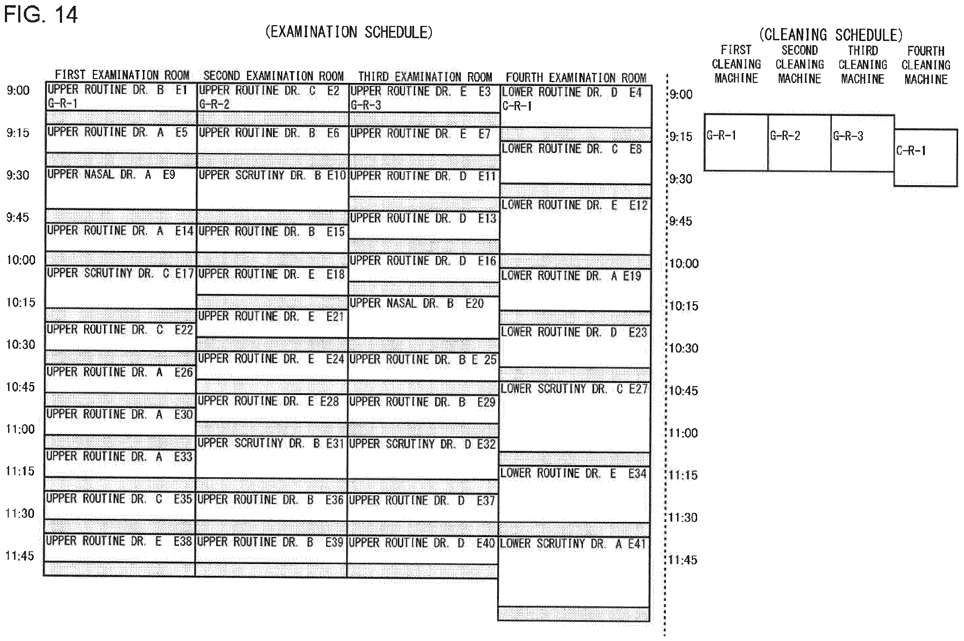

Fig. 14 illustrates a cleaning schedule generated by the cleaning schedule management unit 130. Herein, the results of the assignment by the cleaning machine assignment unit 144 are reflected in the cleaning schedule, and specifically it is registered that between 9:10 and 9:30: the endoscope G-R-1 is cleaned by the first cleaning machine 50a; the endoscope G-R-2 by the second cleaning machine 50b; and the endoscope G-R-3 by the third cleaning machine 50c, and registered that between 9:15 and 9:35 the endoscope C-R-1 is cleaned by the fourth cleaning machine 50d. The cleaning schedule management unit 130 records the updated cleaning schedule in the cleaning schedule holding unit 208.

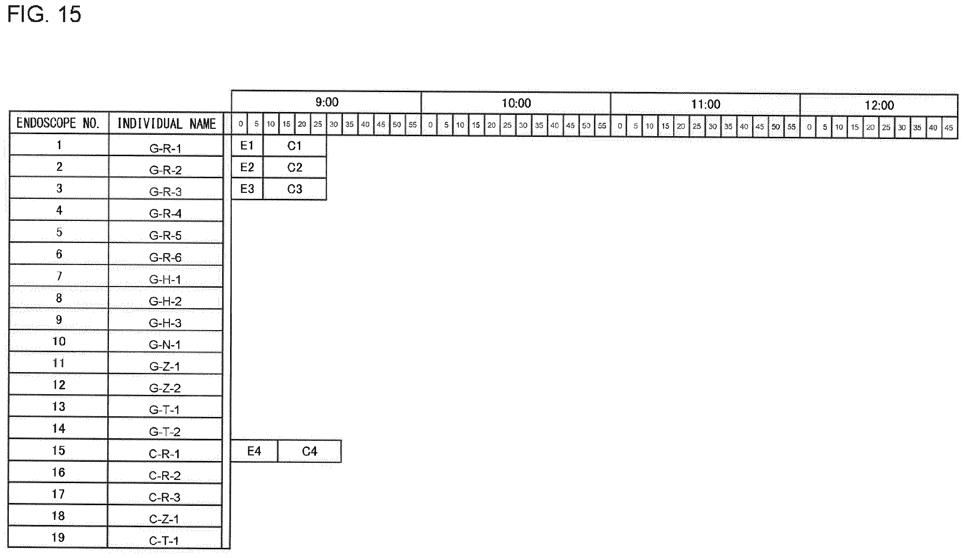

Fig. 15 illustrates schedule information on the individuals of the endoscope 30. Herein, for the sake of easy understanding, an example is illustrated, in which the display processing unit 150 displays an individual schedule at the stage where the above processing is completed, but in fact the display processing unit 150 displays an individual schedule at the stage where all the scheduling are completed. In Fig. 15, C1 indicates that the first cleaning machine 50a is under cleaning, C2 indicates that the second cleaning machine 50b is under cleaning, C3 indicates that the third cleaning machine 50c is under cleaning, and C4 indicates that the fourth cleaning machine 50d is under cleaning. Each of E1, E2, and the like indicates the examination No. of an examination under use. Information indicated by such an individual schedule serves as schedule information on each individual.

Returning to Fig. 7, when the cleaning machine assignment processing of S20 ends, it is determined whether the processing is completed for the examinations with all examination Nos. (S22), and when not completed, the basic flow is repeated by returning to S16. Hereinafter, a process will be described, in which schedule information on the endoscope 30 is generated by repeatedly executing the steps of S16 to S20. The steps of S16 to S20 described above with respect to the examinations E1 to E4 serve as the first time processing.

### <Second Time: S16 to S20>

In S16, the examination extraction unit 122 extracts the examination E5 from the first examination room 20a, the examination E6 from the second examination room 20b, the examination E7 from the third examination room 20c, and the examination E8 from the fourth examination room 20d, thereby specifying the examinations E5 to E8 as the examinations to which the endoscope 30 is to be assigned.

In S18, the endoscope specification unit 124 determines the endoscopes with endoscope Nos. 4 to 14, which are endoscopes for upper examination, as candidate endoscopes for the examinations E5, E6, and E7, and determines the endoscopes with endoscope Nos. 16 to 19, which are endoscopes for lower examination, as candidate endoscopes for the examination E8. Because the statuses of the endoscopes with endoscope Nos. 1 to 3 are "under cleaning" at the scheduled examination start time (9:15) of the examinations E5, E6, and E7, they do not become candidate endoscopes for the examinations E5, E6, and E7. Also, because the status of the endoscope with endoscope No. 15 is "under cleaning" at the scheduled examination start time (9:20) of the examination E8, it dose not become a candidate endoscope for the examination E8. The specified candidate endoscopes are notified to the endoscope assignment unit 126.

In response to the notice from the endoscope specification unit 124, the endoscope assignment unit 126 assigns: an endoscope G-R-4 with endoscope No. 4 to the examination E5; an endoscope G-R-5 with endoscope No. 5 to the examination E6; and an endoscope G-R-6 with endoscope No. 6 to the examination E7. Also, the endoscope assignment unit 126 assigns an endoscope C-R-2 with endoscope No. 16 to the examination E8.

In S20, the cleaning machine assignment unit 144 assigns: the first cleaning machine 50a to the endoscope G-R-4 for the examination E5; the second cleaning machine 50b to the endoscope G-R-5 for the examination E6; the third cleaning machine 50c to the endoscope G-R-6 for the examination E7; and the fourth cleaning machine 50d to the endoscope C-R-2 for the examination E8. The cleaning machine assignment unit 144 sets: the scheduled cleaning start times of the endoscopes G-R-4, G-R-5, and G-R-6 to be 9:30; the scheduled cleaning end times thereof to be 9:50; the scheduled cleaning start time of the endoscope C-R-2 to be 9: 35; and the scheduled cleaning end time thereof to be 9:55.

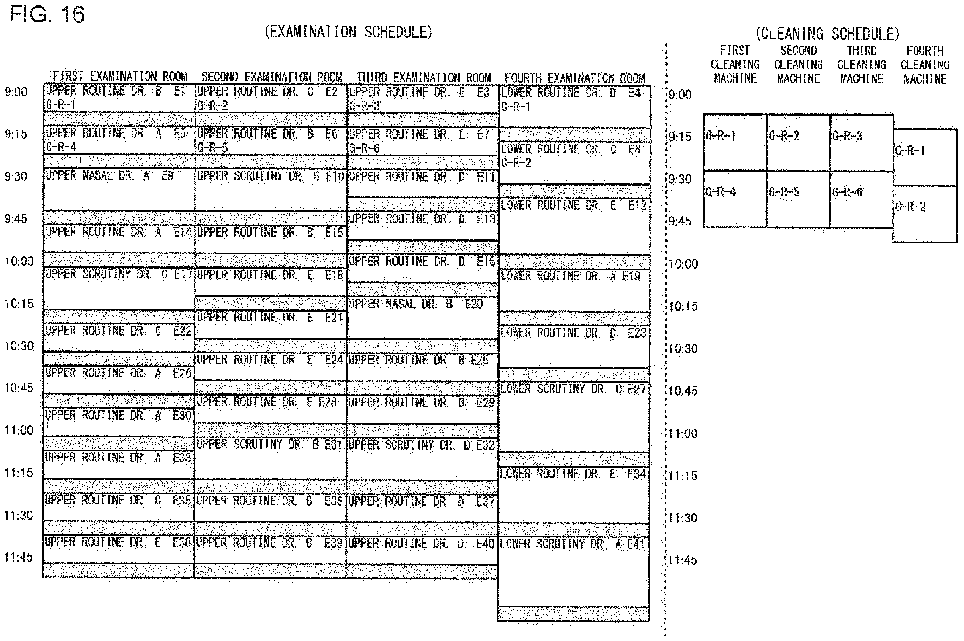

Fig. 16 illustrates an examination schedule generated by the examination schedule management unit 110 and a cleaning schedule generated by the cleaning schedule management unit 130. Herein, the results of the assignment by the endoscope specification unit 124 are reflected in the examination schedule, and those of the assignment by the cleaning machine assignment unit 144 are reflected in the cleaning schedule.

### <Third Time: S16 to S20>

In S16, the examination extraction unit 122 extracts: an examination E9 from the first examination room 20a; an examination E10 from the second examination room 20b; an examination E11 from the third examination room 20c; and an examination E12 from the fourth examination room 20d, thereby specifying the examinations E9 to E12 as the examinations to which the endoscopes 30 are to be assigned.

In S18, the endoscope specification unit 124 determines the endoscopes with endoscope Nos. 1 to 3 and 7 to 14, which are endoscopes for upper examination, as candidate endoscopes for the examinations E9, E10, and E11, and determines the endoscopes with endoscope Nos. 15 and 17 to 19, which are endoscopes for lower examination, as candidate endoscopes for the examination E12. Because the statuses of the endoscopes with endoscope Nos. 4 to 6 are "under cleaning" at the scheduled examination start times (9:30) of the examinations E9, E10, and E11, they do not become candidate endoscopes for the examinations E9, E10, and E11. Also, because the status of the endoscope with endoscope No. 16 is "under cleaning" at the scheduled examination start time (9:40) of the examination E12, it does not become a candidate endoscope for the examination E12. The specified candidate endoscopes are notified to the endoscope assignment unit 126.

The scheduled cleaning end times of the endoscopes with endoscope Nos. 1 to 3 are 9:30 and the cleaning thereof is completed at the time of 9:30, and hence the statuses thereof are "under standby", and they become candidate endoscopes for the examinations E9, E10, and E11. Also, the scheduled cleaning end time of the endoscope with endoscope No. 15 is 9:35, and the status thereof is "under standby" at the time of 9:40, and hence it becomes a candidate endoscope for the examination E12.

In response to the notice from the endoscope specification unit 124, the endoscope assignment unit 126 assigns: the endoscope G-R-1 with endoscope No. 1 to the examination E9; the endoscope G-R-2 with endoscope No. 2 to the examination E10; and the endoscope G-R-3 with endoscope No. 3 to the examination E11. Also, the endoscope assignment unit 126 assigns the endoscope C-R-1 with endoscope No. 15 to the examination E12.

In this way, the endoscope assignment unit 126 can re-assign an endoscope, the cleaning of which is completed and the status of which is "under standby" at the scheduled examination start time, to the examination. In other words, in the scheduling processing, the endoscope assignment unit 126 can assign the endoscope 30 to an endoscopic examination such that a time after the scheduled end time of cleaning by the cleaning machine 50, which is assigned to the endoscope 30 by the cleaning machine assignment unit 144, becomes a scheduled examination start time. Because the cleaning machine assignment unit 144 efficiently assigns the cleaning machine 50 to the endoscope 30 in the third time step, each of the endoscopes G-R-1, G-R-2, G-R-3, and C-R-1 is re-assigned to an examination to be started after the scheduled cleaning end time of each of them, thereby enabling efficient scheduling of the endoscopes 30.

In S20, the cleaning machine assignment unit 144 assigns: the first cleaning machine 50a to the endoscope G-R-1 for the examination E9; the second cleaning machine 50b to the endoscope G-R-2 for the examination E10; the third cleaning machine 50c to the endoscope G-R-3 for the examination E11; and the first cleaning machine 50a to the endoscope C-R-1 for the examination E12. The cleaning machine assignment unit 144 sets: the scheduled cleaning start times of the endoscopes G-R-1, G-R-2, and G-R-3 to be 9:50; the scheduled cleaning end times thereof to be 10:10; the scheduled cleaning start time of the endoscope C-R-1 to be 10:10; and the scheduled cleaning end time thereof to be 10:30.

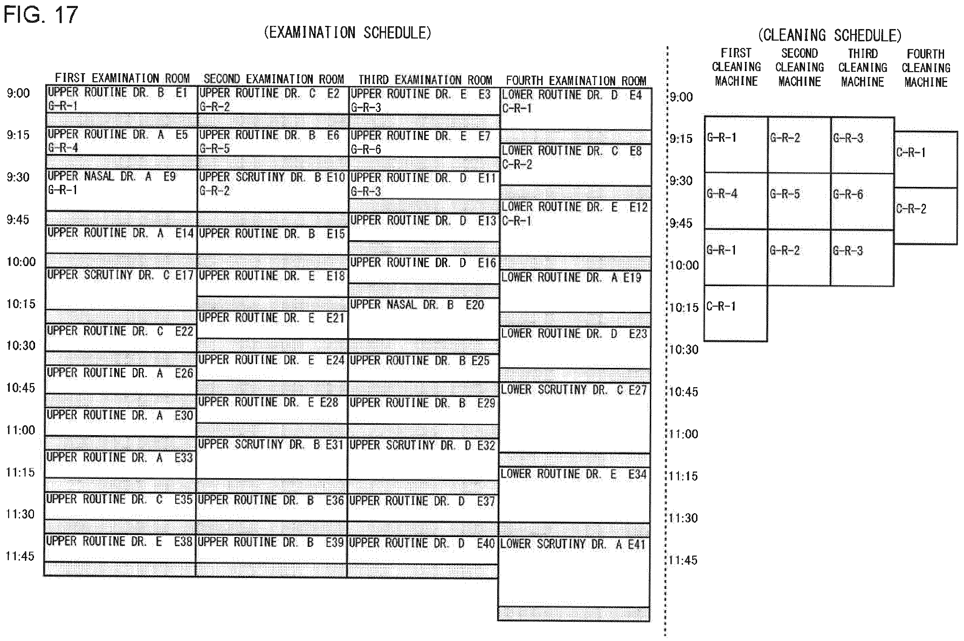

Fig. 17 illustrates an examination schedule generated by the examination schedule management unit 110 and a cleaning schedule generated by the cleaning schedule management unit 130. Herein, the results of the assignment by the endoscope assignment unit 126 are reflected in the examination schedule, and those of the assignment by the cleaning machine assignment unit 144 are reflected in the cleaning schedule.

As illustrated in the examination schedule, the scheduled examination end time of the examination E12 in which the endoscope C-R-1 is to be used is 10:00, on the other hand, according to the cleaning schedule of the fourth cleaning machine 50d, the fourth cleaning machine 50d is available after 9:55. Therefore, the cleaning machine assignment unit 144 can assign, from 10:00, the fourth cleaning machine 50d to the endoscope C-R-1 for the examination E12, but a 5-minute unused time occurs with the fourth cleaning machine 50d, and hence the cleaning machine assignment unit 144 assigns the first cleaning machine 50a to the endoscope C-R-1.

### <Fourth Time: S16 to S20>

In S16, the examination extraction unit 122 extracts: an examination E14 from the first examination room 20a; an examination E15 from the second examination room 20b; an examination E13 from the third examination room 20c; and an examination E19 from the fourth examination room 20d. Herein, when the step of S34 in Fig. 8 is executed, the scheduled examination start time (10:00) of the examination E16 next to the examination E13 in the third examination room 20c is earlier than the scheduled examination start time (10:05) of the examination E19 in the fourth examination room 20d (S34/N). That is, the examination E19 extracted from the fourth examination room 20d is started after the examination E16 in the third examination room 20c that is not yet extracted. Therefore, the examination extraction unit 122 determines that an endoscope should not be assigned to the examination E19 before the examination E16, and excludes the examination E19 from the examinations to which endoscopes are to be assigned (S38). The examinations E13, E14, and E15 are specified as the examinations to which the endoscopes 30 are to be assigned.

In S18, the endoscope specification unit 124 determines the endoscopes with endoscope Nos. 7 to 14, which are endoscopes for upper examination, as candidate endoscopes for the examination E13 whose scheduled examination start time is 9: 45. At this scheduled examination start time (9:45), the statuses of the endoscopes with endoscope Nos. 1 to 3 are "used", and those of the endoscopes with endoscope Nos. 4 to 6 are "under cleaning", and hence they do not become candidate endoscopes for the examination E13. Also, the endoscope specification unit 124 determines the endoscopes with endoscope Nos. 4 to 14, which are endoscopes for upper examination, as candidate endoscopes for the examinations E14 and E15 whose scheduled examination start times are 9:50. At this scheduled examination start time (9:50), the statuses of the endoscopes with endoscope Nos. 1 to 3 are "under cleaning", and hence they do not become candidate endoscopes for the examination E13. The specified candidate endoscopes are notified to the endoscope assignment unit 126.

In response to the notice from the endoscope specification unit 124, the endoscope assignment unit 126 assigns: an endoscope G-H-1 with endoscope No. 7 to the examination E13; the endoscope G-R-4 with endoscope No. 4 to the examination E14; and the endoscope G-R-5 with endoscope No. 5 to the examination E15. The endoscopes G-R-4 and G-R-5 are re-assigned to examinations after being cleaned at 9: 50.

In S20, the cleaning machine assignment unit 144 assigns : the fourth cleaning machine 50d to the endoscope G-H-1 for the examination E13; the second cleaning machine 50b to the endoscope G-R-4 for the examination E14; and the third cleaning machine 50c to the endoscope G-R-5 for the examination E15. The cleaning machine assignment unit 144 sets: the scheduled cleaning start time of the endoscope G-H-1 to be 9: 55; the scheduled cleaning end time thereof to be 10:15; the scheduled cleaning start times of the G-R-4 and G-R-5 to be 10:10; and the scheduled cleaning end times thereof to be 10:30.

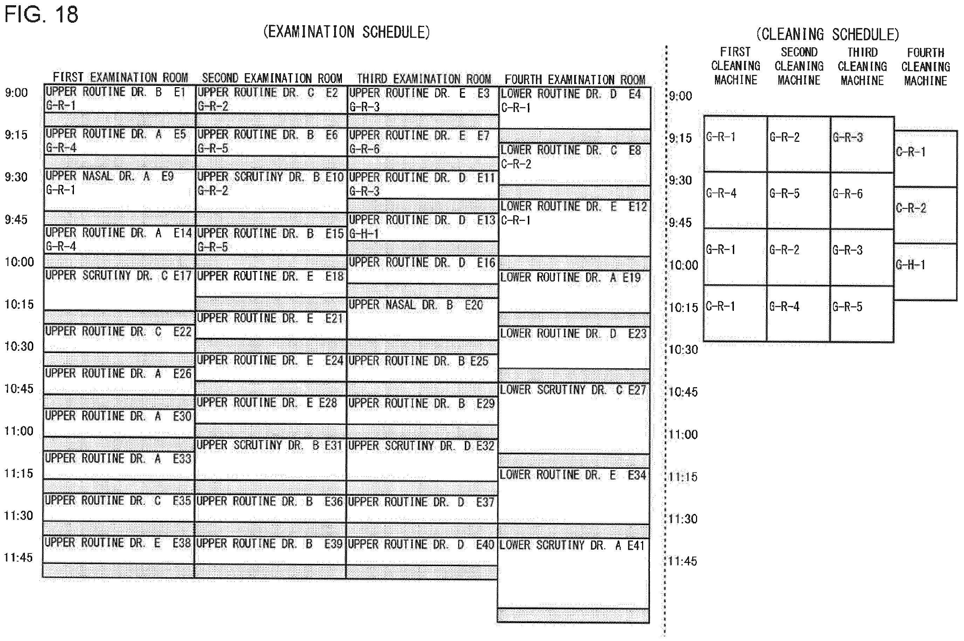

Fig. 18 illustrates an examination schedule generated by the examination schedule management unit 110 and a cleaning schedule generated by the cleaning schedule management unit 130. Herein, the results of the assignment by the endoscope assignment unit 126 are reflected in the examination schedule, and those of the assignment by the cleaning machine assignment unit 144 are reflected in the cleaning schedule.

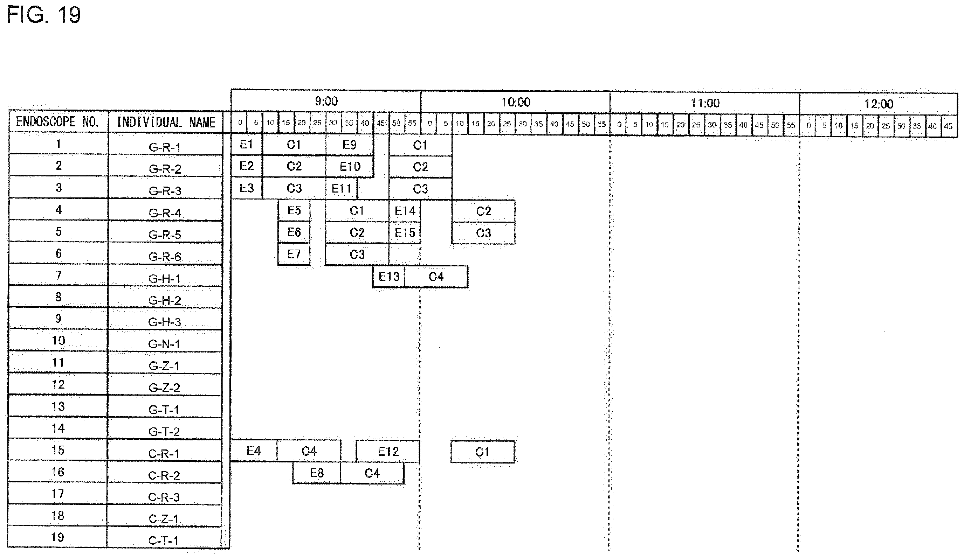

Fig. 19 illustrates an individual schedule of the endoscope 30. In this way, individual schedule information on the endoscope 30 are generated as illustrated in Fig. 19 by repeating four times S16 to S20 of the basic flowchart.

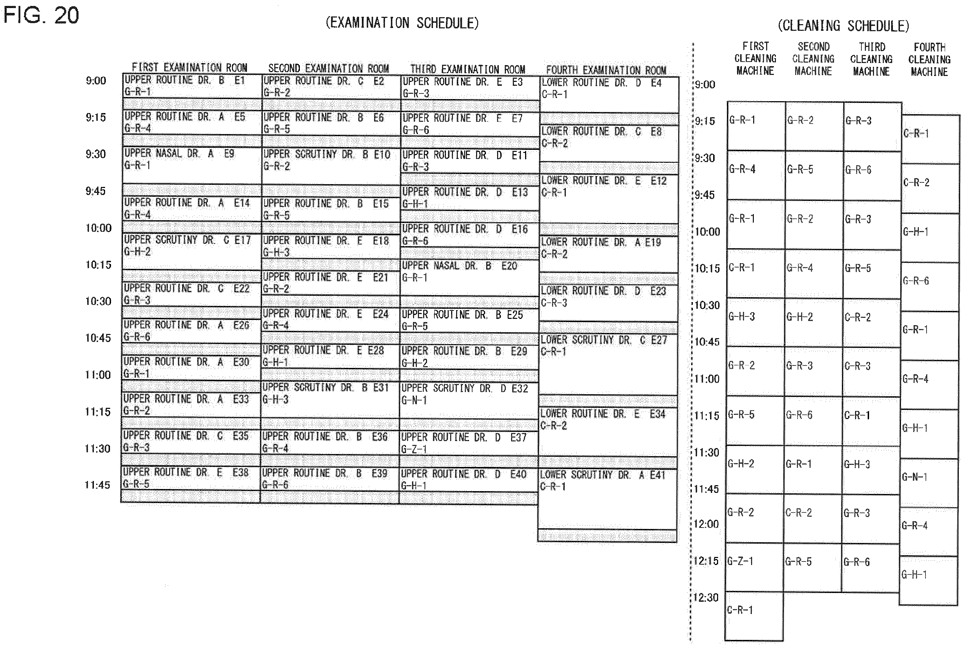

As described above, the processing of S16 to S20 is repeated until the assignment processing for the last examination is completed. Fig. 20 illustrates an examination schedule generated by the examination schedule management unit 110 and a cleaning schedule generated by the cleaning schedule management unit 130. Herein, the results of the assignment by the endoscope assignment unit 126 are reflected in the examination schedule, and those of the assignment by the cleaning machine assignment unit 144 are reflected in the cleaning schedule. As described above, the processing for generating the schedule information on the endoscope 30 is completed when the assignment of the endoscopes 30 to all the examinations is completed and when the assignment of the cleaning machines 50 to the endoscopes 30 is completed.

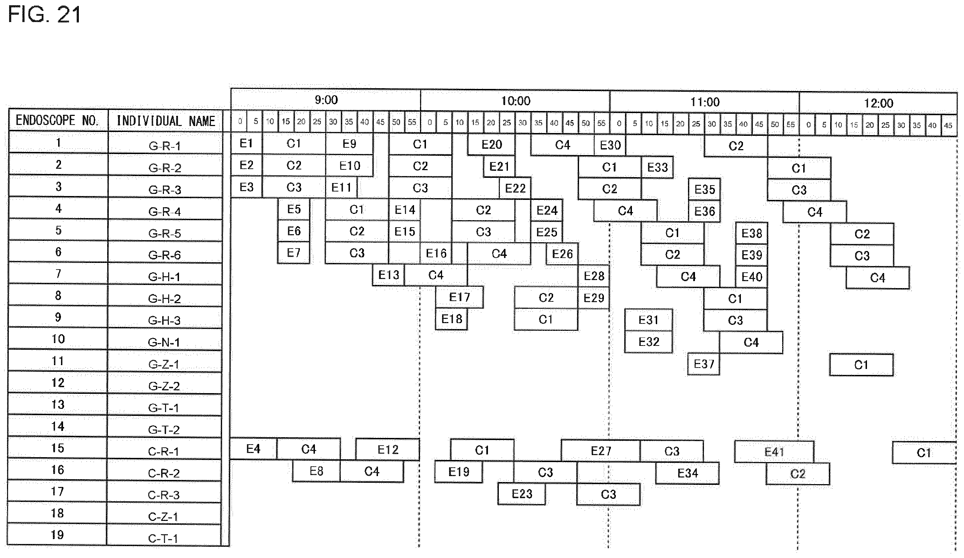

Fig. 21 illustrates an individual schedule of the endoscopes 30 for one day. The results of the assignment of the endoscopes 30 by the endoscope assignment unit 126 and/or the results of the assignment of the cleaning machines 50 by the cleaning machine assignment unit 144 are displayed on the display of the terminal device 12 by the display processing unit 150. For example, the display processing unit 150 may read the examination schedule information from the examination schedule holding unit 206 and display the examination schedule table illustrated in Fig. 20 on the display of the terminal device 12. Also, the display processing unit 150 may read the cleaning schedule information from the cleaning schedule holding unit 208 and display the cleaning schedule table illustrated in Fig. 20 on the display of the terminal device 12. Also, the display processing unit 150 may display the examination schedule table and the cleaning schedule table on the same screen. As a result, a doctor and a person preparing for examination can easily recognize which endoscope 30 is to be used in an examination, and a person preparing for examination can easily recognize which cleaning machine 50 is to be used for the cleaning of the used endoscope 30.

Also, the display processing unit 150 may read the examination schedule information from the examination schedule holding unit 206 and read the cleaning schedule information from the cleaning schedule holding unit 208, and may display the individual schedule table of the endoscopes 30 on the display of the terminal device 12. This individual schedule table is illustrated in Fig. 21, and by generating such an individual schedule table, a person preparing for examination can understand the schedule of each individual of the endoscope 30. In the case of wanting to understand the situation of the endoscope 30 at a certain time, a person preparing for examination can understand, by the individual schedule table, the situation such as whether the endoscope 30 is under cleaning or under use in an examination.

As illustrated in Fig. 21, the endoscope C-R-1 is scheduled to be under cleaning between 12:30 and 12:50. Depending on a medical facility, if the lunch break of persons preparing for examination is defined to be, for example, between 12:30 and 13:30, it may be notified that this cleaning processing is performed outside working hours.

The end time determination unit 146 determines whether, as a result of the assignment of the cleaning machine 50 to the endoscope 30 by the cleaning machine assignment unit 144, the scheduled cleaning end time is after a reference time (12:30). When it is determined by the end time determination unit 146 that the scheduled cleaning end time is after the reference time, the cleaning machine assignment availability confirmation unit 148 may transmit a notice to a user (e.g., an operator) to confirm whether the cleaning machine assignment is permitted. For example, the timing of this notification may be after the assignment processing of the endoscopes 30 is completed for all the examinations. With reference to Fig. 21, the scheduled cleaning end time of the endoscope G-H-1 is 12: 35, and hence also with respect to this cleaning schedule, the cleaning machine assignment availability confirmation unit 148 confirms to a user the availability of cleaning machine assignment.

Schedules of doctors are determined by the examination order, and a doctor grasps the scheduled start time and the examination room, etc., of the next examination according to the examination schedule table. Similarly, the information management device 10 may set a schedule to a person preparing for examination. In this schedule, it may be set that a person preparing for examination brings the endoscope 30 into the examination room 20 before the start of an examination and brings the endoscope 30 into the cleaning room 40 after the end of the examination, etc. , and it also may be set that he/she assists an examination in an examination room, etc.

Hereinafter, various aspects related to the scheduling processing by the information management device 10 of the embodiment will be described.

### <Example 1>

In the embodiment, the endoscope specification unit 124 specifies an available endoscope 30 based on the examination type information on an endoscopic examination to be an assignment target, in the endoscope retrieval processing illustrated in Fig. 11. At this time, the examination type is distinguished depending on whether it is an upper examination or a lower examination, but in Example 1, the endoscope specification unit 124 specifies an available endoscope 30 based on the examination type information indicating more detailed examination contents.

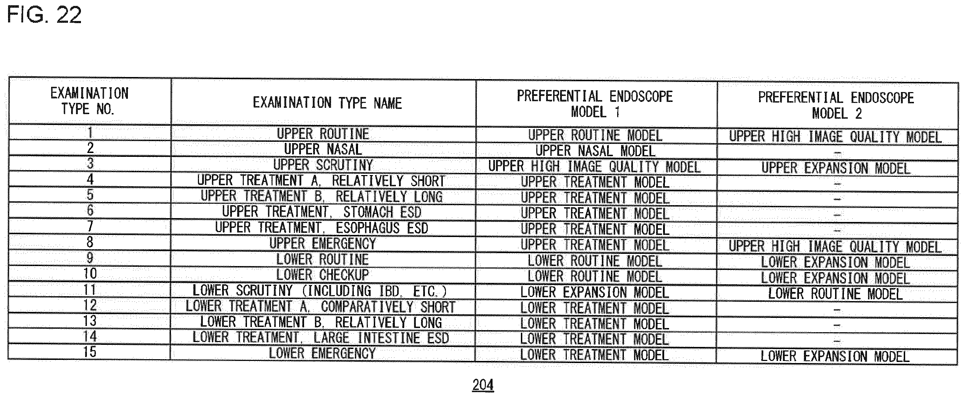

Fig. 22 illustrates an endoscope order table held in an endoscope order holding unit 204. The endoscope order table records endoscope models to be preferentially assigned to the examination types by associating them with each other. Herein, the "preferential endoscope model 1" means information on the models to be assigned most preferentially, and the "preferential endoscope model 2" means information on the models to be assigned with the second highest priority. If an endoscope 30 designated by the "preferential endoscope model 1" is "under standby", the endoscope specification unit 124 specifies the endoscope 30 as a candidate endoscope. On the other hand, if an endoscope 30 designated by the "preferential endoscope model 1" is not "under standby" and an endoscope 30 designated by the "preferential endoscope model 2" is "under standby", the endoscope specification unit 124 specifies the endoscope 30 designated by the "preferential endoscope model 2" as a candidate endoscope. As described above, the endoscope order holding unit 204 holds the priority order of the models of the endoscope 30 to be assigned to the examination types of endoscopic examinations, and the endoscope specification unit 124 specifies the endoscope 30 of a model with higher priority order as a candidate endoscope.

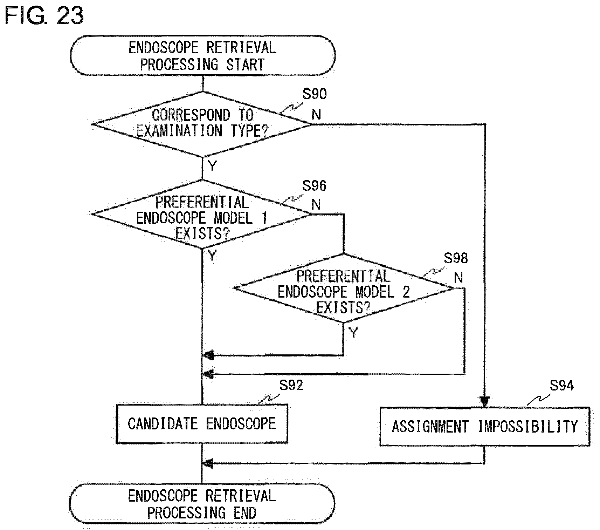

Fig. 23 illustrates a detailed flowchart of endoscope retrieval processing in Example 1. The endoscope specification unit 124 executes retrieval processing on all the endoscopes 30 whose statuses are "under standby." The endoscope specification unit 124 specifies an available endoscope 30 based on the examination type information on the endoscopic examination to be an assignment target. In S90, when the examination type information on an examination indicates an upper examination, it is determined that an endoscope for upper examination corresponds and an endoscope for lower examination does not correspond. Similarly, when the examination type information on an examination indicates a lower examination, it is determined that an endoscope for lower examination corresponds and an endoscope for upper examination does not correspond.

When the first time steps of S16 to S20 in the embodiment are described, the endoscope specification unit 124 determines that the endoscopes with endoscope Nos. 1 to 14, which are endoscopes for upper examination, correspond to the examinations E1, E2, and E3; and determines that the endoscopes with endoscope Nos. 15 to 19, which are endoscopes for lower examination, correspond to the examination E4 (S90).

The examination types of the examinations E1, E2, and E3 are "upper routine examination", and the endoscope specification unit 124 recognizes that a model with the highest priority order (preferential endoscope model 1) is an "upper routine model", by referring to the endoscope order information held in the endoscope order holding unit 204. Therefore, the endoscope specification unit 124 determines whether an "upper routine model" is included in the corresponding endoscopes (S96). In this case, the endoscopes with endoscope Nos. 1 to 6 exist as an upper routine model (S96/Y), and hence the endoscope specification unit 124 determines the endoscopes with endoscope Nos. 1 to 6 as candidate endoscopes (S92).

The examination type of the examinations E4 is a "lower routine examination", and the endoscope specification unit 124 recognizes that a model with the highest priority (preferential endoscope model 1) is a "lower routine model", by referring to the endoscope order information held in the endoscope order holding unit 204. Therefore, the endoscope specification unit 124 determines whether a "lower routine model" is included in the corresponding endoscopes (S96). In this case, the endoscopes with endoscope Nos. 15 to 17 exist as a lower routine model (S96/Y), and hence the endoscope specification unit 124 determines the endoscopes with endoscope Nos. 15 to 17 as candidate endoscopes (S92).

With respect to the examinations E1 to E3, if the endoscope of the preferential endoscope model 1 is not included in the corresponding endoscopes (S96/N), the endoscope specification unit 124 recognizes that the model with the second highest priority order (preferential endoscope model 2) is an "upper high image quality mode l", by referring to the endoscope order information held in the endoscope order holding unit 204. Therefore, the endoscope specification unit 124 determines whether an "upper high image quality model" is included in the corresponding endoscopes (S98), and if it is included (S98/Y), the endoscope specification unit 124 determines the endoscope of an upper high image quality model as a candidate endoscope (S92).

If the endoscope of the preferential endoscope model 2 is also not included in the corresponding endoscopes (S98/N), the endoscope specification unit 124 determines, of the endoscopes determined to correspond to the examination type in S90, an endoscope of a model other than the preferential endoscope models as a candidate endoscope (S92). The endoscope specification unit 124 notifies the endoscope assignment unit 126 of the specified candidate endoscope, and the endoscope assignment unit 126 assigns the endoscope 30 to an examination, as described in the embodiment.

When the endoscope specification unit 124 specifies an endoscope 30 of low priority order model and the endoscope assignment unit 126 intends to assign the specified endoscope to an endoscopic examination, it is preferable that the endoscope assignment availability confirmation unit 128 confirms to a user the availability of endoscope assignment. For example, when the preferential endoscope model 1 is not assigned, the endoscope assignment availability confirmation unit 128 may perform confirmation to a user assuming that a model with low priorityorder is assigned, but when the model set by the endoscope order holding unit 204 is assigned (e.g., when the preferential endoscope model 2 is assigned), the endoscope assignment availability confirmation unit 128 may not perform confirmation to a user. That is, only when the endoscope specification unit 124 determines a model other than the preferential endoscope models as a candidate endoscope and the endoscope assignment unit 126 assigns the candidate endoscope to an examination, the endoscope assignment availability confirmation unit 128 may be caused to confirm to a user the availability of the endoscope assignment. When the endoscope specification unit 124 cannot determine the preferential endoscope model as a candidate endoscope (S98/N), the endoscope specification unit 124 may determine that there is no candidate endoscope and the endoscope assignment availability confirmation unit 128 may notify a user of the fact.

The timing of this confirmation may be after the assignment processing of the endoscopes 30 is completed for all the examinations. Before endoscopic examination work for one day starts, at least a user needs to recognize that there is an examination to which a proper endoscope 30 is not assigned.

When the embodiment and Example 1 are compared with each other, for example, the endoscope G-R-1 that is an upper routine model is assigned to the examination E9 that is an upper nasal examination in the embodiment, as illustrated in Fig. 17. According to Example 1, however, the endoscope specification unit 124 recognizes that the model with the highest priority order (preferential endoscope model 1) for the examination E9 is an "upper nasal model", by referring to the endoscope order information held in the endoscope order holding unit 204, thereby allowing the endoscope specification unit 124 to specify the endoscope G-N-1 with endoscope No. 10 as a candidate endoscope for the examination E9. Similarly, the endoscope specification unit 124 specifies the endoscopes G-H-1 to G-H-3 with endoscope Nos. 7 to 9, which are upper high image quality models, as candidate endoscopes for the examination E10 that is an upper scrutiny examination. Therefore, the endoscope assignment unit 126 assigns the endoscope G-N-1 to the examination E9 and the endoscope G-H-1 to the examination E10. In comparison with the embodiment as described above, an endoscope 30 with higher priority order, that is, an endoscope 30 suitable for an examination can be assigned to an endoscopic examination in Example 1, and hence the degree of perfection of the examination schedule can be enhanced.

### <Example 2>

In the endoscopic examination work support system 1, an endoscope in which wear or aging has progressed is likely to cause functional deterioration or a malfunction. The case where the wear or aging of an endoscope progresses prominently occurs generally when the number of times of use and the use time of the endoscope are extremely larger and longer than other endoscopes, and hence in Example 2, it is aimed that the number of times of use and the use time of a plurality of endoscopes 30 are equalized.

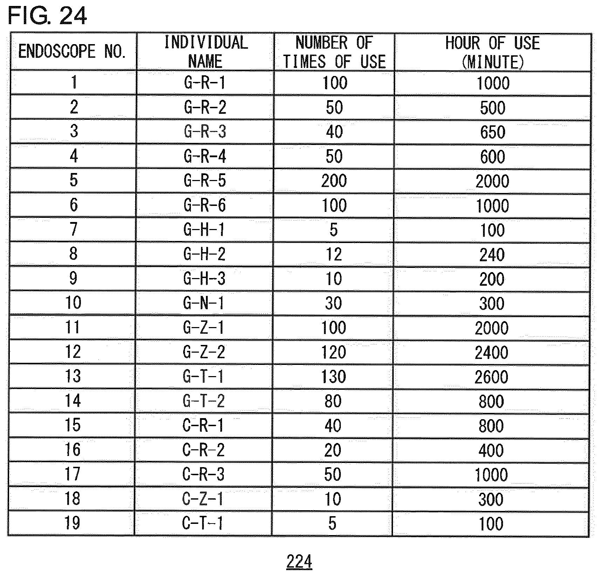

Returning to Fig. 3, the usage condition storage unit 224 stores the past usage conditions of a plurality of the possessed endoscopes 30. Fig. 24 illustrates a usage condition table stored in the usage condition storage unit 224. The usage condition table records the past usage condition by associating with each endoscope 30. These usage condition is absolutely a thing of the past, and updated when the endoscope 30 is actually used. Herein, the usage condition means the "number of times of use" and "use time", and the "use time" indicates the cumulative number of times used in examinations, and the "use time" indicates the cumulative time used in examinations.

Referring to Fig. 1, the endoscope 30 is connected to the endoscopic observation device 22 when an examination is started, and at this time the identification information on the endoscope 30 (endoscope ID) is transmitted to the information management device 10 via the network 2. When an examination end button is operated (or when the endoscope 30 is withdrawn from the endoscopic observation device 22) in the endoscopic observation device 22 at the end of an examination, a notice of end of the examination is transmitted to the information management device 10. In the information management device 10, the usage condition monitoring unit 160 monitors the information transmitted from the endoscopic observation device 22, and derives the time between the transmission of the endoscope ID and the transmission of the notice of end of examination as an examination use time. When the notice of end of examination is transmitted, the usage condition monitoring unit 160 updates the usage condition table by increasing the number of times of use of the corresponding endoscope 30 in the usage condition table by 1 and by adding the currently derived examination use time to the use time, and records them in the usage condition storage unit 224. The usage condition table is generated as described above.

The usage condition table illustrated in Fig. 24 is an example illustrated for ease of understanding. In Fig. 24, for example, the numbers of times of use and the use times of the upper routine models with endoscope Nos. 1 to 6 are greatly different, and in Example 2, a technique for leveling (equalizing) the numbers of times of use and the use times of a plurality of endoscopes is presented in order to prevent such a situation from occurring. Therefore, please note that the usage condition table illustrated in Fig. 24 is merely an example of the usage conditions.

In Example 2, when there are a plurality of endoscopes 30 that can be assigned to the respective endoscopic examinations managed by the examination schedule management unit 110, the endoscope assignment unit 126 preferentially assigns the endoscope 30, the past number of times of use or past use time of which is relatively small or short, to an endoscopic examination by referring to the usage conditions stored in the usage condition storage unit 224.

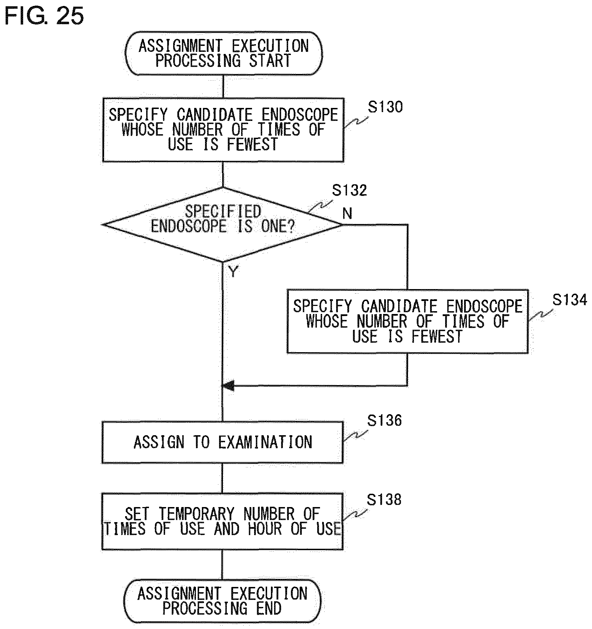

Fig. 25 illustrates a detailed flowchart of S56 in the endoscope assignment processing illustrated in Fig. 9. The endoscope assignment unit 126 specifies, of the candidate endoscopes notified from the endoscope specification unit 124, the candidate endoscope whose past number of times of use is smallest, by referring to the usage conditions stored in the usage condition storage unit 224 (S130).

Hereinafter, an example will be described, in which candidate endoscopes for the examinations E1, E2, and E3 are specified by the endoscope retrieval processing described in Example 1. In Example 1, the endoscope specification unit 124 specifies the endoscope with endoscope Nos. 1 to 6 as the candidate endoscopes for the examinations E1, E2, and E3, and notifies the endoscope assignment unit 126.

The endoscope assignment unit 126 specifies, of the endoscopes with endoscope Nos. 1 to 6, the endoscope G-R-3 with endoscope No. 3 for the examination E1 as the endoscope whose number of times of use is smallest, by referring to the usage conditions of the endoscopes with endoscope Nos. 1 to 6 stored in the usage condition storage unit 224 (S130). The number of times of use of the endoscope G-R-3 is 40, which is relatively smaller than those of the endoscopes G-R-1, G-R-2, G-R-4, G-R-5 and G-R-6, and there is no other endoscope whose number of times of use is 40 (S132/Y), and hence the endoscope assignment unit 126 assigns the endoscope G-R-3 to the examination E1 (S136). The endoscope assignment unit 126 contributes to equalization of the numbers of times of use of the endoscopes by preferentially assigning G-R-3 whose number of times of use is smallest to the examination E1.

When having assigned an endoscope to an examination, the endoscope assignment unit 126 sets the temporary number of times of use and use time (temporary usage condition) as the usage condition of the endoscope (S138). In this case, the number of times of use of the endoscope with endoscope No. 3 is temporarily increased by 1, and the use time is temporarily increased by 10 minutes (scheduled examination time of an upper routine examination is 10 minutes) by referring to the examination type master table 210 illustrated in Fig. 5. As a result, the temporary number of times of use of the endoscope with endoscope No. 3 becomes "41", and the temporary use time becomes "660 minutes." This temporary usage condition will be used later in the assignment execution processing illustrated in Fig. 25.

The temporary number of times of use and use time are not reflected in the usage condition table in the usage condition storage unit 224. The temporary usage condition is set only for the purpose of scheduling the endoscope 30, and may be discarded when the scheduling of the endoscopes 30 is completed for all the examinations.

Next, the endoscope assignment unit 126 specifies, of the endoscopes with endoscope Nos. 1, 2, and 4 to 6, the endoscope G-R-2 with endoscope No. 2 and the endoscope G-R-4 with endoscope No. 4 for the examination E2 as the endoscopes whose numbers of times of use are smallest, by referring to the usage conditions of the endoscopes with endoscope Nos. 1, 2, and 4 to 6 stored in the usage condition storage unit 224 (S130). The numbers of times of use of the endoscopes G-R-2 and G-R-4 are 50, which is relatively smaller than those of the endoscopes G-R-1, G-R-5, and G-R-6, and two endoscopes, the numbers of times of use of which are equal to each other, are specified (S132/N). Herein, the endoscope assignment unit 126 specifies, of the two endoscopes, the endoscope G-R-2 with endoscope No. 2 as the endoscope whose use time is shortest, by referring to the endoscope usage conditions of the endoscopes with endoscope Nos. 2 and 4 stored in the usage condition storage unit 224 (S134), and assigns the endoscope G-R-2 to the examination E2 (S136). The endoscope assignment unit 126 contributes to equalization of the use times of endoscopes by preferentially assigning, of the candidate endoscopes, G-R-2 whose number of times of use is smallest and whose use time is shortest to the examination E2. The endoscope assignment unit 126 sets the temporary number of times of use and use time (temporary usage condition) as the usage condition of the endoscope with endoscope No. 2 (S138). That is, the temporary number of times of use of the endoscope with endoscope No. 2 becomes "51", and the temporary use time becomes "510 minutes."

Next, the endoscope assignment unit 126 specifies, of the endoscopes with endoscope Nos. 1 and 4 to 6, the endoscope G-R-4 with endoscope No. 4 for the examination E3 as the endoscope whose number of times of use is smallest, by referring to the usage conditions of the endoscopes with endoscope Nos. 1 and 4 to 6 stored in the usage condition storage unit 224 (S130). The number of times of use of G-R-4 is 50, which is relatively smaller than those of the endoscopes G-R-1, G-R-5, and G-R-6, and hence the endoscope assignment unit 126 assigns the endoscope G-R-4 to the examination E3 (S136). The endoscope assignment unit 126 contributes to equalization of the numbers of times of use of endoscopes by preferentially assigning, of the candidate endoscopes, G-R-4 whose number of times of use is smallest to the examination E3. The endoscope assignment unit 126 sets the temporary number of times of use and use time (temporary usage condition) as the usage condition of the endoscope with endoscope No. 4 (S138).

As described above, when there are a plurality of the endoscopes 30 that can be assigned to respective endoscopic examinations, the endoscope assignment unit 126 preferentially assigns the endoscope 30, the past number of times of use or past use time of which is relatively small or short, to an endoscopic examination by referring to the usage condition stored in the usage condition storage unit 224, thereby contributing to equalization of the numbers of times of use or use times. In Fig. 25, a candidate endoscope whose number of times of use is smallest is specified in S130, and a candidate endoscope whose use time is shortest is specified in S134, but this order may be reversed. When there are a plurality of candidate endoscopes whose numbers of times of use and use times are equal to each other, the endoscope assignment unit 126 may assign any one of them to an endoscopic examination.

In the above Examples 1 and 2, the modes of assigning the endoscope 30 in an examination schedule have been described. In the following Examples 3, the modes of assigning the cleaning machine 50 in a cleaning schedule will be described.

### <Example 3>

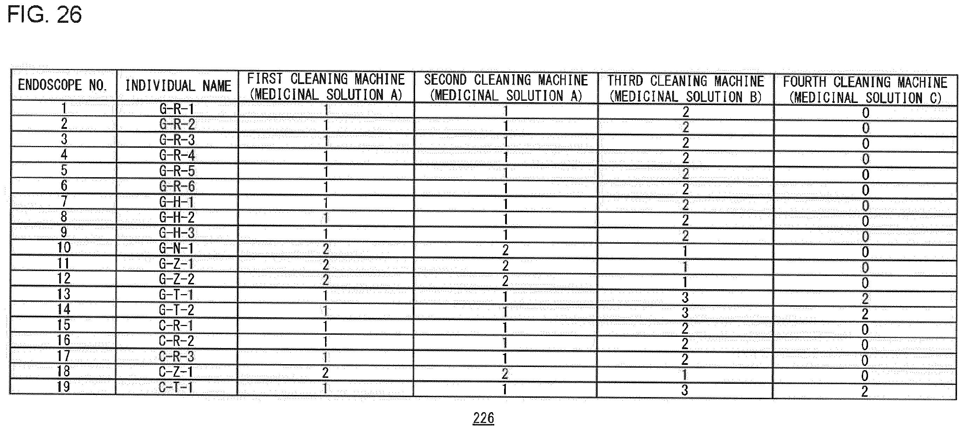

Returning to Fig. 3, the cleaning machine order holding unit 226 holds the priority order of the cleaning machines 50 to be assigned to the endoscopes 30. Fig. 26 illustrates a cleaning machine order table held in the cleaning machine order holding unit 226. The cleaning machine order table records the priority order of the cleaning machines 50 to be assigned, by associating with the endoscopes 30. In this cleaning machine order table, the respective endoscopes are recorded along the vertical axis and the respective cleaning machines are along the horizontal axis, and priority orders are set for the combinations of the endoscopes and the cleaning machines. In this example, it is assumed that: the first cleaning machine 50a and the second cleaning machine 50b use a medicinal solution A; the third cleaning machine 50c uses a medicinal solution B; and the fourth cleaning machine 50d uses a medicinal solution C. In the cleaning machine order table, the priority order of the cleaning machines 50 to be assigned may be associated with the endoscope models, instead of the endoscope individuals.

In Example 3, the model of the first cleaning machine 50a and that of the second cleaning machine 50b may or may not be the same as each other. The first and second cleaning machines 50a and 50b, the third cleaning machine 50c, and the fourth cleaning machine 50d use different medicinal solutions, and therefore these models are different from each other. In the case where a plurality of the cleaning machines 50 are formed by different models in this way, the cleaning machine order holding unit 226 holds the priority order of the cleaning machine models.

In the cleaning machine order table illustrated in Fig. 26, a set value 1 indicates that the priority order is the highest, and a set value 2 indicates that the priority order the second highest. A set value 3 indicates that the priority order is the third highest. A set value 0 indicates that assignment to the endoscope 30 is prohibited.

In the cleaning machine order table illustrated in Fig. 26, the set value 0 is provided to the fourth cleaning machine 50d that uses the medicinal solution C. This is because there is a situation in which: for example, the medicinal solution C has a strong attack property by which an endoscope member is likely to be deteriorated, and hence the use of it for cleaning many endoscopes 30 is prohibited in medical facilities. In this example, the set value 2 is provided to the upper treatment models G-T-1 and G-T-2 and the lower treatment model C-T-1, but the set value 0 is provided to the other endoscopes, which prohibits the use for the endoscopes. The medicinal solution C may be, for example, strongly acidic electrolyzed water.

In the example illustrated in Fig. 26, the priority order of the cleaning machines 50 to be assigned is thus set based on the attack property of a medicinal cleaning solution to be used in the cleaning machine 50. A cleaning machine order table is appropriately generated according to the policy of a medical facility, and long life of the endoscope 30 can be expected by generating the cleaning machine order table depending on the strength of an attack property. On the other hand, for example, strongly acidic electrolyzed water has the merit that it is very inexpensive as a medicinal cleaning solution. Therefore, according to a policy focused on the cost of a medicinal solution, it is also possible to provide a set value other than the set value 0 to the cleaning machine 50 that uses strongly acidic electrolyzed water. Thus, the priority order of the cleaning machines 50 held in the cleaning machine order holding unit 226 is set based on a medicinal solution to be used in the cleaning machine 50.

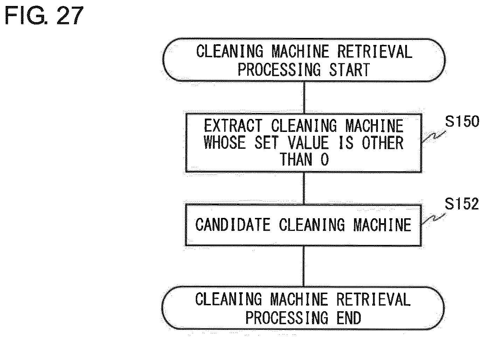

Fig. 27 illustrates a detailed flowchart of S110 in the cleaning machine assignment processing illustrated in Fig. 13. In S110, the cleaning machine specification unit 142 executes retrieval processing on the possessed cleaning machines 50 for the respective endoscopes 30 assigned by the endoscope assignment processing of S18 (see Fig. 7), and specifies an available cleaning machine 50.

Hereinafter, an example will be described, in which the endoscopes G-R-1, G-R-2, G-R-3 and C-R-1 are respectively assigned to the examinations E1, E2, E3 and E4 by the endoscope assignment processing described in the embodiment. That is, the cleaning machine assignment processing is started in the state where the examination schedule illustrated in Fig. 12 is set.

Returning to Fig. 3, in the second assignment processing unit 140, the cleaning machine specification unit 142 extracts the cleaning machines 50 whose set values are other than 0 for the endoscopes G-R-1, G-R-2, G-R-3, and C-R-1 by referring to the cleaning machine order information held in the cleaning machine order holding unit 226 (S150). In S150, cleaning machines 50, the use of which is not prohibited, are extracted. Herein, for the endoscopes G-R-1, G-R-2, and G-R-3, which are upper routine models, the set values of the first cleaning machine 50a, the second cleaning machine 50b, and the third cleaning machine 50c are not 0, and similarly, for the endoscope C-R-1, which is a lower routine model, the set values of the cleaning machine 50a, the second cleaning machine 50b, and the third cleaning machine 50c are not 0. Therefore, the cleaning machine specification unit 142 specifies the first cleaning machine 50a, the second cleaning machine 50b, and the third cleaning machine 50c as candidate cleaning machines for the respective examinations E1 to E4 (S152). The specified candidate cleaning machines are notified to the cleaning machine assignment unit 144.

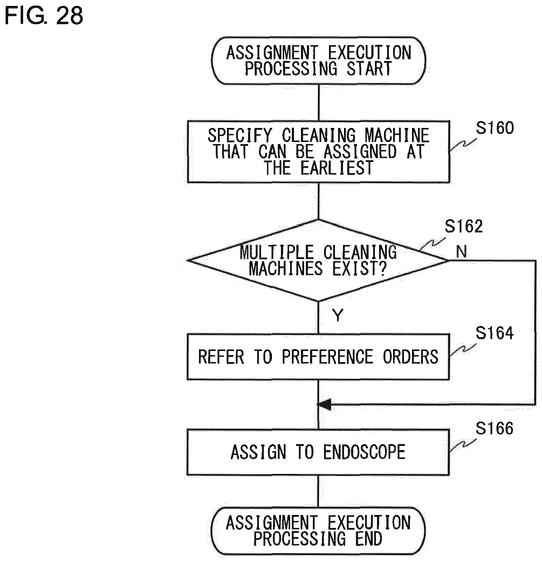

Fig. 28 illustrates a detailed flowchart of S114 in the cleaning machine assignment processing illustrated in Fig. 13. In S114, the cleaning machine assignment unit 144 assigns the cleaning machine 50 to the endoscope 30.

Initially, the cleaning machine assignment unit 144 assigns a cleaning machine to the endoscope G-R-1 for the examination E1. The cleaning machine assignment unit 144 specifies, of the candidate cleaning machines, the cleaning machine 50 that can be assigned earliest, by referring to the cleaning schedule held in the cleaning schedule holding unit 208 (S160). As described in the embodiment, when the cleaning machine specification unit 142 notifies the cleaning machine assignment unit 144 of a time zone when the candidate cleaning machine can be used, the cleaning machine assignment unit 144 may specify the cleaning machine 50 that can be assigned earliest by referring to the notified time zone.

Herein, the candidate cleaning machines, i.e., the first cleaning machine 50a, the second cleaning machine 50b, and the third cleaning machine 50c can be used in all the time zones in their initial states, that is, the statuses in all the time zones become "under standby". Then, the cleaning machine assignment unit 144 specifies that all of the first cleaning machine 50a, the second cleaning machine 50b, and the third cleaning machine 50c can be assigned earliest (S160), and determines that there are a plurality of cleaning machines that can be assigned earliest (S162/Y). Herein, the cleaning machine assignment unit 144 recognizes that the set value 1 is provided to the first cleaning machine 50a and the second cleaning machine 50b for the endoscope G-R-1 for the examination E1, by referring to the priority orders held in the cleaning machine order holding unit 226 (S164). Thereby, the cleaning machine assignment unit 144 assigns the first cleaning machine 50a to the endoscope G-R-1 (S166). In this way, the cleaning machine assignment unit 144 assigns the first cleaning machine 50a with high priority order to the endoscope G-R-1, whereby it becomes possible to clean by a cleaning machine suitable for an endoscope. The scheduled cleaning start time is set to be 9:10 and the scheduled cleaning end time to be 9:30, which are registered in the cleaning schedule. Thereby, the status of the first cleaning machine 50a between 9:10 and 9:30 becomes "under use."

Next, the cleaning machine assignment unit 144 assigns a cleaning machine to the endoscope G-R-2 for the examination E2. The cleaning machine assignment unit 144 specifies, of the candidate cleaning machines, the cleaning machine 50 that can be assigned earliest, by referring to the cleaning schedule held in the cleaning schedule holding unit 208 (S160). Herein, the first cleaning machine 50a is set to be scheduled to be used between 9:10 and 9:30, and hence the cleaning machine assignment unit 144 specifies that the second cleaning machine 50b and the third cleaning machine 50c can be assigned earliest (S160), and determines that there are a plurality of cleaning machines that can be assigned earliest (S162/Y). Herein, the cleaning machine assignment unit 144 recognizes that the set value 1 is provided to the second cleaning machine 50b for the endoscope G-R-2 for the examination E2, by referring to the priority orders held in the cleaning machine order holding unit 226 (S164). Thereby, the cleaning machine assignment unit 144 assigns the second cleaning machine 50b to the endoscope G-R-2. Thus, the cleaning machine assignment unit 144 assigns the second cleaning machine 50b with high priority order to the endoscope G-R-2. The scheduled cleaning start time is set to be 9:10 and the scheduled cleaning end time to be 9:30, which are registered in the cleaning schedule.

Next, the cleaning machine assignment unit 144 assigns a cleaning machine to the endoscope G-R-3 for the examination E3. The cleaning machine assignment unit 144 specifies, of the candidate cleaning machines, the cleaning machine 50 that can be assigned earliest, by referring to the cleaning schedule held in the cleaning schedule holding unit 208 (S160). Herein, the first cleaning machine 50a and the second cleaning machine 50b are set to be scheduled to be used between 9:10 and 9:30, and hence the cleaning machine assignment unit 144 specifies that the third cleaning machine 50c can be assigned earliest (S160), and determines that one cleaning machine 50 is specified (S162/N). Thereby, the cleaning machine assignment unit 144 assigns the third cleaning machine 50c to the endoscope G-R-3. The scheduled cleaning start time is set to be 9:10 and the scheduled cleaning end time to be 9:30, which are registered in the cleaning schedule.

With respect to the endoscope G-R-3, the set value of the third cleaning machine 50c is 2, and hence the priority order thereof is lower than those of the first cleaning machine 50a and the second cleaning machine 50b. Therefore, it is also possible to assign the first cleaning machine 50a or the second cleaning machine 50b to the endoscope G-R-3 at a time when the first cleaning machine 50a or the second cleaning machine 50b can be used. In such a case, however, the cleaning of the endoscope G-R-3 is delayed, which is not preferable from the viewpoint of working efficiency. Therefore, unless assignment is prohibited, the cleaning machine assignment unit 144 positively assigns the cleaning machine 50, even if the priority order thereof is low.

Next, the cleaning machine assignment unit 144 assigns a cleaning machine to the endoscope C-R-1 for the examination E4. The cleaning machine assignment unit 144 specifies, of the candidate cleaning machines, the cleaning machine 50 that can be assigned earliest, by referring to the cleaning schedule held in the cleaning schedule holding unit 208 (S160). Herein, the first cleaning machine 50a, the second cleaning machine 50b, and the third cleaning machine 50c are set to be scheduled to be used between 9:10 and 9:30, and hence the cleaning machine assignment unit 144 specifies that all of the first cleaning machine 50a, the second cleaning machine 50b, and the third cleaning machine 50c can be assigned earliest (S160), and determines that there are a plurality of cleaning machines that can be assigned earliest (S162/Y).

Herein, the cleaning machine assignment unit 144 recognizes that the set value 1 is provided to the first cleaning machine 50a and the second cleaning machine 50b for the endoscope C-R-1 for the examination E4, by referring to the priority orders held in the cleaning machine order holding unit 226 (S164). Thereby, the cleaning machine assignment unit 144 assigns the first cleaning machine 50a to the endoscope C-R-1 (S166). In this way, the cleaning machine assignment unit 144 assigns the first cleaning machine 50a with high priority order to the endoscope C-R-1, whereby it becomes possible to clean by a cleaning machine suitable for an endoscope. The scheduled cleaning start time is set to be 9:30 and the scheduled cleaning end time to be 9:50, which are registered in the cleaning schedule.

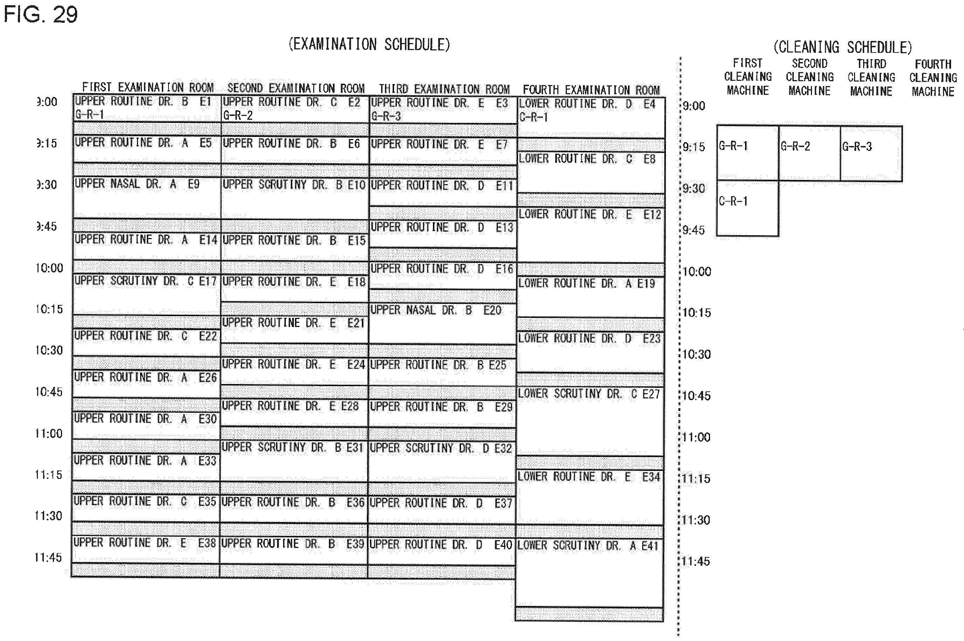

Fig. 29 illustrates a cleaning schedule generated by the cleaning schedule management unit 130 in Example 3. Herein, the results of the assignment by the cleaning machine assignment unit 144 are reflected in the cleaning schedule, and specifically it is registered that between 9:10 and 9:30: the endoscope G-R-1 is cleaned by the first cleaning machine 50a; the endoscope G-R-2 is cleaned by the second cleaning machine 50b; and the endoscope G-R-3 is cleaned by the third cleaning machine 50c, and registered that between 9:30 and 9:50 the endoscope C-R-1 is cleaned by the first cleaning machine 50a. The cleaning schedule management unit 130 records the updated cleaning schedule in the cleaning schedule holding unit 208.

When compared with Fig. 14 described in the embodiment, the fourth cleaning machine 50d is not used in the cleaning schedule illustrated in Fig. 29. This is because: the use of the fourth cleaning machine 50d, in other words, the use of the medicinal solution C is prohibited for the endoscope C-R-1, and hence a state where an endoscope cannot be assigned to the fourth cleaning machine 50d occurs. In Fig. 26, for the endoscopes G-T-1, G-T-2, and C-T-1, the set value of the fourth cleaning machine 50d is 2, and therefore the fourth cleaning machine 50d may be assigned in scheduling the cleaning of these endoscopes.

In the above description, the case where the use of the fourth cleaning machine 50d, the set value of which is 0 in the cleaning machine order holding unit 226, is prohibited has been described; however, the processing for generating a cleaning schedule may be performed by loosening this restriction. It is because, in the above processing in which the use of the fourth cleaning machine 50d is prohibited, it is assumed that a situation may occur in which: the fourth cleaning machine 50d is not usually used; the cleaning processing of the endoscopes 30 is not performed efficiently; and the number of the endoscopes 30 waiting for cleaning is increased. Therefore, on the cleaning machine 50 whose set value is 0, gentle restriction in which the assignment to the endoscope 30 is avoided as much as possible may be imposed, not severe restriction in which the assignment to the endoscope 30 is prohibited. The severe restriction and the gentle restriction may be determined according to, for example, a scheduling mode, and when the efficiency of the cleaning processing is intended to be promoted, a user may assign the cleaning machine 50 whose set value is 0 to the endoscope 30 by selecting the gentle restriction mode.

In this case, it is preferable that the usage condition storage unit 224 (or the later-described history recording unit 232) stores, for each endoscope 30, the number of times of cleaning by the cleaning machine 50 whose set value is 0. It is preferable that the cleaning machine assignment unit 144 assigns the cleaning machines 50 to the endoscopes 30 such that the number of times of cleaning by the cleaning machine 50 whose set value is 0 does not prominently become large, that is, such that the numbers of times of cleaning by the cleaning machines 50 whose set value are 0 become equal. An upper limit (e.g., 20 times) may be set to the number of times of cleaning by the cleaning machine 50 whose set value is 0, and the cleaning machine assignment unit 144 may not assign the cleaning machine 50 whose set value is 0 multiple times more than this upper limit.

### <Example 4>

In Example 4, assignment processing is performed in scheduling the endoscope 30, in which a certain endoscope 30 is used by a specific doctor as much as possible. By making a set of the endoscope 30 and a doctor using it, it can be analyzed that, for example, an endoscope 30, the good condition of which is maintained for a long time, has been skillfully operated by a doctor frequently using it; on the other hand, it can also be analyzed that an endoscope 30, etc., which is likely to cause a malfunction, is problematically operated by a doctor frequently using it, etc.

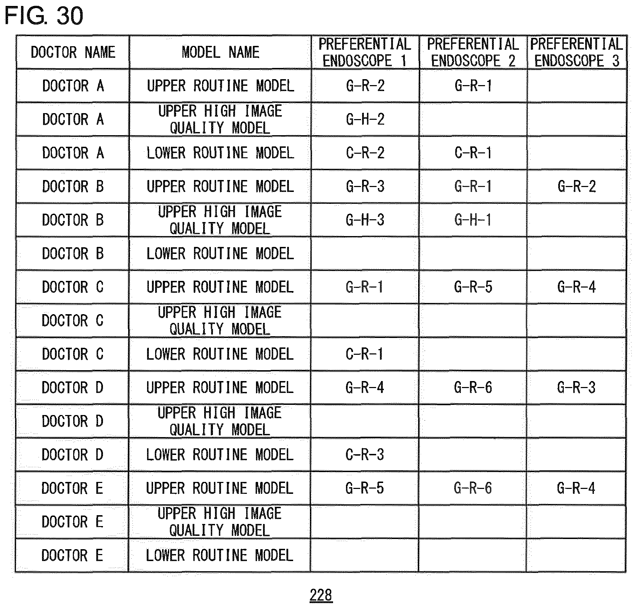

Returning to a Fig. 3, the assigned endoscope information holding unit 228 holds preferential endoscope information on the endoscope preferentially assigned to a doctor. Fig. 30 illustrates a preferential endoscope table stored in the assigned endoscope information holding unit 228. In the preferential endoscope table, preferential endoscope information that define the priority orders of the endoscopes 30 to be assigned are recorded for each primary doctor in charge of an endoscopic examination and for each endoscope model. Herein, the priority order is provided on the premise that a medical facility possesses a plurality of endoscopes 30 of the same model, and the assigned endoscope information holding unit 228 holds, of the plurality of endoscopes 30 of the same model, the endoscopes to be preferentially assigned to doctors as preferential endoscope information. In Fig. 30, the "preferential endoscope 1" means an endoscope with the highest assignment priority order, the "preferential endoscope 2" means an endoscope with the second highest assignment priority order, and the "preferential endoscope 3" means an endoscope with the third highest assignment priority order.

For example, for a doctor A with respect to an upper routine model, the assignment priority order of the endoscope G-R-2 is set to be the highest, and that of the endoscope G-R-1 to be the second highest. This preferential endoscope information is information by which a preferential endoscope is preferentially assigned to a doctor in the case where such an action can be taken, and a preferential endoscope should not necessarily be assigned to a doctor. For example, in an examination scheduled to be performed by the doctor A, if the statuses of the endoscopes G-R-2 and G-R-1 are not "under standby" at the scheduled examination start time, the endoscope assignment unit 126 assigns another upper routine model in order to prevent a delay in examinations.

In Example 4, the endoscope assignment unit 126 determines the endoscope 30 to be assigned to an endoscopic examination, based on the preferential endoscope information held in the assigned endoscope information holding unit 228 and the information on primary doctors of endoscopic examinations that the examination schedule management unit 110 manages.

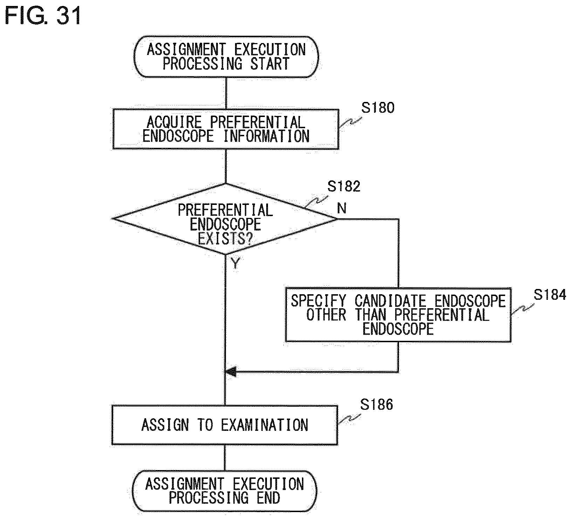

Fig. 31 illustrates a detailed flowchart of S56 in the endoscope assignment processing illustrated in Fig. 9. The endoscope assignment unit 126 acquires the preferential endoscope information held in the assigned endoscope information holding unit 228, based on a primary doctor of an endoscopic examination (S180).

Hereinafter, an example will be described, in which candidate endoscopes for the examinations E1, E2, E3, and E4 are specified by the endoscope retrieval processing described in Example 1. In Example 1, the endoscope specification unit 124 specifies the endoscopes with endoscope Nos. 1 to 6 as candidate endoscopes for the examinations E1, E2, and E3, and specifies the endoscopes with endoscope Nos. 15 to 17 as candidate endoscopes for the examination E4, and notifies the endoscope assignment unit 126.

The endoscope assignment unit 126 acquires, for each of the examinations E1, E2, E3, and E4, the preferential endoscope information on an endoscope model to be used from the assigned endoscope information holding unit 228, based on the primary doctor information on each of the examinations (S180).

With reference to the examination schedule of Fig. 4, the doctor B is a primary doctor of the examination E1, and the endoscope assignment unit 126 recognizes that: the preferential endoscope 1 of an upper routine model is G-R-3; the preferential endoscope 2 is G-R-1; and the preferential endoscope 3 is G-R-2, by referring to the preferential endoscope table. The doctor C is a primary doctor of the examination E2, and the endoscope assignment unit 126 recognizes that: the preferential endoscope 1 of an upper routine model is G-R-1; the preferential endoscope 2 is G-R-5; and the preferential endoscope 3 is G-R-4. The doctor E is a primary doctor of the examination E3, and the endoscope assignment unit 126 recognizes that: the preferential endoscope 1 of an upper routine model is G-R-5; the preferential endoscope 2 is G-R-6; and the preferential endoscope 3 is G-R-4. Also, the doctor D is a primary doctor of the examination E4, and the endoscope assignment unit 126 recognizes that the preferential endoscope 1 of a lower routine model is C-R-3.

With respect to the examination E1, the endoscope assignment unit 126 determines that the endoscope G-R-3, the preferential endoscope 1, is included in the candidate endoscopes with endoscope Nos. 1 to 6 (S182/Y), and therefore assigns the endoscope G-R-3 to the examination E1 (S186). Thereby, the doctor B can use the endoscope G-R-3 in the examination E1.

Next, with respect to the examination E2, the endoscope assignment unit 126 determines that the endoscope G-R-1, the preferential endoscope 1, is included in the candidate endoscopes with endoscope Nos. 1, 2, and 4 to 6 (S182/Y), and therefore assigns the endoscope G-R-1 to the examination E2 (S186). Thereby, the doctor C can use the endoscope G-R-1 in the examination E2.

Next, with respect to the examination E3, the endoscope assignment unit 126 determines that the endoscope G-R-5, the preferential endoscope 1, is included in the candidate endoscopes with endoscope Nos. 2 and 4 to 6 (S182/Y), and therefore assigns the endoscope G-R-5 to the examination E3 (S186). Thereby, the doctor E can use the endoscope G-R-5 in the examination E3.

Finally, with respect to the examination E4, the endoscope assignment unit 126 determines that the endoscope C-R-3, the preferential endoscope 1, is included in the candidate endoscopes with endoscope Nos. 15 to 17 (S182/Y), and therefore assigns the endoscope G-R-3 to the examination E4 (S186). Thereby, the doctor D can use the endoscope C-R-3 in the examination E4.

In this way, when there are a plurality of preferential endoscopes in candidate endoscopes, the endoscope assignment unit 126 assigns an endoscope with higher priority order to an examination. When a preferential endoscope is not included in the candidate endoscopes, that is, when neither of the preferential endoscope 1, the preferential endoscope 2, and the preferential endoscope 3 is included (S182/N), the endoscope assignment unit 126 specifies a candidate endoscope other than the preferential endoscopes (S184), and assigns to an examination (S186). In this way, if there is no assignable preferential endoscope at the scheduled examination start time, it is preferable to assign another endoscope rather than to wait for the preferential endoscopes to become available, thereby allowing an efficient examination schedule to be generated. If there is no candidate endoscope, it is preferable to notify a user of the fact.

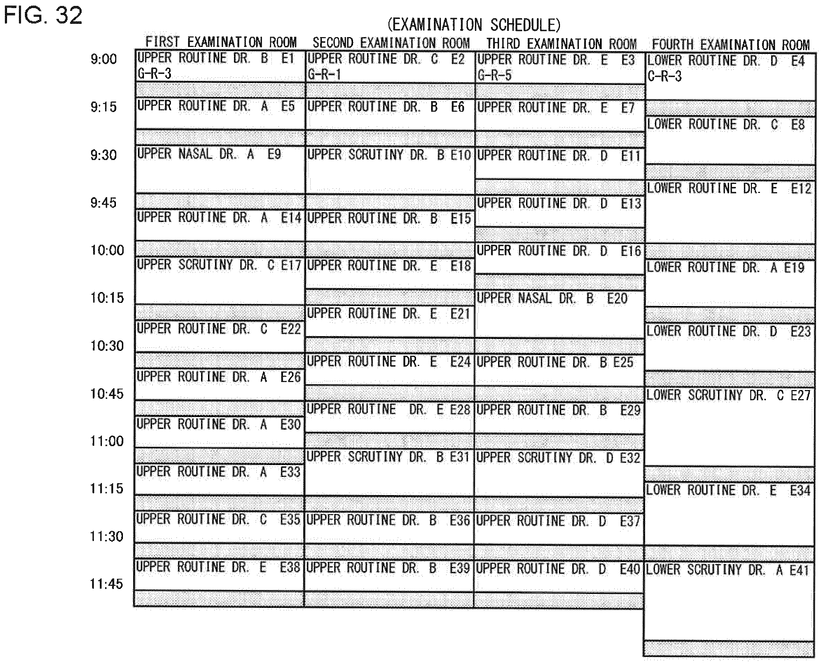

Fig. 32 illustrates an examination schedule updated by the examination schedule management unit 110. When the results of the assignment are notified from the endoscope assignment unit 126, the examination schedule management unit 110 registers the assigned endoscope 30 in the corresponding examination. Herein, it is registered that: the endoscope G-R-3 is used in the examination E1; the endoscope G-R-1 in the examination E2; the endoscope G-R-5 in the examination E3; and the endoscope C-R-3 in the examination E4. The examination schedule management unit 110 records the updated examination schedule in the examination schedule holding unit 206.

In Example 4, the endoscope assignment unit 126 preferentially assigns, as much as possible, a specific endoscope 30 to the examinations that a specific doctor takes charge of, and hence the use frequency of the endoscope 30 by the doctor becomes high.

The usage condition monitoring unit 160 monitors the usage condition of the endoscope 30 used in the actually performed endoscopic examinations, and records it in the history recording unit 232. As a result, the history recording unit 232 records the usage history information on the endoscope 30 used in the actual endoscopic examinations. With respect to the endoscope 30, the history recording unit 232 records information on the examination using it, the doctor using it, information on use date and time, and the like by associating them with each other.

The history recording unit 232 also records a history relating to a malfunction of the endoscope 30. For example, the malfunction history may include the doctor who operated when a malfunction occurred, examination type information, and information on date and time.

The display processing unit 150 displays, in a comparable format, the usage history information on a plurality of the endoscopes 30 recorded in the history recording unit 232. At this time, the display content derivation unit 152 calculates a statistical amount based on the usage history information recorded in the history recording unit 232. Herein, the statistical amount means the number of times of use of the endoscope 30, the use time thereof, or the like calculated for each doctor, and the display content derivation unit 152 has the function of deriving the statistical amount in accordance with the contents to be displayed. The display processing unit 150 displays the statistical amount calculated by the display content derivation unit 152.

The period designation unit 154 designates a period for the usage history information. This period is specified by an input by a user into an input frame provided on the screen of the terminal device 12. When the period designation unit 154 designates a period, the display content derivation unit 152 extracts the usage history information during the period from the history recording unit 232 and calculates a statistical amount to be displayed, and the display processing unit 150 displays the usage history information during the designated period, i.e., the statistical amount calculated by the display content derivation unit 152 on the display of the terminal device 12.

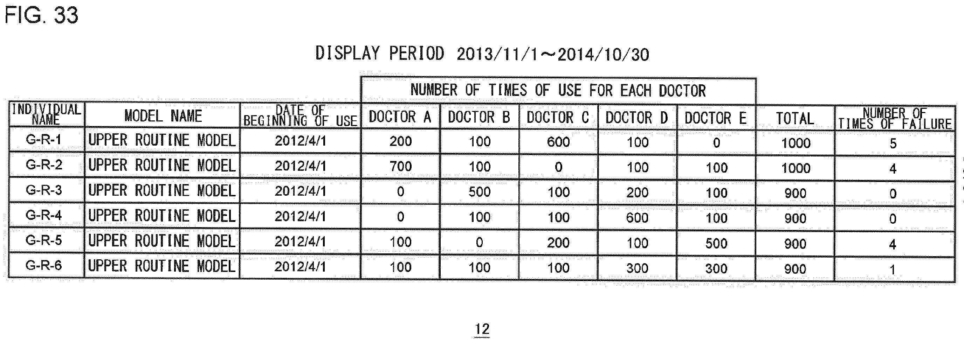

Fig. 33 illustrates one example of the usage history information to be displayed on the terminal device 12. When a user inputs the period between 2013/11/1 and 2014/10/30 as a display period, the period designation unit 154 designates this period, and the display content derivation unit 152 extracts the usage history information during this period from the history recording unit 232. Herein, the display content derivation unit 152 generates a number of times of use table by calculating the number of times of use of an upper routine model for each doctor, and the display processing unit 150 displays it on the display of the terminal device 12. The display content derivation unit 152 may calculate the number of times of malfunctions by generating a list of malfunction histories during this period, and the display processing unit 150 may collectively display the number of times of malfunctions and the malfunction histories.

With this number of times of use table, a user can specify an endoscope that caused less malfunctions and a doctor who frequently uses the endoscope. Conversely, a user can specify an endoscope that caused more malfunctions and a doctor who frequently uses the endoscope. As described above, the endoscope assignment unit 126 performs the endoscope assignment processing such that a specific doctor preferentially uses a specific endoscope, whereby the history information in which the endoscope 30 was actually used serves as useful information when failure analysis, etc., is performed. Additionally, because the display processing unit 150 displays the usage history information on a plurality of the endoscopes 30 in a comparable format, a user can recognize at a glance differences among the usage conditions of the endoscopes 30.

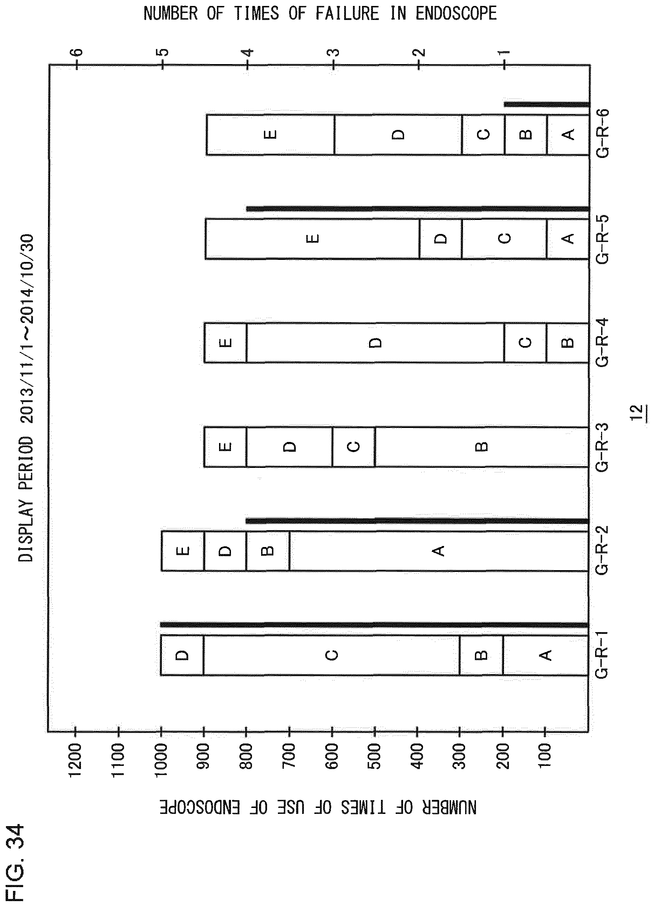

Fig. 34 illustrates one example of the usage history information to be displayed on the terminal device 12. This number of times of use graph expresses in graph form the number of times of use table illustrated in Fig. 33. By expressing in graph form in this way, it becomes possible to understand the differences among the usage conditions of the endoscopes 30 at a glance.

In Figs. 33 and 34, the display processing unit 150 displays the number of times of use of the endoscope 30 for each doctor as the usage history information, but may display, for example, the use time of the endoscope for each doctor as the usage history information. Additionally, the display processing unit 150 may display, for each doctor, the number of times of use or use time of the endoscope 30 used by a doctor.

### <Example 5>

In Example 5, when the scheduling of the endoscope 30 is performed, the processing for assigning cleaning work to a person preparing for examination is performed such that a certain endoscope 30 is cleaned by a specific person preparing for examination as much as possible. By making a set of the cleaning work of the endoscope 30 and a person preparing for examination who cleans it, it can be analyzed that, for example, an endoscope 30, the good condition of which is maintained for a long time, has been skillfully cleaned by a person-in-charge who has frequently experienced cleaning work; on the other hand, it can also be analyzed that an endoscope 30, etc., which is likely to cause a malfunction, is problematically handled in a cleaning step, etc.

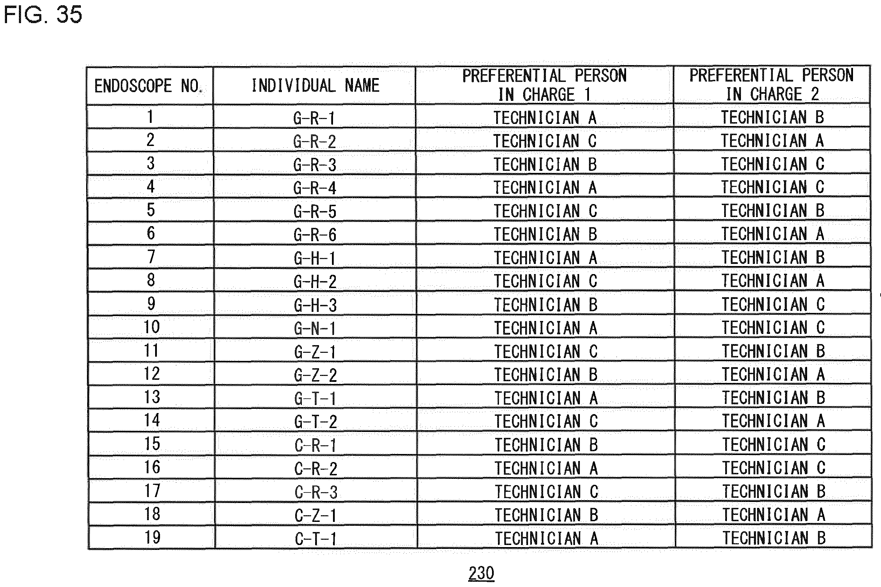

Returning to Fig. 3, the assigned person-in-charge information holding unit 230 holds the preferential person-in-charge information on a person-in-charge to whom cleaning work of the endoscope 30 is preferentially assigned. Fig. 35 illustrates a preferential person-in-charge table stored in the assigned person-in-charge information holding unit 230. The preferential person-in-charge table records, for each endoscope 30, preferential person-in-charge information in which the priority order of the persons preparing for examination to whom cleaning work is to be assigned (hereinafter, also referred to as a "person-in-charge"). That is, the assigned person-in-charge information holding unit 230 holds, for one endoscope 30, the priority order of the person-in-charge to whom cleaning work is to be assigned. In Fig. 35, the "Preferential person-in-charge 1" means a person-in-charge whose assignment priority order is the highest, and the "Preferential person-in-charge 2" a person-in-charge whose assignment priority order is the second highest.

For example, for the endoscope G-R-1, the assignment priority order of a technician A is set to be the highest, and that of a technician B to be the second highest. This preferential person-in-charge information is one by which it is designated that when a preferential person-in-charge can be assigned to cleaning work, he/she is preferentially assigned; and a person preparing for examination, who is designated as a preferential person-in-charge, should not necessarily be assigned to the cleaning work of the endoscope. For example, when an examination using the endoscope G-R-1 ends and the endoscope is to be cleaned, and when the technicians A and B are performing other work at the time of cleaning the endoscope, the person-in-charge assignment unit 149 assigns another technician (e.g., technician C) to the cleaning work in order to prevent a delay in cleaning work.

In Example 5, the person-in-charge assignment unit 149 determines a person-in-charge of the cleaning work of an endoscope based on the preferential person-in-charge information held in the assigned person-in-charge information holding unit 230 and the endoscope information managed by the examination schedule management unit 110.

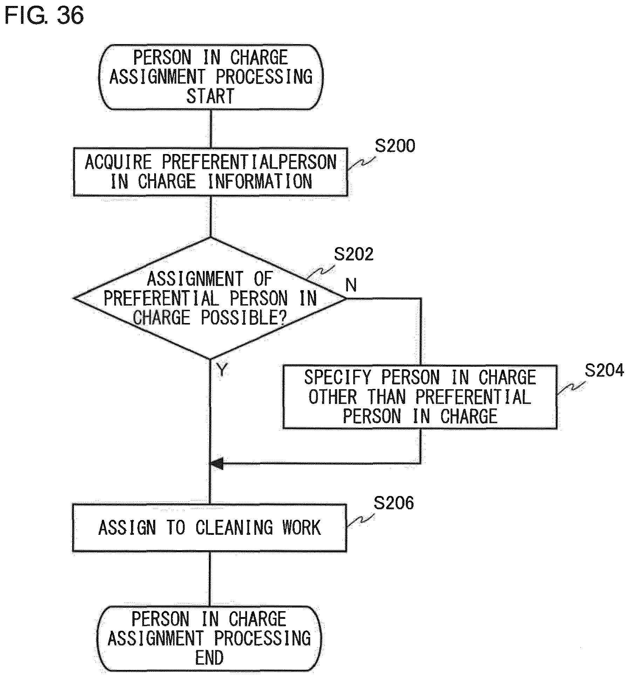

Fig. 36 illustrates a flowchart of person-in-charge assignment processing. The person-in-charge assignment processing illustrated in Fig. 36 is added as processing between S118 and S120 in the cleaning machine assignment processing illustrated in Fig. 13. In the flowchart illustrated in Fig. 13, after the cleaning machine assignment unit 144 assigns the cleaning machine 50 to the used endoscope (S114) and the scheduled cleaning start time and the scheduled cleaning end time are set (S118), the person-in-charge assignment unit 149 acquires the preferential person-in-charge information held in the assigned person-in-charge information holding unit 230 based on the endoscope 30 scheduled to be cleaned (S200).

Hereinafter, an example will be described, in which the endoscopes G-R-3, G-R-1, G-R-5 and C-R-3 are respectively assigned to the examinations E1, E2, E3 and E4 by the endoscope assignment processing described in Example 4.

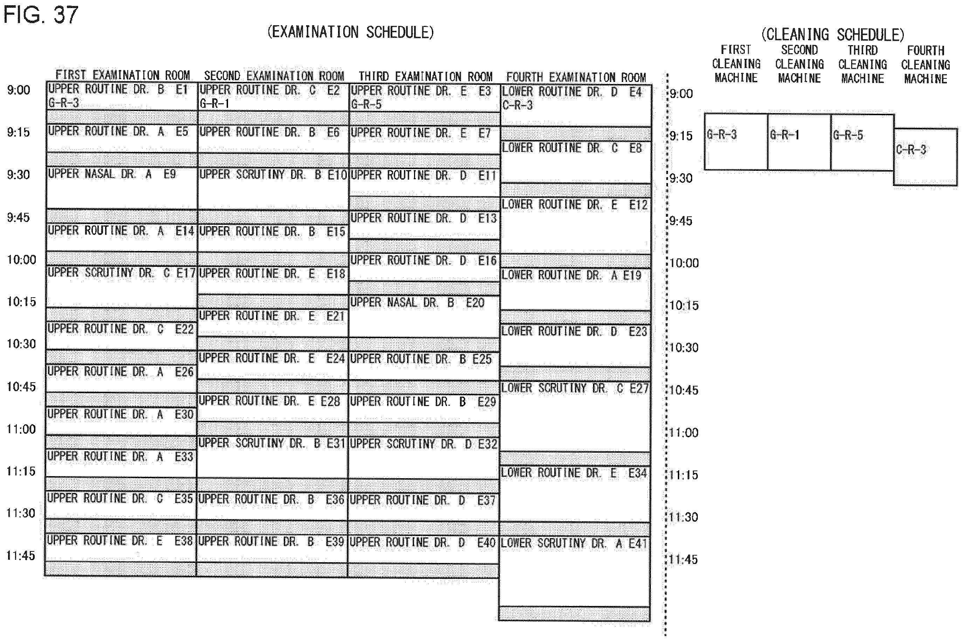

Fig. 37 illustrates a cleaning schedule generated by the cleaning schedule management unit 130. Herein, the results of the assignment by the cleaning machine assignment unit 144 are reflected in the cleaning schedule, and specifically it is registered that between 9:10 and 9:30: the endoscope G-R-3 is cleaned by the first cleaning machine 50a; the endoscope G-R-1 is cleaned by the second cleaning machine 50b; and the endoscope G-R-5 is cleaned by the third cleaning machine 50c, and registered that between 9:15 and 9:35 the endoscope C-R-3 is cleaned by the fourth cleaning machine 50d.

The cleaning schedule management unit 130 of Example 5 manages a cleaning schedule of a plurality of endoscopes, including the cleaning machine 50, information on scheduled cleaning start time, that on scheduled cleaning end time, and person-in-charge of cleaning who take a charge of cleaning work. Hereinafter, a method for registering a person-in-charge of cleaning in the cleaning schedule will be described.

The person-in-charge assignment unit 149 acquires, for each endoscope 30, preferential person-in-charge information from the assigned person-in-charge information holding unit 230, based on the endoscope information assigned to each of the examinations E1, E2, E3, and E4 (S200).

With reference to the preferential person-in-charge table of Fig. 35, the preferential person-in-charge 1 of the endoscope G-R-3 is the technician B, the preferential person-in-charge of the endoscope G-R-1 is the technician A, the preferential person-in-charge of the endoscope G-R-5 is the technician C, and the preferential person-in-charge 1 of the endoscope C-R-3 is the technician C.

With respect to the endoscope G-R-3, the person-in-charge assignment unit 149 determines whether the technician B, preferential person-in-charge 1, can take charge of the cleaning work of the endoscope G-R-3 (S202). In Example 5, the person-in-charge schedule is set for each technician, and the person-in-charge assignment unit 149 determines whether the technician B can take charge of the cleaning work of the endoscope G-R-3 by determining whether there is a vacancy between the scheduled cleaning start time and the scheduled cleaning end time in the person-in-charge schedule. If there is no other work between the scheduled cleaning start time and the scheduled cleaning end time of the endoscope G-R-3, the person-in-charge assignment unit 149 determines that the cleaning work of the endoscope G-R-3 can be assigned to the technician B (S202/Y), and assigns the work to the technician B (S206). If another work is scheduled to be performed either at the scheduled cleaning start time or the scheduled cleaning end time in the person-in-charge schedule, the person-in-charge assignment unit 149 determines that the cleaning work of the endoscope G-R-3 cannot be assigned to the technician B (S202/N). If the work cannot be assigned also to the technician C, preferential person-in-charge 2, the person-in-charge assignment units 149 specifies, of persons-in-charge other than the preferential persons-in-charge, a person-in-charge who does not have work at the time (S204), and assigns the work to this person-in-charge (S206).

Next, with respect to the endoscope G-R-1, the person-in-charge assignment unit 149 determines whether the technician A, preferential person-in-charge 1, can take charge of the cleaning work of the endoscope G-R-1 (S202). When the cleaning work of the endoscope G-R-1 can be assigned to the technician A (S202/Y), the person-in-charge assignment unit 149 assigns the cleaning work of the endoscope G-R-1 to the technician A (S206). If another work is scheduled to be performed either at the scheduled cleaning start time or the scheduled cleaning end time in the person-in-charge schedule, the person-in-charge assignment unit 149 determines that the cleaning work of the endoscope G-R-1 cannot be assigned to the technician A (S202/N). At this time, if the work cannot be assigned also to the technician B, preferential person-in-charge 2, the person-in-charge assignment units 149 specifies, of persons-in-charge other than the preferential persons-in-charge, a person-in-charge who does not have work at the time (S204), and assigns the work to this person-in-charge (S206).

Next, with respect to the endoscope G-R-5, the person-in-charge assignment unit 149 determines whether the technician C, preferential person-in-charge 1, can take charge of the cleaning work of the endoscope G-R-5 (S202). When the cleaning work of the endoscope G-R-5 can be assigned to the technician C (S202/Y), the person-in-charge assignment unit 149 assigns the cleaning work of the endoscope G-R-5 to the technician C (S206). If another work is scheduled to be performed either at the scheduled cleaning start time or the scheduled cleaning end time in the person-in-charge schedule, the person-in-charge assignment unit 149 determines that the cleaning work of the endoscope G-R-5 cannot be assigned to the technician C (S202/N). At this time, if the work cannot be assigned also to the technician B, preferential person-in-charge 2, the person-in-charge assignment units 149 specifies, of persons-in-charge other than the preferential persons-in-charge, a person-in-charge who does not have work at the time (S204), and assigns the work to this person-in-charge (S206).

Next, with respect to the endoscope C-R-3, the person-in-charge assignment unit 149 determines whether the technician C, preferential person-in-charge 1, can take charge of the cleaning work of the endoscope C-R-3 (S202). When the cleaning work of the endoscope C-R-3 can be assigned to the technician C (S202/Y), the person-in-charge assignment unit 149 assigns the cleaning work of the endoscope C-R-3 to the technician C (S206). If another work is scheduled to be performed either at the scheduled cleaning start time or the scheduled cleaning end time in the person-in-charge schedule, the person-in-charge assignment unit 149 determines that the cleaning work of the endoscope C-R-3 cannot be assigned to the technician C (S202/N). At this time, if the work cannot be assigned also to the technician B, preferential person-in-charge 2, the person-in-charge assignment units 149 specifies, of persons-in-charge other than the preferential persons-in-charge, a person-in-charge who does not have work at the time (S204), and assigns the work to this person-in-charge (S206).

When both the preferential person-in-charge 1 and the preferential person-in-charge 2 can be assigned to certain cleaning work, the person-in-charge assignment unit 149 assigns a person-in-charge with higher priority order to the cleaning work. The assigned person-in-charge information is notified to the cleaning schedule management unit 130.

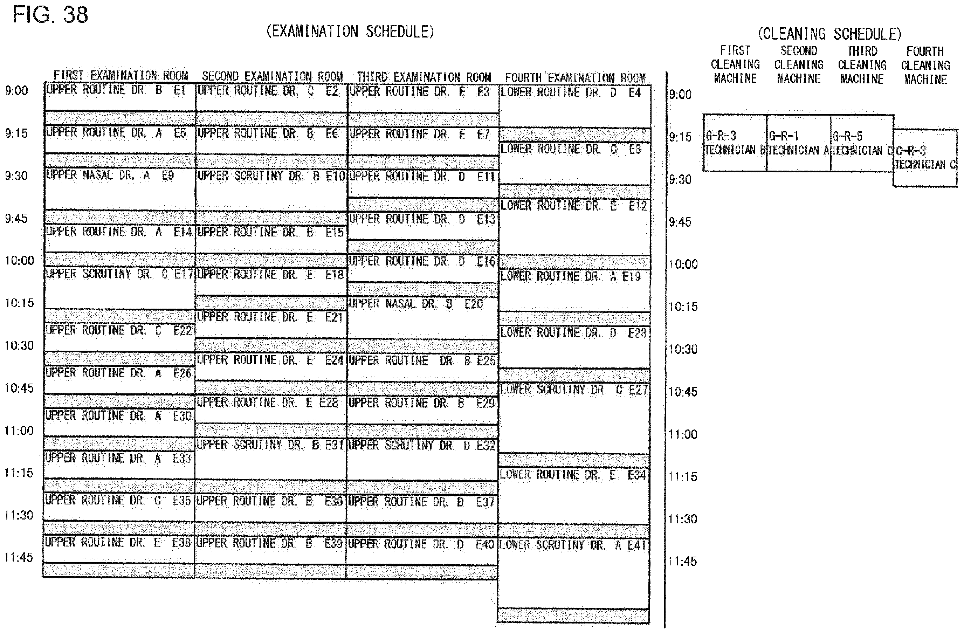

Fig. 38 illustrates a cleaning schedule updated by the cleaning schedule management unit 130. When the results of the assignment are notified from the person-in-charge assignment unit 149, the cleaning schedule management unit 130 registers the assigned person-in-charge in the corresponding cleaning processing. Herein, it is registered that: the technician B takes charge of the cleaning work of the endoscope G-R-3: the technician A takes charge of the cleaning work of the endoscope G-R-1; the technician C takes charge of the cleaning work of the endoscope G-R-5; and the technician C takes charge of the cleaning work of endoscope C-R-3. In this way, the cleaning schedule management unit 130 also adds a person-in-charge who performs cleaning work to the cleaning schedule, and records the updated cleaning schedule in the cleaning schedule holding unit 208.

In Example 5, the person-in-charge assignment unit 149 preferentially assigns the cleaning work of a specific endoscope 30 such that a specific person preparing for examination takes charge of the work as much as possible, and hence the cleaning work of the endoscope 30 is performed by the person-in-charge more frequently.

The usage condition monitoring unit 160 monitors the actually performed cleaning processing condition of the endoscope 30, and records it in the history recording unit 232. For example, each cleaning machine 50 is provided with a reading means for reading a person-in-charge ID, so that a person-in-charge performing cleaning work is specified by the person-in-charge causing the reading means to read an ID card, or the like. The usage condition monitoring unit 160 monitors this cleaning processing condition, and the history recording unit 232 records the cleaning history information on the cleaned endoscope 30. With respect to the endoscope 30, the history recording unit 232 records information on the date and time when it was cleaned, the person-in-charge who cleaned it, and the like by associating them with each other.

The history recording unit 232 also records histories relating to malfunctions and maintenance of the endoscope 30. The above histories may also include, for example, the person-in-charge who worked when a malfunction occurred or maintenance was performed, and information on date and time.

The display processing unit 150 displays, in a comparable format, the cleaning history information on a plurality of the endoscopes 30 recorded in the history recording unit 232. At this time, the display content derivation unit 152 calculates a statistical amount based on the cleaning history information recorded in the history recording unit 232. Herein, the statistical amount means the number of times of cleaning of the endoscope 30, the cleaning time thereof, or the like calculated for each person-in-charge, and the display content derivation unit 152 has the function of deriving the statistical amount in accordance with the contents to be displayed. The display processing unit 150 displays the statistical amount calculated by the display content derivation unit 152.

The period designation unit 154 designates a period for the cleaning history information. This period is specified by an input by a user into an input frame provided on the screen of the terminal device 12. When the period designation unit 154 designate a period, the display content derivation unit 152 extracts the cleaning history information during the period from the history recording unit 232 and calculates the statistical amount to be displayed, and the display processing unit 150 displays the cleaning history information during the designated period, i.e., the statistical amount calculated by the display content derivation unit 152 on the display of the terminal device 12.

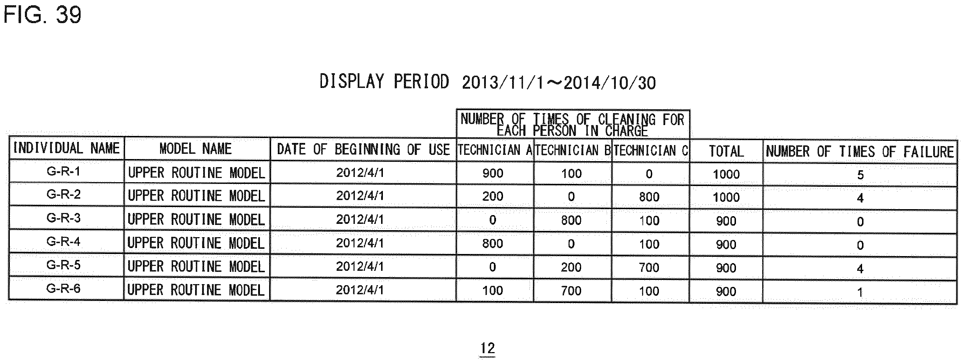

Fig. 39 illustrates one example of the cleaning history information to be displayed on the terminal device 12. When a user inputs the period between 2013/11/1 and 2014/10/30 as a display period, the period designation unit 154 designates this period, and the display content derivation unit 152 extracts the cleaning history information during this period from the history recording unit 232. Herein, the display content derivation unit 152 generates a number of times of cleaning table by calculating the number of times of cleaning of an upper routine model for each person-in-charge, and the display processing unit 150 displays it on the display of the terminal device 12. The display content derivation unit 152 may calculate the number of times of malfunctions by generating a list of malfunction histories during this period, and the display processing unit 150 may collectively display the number of times of malfunctions and the malfunction histories.

With this number of times of cleaning table, a user can specify an endoscope that caused less malfunctions and a technician who frequently cleans the endoscope. Conversely, a user can specify an endoscope that caused more malfunctions and a technician who frequently cleans the endoscope. In this way, by the person-in-charge assignment unit 149 preferentially assigning the cleaning work of a specific endoscope to a specific person-in-charge, the information on the histories in which the endoscope 30 was actually cleaned serve as useful information when failure analysis, etc., is performed. Additionally, because the display processing unit 150 displays the cleaning history information on a plurality of the endoscopes 30 in a comparable format, a user can recognize at a glance differences among the usage conditions of the endoscopes 30.

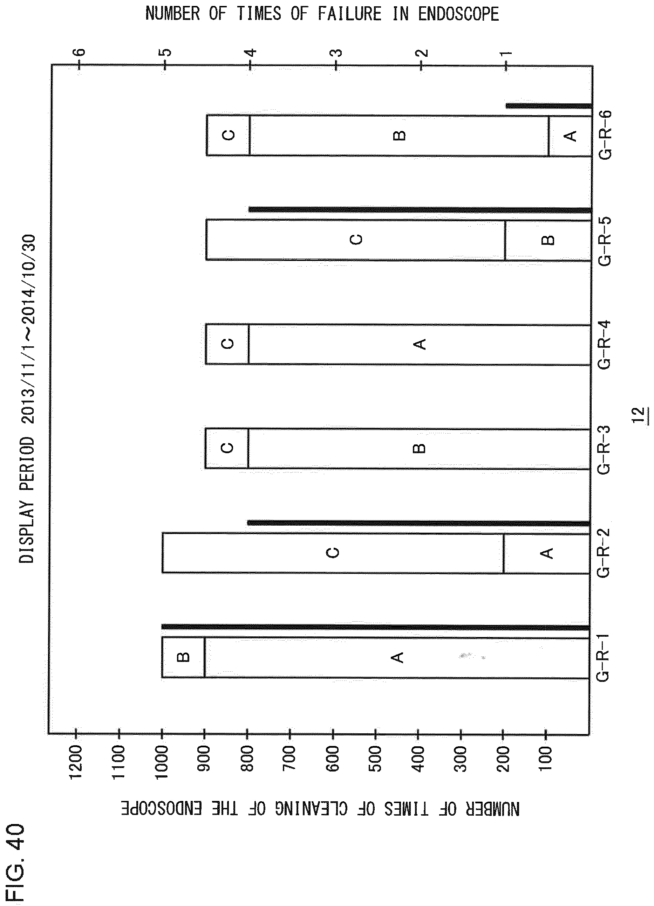

Fig. 40 illustrates one example of the cleaning history information to be displayed on the terminal device 12. This number of times of cleaning graph expresses in graph form the number of times of cleaning table illustrated in Fig. 39. By expressing in graph form in this way, it becomes possible to understand the differences among the usage conditions of the endoscopes 30 at a glance.

In Figs. 39 and 40, the display processing unit 150 displays the number of times of cleaning of the endoscope 30 for each person-in-charge as the cleaning history information, but may display, for example, the cleaning time of the endoscope for each person-in-charge as the cleaning history information. Additionally, the display processing unit 150 may display, for each person-in-charge, the number of times of cleaning or cleaning time of the endoscope 30 cleaned by a person-in-charge.

The configuration with respect to the scheduling processing of the present invention has been described above based on the embodiment and Examples 1 to 5. It should be understood by those skilled in the art that the embodiment and Examples 1 to 5 with respect to the scheduling processing are described for exemplary purposes only, and that various modifications can be made to combinations of the constituent elements and respective processes, and that such modifications are also within the scope of the present invention. In particular, the use history of an endoscope and the cleaning history thereof are recorded in Examples 4 and 5, respectively, and it is very meaningful to combine both Examples.

### <Rescheduling Processing 1>

By the processing described with respect to the embodiment and Examples 1 to 5, an examination schedule and a cleaning schedule are generated before the start of endoscopic examination work for one day. In a medical facility, it is ideal that the examination work proceeds according to the generated examination schedule and cleaning schedule, however, at a work site, the examination work may not proceed as scheduled in the examination schedule and/or the cleaning schedule due to various factors.

One of the typical factors is that the time at which an examination actually is started or is ended becomes earlier or later than the time set in the schedules. In the examination type master table 210 illustrated in Fig. 5, for example, the scheduled examination time of the "upper routine examination" with examination type No. 1 is set to be 10 minutes, so the "upper routine examination" is incorporated into the examination schedule, assuming that the time between the start and the end of the examination is 10 minutes. However, the actual examination time may be shorter or longer than the scheduled time of 10 minutes, and in particular when it is longer than the scheduled time, it is necessary to reset the subsequent schedules.

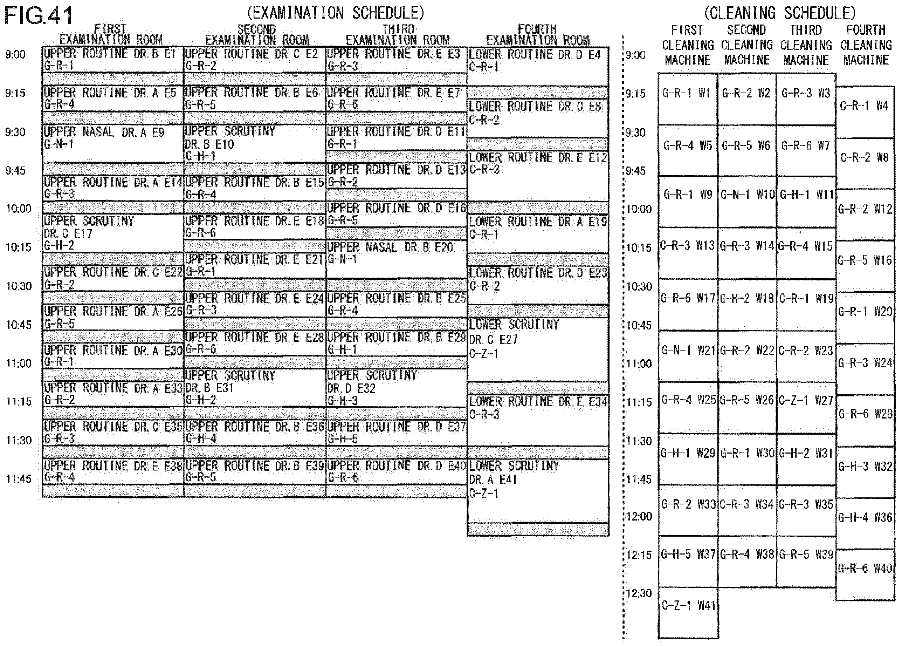

Fig. 41 illustrates examples of an examination schedule generated by the examination schedule management unit 110 and a cleaning schedule generated by the cleaning schedule management unit 130. The examination schedule illustrated in Fig. 41 is generated by assigning an endoscope to each examination with the endoscope assignment processing described with respect to Example 1 and according to the endoscope order table. The cleaning schedule illustrated in Fig. 41 is generated by the cleaning machine assignment processing described with respect to the embodiment. In the rescheduling processing described in the following Examples 6 to 8, the elements included in the examination schedule and cleaning schedule illustrated in Fig. 41 are subjected to the rescheduling processing, but this is only one example and the elements included in the examination schedule and cleaning schedule illustrated in Fig. 20 may be subjected to. In either case, it is premised in the rescheduling processing that an examination schedule and a cleaning schedule are generated in advance. For convenience of description, cleaning Nos. of W1 to W41 are attached to the cleaning processing of the respective endoscopes in the cleaning schedule.

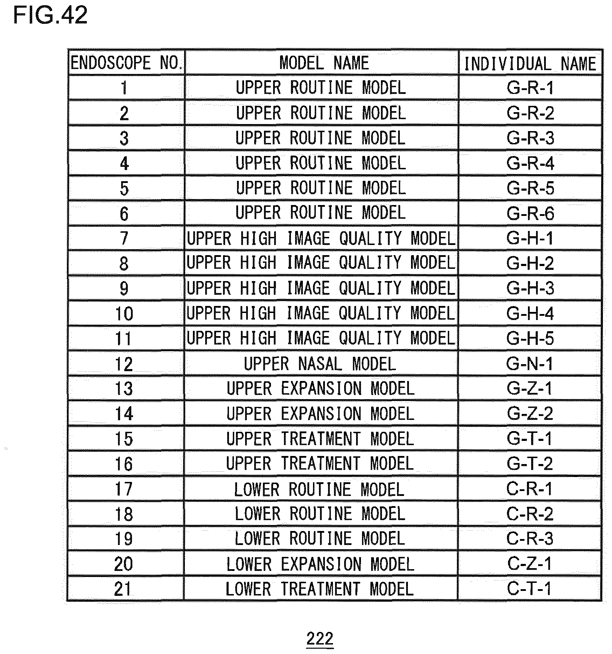

In Fig. 41, it is also premised that a medical facility possesses five upper high image quality models. Fig. 42 illustrates one example of the possessed endoscope master table 222. The possessed endoscope master table 222 is different from that illustrated in Fig. 6 in that a medical facility possesses five upper high image quality models. That is, the possessed endoscope master table 222 illustrated in Fig. 42 is different from that illustrated in Fig. 6 in that two upper high image quality models G-H-4 and G-H-5 are added, and in the examination schedule in Fig. 41, the endoscope G-H-4 is assigned to the examination E36 and the endoscope G-H-5 to the examination E37, respectively.

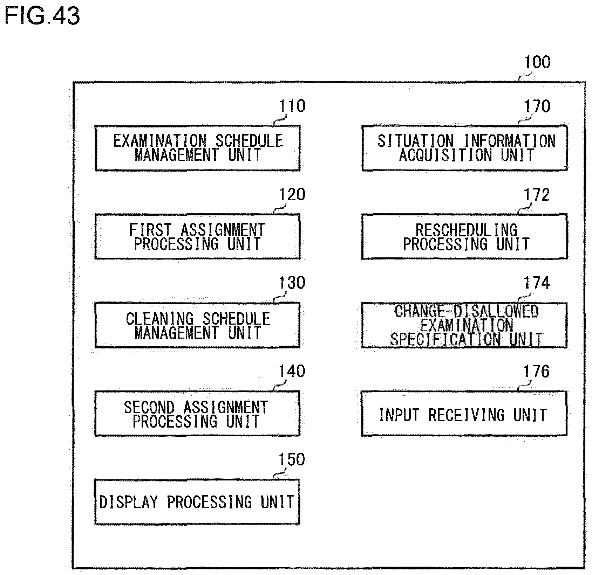

Fig. 43 illustrates, of the configuration of the information management device 10, the configuration of the processing unit 100 having the function of executing rescheduling processing. The processing unit 100 includes the examination schedule management unit 110, the first assignment processing unit 120, the cleaning schedule management unit 130, the second assignment processing unit 140, the display processing unit 150, a situation information acquisition unit 170, a rescheduling processing unit 172, a change-disallowed examination specification unit 174, and an input receiving unit 176. Although not illustrated in Fig. 43, the processing unit 100 is configured to also include the display content derivation unit 152, the period designation unit 154, and the usage condition monitoring unit 160, as illustrated in Fig. 3; the first assignment processing unit 120 is configured to include the examination extraction unit 122, the endoscope specification unit 124, the endoscope assignment unit 126, the endoscope assignment availability confirmation unit 128, and the doctor assignment unit 129; and the second assignment processing unit 140 is configured to include the cleaning machine specification unit 142, the cleaning machine assignment unit 144, the end time determination unit 146, the cleaning machine assignment availability confirmation unit 148, and the person-in-charge assignment unit 149.

Each component of the processing unit 100 can be realized by a CPU, memory, or other LSIs of an arbitrary computer in terms of hardware, and realized by a program or the like loaded in a memory in terms of software, but herein functional blocks realized by the cooperation of hardware and software are depicted. Therefore, it is to be understood by those skilled in the art that these functional blocks can be realized in various forms, namely, solely in hardware, solely in software, or through a combination of hardware and software. The following rescheduling processing is performed in accordance with the actual situation of an endoscopic examination after the start of the endoscopic examination work.

### <Example 6>

The situation information acquisition unit 170 acquires situation information on the situation of an examination. The situation information acquisition unit 170 includes a communication unit that communicates with the endoscopic observation device 22, and may have the function of acquiring the information transmitted from the endoscopic observation device 22. In this respect, the situation information acquisition unit 170 may include the function of the usage condition monitoring unit 160 illustrated in Fgi. 3.

The endoscope 30 is connected to the endoscopic observation device 22 before the start of an examination, and at the time the identification information on the endoscope 30 (endoscope ID) is transmitted to the information management device 10 via the network 2 along with information for identifying the endoscopic observation device 22 (observation device ID) and information for identifying an examination (examination ID). When an examination start button is operated in the endoscopic observation device 22 to start an examination, an examination start notice is transmitted to the information management device 10 along with the examination ID and the observation device ID. When an examination end button is operated in the endoscopic observation device 22 to end an examination (or when the endoscope 30 is withdrawn from the endoscopic observation device 22), an examination end notice is transmitted to the information management device 10 along with the examination ID and the observation device ID. In the information management device 10, the situation information acquisition unit 170 monitors the information transmitted from the endoscopic observation device 22, and acquires, as the situation information on the implementation situation of an examination, the information on the endoscope ID, examination start notice information, and examination end notice information. The situation information acquisition unit 170 immediately (in real time) provides the acquired situation information to the rescheduling processing unit 172 along with the acquired time information. Alternatively, the situation information acquisition unit 170 may periodically provide the acquired situation information to the rescheduling processing unit 172 along with the acquired time information.

The examination start notice information and the examination end notice information may be input by a user and received by the input receiving unit 176 along with the time information, and the input receiving unit 176 may transfer both the notice information to the situation information acquisition unit 170 as the situation information. Alternatively, the examination start notice information and the examination end notice information may be input to an in-hospital information system, such as an ordering system, and the situation information acquisition unit 170 may acquire both the notice information from the in-hospital information system as the situation information. As described above, the situation information acquisition unit 170 only has to acquire the implementation situation of the actual examination by some means, but in order to execute efficient rescheduling processing, it is preferable to acquire the situation information in real time and to provide it to the rescheduling processing unit 172 along with the acquired time information.

The rescheduling processing unit 172 determines based on the situation information whether it is necessary to change an element included in the examination schedule and/or the cleaning schedule illustrated in Fig. 41. For example, when the time at which the situation information acquisition unit 170 acquires the examination end notice information is later than the scheduled examination end time of the examination, the rescheduling processing unit 172 determines that it is necessary to change the examination schedule and/or the cleaning schedule.

In order to change the examination schedule, it is necessary to know the time when the situation information acquisition unit 170 acquired the examination end notice information, but when the situation information acquisition unit 170 did not acquire the examination end notice information at a time later than the scheduled examination end time, the rescheduling processing unit 172 can determine that it is necessary to change at least the examination schedule. Therefore, when the situation information acquisition unit 170 does not acquire the examination end notice information at a time later than the scheduled examination end time, which is known by referring to the examination schedule, the situation information acquisition unit 170 may provide information indicating this fact to the rescheduling processing unit 172.

When determining based on the situation information that it is necessary to change the examination schedule and/or the cleaning schedule, the rescheduling processing unit 172 instructs at least one of the examination schedule management unit 110 and the cleaning schedule management unit 130 to change an element included in the examination schedule and/or the cleaning schedule. Herein, the element included in the examination schedule is an examination specified by an examination ID, an examination room assigned to an examination, information on scheduled examination start time, that on scheduled examination end time, a primary doctor, or an endoscope 30; while the element included in the cleaning schedule is a cleaning machine assigned to an endoscope 30, information on scheduled cleaning start time, or that on scheduled cleaning end time. When a person-in-charge is assigned to cleaning processing in the cleaning schedule, as illustrated in Example 5 (Fig. 38), person-in-charge information is also included in the element included in the cleaning schedule.

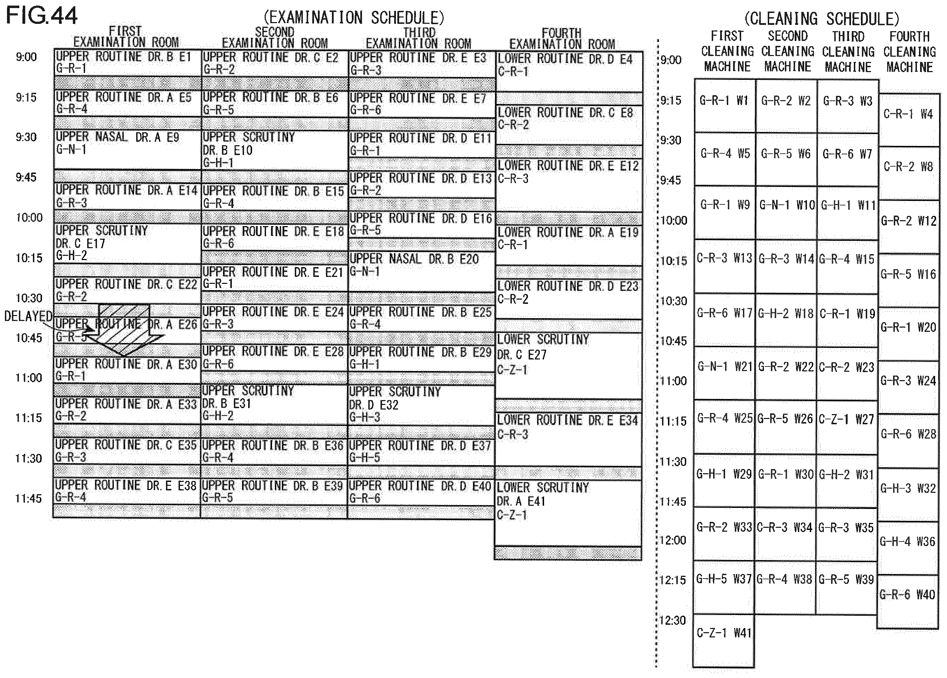

Hereinafter, specific rescheduling processing will be described with reference to Figs. 44 to 47. Fig. 44 is a view illustrating an example in which an actual examination is delayed than scheduled. The examination E22 is scheduled to be performed between 10:25 as the scheduled examination start time and 10:35 as the scheduled examination end time, but the actual examination end time thereof is later than the scheduled end time by 20 minutes. Therefore, the situation information acquisition unit 170 acquires examination end notice information from the endoscopic observation device 22a in the first examination room 20a at 10: 55. Upon acquiring the examination end notice information, the situation information acquisition unit 170 immediately notifies the rescheduling processing unit 172 of the acquisition of the examination end notice information on the examination E22 at 10:55.

When detecting, in response to the notice from the situation information acquisition unit 170, that the end time of the examination E22 is later than the scheduled time and a delay occurs, the rescheduling processing unit 172 determines that it is necessary to change the examination schedule and/or the cleaning schedule. When determining that it is necessary to change a schedule, the rescheduling processing unit 172 instructs at least one of the examination schedule management unit 110 and the cleaning schedule management units 130 to change an element included in the schedule. In this Example 6, the rescheduling processing unit 172 instructs the examination schedule management unit 110 to change an element included in the examination schedule, and the examination schedule management unit 110 performs processing for moving the time frame of an examination designated by information on scheduled examination start time and that on scheduled examination end time as an element of the examination schedule.

At this time, the rescheduling processing unit 172 may specify, of the elements included in the examination schedule, information on scheduled examination end time of the examination E22 as an element to be changed, and may instruct the examination schedule management unit 110 to change the specified element. The rescheduling processing unit 172 may specify an element to be changed based on the situation information in this way, but the examination schedule management unit 110 may specify an element to be changed by being notified of the end time of the examination E22 from the rescheduling processing unit 172.

It has been described that when the situation information acquisition unit 170 does not acquire the examination end notice information at a time later than the scheduled examination end time, which is known by referring to the examination schedule, the situation information acquisition unit 170 may provide information indicating that a delay occurs in an examination to the rescheduling processing unit 172, but at the time the rescheduling processing unit 172 may notify the examination schedule management unit 110 that a delay occurs in the examination E22. As a result, the examination schedule management unit 110 recognizes, as the elements that need to be reset, the information on scheduled examination start time and that on scheduled examination end time of each of the examinations E26, E30, E33, E35, and E38 that are schedulded to be performed in the same examination room as the examination E22 and after the examination E22, and may perform processing for moving the time frames of these examinations at a time when the end time of the examination 22 is known.

Even if not notified from the rescheduling processing unit 172 that a delay occurs in the examination E22, the examination schedule management unit 110 can determine by itself that a delay occurs in the examination 22 by referring to the examination schedule it manages. Therefore, the examination schedule management unit 110 can also recognize, at a time later than the scheduled end time of the examination 22, the information on scheduled examination start time and that on scheduled examination end time of each of the examinations E26, E30, E33, E35, and E38, which are scheduled to be performed after the examination E22, as the elements that need to be reset.

Doctors, etc., confirm the schedule information before the start of examination work for one day, and often put into their mind what types of examinations are to be performed in which examination rooms and at which times. Therefore, rescheduling processing is executed in Example 6, in such a way that the schedules preset before the start of the work are basically taken over, not changed greatly.

Under such a policy, the examination schedule management unit 110 specifies an examination room by which it becomes necessary to change the examination schedule, based on a schedule element change instruction provided from the rescheduling processing unit 172, and changes the information on scheduled examination start time and that on scheduled examination end time of an endoscopic examination in the specified examination room. Specifically, the schedule element change instruction includes the examination end time of the examination E22, and in response to this change instruction, the examination schedule management unit 110 specifies the first examination room 20a where the examination E22 was performed, and resets the information on scheduled examination start time and that on scheduled examination end time of each of the examinations E26, E30, E33, E35, and E38, which are scheduled to be performed in the first examination room 20a after the examination E22, to be delayed by the delayed time of the examination E22, i.e., in this example to be later than the scheduled times by 20 minutes.

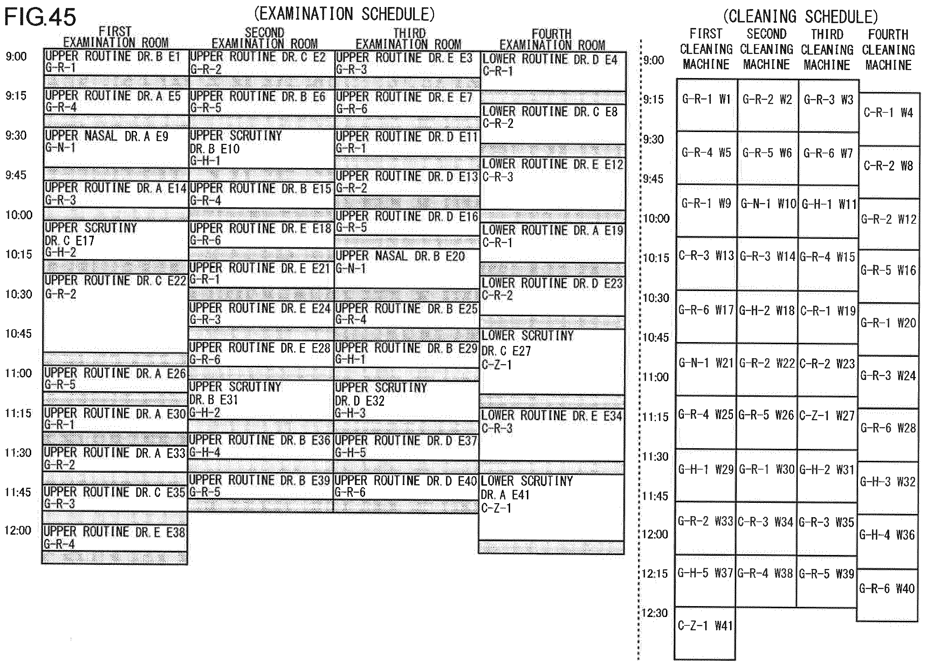

Fig. 45 illustrates a state where the scheduled examination start time and the scheduled examination end time of each of the examinations E26, E30, E33, E35, and E38 are delayed by 20 minutes, respectively. Although it goes back and forth in time, the scheduled examination start time of the examination E27 is set to be 10:45 in the examination schedule. The primary doctor of the examination E27 is the doctor C, however, the doctor C is still performing the examination E22 at the time of 10:45, so the examination E27 is not started at the scheduled time of 10:45. If the situation information acquisition unit 170 did not acquire, at 10: 45, the examination start notice information on the examination E27 from the endoscopic observation device 22d in the fourth examination room 20d, the rescheduling processing unit 172 recognizes that the examination E27 is not started as scheduled, and notifies the examination schedule management unit 110 that the examination E27 is not started. As a result, the examination schedule management unit 110 recognizes the information on scheduled examination start time and that on scheduled examination end time of each of the examinations E27, E34, and E41 in the fourth examination room 20d as the elements that need to be reset.

When the end of the examination E22 is delayed from the schedule in this way, there is the possibility that not only in the same examination room as the examination E22 but also in other examination rooms, the examination schedules and/or the cleaning schedules after the scheduled examination end time (10:35) of the examination E22 may be affected. This example describes the case where the schedule of the doctor C is affected, but other than this, the use schedule of the endoscope 30 and that of the cleaning machine 50 may be affected. Therefore, in Example 6, the examination schedule management unit 110 first investigate, by paying attention to the time frames of examinations, whether an inconsistency occurs in the doctor information or the endoscope 30, which are elements assigned to a time frame whose scheduled start time is later than the scheduled examination end time of the examination E22. Herein, the case where an inconsistency occurs means: with respect to the doctor information, the case where the same doctor is assigned to a plurality of examinations overlapping each other in time; and with respect to the endoscope 30, the case where one endoscope 30 is assigned to a plurality of examinations overlapping each other in time, or the case where one endoscope 30 is assigned to an examination and cleaning at the same time.

Specifically, the examination schedule managing unit 110 investigates, at the time of 10:55 when the examination end notice information on the examination E22 is received, whether the time fames of examinations, in which a doctor or an endoscope 30 overlaps in time, exist, as a result that the time frames of examinations after the examination E26 in the first examination room 20a are delayed by 20 minutes, respectively, as illustrated in Fig. 45. Herein, the examination schedule management unit 110 already recognizes, at the time of 10:45, the information on scheduled examination start time and that on scheduled examination end time of the examination E27 as the elements that need to be reset, and resets the information on scheduled examination start time of the examination E27 to be later than 10:55 because the doctor C can perform the examination at the time of 10: 55. At this time, the examinations after the examination E27 in the fourth examination room 20d are similarly delayed, that is, the examination schedule management unit 110 specifies the fourth examination room 20d in which an examination is to be delayed, so that the information on scheduled examination start time and that on scheduled examination end time of each of the examinations E27, E34, and E41 are reset, respectively.

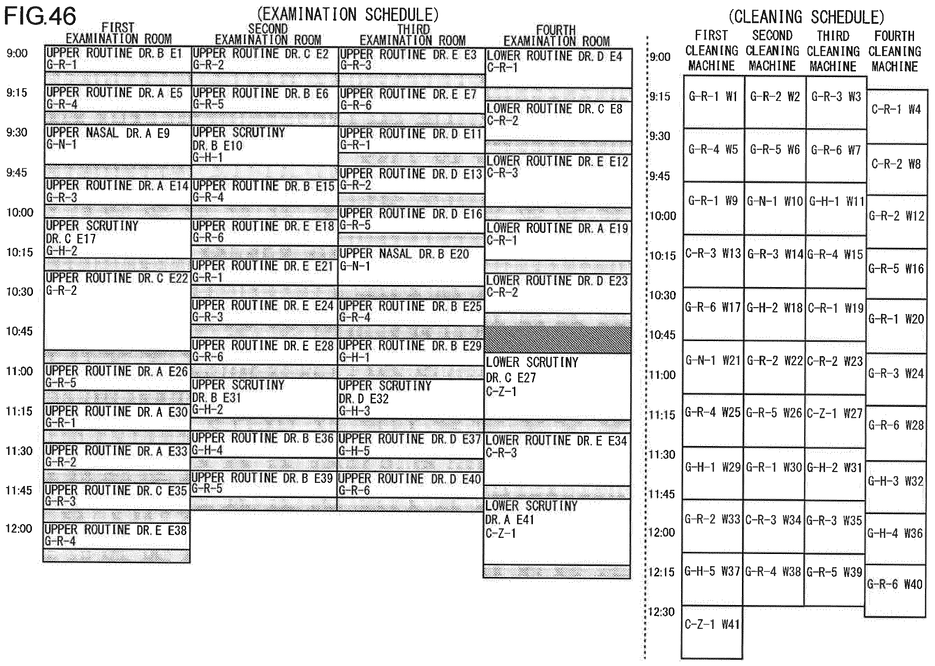

Fig. 46 illustrates a state where the scheduled examination start time and the scheduled examination end time of each of the examinations E27, E34, and E41 are delayed by 10 minutes, respectively. In this example, the scheduled examination start time of the examination E27 is reset such that the doctor C starts the examination E27 immediately after the end (10:55) of the examination E22, but the scheduled examination start time thereof may be reset such that the examination E27 starts after a 5-minute interval, i.e., at 11:00.

Specifically, when resetting the time frame of an examination, the examination schedule management unit 110 excludes, from investigation targets, the time frame of an examination being performed as scheduled. It is known, at the time when the examination end notice information on the examination E22 is received, that examinations are being performed as scheduled in the second examination room 20b and the third examination room 20c, because the examination start notice information on the examination E28 in the second examination room 20b and the examination start notice information on the examination E29 in the third examination room 20c are already provided from the situation information acquisition unit 170 via the rescheduling processing unit 172 at the time of 10:50. Therefore, with respect to the examinations E26, E31, E32, and E27 in each examination room, the scheduled examination start times of which are later than the scheduled examination end time (10:35) of the examination E22 and which are not yet started, the examination schedule management unit 110 investigates whether there is any examination in which a primary doctor or an endoscope overlaps in time. As a result, the scheduled start time of the examination E27 in the fourth examination room 20d is reset to be 10:55, i.e., is corrected to the examination schedule illustrated in Fig. 46.

Next, the cleaning schedule management unit 130 investigates whether an inconsistency occurs in the cleaning schedule of an endoscope whose scheduled cleaning start time is later than the scheduled examination end time (10:35) of the examination E22. Herein, the case where an inconsistency occurs means the case where cleaning of an endoscope 30 is to be started although the endoscope 30 is being used in an examination at a time. Herein, cleaning W20 to W27 are extracted as the cleaning schedules of endoscopes whose scheduled cleaning start times are later than the scheduled examination end time (10:35) of the examination E22.

The cleaning schedule management unit 130 is monitoring whether cleaning is performed as the cleaning schedule by the time (10:55) when the examination E22 was actually ended, similarly to the examination schedule management unit 110. For example, the situation information acquisition unit 170 includes a communication unit that communicates with the cleaning machine 50, and may have the function of acquiring the information transmitted from the cleaning machine 50. A means for reading the endoscope ID of an endoscope 30 to be cleaned is provided in the cleaning machine 50, and a person-in-charge of cleaning causes the reading means to read the endoscope ID of an endoscope 30 before the start of cleaning. When a cleaning start button is operated, the cleaning machine 50 transmits the cleaning start notice information to the information management device 10 via the network 2, along with the endoscope ID and the information for identifying a cleaning machine 50 (cleaning machine ID). When the cleaning ends, the cleaning machine 50 transmits the cleaning end notice information to the information management device 10 via the network 2, along with the endoscope ID and the cleaning machine ID. The situation information acquisition unit 170 monitors the information transmitted from the cleaning machine 50, and acquires the cleaning start notice information and the cleaning end notice information as the situation information on the implementation situation of cleaning. The situation information acquisition unit 170 immediately (in real time) provides the acquired situation information to the rescheduling processing unit 172 along with the acquired time information, and the rescheduling processing unit 172 notifies the cleaning schedule management unit 130 of the situation information and the time information.

Therefore, if there is any cleaning that is not performed as scheduled between the scheduled end time (10:35) and the actual end time (10:55) of the examination E22, the cleaning schedule management unit 130 recognizes, at the time of 10:55, the cleaning as an element that needs to be reset. In this example, the cleaning W20 to W23 are ones whose scheduled cleaning start times are between 10:35 and 10:55, but the endoscope G-R-2 in the cleaning W22 is still being actually used in the examination E22 at the time of 10:50 when the cleaning thereof is scheduled to be started. Therefore, the cleaning schedule management unit 130 recognizes, at the time of 10:50, the information on scheduled cleaning start time and that on scheduled cleaning end time, which are elements of the cleaning W22, as the elements that need to be reset.

When the rescheduling processing unit 172 issues, at the time of 10:55, a cleaning schedule element change instruction to the cleaning schedule management unit 130, the cleaning schedule management unit 130 resets the scheduled cleaning start time and scheduled cleaning end time of the cleaning W22. This cleaning schedule resetting processing is executed after the above examination schedule resetting processing by the examination schedule management unit 110 is performed. The cleaning schedule management unit 130 instructs the second assignment processing unit 140 to perform the cleaning machine reassignment processing of the endoscope G-R-2 in the cleaning W22, and in response to this instruction, the cleaning machine assignment unit 144 of the second assignment processing unit 140 assigns the second cleaning machine 50b to the endoscope G-R-2 by changing the scheduled start time of W22 to be shifted 5 minutes later.

Thereafter, the cleaning schedule management unit 130 investigates whether an inconsistency occurs in the cleaning W24 to W27 whose scheduled start times are later than 10:55. Herein, the start of the examination E27 is delayed, and hence the status of the endoscope C-Z-1 in the cleaning W27 is under use in the examination E27 at the time of 11:10 that is the scheduled cleaning start time of the endoscope C-Z-1. Therefore, the cleaning schedule management unit 130 resets the scheduled cleaning start time of the cleaning W27 to be equal to the scheduled end time (11:20) of the examination E27, that is, reschedules the cleaning schedule by delaying the scheduled start time by 10 minutes.

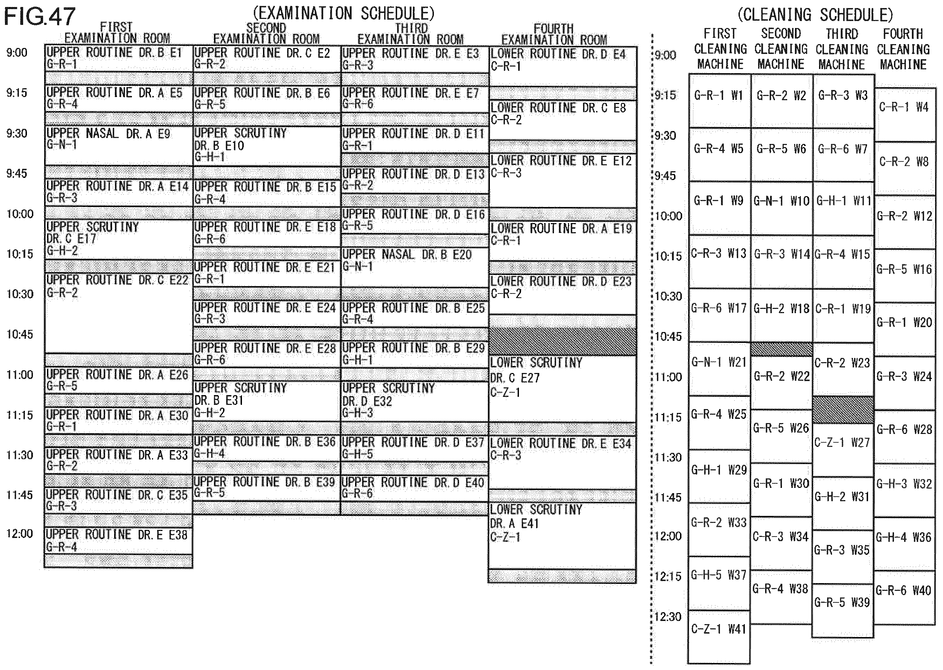

Fig. 47 illustrates a state where the scheduled cleaning start time and scheduled cleaning end time of the cleaning W22 are delayed by 5 minutes and the scheduled cleaning start time and scheduled cleaning end time of the cleaning W27 are delayed by 10 minutes. Subsequent cleaning W26, W30, W34, and W38 in the same cleaning machine as the cleaning W22 are also similarly delayed by 5 minutes, respectively, and subsequent cleaning W31, W35, and W39 in the same cleaning machine as the cleaning W27 are also similarly delayed by 10 minutes, respectively.

Next, with respect to the examinations E30, E36, E37, and E34 in each examination room that is not yet investigated, the examination schedule management unit 110 investigates whether there is any examination in which a primary doctor or an endoscope overlaps in time. If there is any examination in which it overlaps, the scheduled start time of the examination is delayed, but in this example it does not overlap in time, and hence the schedules are fixed. Similarly, with respect to cleaning W28 to W31 in each cleaning machine that is not yet investigated, the cleaning schedule management unit 130 investigates whether an inconsistency occurs, and if there is any endoscope that overlaps in time, the scheduled start time of the cleaning is delayed. In this example, no endoscope overlaps in time, and hence the schedules of these cleaning are fixed. The examination schedule and the cleaning schedule can be reset by repeating the above processing. This steps of repeating the processing may be performed according to the steps described in the embodiment.

As described above, the examination schedule management unit 110 and the cleaning schedule management unit 130 reschedule, in real time, the examination schedule and the cleaning schedule that have been generated in advance, depending on the actual situation of an examination and based on a schedule element change instruction from the rescheduling processing unit 172. In Example 6, only the time frames of examinations and cleaning are changed, but neither the orders of the examinations and cleaning nor the examination rooms and cleaning machines to be used are changed. As described above, by executing rescheduling processing in a way in which the schedule information before the start of examination work is taken over and schedule elements, such as an examination order and an examination room, are not changed, doctors, etc., only have to move from one examination room to another basically according to the schedule information confirmed before the start of examination work, although it goes back and forth in time, whereby work efficiency can be improved.

In Example 6, an example has been described in which the examination E22 takes longer than scheduled and the end time is later than the scheduled time. Even in the case where, for example, preparation for an examination takes time and the start of the examination is delayed from the scheduled time, the rescheduling processing described in Example 6 can be applied. Also, even in the case where the start time or end time of an examination is earlier than the scheduled time, the rescheduling processing described in Example 6 can be applied, whereby work efficiency can also be improved. Also, even in the case: where, for example, the examination room 20 cannot be used due to a malfunction, or the like of the endoscopic observation device 22 in the examination room 20; where the cleaning machine 50 cannot be used; or the like, the rescheduling processing described in Example 6 can be applied, whereby the examination schedule and the cleaning schedule can be efficiently reconstructed.

### <Example 7>

Example 6 has been described the case where the rescheduling processing is needed because the actual start time or end time of an examination is earlier or later than the time scheduled in the schedules. In Example 7, a case is assumed where the scheduled examination time is changed because, for example, the arrival of an outpatient at a medical facility is delayed.

Even in the case where a pretreatment is too late for the scheduled examination start time because the arrival of an outpatient is delayed, it is preferable to perform the examination during the day by adjusting time because the patient also prepares for the examination by fasting from the previous day. In order to achieve this, the examination of the patient is to be interrupted into the schedule, but on the other hand, the start of examinations for other patients are delayed from the scheduled times due to the interruption of the examination. Therefore, it is preferable that rescheduling processing is performed in such a way that an examination is started at a time close to the scheduled time as much as possible, in other words, at a time when a start delay is suppressed as much as possible.

An examination, in which an assigned element cannot be changed, also exists in the examination schedule. If there is a circumstance in which an examination can be performed only in a certain time zone depending on, for example, the convenience of a patient or a doctor, the scheduled examination start time of the examination cannot be moved. In addition, if an examination requires a special skill, only a doctor having the skill can take charge of the examination, and hence at least the primary doctor of the examination cannot be changed. In addition, even with the endoscopes 30 of the same type, there are various models ranging from the latest one to an old-fashioned one, but for example, in a special examination, only the latest endoscope 30 may be used.

Therefore, in Example 7, the change-disallowed examination specification unit 174 specifies an endoscopic examination in which the change of at least one element, of a plurality of elements assigned in advance to an endoscopic examination in the examination schedule, is made disallowed. An input interface, such as a keyboard or a mouse, is connected to the terminal device 12, and the input receiving unit 176 receives an input operation from a user via the terminal device 12. For example, when an examination schedule is generated and when a user designates an examination in which the change of an element is made disallowed, the input receiving unit 176 receives the designating operation from the user, and the change-disallowed examination specification unit 174 specifies an endoscopic examination in which the change of an element is made disallowed. Alternatively, it may be configured such that a user can designate, of a plurality of elements that form the schedule of an endoscopic examination, an element, the change of which is made disallowed.

Herein, the elements that form the examination schedule include at least an assigned examination room, information on scheduled examination start time, that on scheduled examination end time, primary doctor, and endoscope ID. Of these elements, a user can designate that the changes of, for example, the primary doctor and the scheduled examination start time are prohibited. When such designation is made, an examination room and an endoscope to be used can be changed, and the change-disallowed examination specification unit 174 specifies an examination element, the change of which is made disallowed, so that such an element is not changed when the rescheduling processing is performed.

In the following description, a case will be described in which all of the schedule elements of the examination E17 illustrated in Fig. 41, that is, the first examination room 20a to be used, the primary doctor C, the endoscope G-H-2 to be used, the scheduled examination start time, and the scheduled examination end time are designated such that the changes thereof are disallowed. In addition, Example 7 describes the case where an examination cannot be started from the scheduled start time of 9:50 because the arrival of a patient of the examination E15 is delayed.

When a user in a medical facility knows in advance that the examination E15 cannot be started at the scheduled start time, the user performs a reservation change operation for the examination E15 by operating the input interface. For example, the display processing unit 150 reads the examination schedule information from the examination schedule holding unit 206 and displays the examination schedule table illustrated in Fig. 41 on the display of the terminal device 12. It is configured that the examination schedule table is displayed as a GUI that can be operated by a user, so that a user can designate an examination, the reservation for which is to be changed, on the examination schedule table.

When the input receiving unit 176 receives the reservation change operation for the examination E15, the situation information acquisition unit 170 acquires the reservation change information from the input receiving unit 176 as the situation information on the situation of an examination, and provides it to the rescheduling processing unit 172. The rescheduling processing unit 172 determines based on the situation information that it is necessary to change the examination schedule and/or the cleaning schedule, and instructs at least one of the examination schedule management unit 110 and the cleaning schedule management unit 130 to change an element included in the examination schedule and/or the cleaning schedule. This change instruction is one for changing the scheduled start time of the exdamination E15, and in response to this change instruction, the examination schedule management unit 110 temporarily deletes the schedule information on the examination E15 from the examination schedule.

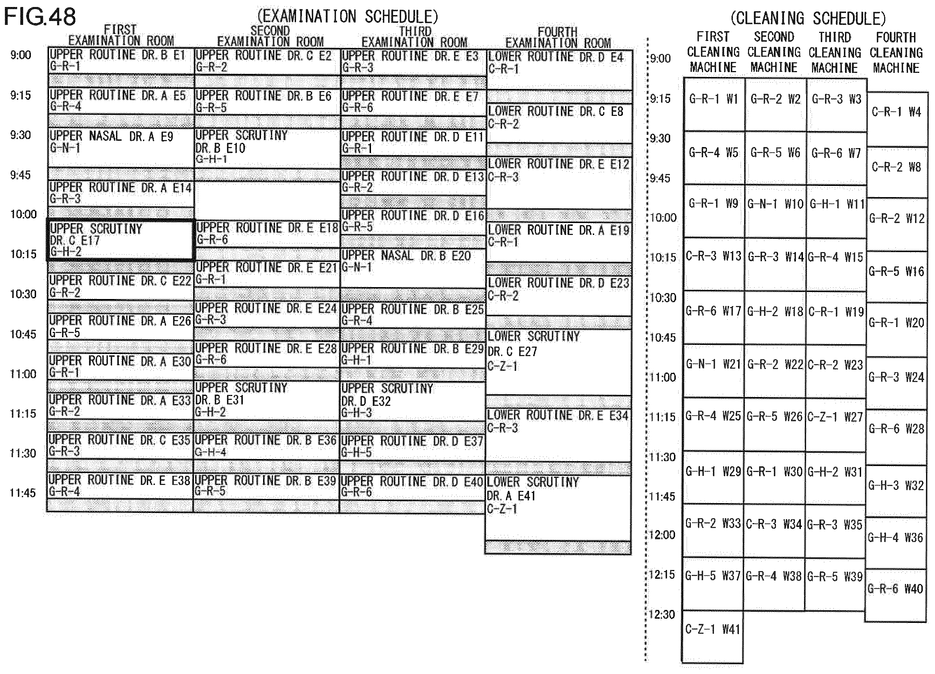

Fig. 48 illustrates an examination schedule table and a cleaning schedule table to be displayed on a display. The display processing unit 150 may display the time frame of the examination E17, which is specified by the change-disallowed examination specification unit 174 and the change of which is prohibited, so that a user can recognize. In Fig. 48, the time frame of the examination E17 is displayed by a thick frame, so that a user can recognize such that the change of which is disallowed. Also, in the examination schedule table, it is shown that the schedule information on the examination E15 is temporarily deleted.

When knowing that the examination E15 can be started from 10:05, a user sets the scheduled examination start time of the examination E15 such that the patient is examined as soon as possible. For example, when a nurse is performing a pretreatment on a patient of the examination E15, the user can know that the examination E15 can be started from 10:05 by receiving from the nurse a report indicating that the pretreatment is completed at 10:00, whereby the scheduled examination start time can be set.

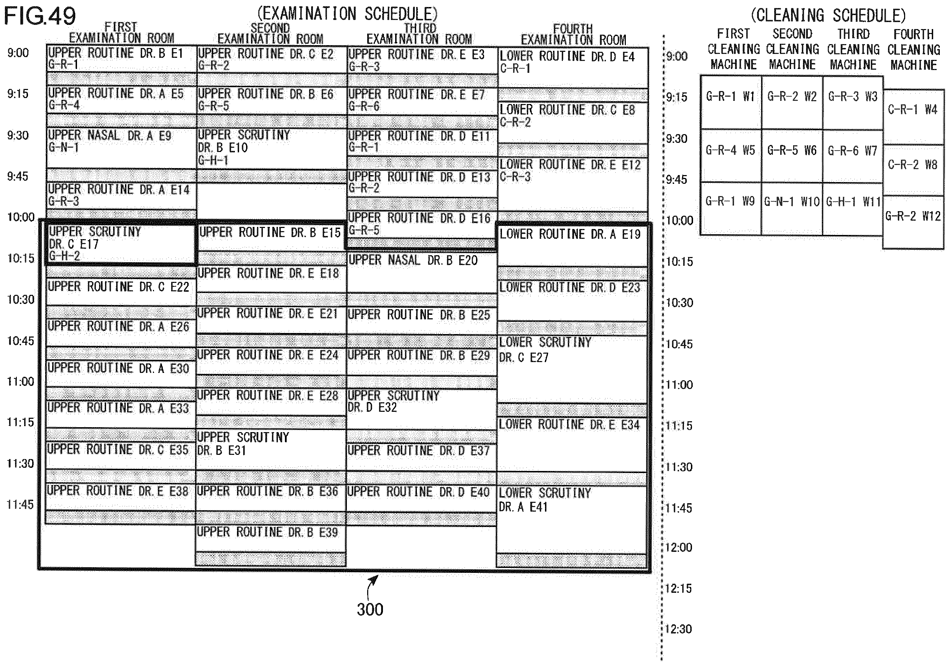

Fig. 49 illustrates a state where a user designates the examination E15 such that the scheduled examination start time thereof becomes equal to 10:05 in the second examination room 20b. When the input receiving unit 176 receives designation information on the scheduled examination start time of the examination E15, the situation information acquisition unit 170 acquires the information received by the input receiving unit 176 as the situation information on the situation of the examination E15, and provides it to the rescheduling processing unit 172. The rescheduling processing unit 172 notifies, based on this situation information, the examination schedule management unit 110 of the information on scheduled examination start time of the examination E15, and instructs to change at least an element of the examination E15.

In response to this change instruction, the examination schedule management unit 110 resets the scheduled examination start time of the examination E15 to be 10:05. At the time, the examination schedule management unit 110 specifies, based on the schedule element change instruction, an examination room by which it becomes necessary to change the examination schedule, and changes the information on scheduled examination start time and that on scheduled examination end time of an examination in the specified examination room. The schedule element change instruction includes that the examination E15 should be started in the second examination room 20b and from 10:05, and therefore the examination schedule management unit 110 specifies the second examination room 20b by which it becomes necessary to change the examination schedule, and specifies an examination by which it becomes necessary to change the schedule by starting the examination E15 from 10:05. With reference to the examination schedule in Fig. 48, it is specified herein that it is necessary to change the schedules of the examinations E18, E21, E24, E28, E31, E36, and E39 that are scheduled in the second examination room 20b, and therefore the examination schedule management unit 110 adjusts the time frames of the examinations E18, E21, E24, E28, E31, E36, and E39 so as to be shifted backward in time, as illustrated in Fig. 49. With it, the examination schedule management unit 110 extracts examinations that are scheduled to be started later than the scheduled examination start time (10:05) of the examination E15 as examinations to be reassigned, and assignment of endoscopes are cleared. In Fig. 49, the examinations enclosed by a frame 300 are ones to which endoscopes are to be reassigned.

At this time, the change-disallowed examination specification unit 174 specifies an examination in which the change of at least one element, of the elements assigned in advance to the examination, is made disallowed. Herein, the change-disallowed examination specification unit 174 recognizes that all the elements assigned to the examination E17 are designated such that the changes of which are disallowed, and specifies the examination E17, the changes of the elements of the schedule information of which are prohibited. The rescheduling processing unit 172 notifies the examination schedule management unit 110 of the elements of the examination E17, which are specified by the change-disallowed examination specification unit 174 and the changes of which are made disallowed, so that the changes of the elements are prohibited. In this example, changes of all of the elements of the examination E17 are prohibited, and hence the rescheduling processing unit 172 notifies the examination schedule management unit 110 that changes of all of the elements of the examination E17 are prohibited, and in response to this notice and when the endoscope assignment is cleared, the rescheduling processing unit 172 does not clear the endoscope assignment to the examination E17, but clears those to the examinations other than the examination E17.

The cleaning schedule management unit 130 deletes the cleaning W15 for the endoscope G-R-4, which was scheduled to be used in the examination E15 in the examination schedule before the reassignment, from the cleaning schedule. Further, the cleaning schedule management unit 130 deletes the cleaning W13, W14, and W16 to W41, which are scheduled to be started later than the scheduled examination start time (10:05) of the examination E15 after the reassignment, from the cleaning schedule. Thereby, the examination schedule and the cleaning schedule, which are illustrated in Fig. 49, are generated.

Thereafter, the processing unit 100 repeats the processing in which the endoscope 30 is assigned to each examination and the cleaning machine 50 is assigned to the endoscope 30 used in the examination, from the examination schedule and the cleaning schedule illustrated in Fig. 49, whereby the examination schedule and the cleaning schedule are reconstructed, as described with respect to the embodiment, etc. Specifically, the endoscope assignment unit 126 in the first assignment processing unit 120 assigns the endoscope 30 to an examination whose information on scheduled examination start time and that on scheduled examination end time were changed, and the cleaning machine assignment unit 144 in the second assignment processing unit 140 assigns the cleaning machine 50 for cleaning the endoscope 30 such that a time after the scheduled examination end time of the endoscope 30 assigned to the examination becomes equal to the scheduled cleaning start time. Further, the endoscope assignment unit 126 assigns the endoscope 30 to a subsequent examination such that a time after the scheduled cleaning end time of cleaning, the cleaning being performed by the cleaning machine 50 assigned to the endoscope 30 by the cleaning machine assignment unit 144, becomes equal to the scheduled examination start time of the examination. As described with respect to the embodiment, etc., the examination schedule and the cleaning schedule are constructed by the first assignment processing unit 120 and the second assignment processing unit 140 by repeating this processing.

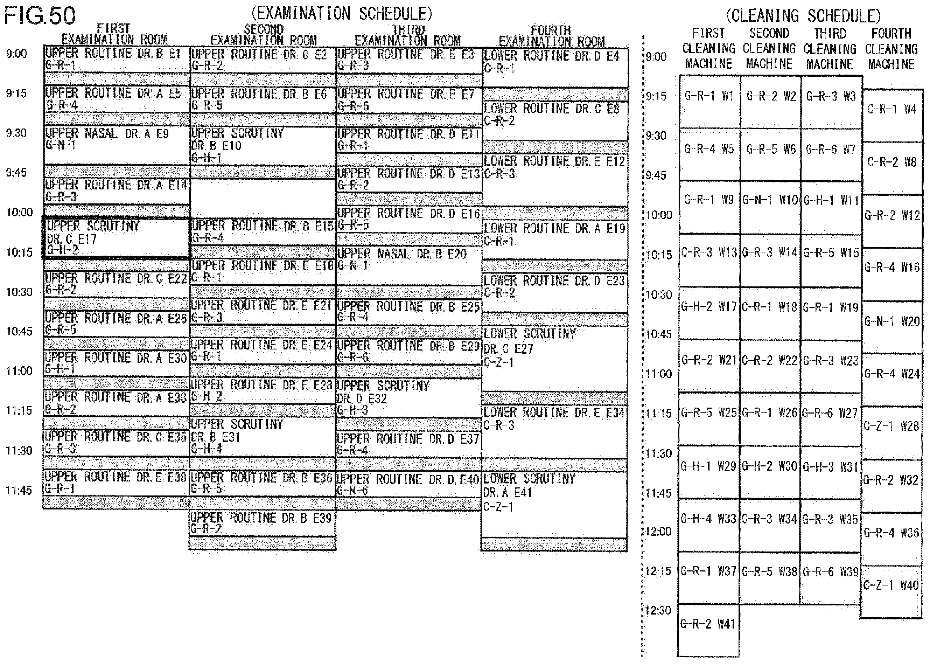

Fig. 50 illustrates an examination schedule and a cleaning schedule in which the rescheduling processing has been performed. As described above, the change of a schedule element of the examination E17 is prohibited, and the first assignment processing unit 120 does not change an element of the schedule information. In Example 7, the schedule elements of an examination, in which the change of a schedule element is prohibited, are fixed, and the schedule elements of other examinations are changed, as described above. In the example illustrated in Fig. 50, the assignment of the endoscope 30 is changed, and the assignment of a primary doctor is not changed but the time frame of an examination is changed by the examination schedule management unit 110, and hence when one doctor is assigned to a plurality of examinations overlapping each other in time, the doctor assignment unit 129 in the first assignment processing unit 120 needs to reassign a primary doctor.

Also in Example 7, even when the examination E15 is interrupted into an arbitrary position in the examination schedule, the examination schedule management unit 110 does not change the order, examination rooms, and the like of the examinations after the interruption. As a result, the start times of the subsequent examinations in the examination room, into which an examination is interrupted, are delayed, but the order of the examinations is not changed, and hence the scheduled start times thereof are delayed by minimum amounts. Herein, the examination E15 is interrupted into an arbitrary position in the examination schedule, but it may be adjusted such that the examination E15 is applied, for example, to the time frame of another examination.

When an inconsistency occurs in the schedule of a primary doctor, the doctor assignment unit 129 may reassign a primary doctor, as described above, but as described in Example 6, it is also possible to fix the assignment of a primary doctor by adjusting the time frame of an examination. In this case, doctors only have to move from one examination room to another, and the like, basically according to the schedule information confirmed before the start of examination work, although it goes back and forth in time, whereby work efficiency can be improved.

Example 7 has described the case where the examination of a patient is delayed, but the rescheduling processing described in Example 7 can also be applied to the case where the examination of an urgent patient or the like is interrupted. It also becomes possible to replace with each other the orders of the examinations of two patients in the same examination room or to replace with other the examinations of two patients in different examination rooms, by using the rescheduling processing described in Example 7. Fig. 41 illustrates an examination schedule in which examinations are closely assigned, but when there is sufficient free time, for example, in the examination room 20, it is also possible to perform the reassignment processing of an endoscope by interrupting an examination into the free time.

Example 7 has described the change-disallowed examination specification unit 174 that specifies an examination in which the change of an element is made disallowed, but according to an idea opposite to the change-disallowed examination specification unit 174, a change-allowed examination specification unit, which specifies an examination in which the change of an element is made allowed, may be provided. In this case, a user designates an examination, in which the change of an element is made allowed, by operating the input interface, and with respect to an examination specified by the change-allowed examination specification unit, the first assignment processing unit 120 may change an element, such as an endoscope or a primary doctor.

Further, it has been described that an examination, in which the change of a schedule element is made disallowed, is specified by the change-disallowed examination specification unit 174 and the rescheduling processing unit 172 notifies the examination schedule management unit 110 of the schedule element, the change of which is made disallowed; however, there is the possibility that when the examination schedule management unit 110 changes elements of the examination schedules, the changes of which are not made disallowed, the endoscopes 30 to be assigned may run short, so that a situation where a schedule is greatly delayed, or the like may occur.

Therefore, the examination schedule management unit 110 holds a predetermined rescheduling condition with respect to the rescheduling, and notifies a user that an examination schedule cannot be changed when this rescheduling condition is not satisfied. The rescheduling condition may be set such that a delay of the start of an examination falls within a predetermined time (e.g., one hour). When there is a schedule element, the change of which is made disallowed by the change-disallowed examination specification unit 174, and when the first assignment processing unit 120 assigns the endoscope 30 or a doctor, a restriction is also placed on the flexibility of the assignment, because there is a schedule element, the change of which is prohibited. Therefore, when the examination schedule management unit 110 causes the first assignment processing unit 120 to execute the schedule element reassignment processing in order to change an element of the examination schedule, and when the predetermined rescheduling condition is not satisfied, it is preferable to notify a user that the examination schedule cannot be changed.

Even if the rescheduling condition is satisfied and an examination schedule can be changed, but when the time frame of an examination was adjusted, it is preferable for the examination schedule management unit 110 to notify a user that the time frame thereof was adjusted. As a result, a user, such as a doctor, can recognize that the end of an examination is delayed than scheduled, and it becomes possible to take action, such as to review the schedule of himself/herself after the end of the examination.

In Example 7, the examination schedule and cleaning schedule illustrated in Fig. 50 are reconstructed by the rescheduling processing. The result of this reconstruction is one example, and as described with respect, for example, to the embodiment and Examples 1 to 6, plural types of examination schedules and cleaning schedules can also be generated by various scheduling algorithms proposed in the present application. Therefore, the display processing unit 150 may display the generated plural types of examination schedules and cleaning schedules on the display of the terminal device 12 such that a user can select which one to adopt.

### <Example 8>

Examples 6 and 7 have described the case where an example cannot be performed as scheduled and the time frame of an examination is adjusted, but in Example 8, a case is assumed where an examination schedule is reconstructed in a way in which because an examination is canceled, other examinations are made earlier than scheduled.

When an examination is canceled, it is preferable to effectively utilize resources such as the examination room 20, the endoscope 30, and the doctor that were scheduled to be used in the canceled examination. Therefore, in Example 8, it is aimed to achieve effective utilization of the resources and shortening of examination waiting time of a patient by executing rescheduling processing when an examination is canceled. Also in Example 8, it is premised that the examination schedule and the cleaning schedule, which are illustrated in Fig. 41, are generated in advance before the start of examination work, similarly to Examples 6 and 7.

With reference to Fig. 41, a case is assumed where cancellation of the examination E22 is notified to the information management device 10 before the scheduled examination start time (10:25) of the examination E22. When a user inputs a cancel operation for the examination E22 from the input interface connected to the terminal device 12, the input reception unit 176 receives the cancel operation for the examination E22. The situation information acquisition unit 170 acquires, from the input reception unit 176, reservation cancellation information on the examination E22 as the situation information on the situation of an examination, and provides it to the rescheduling processing unit 172.

The rescheduling processing unit 172 determines based on the situation information that it is necessary to change the examination schedule and/or the cleaning schedule, and instructs at least one of the examination schedule management unit 110 and the cleaning schedule management unit 130 to change an element included in the examination schedule and/or the cleaning schedule. In Example 8, the examination schedule management unit 110 deletes, in response to this change instruction, the schedule information on the examination E22 from the examination schedule.

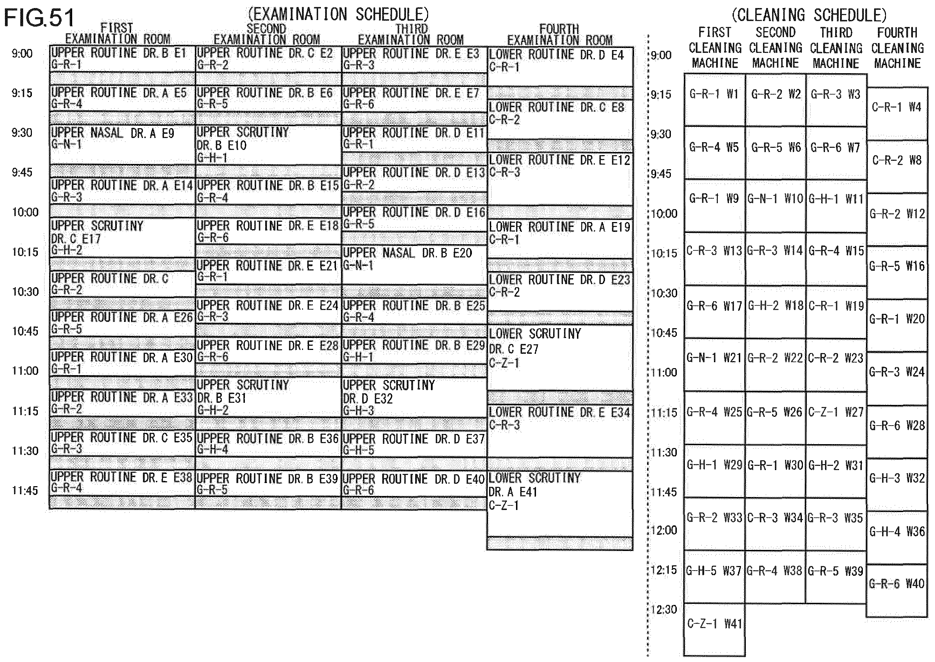

Fig. 51 illustrates a state where the examination E22 is deleted from the examination schedule. In Example 8, rescheduling processing is proposed in which in order to effectively utilize the resources of the examination canceled from the examination schedule, examinations, the scheduled start times of which are later than the scheduled start time of the canceled examination, can be performed ahead of schedule.

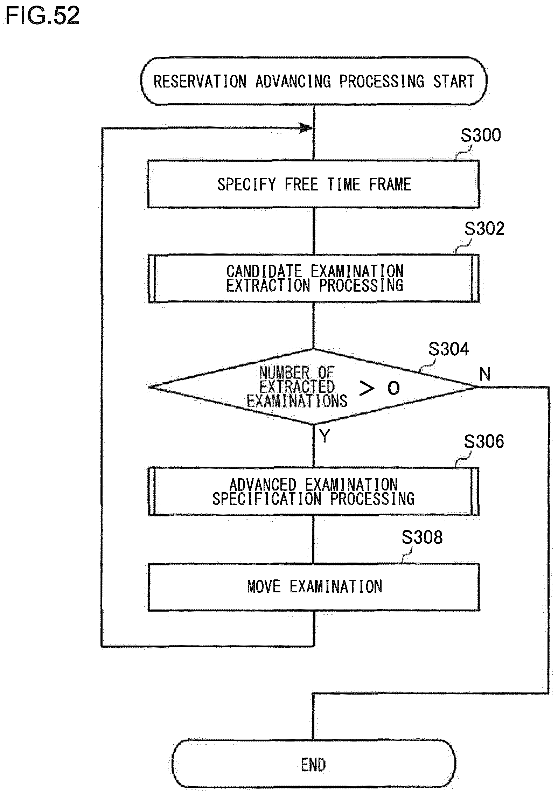

Fig. 52 illustrates a flowchart of the reservation advancing processing in Example 8. The examination schedule management unit 110 specifies the time frame of the canceled examination as a free time frame (S300), and performs processing for extracting candidate examinations to be moved to the free time frame (S302). If one or more examinations, which can be moved to the free time frame, are extracted by the candidate examination extraction processing (S304/Y), the examination schedule management unit 110 performs processing for specifying an examination to be moved (S306). When the examination to be moved is specified, it is moved to the free time frame (S308). The resources of the time frame of the canceled examination can be utilized for another examination by this movement processing. In this reservation advancing processing, the time frame of the moved examination is further specified as a free time frame (S300), and processing for assigning another examination to the free time frame is repeated. If an examination, which can be moved to the free time frame, cannot be extracted by the candidate examination extraction processing (S304/N), the reservation advancing processing ends.

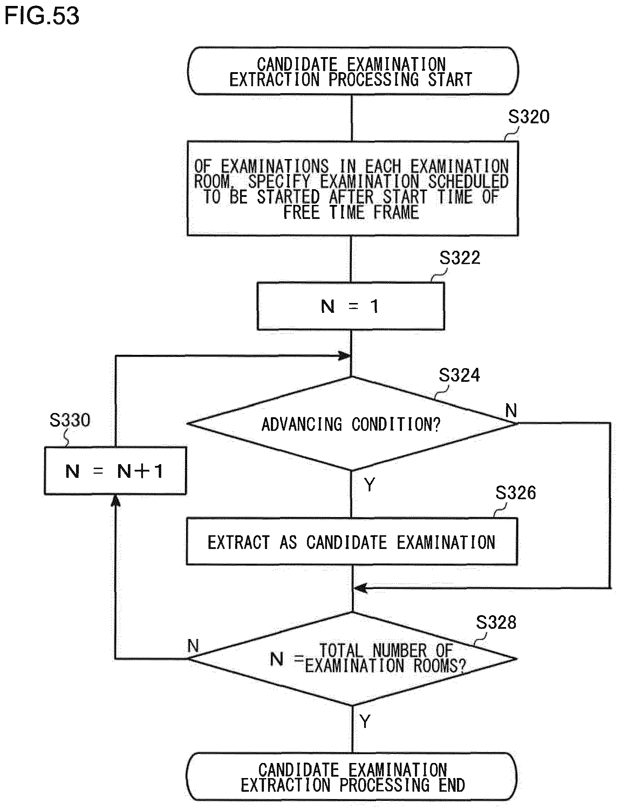

Fig. 53 illustrates a detailed flowchart of the candidate examination extraction processing indicated of S302. The examination schedule management unit 110 specifies, of the examinations in each examination room 20 in the examination schedule, an examination scheduled to be started after the start time of the free time frame specified in S300 (S320). In advancing an examination reservation, it is often not realistic to perform ahead of schedule an examination that is scheduled, for example, 2 hours later, because a pretreatment may not be in time. Therefore, the examination schedule management unit 110 specifies, of the examinations in each examination room 20, an examination scheduled to be started earliest after the start time of the free time frame.

Subsequently, the examination schedule management unit 110 sets "N=1" (S322), and determines whether the examination specified in the N-th examination room satisfies predetermined advancing conditions (S324). Herein, one of the advancing conditions is set such that the examination type of the examination scheduled in the free time frame coincides with that of the examination specified in the N-th examination room. If both the examination types coincide with each other (S324/Y), the scheduled examination times are equal to each other and the models of the endoscopes 30 to be used are the same as each other, and hence the examination specified in the N-th examination room can be moved to the free time frame, without changing the schedule elements set in the free time frame, such as the endoscope 30. Conditions with respect to the skill of a doctor may be set as another advancing condition. For example, if the examination to be moved to the free time frame requires a special skill, an inexperienced young doctor may not deal with that. Therefore, when the skill of a primary doctor set in the free time frame is compared with the skill required for the examination to be moved and the skill of the primary doctor can sufficiently deal with (S324/Y), it is determined that the examination can be moved to the free time frame. When the above advancing conditions are not satisfied (S324/N), the processing proceeds to S328.

If the predetermined advancing conditions are satisfied (S324/Y), the examination schedule management unit 110 extracts the examination as a candidate examination that can be moved to the free time frame (S326). Subsequently, it is determined whether N is equal to the total number of examination rooms (in this example, the total number thereof=4) (S328), and when N does not reach the total number of examination rooms (S328/N), N is incremented by 1 (S330) and the processing returns to S324.

As described above, the candidate examination extraction processing of S302 is performed until N reaches the total number of examination rooms (S328/Y), that is, executed on all the examinations extracted from each examination room 20.

Returning to Fig. 52, the examination schedule management unit 110 determines whether there is the extracted candidate examination (S304). Herein, if the candidate examination is not extracted (S304/N), the reservation advancing processing ends. On the other hand, if one or more candidate examinations are extracted (S304/Y), the examination schedule management unit 110 performs processing for specifying an examination to be moved to the free time frame (S306).

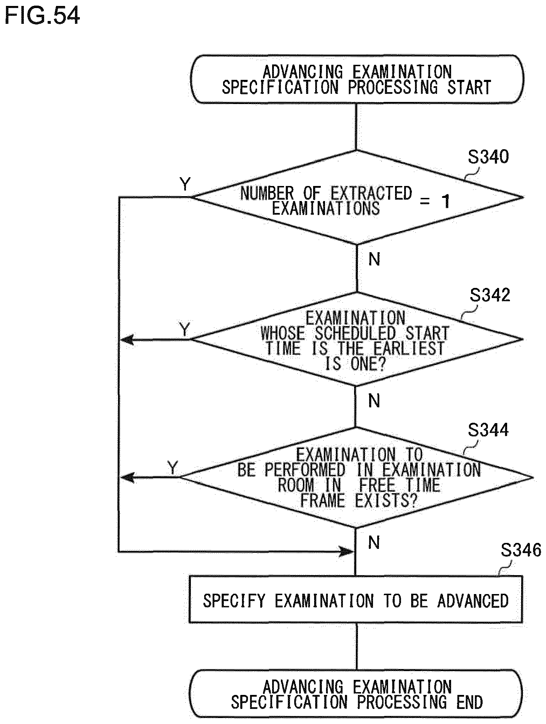

Fig. 54 illustrates a detailed flowchart of the advancing examination specification processing of S306. When there is one examination extracted in S304 (S340/Y), the examination schedule management unit 110 specifies the one examination as the examination to be moved to the free time frame (S346). When there are a plurality of examinations extracted in S304 (S340/N), and when there is one examination whose scheduled start time is earliest (S342/Y), the examination schedule management unit 110 specifies the examination whose scheduled start time is earliest as the examination to be moved to the free time frame (S346). When there are a plurality of examinations whose scheduled start times are earliest (S342/N), and when, of the examinations, there is an examination scheduled in the same examination room as the examination room of the free time frame (S344/Y), the examination schedule management unit 110 specifies the examination scheduled in the same examination room as the examination to be moved to the free time frame (S346). If there is no examination scheduled in the same examination room (S344/N), the examination schedule management unit 110 may specify, for example, an examination in an examination room with smaller room No. as the examination to be moved to the free time frame (S346).

Returning to Fig. 52, the examination schedule management unit 110 moves the examination specified in S306 to the free time frame (S308). The flow returns to S300, and the examination schedule management unit 110 specifies the time frame of the moved examination as a free time frame (S300), so that the processing of S302 to S308 are repeated. These processing are repeated until a candidate examination is no longer extracted in S302.

Hereinafter, specific processing to be performed when the examination E22 is canceled will be described. When the input receiving unit 176 receives a cancel operation for the examination E22, the situation information acquisition unit 170 acquires, from the input receiving unit 176, reservation cancellation information on the examination E22 as the situation information on the situation of an examination, and provides it to the rescheduling processing unit 172.

The rescheduling processing unit 172 determines based on the situation information that it is necessary to change the examination schedule and/or the cleaning schedule, and instructs at least one of the examination schedule management unit 110 and the cleaning schedule management unit 130 to change an element included in the examination schedule and/or the cleaning schedule. In Example 8, the examination schedule management unit 110 deletes, in response to this change instruction, the examination E22 from the examination the schedule. The examination schedule management unit 110 deletes the examination ID of the examination E22 from the examination schedule, and leaves other schedule information, that is, the primary doctor, the endoscope ID, the information on scheduled examination start time, and that on scheduled examination end time, which are assigned to the examination E22. As a result, the time frame, to which the examination E22 has been assigned, becomes free, and the examination schedule management unit 110 specifies the time frame between 10:25 and 10:35 in the first examination room 20a as a free time frame (S300) .

Subsequently, the examination schedule management unit 110 extracts candidate examinations to be moved to the free time frame (S302). With reference to Fig. 53, the examination schedule management unit 110 specifies, for every examination room, an examination scheduled to be started after the start time of the free time frame (S320). The examinations specified herein are the examinations E26, E24, E25, and E27.

Hereinafter, it is assumed that the advancing condition to be used in S324 is related to an examination type, that is, it is the same as the examination type of the examination set in the free time frame. First, the examination type of the examination E26 in the first examination room 20a is the same as that of the canceled examination E22 (upper routine examination) (S324/Y), and therefore the examination schedule management unit 110 extracts the examination E26 as a candidate examination (S326). Similarly, the examination types of the examination E24 in the second examination room 20b and the examination E25 in the third examination room 20c are also the same as that of the examination E22 (S324/Y), and therefore the examination schedule management unit 110 also extracts the examinations E24 and E25 as candidate examinations (S326). On the other hand, the examination type of the examination E27 in the fourth examination room 20d is different from that of the examination E22 (S324/N), and hence the examination E27 is not extracted as a candidate examination. Thereby, the candidate examinations are extracted as the examinations E26, E24, and E25.

Returning to Fig. 52, the number of the extracted candidate examinations is three (S304/Y), and hence the examination schedule management unit 110 executes the advanced examination specification processing illustrated in Fig. 54. Herein, the number of the extracted examinations is three (S340/N), and the scheduled start time of the examination E26 is 10:40, and those of the examinations E24 and E25 are 10:35, respectively. Therefore, there are two examinations E24 and E25 as the examination whose scheduled start time is earliest (S342/N). Because the examinations E24 and E25 are performed in examination rooms different from the examination room that is the same as the free time frame (i.e., first examination room 20a) (S344/N), the examination schedule management unit 110 specifies the examination E24 with smaller examination room No. as the examination to be moved (S346). Returning to Fig. 52, the examination schedule management unit 110 moves the specified examination E24 to the free time frame (S308). Specifically, the examination schedule management unit 110 determines that the examination E24 is to be performed in the free time frame, and thereby it is defined as the schedule information on the examination E24 that the primary doctor C uses the endoscope G-R-2 in the first examination room 20a between 10:25 and 10:35.

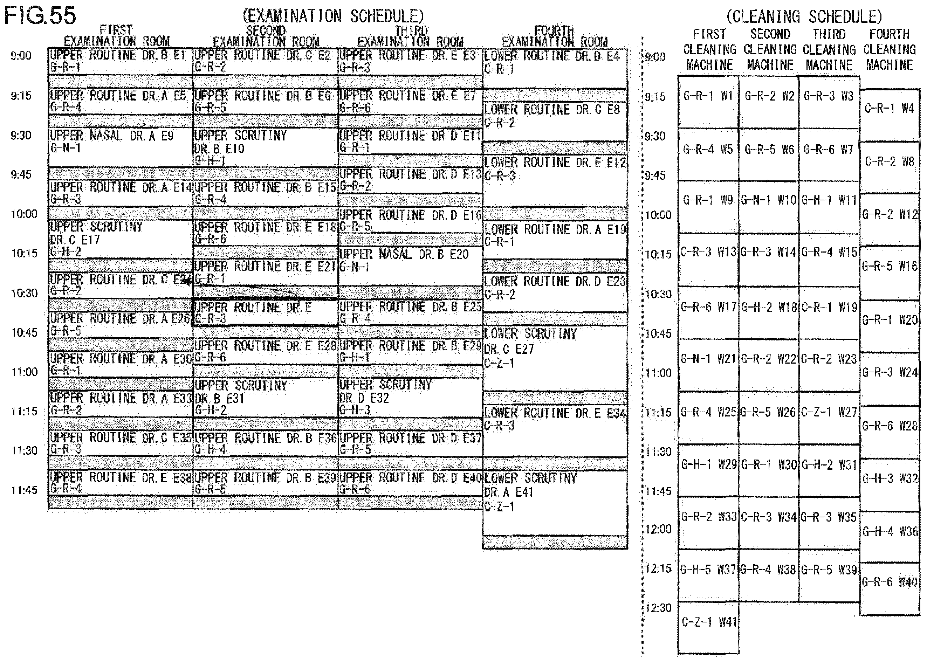

Fig. 55 illustrates a state where the examination E24 is moved to the free time frame. Subsequently, the examination schedule management unit 110 specifies, as a free time frame, the time frame between 10:35 and 10: 45 in the second examination room 20b assigned to the examination E24 before moved (S300), and extracts a candidate examination to be moved to the free time frame (S302). With reference to Fig. 53, the examination schedule management unit 110 specifies, for every examination room, an examination scheduled to be started after the start time of the free time frame (S320). The examinations specified herein are the examinations E26, E28, E29, and E27.

Of the above examinations, the examination E27 does not satisfy the advancing condition (S324/N), but the examinations E26, E28, and E29 satisfy the advancing condition (S324/Y), and hence the examination schedule management unit 110 extracts the examinations E26, E28, and E29 as candidate examinations (S326).

Herein, with reference to Fig. 54, the number of the extracted examinations is three (S340/N), and the scheduled start time of the examination E26 is 10:40, and those of the examinations E28 and E29 are 10:50, respectively. In this case, the examination whose scheduled start time is earliest is one examination E26 (S342/Y), and hence the examination schedule management unit 110 specifies the examination E26 as the examination to be moved (S346). Thereby, with reference to Fig. 52, the examination schedule management unit 110 moves the specified examination E26 to the free time frame (S308).

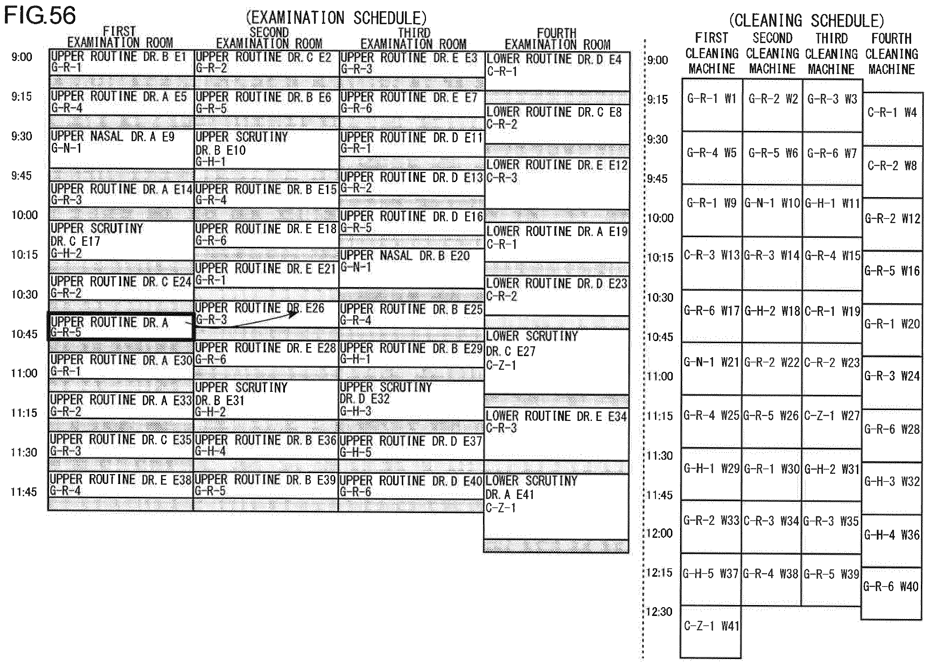

Fig. 56 illustrates a state where the examination E26 is moved to the free time frame. Subsequently, the examination schedule management unit 110 specifies, as a free time frame, the time frame between 10:40 and 10:50 in the first examination room 20a assigned to the examination E26 before moved (S300), and extracts a candidate examination to be moved to the free time frame (S302). With reference to Fig. 53, the examination schedule management unit 110 specifies, for every examination room, an examination scheduled to be started after the start time of the free time frame (S320). The examinations specified herein are the examinations E30, E28, E29, and E27.

Of the above examinations, the examination E27 does not satisfy the advancing condition (S324/N), but the examinations E30, E28, and E29 satisfy the advancing condition (S324/Y), and hence the examination schedule management unit 110 extracts the examination E30, E28, and E29 as candidate examinations.

Herein, with reference to Fig. 54, the number of the extracted examinations is three (S340/N), and the scheduled start time of the examination E30 is 10:55, and those of the examinations E28 and E29 are 10:50, respectively. In this case, there are two examinations E28 and E29 as the examination whose scheduled start time is earliest (S342/N), and the examinations E28 and E29 are performed in examination rooms different from the examination room that is the same as the free time frame (i.e., first examination room 20a) (S344/N), and hence the examination schedule management unit 110 specifies the examination E28 with smaller examination room No. as the examination to be moved (S346). Returning to Fig. 52, the examination schedule management unit 110 moves the specified examination E28 to the free time frame (S308).

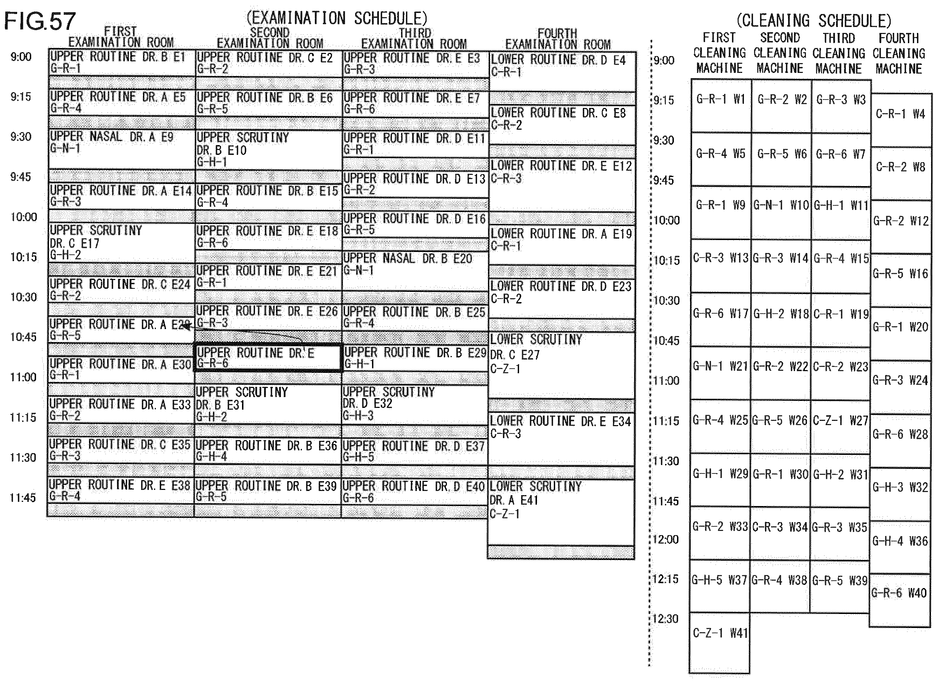

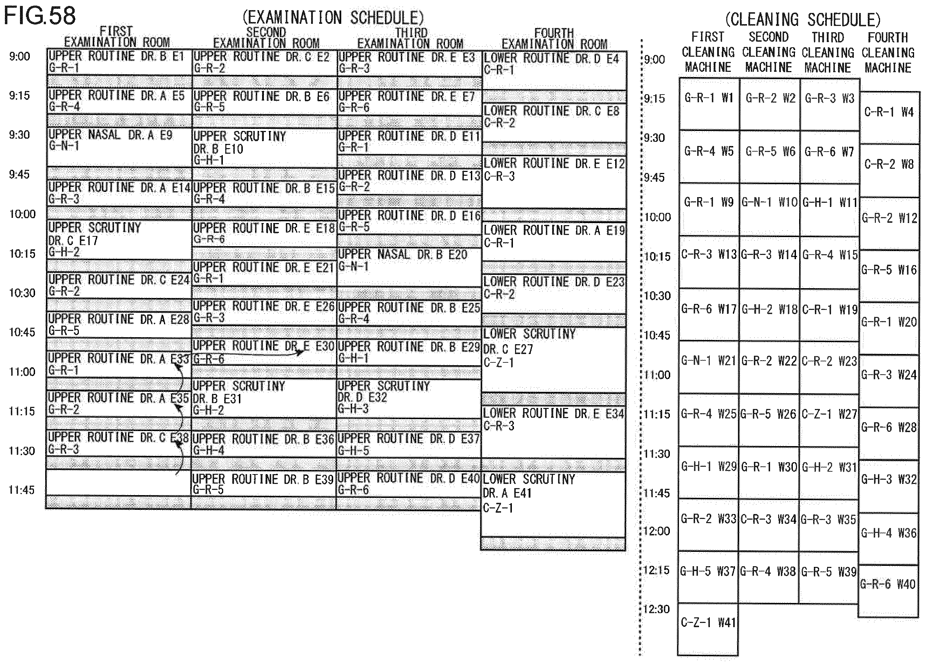

Fig. 57 illustrates a state where the examination E28 is moved to the free time frame. The examination reservation advancing processing is completed by repeating the above processing. Fig. 58 illustrates the reconstructed examination schedule. When an examination reservation is canceled, an efficient examination schedule can be reconstructed by advancing an examination of the same type scheduled later than the canceled examination, as described in Example 8. By advancing an examination of the same type, the resources, such as the assigned endoscope 30, can be used as they are, without having to change the resources, and therefore in terms of a primary doctor, there is almost no change in the schedule itself of the doctor although there is a change in an examination (patient), whereby examination work can be progressed.

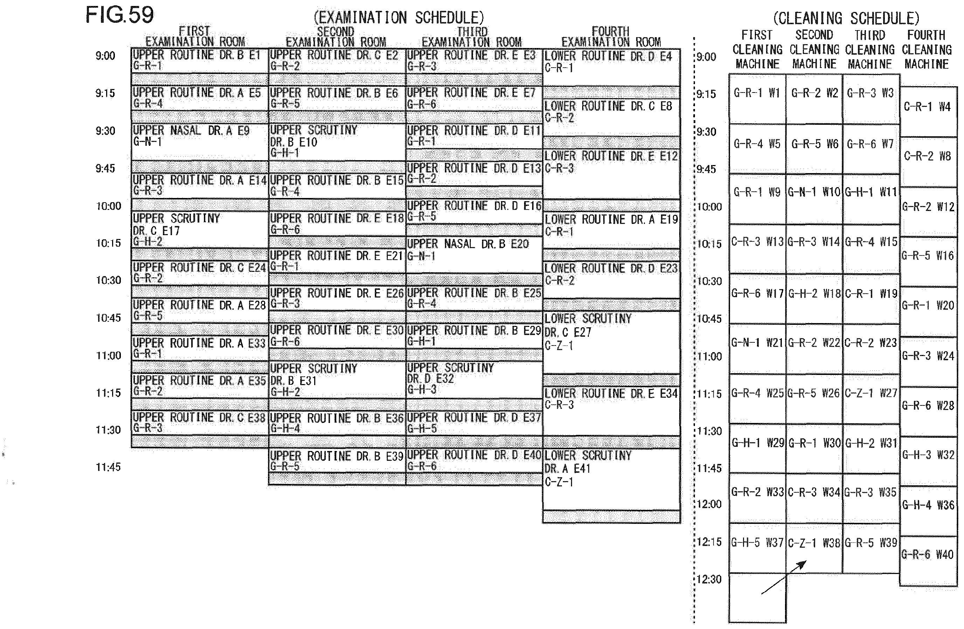

In response to the fact that the use schedule of the endoscope G-R-4, which was scheduled to be used in the examination E38 in the original examination schedule, was canceled, the cleaning schedule management unit 130 cancels the cleaning schedule of the endoscope G-R-4 in the cleaning W38. With this cancellation, the cleaning schedule management unit 130 determines that the endoscope C-Z-1, which is scheduled in the cleaning W41, can be advanced to the time frame of the cleaning W38, and changes the cleaning schedule. Fig. 59 illustrates the reconstructed cleaning schedule.

The rescheduling processing of the present invention has been described above based on Examples 6 to 8. These examples are illustrative in nature, and it should be appreciated by a person skilled in the art that various modifications can be made to the combinations of the components and the processing processes and such modifications also fall within the endoscope of the invention.

Examples 6 to 8 have described that the examination schedule and the cleaning schedule are reconstructed by the rescheduling processing. In the invention of the present application, plural types of candidates for the examination schedule and the cleaning schedule can also be generated by the proposed various rescheduling algorithms. Therefore, the display processing unit 150 has the function of displaying the changed examination schedule and/or cleaning schedule on the display of the terminal device 12, and when there are a plurality of candidates for the examination schedule and/or the cleaning schedule, each candidate may be displayed on the display such that a user can select any one of the candidates. When a user selects any one of the candidates, the examination schedule management unit 110 and the cleaning schedule management unit 130 fix the changes of the selected examination schedule and/or cleaning schedule.

The above embodiment and examples have described, for example, the doctor assignment unit 129 that assigns a doctor to an examination and the person-in-charge assignment unit 149 that assigns a person-in-charge of cleaning work. For example, an auxiliary work practitioner assignment unit, which assigns an auxiliary work practitioner who assists an examination to an examination, may be further provided in the information management device 10.

### <Rescheduling Processing 2>

With the processing described with respect to the embodiment and Examples 1 to 5, an examination schedule and a cleaning schedule are generated before the start of endoscopic examination work for one day. In a medical facility, it is ideal that the examination work proceeds according to the generated examination schedule and cleaning schedule, however, at a work site, the work may not proceed as scheduled in the examination schedule and/or the cleaning schedule due to various factors.

One of the typical factors is that a nurse erroneously prepares an endoscope 30 different from the scheduled one and an examination is started as it is. In the examination schedule, the endoscope 30 scheduled to be used is assigned to an examination, and a nurse originally has to prepare the scheduled endoscope 30 by confirming the examination schedule. At a busy work site, however, an endoscope 30 different from the scheduled one may be prepared due to a mistake in vision on an examination schedule table or on the print tape attached to the endoscope 30, and an examination may be started without noticing the mistake.

Also, even if a mistake is noticed before the start of an examination, it takes a certain amount of time to take the endoscope 30 by going into the cleaning room 40 or the like, and hence when it is intended to start an examination after repreparing the endoscope 30, the start of the examination may be delayed from the scheduled time. In such a case, it is considered that examination work can be efficiently progressed by rescheduling the assignment of the endoscope 30 to the examination, by giving priority to the start of an examination.

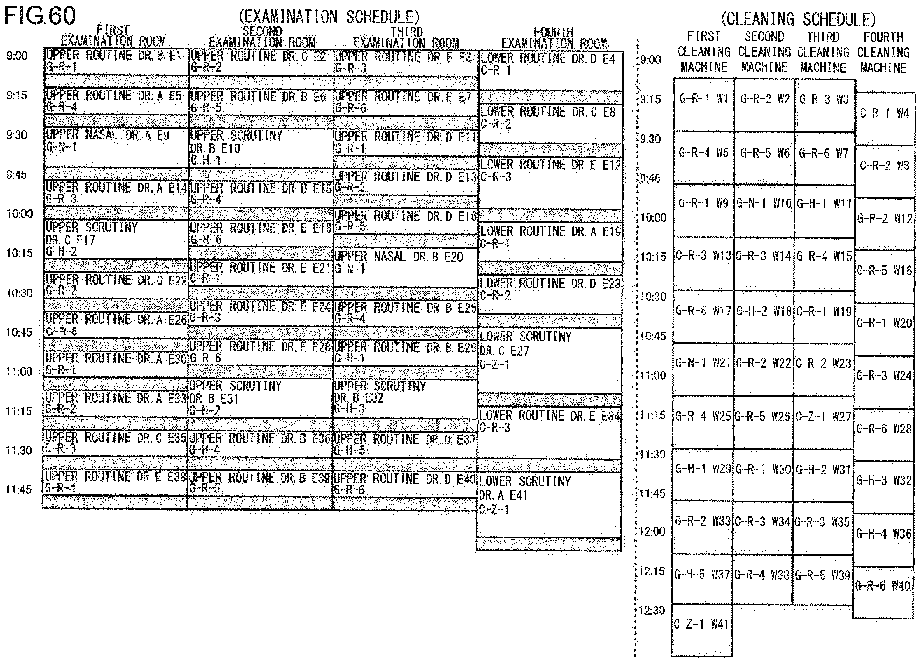

Fig. 60 illustrates examples of an examination schedule generated by the examination schedule management unit 110 and a cleaning schedule generated by the cleaning schedule management unit 130. The examination schedule illustrated in Fig. 60 is generated by assigning an endoscope to each examination by the endoscope assignment processing described with respect to Example 1 and according to the endoscope order table. The cleaning schedule illustrated in Fig. 60 is generated by the cleaning machine assignment processing described with respect to the embodiment. In the rescheduling processing described in the following Examples 9 to 11, the elements included in the examination schedule and the cleaning schedule, which are illustrated in Fig. 60, are subjected to the rescheduling processing, but this is only one example and the elements included in the examination schedule and cleaning schedule illustrated in Fig. 20 may be subjected to. In either case, it is premised in the rescheduling processing that an examination schedule and a cleaning schedule are generated in advance. For convenience of description, cleaning Nos. of W1 to W41 are attached in the cleaning schedule in order to specify respective cleaning processing.

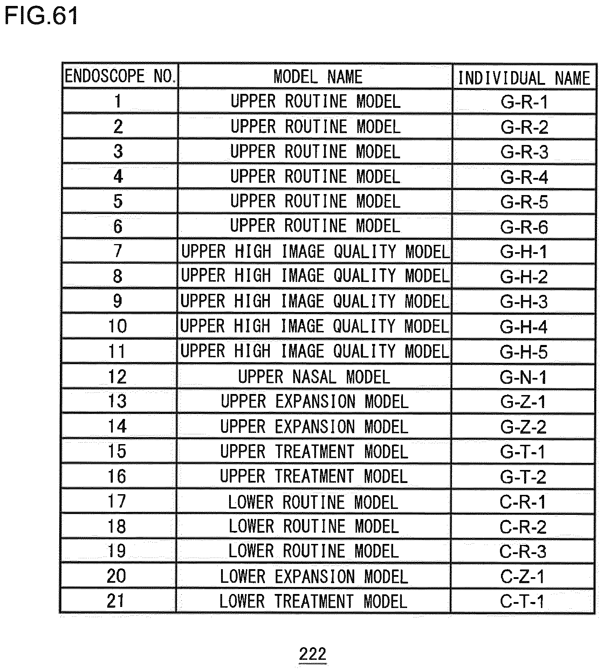

Also, in Fig. 60, it is premised that a medical facility possesses five upper high image quality models. Fig. 61 illustrates one example of the possessed endoscope master table 222. The possessed endoscope master table 222 is different from that illustrated in Fig. 6 in that a medical facility possesses five upper high image quality models. That is, the possessed endoscope master table 222 illustrated in Fig. 61 is different from that illustrated in Fig. 6 in that two upper high image quality models G-H-4 and G-H-5 are added, and in the examination schedule in Fig. 60, the endoscope G-H-4 is assigned to the examination E36 and the endoscope G-H-5 to the examination E37, respectively.

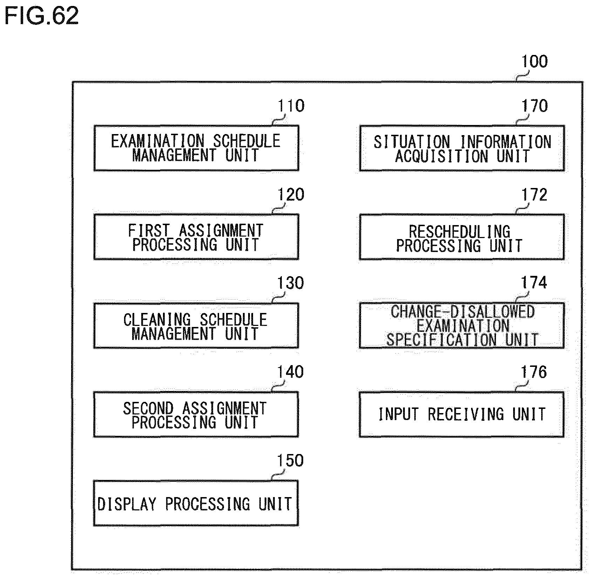

Fig. 62 illustrates, of the configuration of the information management device 10, the configuration of the processing unit 100 having the function of executing rescheduling processing. The processing unit 100 includes the examination schedule management unit 110, the first assignment processing unit 120, the cleaning schedule management unit 130, the second assignment processing unit 140, the display processing unit 150, the situation information acquisition unit 170, the rescheduling processing unit 172, the change-disallowed examination specification unit 174, and the input receiving unit 176. Although not illustrated in Fig. 62, the processing unit 100 is configured to also include the display content derivation unit 152, the period designation unit 154, and the usage condition monitoring unit 160, as illustrated in Fig. 3; the first assignment processing unit 120 is configured to include the examination extraction unit 122, the endoscope specification unit 124, the endoscope assignment unit 126, the endoscope assignment availability confirmation unit 128, and the doctor assignment unit 129; and the second assignment processing unit 140 is configured to include the cleaning machine specification unit 142, the cleaning machine assignment unit 144, the end time determination unit 146, the cleaning machine assignment availability confirmation unit 148, and the person-in-charge assignment unit 149.

Each component of the processing unit 100 can be realized by a CPU, memory, or other LSIs of an arbitrary computer in terms of hardware, and realized by a program or the like loaded in a memory in terms of software, but herein functional blocks realized by the cooperation of hardware and software are depicted. Therefore, it is to be understood by those skilled in the art that these functional blocks can be realized in various forms, namely, solely in hardware, solely in software, or through a combination of hardware and software. The following rescheduling processing is performed in accordance with the actual situation of an endoscope after the start of endoscopic examination work.

### <Example 9>

The situation information acquisition unit 170 acquires situation information on the situation of an endoscope. The situation information acquisition unit 170 may have a communication unit for communicating with the endoscopic observation device 22 so as to have the function of acquiring the information transmitted from the endoscopic observation device 22. In this respect, the situation information acquisition unit 170 may include the function of the usage condition monitoring unit 160 illustrated in Fig. 3.

The endoscope 30 is connected to the endoscopic observation device 22 before the start of an examination, and at the time the identification information on the endoscope 30 (endoscope ID) is transmitted to the information management device 10 via the network 2 along with information for identifying the endoscopic observation device 22 (observation device ID) and information for identifying an examination (examination ID). When an examination start button is operated in the endoscopic observation device 22 to start an examination, an examination start notice is transmitted to the information management device 10 along with the examination ID and the observation device ID. The endoscope ID may be transmitted to the information management device 10 along with the examination start notice.

When an examination end button is operated in the endoscopic observation device 22 to end an examination, an examination end notice is transmitted to the information management device 10 along with the examination ID and the observation device ID. Also, when an endoscope is exchanged during an examination and another endoscope is connected to the endoscopic observation device 22, the endoscope ID of the newly connected endoscope 30 is transmitted to the information management device 10 along with the examination ID and the observation device ID.

In the information management device 10, the situation information acquisition unit 170: monitors the information transmitted from the endoscopic observation device 22; acquires the endoscope ID specifying the endoscope 30 to be used as the situation information indicating the use of the endoscope; and acquires examination start notice information and examination end notice information as the situation information on the implementation situation of an examination. The situation information acquisition unit 170 immediately (in real time) provides the acquired situation information to the rescheduling processing unit 172 along with the acquired time information. Alternatively, the situation information acquisition unit 170 may periodically provide the acquired situation information to the rescheduling processing unit 172 along with the acquired time information.

Alternatively, a means for reading the endoscope ID of an endoscope 30 may be provided in the endoscopic observation device 22 such that a nurse is caused to read the endoscope ID thereof with the reading means before the start of an examination. For example, an RFID tag or the like is built in the endoscope 30, and a tag reading means provided in the endoscope observation device 22 acquires the endoscope ID in the RFID tag. The endoscopic observation device 22 transmits the acquired endoscope ID to the information management device 10 along with the examination ID and the observation device ID. Alternatively, the endoscope ID may be input from a terminal device in the examination room 20 by a nurse so as to be transmitted to the information management device 10.

The situation information acquisition unit 170 only has to be able to acquire the situation information on the situations of an endoscope and an examination by some means, but in order to execute efficient rescheduling processing, it is preferable to acquire the situation information in real time and to immediately provide it to the rescheduling processing unit 172 along with the acquired time information.

The rescheduling processing unit 172 determines based on the situation information whether it is necessary to change an element included in the examination schedule and/or the cleaning schedule, which are illustrated in Fig. 60. For example, at the time when the situation information acquisition unit 170 receives the examination start notice information, if the endoscope ID acquired in advance for an examination is different from the endoscope ID in the examination schedule that is assigned to the same examination by the endoscope assignment unit 126, the rescheduling processing unit 172 determines that it is necessary to change the examination schedule and/or the cleaning schedule.

When determining based on the situation information that it is necessary to change the examination schedule and/or the cleaning schedule, the rescheduling processing unit 172 instructs at least one of the examination schedule management unit 110 and the cleaning schedule management unit 130 to change an element included in the examination schedule and/or the cleaning schedule. Herein, the element included in the examination schedule is an examination specified by an examination ID, an examination room assigned an the examination, information on scheduled examination start time, that on scheduled examination end time, a primary doctor, or identification information on an endoscope 30 (endoscope ID); and the element included in the cleaning schedule is a cleaning machine assigned to an endoscope 30, information on scheduled cleaning start time, or that on scheduled cleaning end time. When a person-in-charge is assigned to cleaning processing in the cleaning schedule, as illustrated in Example 5 (Fig. 38), person-in-charge information is also included in the element included in the cleaning schedule.

An endoscope ID for identifying an endoscope is added to the endoscope 30, and this endoscope ID is a serial number formed of numerals and alphabets, and is different from an individual name such as G-R-1 or G-R-2. However, this individual name is used in the present specification as a name for uniquely specifying the endoscope 30, and hereinafter description will be made, assuming, for the sake of convenience, that individual names, such as G-R-1 and G-R-2, are endoscope IDs for identifying endoscopes.

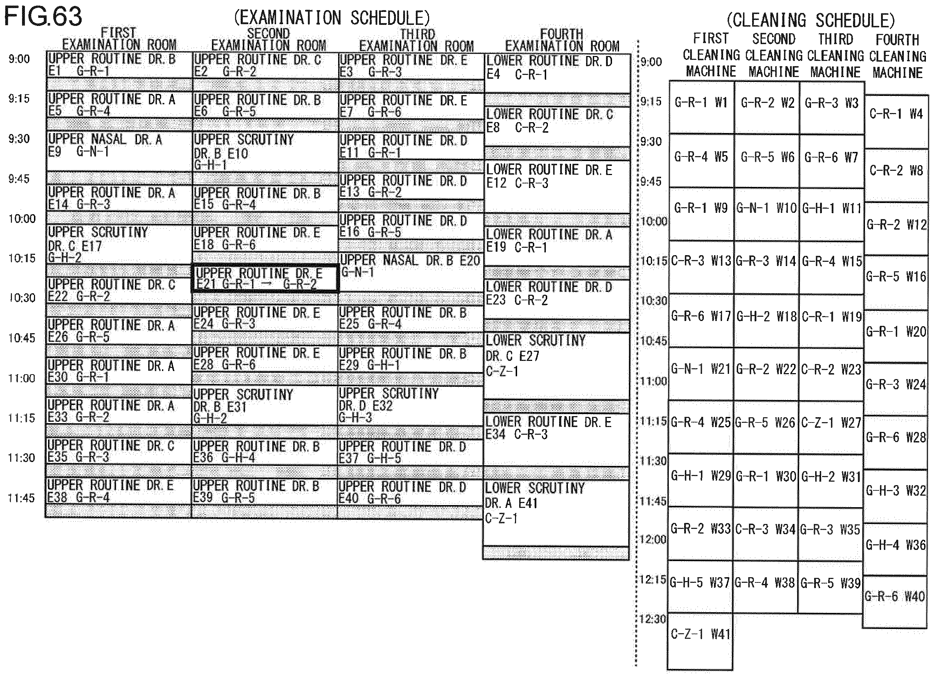

Hereinafter, specific rescheduling processing in Example 9 will be described. Fig. 63 illustrates an example in which an endoscope different from the scheduled one is used in the actual examination. Although the endoscope G-R-1 has been assigned to the examination E21 in the examination schedule, the examination E21 is started in the second examination room 20b, in which the unscheduled endoscope G-R-2 is connected to the endoscopic observation device 22b. The examination E21 is assumed to be started at 10:20 as scheduled.

A nurse or the like brings into the second examination room 20b the endoscope 30 during a preparation period (10:15 to 10:20) of the examination E21, and connects to the endoscopic observation device 22b. When the endoscope 30 is connected, the endoscopic observation device 22b transmits the endoscope ID, the examination ID, and the observation device ID to the information management device 10. In the information management device 10, the situation information acquisition unit 170 acquires the endoscope ID, the examination ID, and the observation device ID as the situation information on the situation of the endoscope. The situation information acquisition unit 170 provides the acquired situation information to the rescheduling processing unit 172.

When the scheduled start time (10:20) of the examination is reached, the examination start button is operated, and an examination start notice is transmitted to the information management device 10 along with the examination ID and the observation device ID. The situation information acquisition unit 170 acquires the examination start notice information, the examination ID, and the observation device ID as the situation information on the implementation situation of the examination. The situation information acquisition unit 170 provides the acquired situation information to the rescheduling processing unit 172.

Alternatively, the endoscopic observation device 22 may transmit the endoscope ID to the information management device 10 along with the examination start notice information, the examination ID, and the observation device ID when the examination start button is operated, as described above. In this case, the situation information acquisition unit 170 acquires the endoscope ID and the examination start notice information at the same timing.

When receiving the examination start notice information from the situation information acquisition unit 170, the rescheduling processing unit 172 determines whether the endoscope ID of the endoscope 30 actually used in the examination E21 coincides with the endoscope ID assigned to the examination E21 in the examination schedule. At this time, the endoscope ID acquired by the situation information acquisition unit 170 is "G-R-2", while the endoscope ID assigned to the examination E21 is "G-R-1." Therefore, the rescheduling processing unit 172 detects that the endoscope in use is different from the scheduled endoscope, and determines that it is necessary to change the examination schedule and/or the cleaning schedule.

When determining that it is necessary to change a schedule, the rescheduling processing unit 172 instructs at least one of the examination schedule management unit 110 and the cleaning schedule management units 130 to change an element included in the schedule. In Example 9, the rescheduling processing unit 172 instructs the cleaning schedule management unit 130 to change an element included in the cleaning schedule, and the cleaning schedule management unit 130 changes the cleaning W20 of the endoscope G-R-1 scheduled to be used in the examination E21 to the cleaning of the endoscope G-R-2. Further, the rescheduling processing unit 172 instructs the examination schedule management unit 110 to change an element included in the examination schedule, and the examination schedule management unit 110 changes, as an element in the examination schedule, the endoscope assigned to the examination E21 to the endoscope G-R-2, and investigates whether endoscopes can be used as scheduled in the examinations that are to be started after the start time of the examination E21. The endoscope G-R-2 is actually in use in the examination E21, and it becomes possible to investigate the subsequent schedule elements by registering the endoscope G-R-2 as an schedule element.

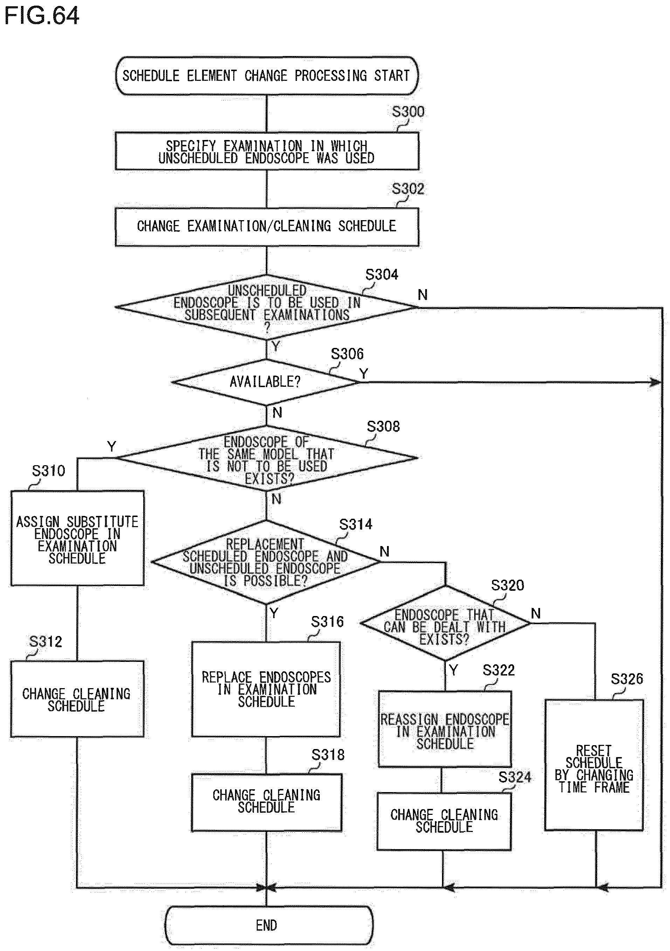

Fig. 64 illustrates a flowchart of the schedule element change processing in Example 9. Hereinafter, in an examination in which the endoscope 30 is erroneously used, the endoscope scheduled to be used in the examination is referred to as a "scheduled endoscope", and the endoscope erroneously used to an "unscheduled endoscope." When the endoscope G-R-2 is used in the examination E21, as described above, the endoscope G-R-1 is a scheduled endoscope and the endoscope G-R-2 is an unscheduled endoscope.

The rescheduling processing unit 172 compares the endoscope ID acquired by the situation information acquisition unit 170 with the ID of the endoscope scheduled to be used in the examination schedule, and when both the endoscope IDs are different from each other, the rescheduling processing unit 172 specifies an examination in which an unscheduled endoscope is used (S300), and determines that it is necessary to change the examination schedule and/or the cleaning schedule. Herein, when the endoscope G-R-2 is used in the examination E21, the rescheduling processing unit 172 recognizes that the unscheduled endoscope G-R-2 is used although the scheduled endoscope is G-R-1, and instructs at least one of the examination schedule management unit 110 and/or the cleaning schedule management unit 130 to change an element included in the examination schedule and/or the cleaning schedule.

The rescheduling processing unit 172 instructs the examination schedule management unit 110 to change the endoscope ID assigned to the examination E21. This change instruction includes the examination ID and the used endoscope ID, and therefore the examination schedule management unit 110 changes the endoscope ID of the examination E21 to "G-R-2" (S302). Further, the rescheduling processing unit 172 instructs the cleaning schedule management unit 130 to change the endoscope ID assigned to the cleaning processing of the endoscope that was scheduled to be used in the examination E21 in the cleaning schedule. The cleaning schedule management unit 130 specifies that the cleaning processing of the endoscope G-R-1, which is set after the scheduled end time of the examination E21, is the cleaning W20, and changes the endoscope ID of the cleaning W20 to "G-R-2" (S302).

After changing the endoscope ID of the examination E21 to "G-R-2", the examination schedule management unit 110 investigates whether the unscheduled endoscope G-R-2 is scheduled to be used in an examination scheduled to be performed after the examination E21 (S304). Herein, when the unscheduled endoscope G-R-2 is not scheduled to be used after the examination E21 (S304/N), the examination schedule management unit 110 confirms that there is no schedule change resulting from having used the endoscope G-R-2 in the examination E21, and ends this flow.

On the other hand, when the unscheduled endoscope G-R-2 is scheduled to be used in an examination after the examination 21 (S304/Y), it is determined whether the endoscope G-R-2 can be used as scheduled, in the examination in which the endoscope is scheduled to be used for the first time (S306). Herein, the case where an endoscope can be used as scheduled means the case where the scheduled cleaning end time of the endoscope G-R-2 in the cleaning W20 is a time before the scheduled start time of the examination in which the endoscope G-R-2 is used, that is, the case where the cleaning of the endoscope G-R-2 in the cleaning W20 ends before the start of the examination in which the endoscope G-R-2 is scheduled to be used. When determining that the endoscope G-R-2 can be used in the examination in which the endoscope is scheduled to be used for the first time (S306/Y), the examination schedule management unit 110 confirms that there is no schedule change resulting from having used the endoscope G-R-2 in the examination E21, and ends this flow.

On the other hand, when it is determined that the endoscope G-R-2 cannot be used as scheduled, in the examination in which the endoscope G-R-2 is scheduled to be used for the first time (S306/N), the examination schedule management unit 110 confirms that the examination schedule should be changed. With reference to Fig. 63, of the examinations after the examination E21, the examination in which the endoscope G-R-2 is scheduled to be used for the first time is the examination E22, and the scheduled examination start time of the examination E22 is 10:25 and the scheduled cleaning end time of the cleaning W20 is 10: 55; and hence the examination schedule management unit 110 determines that the endoscope G-R-2 cannot be used in the examination E22.

When confirming that the unscheduled endoscope G-R-2 cannot be used in the examination E22, the examination schedule management unit 110 determines whether there is an endoscope of the same model that is not scheduled to be used in an examination after the examination E22 (S308). Herein, the endoscope G-R-2 is an upper routine model, and with reference to the examination schedule, the examination schedule management unit 110 searches for, of the possessed upper routine models, an upper routine model that is not scheduled to be used after the examination E22.

Herein, if an upper routine model that is not scheduled to be used after the examination E22 exists (S308/Y), the examination schedule management unit 110 assigns the upper routine model, which is not scheduled to be used, to the examination E22 as a substitute endoscope (S310), and the cleaning schedule management unit 130 assigns the substitute endoscope to the cleaning W22 that is the cleaning processing of the endoscope used in the examination E22 (S312), and ends this flow. Because this substitute endoscope is not scheduled to be used thereafter, the subsequent schedule is not affected, even if the substitute endoscope is assigned to the examination E22 and is assigned to the cleaning W22. Therefore, when an upper routine model that is not scheduled to be used after the examination E22 exists, the schedule element change processing can be simply achieved by using the upper routine model.

Although not illustrated in the possessed endoscope master table 222 of Fig. 61, when a medical facility further has one more G-R-7 in addition to G-R-1 to G-R-6 that are upper routine models, the endoscope G-R-7 is not scheduled to be used after the examination E22. Therefore, if the medical facility has the endoscope G-R-7, the examination schedule management unit 110 can determine the endoscope G-R-7 as a substitute endoscope, so that it can be assigned to the examination E22.

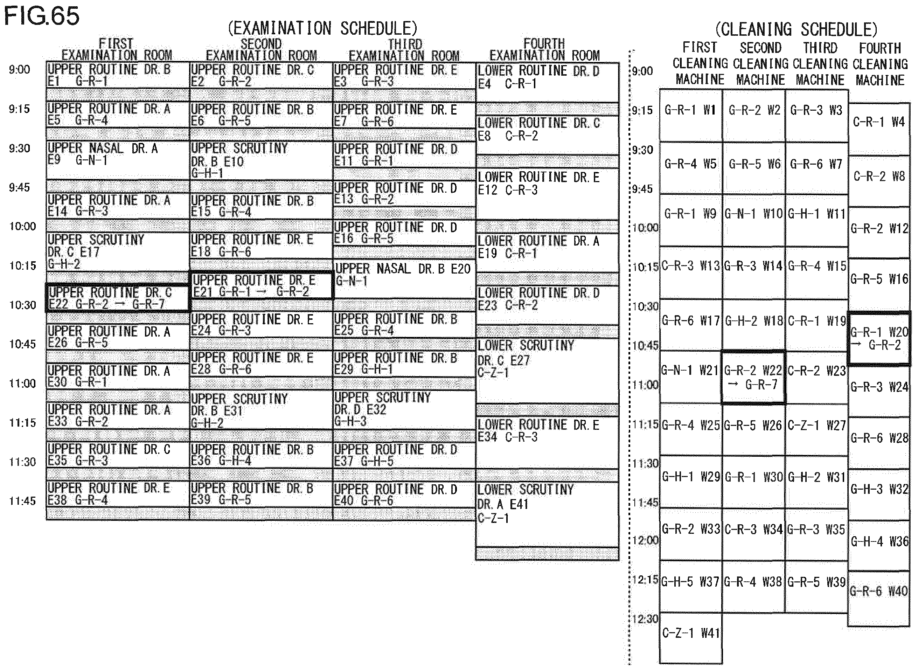

Fig. 65 illustrates an example in which the assignment of an endoscope in the examination E22 is changed. As illustrated in Fig. 65, the endoscope G-R-2 scheduled in the examination E22 and the cleaning W22 is replaced with the endoscope G-R-7 that is a substitute endoscope. In this example, the endoscope G-R-7, which is not originally scheduled to be used in an examination, is used as a substitute endoscope; however, there are some endoscopes that are, for example, in the last stage of the examinations for one day, not scheduled to be used after being cleaned and returned to the endoscope cabinet 14 after being cleaned. Therefore, of G-R-1 to G-R-6 that are upper routine models, an endoscope that is not scheduled to be used after being cleaned can be used as a substitute endoscope in the last stage of the examinations.

On the other hand, if there is no upper routine model that is not scheduled to be used after the examination E22 (S308/N), the examination schedule management unit 110 determines whether the scheduled endoscope G-R-1 and the unscheduled endoscope G-R-2 can be replaced with each other (S314). Whether they can be replaced may be determined by whether the models of the scheduled endoscope and the unscheduled endoscope are the same as each other. In this example, both the scheduled endoscope and the unscheduled endoscope are upper routine models and the models thereof are the same (S314/Y), and hence the examination schedule management unit 110 replaces the endoscopes G-R-1 and G-R-2 with each other in the examination schedule (S316), and the cleaning schedule management unit 130 also replaces the endoscopes G-R-1 and G-R-2 with each other in the cleaning schedule (S318), whereby the schedule elements are changed, respectively.

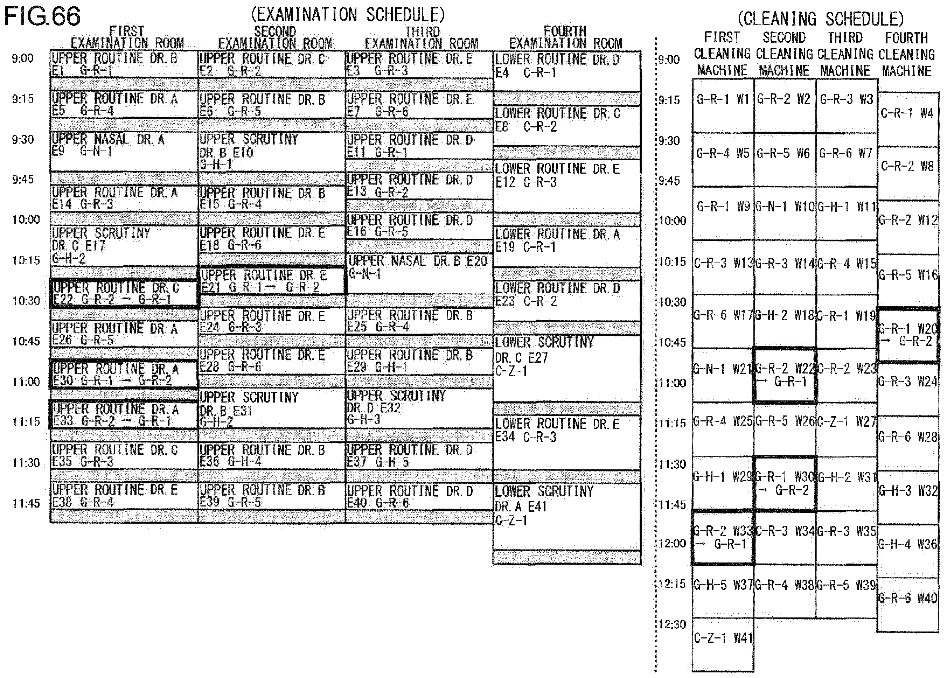

Fig. 66 illustrates an example in which replacement processing of endoscopes is performed. As illustrated in Fig. 66, the examination schedule management unit 110 changes: the endoscope G-R-2 assigned in the examination E22 to the endoscope G-R-1; the endoscope G-R-1 assigned in the examination E30 to the endoscope G-R-2; and the endoscope G-R-2 assigned in the examination E33 to the endoscope G-R-1. Further, the cleaning schedule management unit changes: the endoscope G-R-2 assigned in the cleaning W22 to the endoscope G-R-1; the endoscope G-R-1 assigned in the cleaning W30 to the endoscope G-R-2; and the endoscope G-R-2 assigned in the cleaning W33 to the endoscope G-R-1. When the model of a scheduled endoscope and that of an unscheduled endoscope are the same as each other, both the endoscopes have the same function, and hence rescheduling processing for changing schedule elements can be achieved by replacing both the endoscopes with each other in an examination after the examination E21.

On the other hand, when the model of a scheduled endoscope and that of an unscheduled endoscope are different from each other (S314/N), the examination schedule management unit 110 investigates whether there is any endoscope that is scheduled to be used in an examination after the examination E22 and the time frame of the examination schedule does not need to be changed when the endoscope is used in the examination E22 and cleaned in the cleaning W22 (S320). At this time, the examination schedule management unit 110 investigates not only an upper routine model, which is the same model as the unscheduled endoscope G-R-2, but also an upper high image quality model, which is a different model from the unscheduled endoscope G-R-2 and is associated with the examination E22 (upper routine examination) in the endoscope order holding unit 204 as a preferential endoscope model. In this investigation, it is investigated whether when an endoscope is used in the examination E22, the cleaning of the endoscope is completed before an examination in which the endoscope is scheduled to be subsequently used. Herein, the scheduled use times of the endoscopes G-H-4 and G-H-5, which are, for example, upper high image quality models, are later than the scheduled cleaning end time of the cleaning W22, and hence the examination schedule management unit 110 determines that the endoscopes G-H-4 and G-H-5 can be used in the examination E22 (S320/Y). Therefore, the examination schedule management unit 110 reassigns the endoscope G-H-4 or G-H-5 to the examination E22 in the examination schedule (S322), and the cleaning schedule management unit 130 assigns the endoscope G-H-4 or G-H-5 to the cleaning W22 in the cleaning schedule (S324), whereby schedule elements are changed without changing the time frame of an examination.

On the other hand, when there is no endoscope that can be assigned to the examination E22 without changing a time frame (S320/N), the examination schedule management unit 110 adjusts an examination, which is to be performed in the first examination room 20a and after the examination E22, by shifting it backward until the time when the endoscope G-R-2 can be used, without changing the endoscope G-R-2 assigned to the examination E22, whereby a schedule element is reset (S326).

When the examination schedule management unit 110 changes the examination time of an examination after the examination E22 in the first examination room 20a, the examination schedules between examination rooms and the cleaning schedule may be affected. Therefore, the examination schedule management unit 110 investigates whether an inconsistency occurs between the examination schedules between the examination rooms, and the cleaning schedule management unit 130 investigates whether an inconsistency occurs between the examination schedule and the cleaning schedule. Herein, the case where an inconsistency occurs means: with respect to the doctor information, the case where the same doctor is assigned to a plurality of examinations overlapping each other in time; and with respect to the endoscope 30, the case where one endoscope 30 is assigned to a plurality of examinations overlapping each other in time, the case where an endoscope 30, the cleaning of which is not yet completed, is assigned to an examination, or the case where the endoscope 30, which is being used in an examination, is assigned to cleaning.

When the same doctor is assigned to a plurality of examinations overlapping each other in time, or when one endoscope 30 is assigned to a plurality of examinations overlapping each other in time, the examination schedule management unit 110 reschedules the examination schedule so as to resolve the inconsistency by adjusting such that an examination, the scheduled start time of which is late, is shifted backward. When one endoscope 30 is assigned to an examination and cleaning at the same time, the cleaning schedule management unit 130 reschedules the cleaning schedule so as to resolve the inconsistency by changing the cleaning time such that the scheduled start time of the cleaning is later than the scheduled end time of the examination. The examination schedule management unit 110 and the cleaning schedule management unit 130 reschedule the schedules by adjusting so as to alternately shift schedules backward in time such that inconsistencies are resolved, respectively, whereby schedules, in which an inconsistency is finally eliminated, can be reset.

As described above, the examination schedule management unit 110 and the cleaning schedule management unit 130 reschedule, in real time, the examination schedule and the cleaning schedule that have been generated in advance, depending on the actual usage condition of the endoscopes 30 and based on a schedule element change instruction from the rescheduling processing unit 172. In Example 9, the assignment of an endoscope in the examination schedule is reset such that a time frame in the examination schedule is not changed as much as possible. By thus executing the rescheduling processing in a way in which the schedule information before the start of examination work is taken over, a doctor basically only has to move from one examination room to another, and the like according to the schedule information confirmed before the start of examination work, whereby work efficiency can be improved.

### <Example 10>

Example 9 has described the case where due to erroneous use of the endoscope 30 in preparation for an examination, the endoscope 30 in the subsequent examination is reassigned. In Example 10, a case will be described in which the endoscope 30 is used as scheduled in an examination but another endoscope 30 is additionally used. In Example 10, when an inconsistency occurs in setting a schedule element due to the use of the additional endoscope 30 without a change of the endoscope assigned to an examination, rescheduling processing is performed so as to change the time of the time frame of the examination in order to resolve the inconsistency.

For example, when a doctor intends to observe a certain part in detail during an upper routine examination, an upper routine model connected to the endoscopic observation device 22 is removed and an upper high image quality model or an upper expansion model is connected. In such a case, it is preferable to cause the examination schedule management unit 110 search for an upper high image quality model or an upper expansion model that is not scheduled to be used thereafter, as described in Example 9; however, from the viewpoint of examination efficiency, a free endoscope that can be prepared immediately is frequently used at a work site. When an additional endoscope is used in an examination in this way, at least the cleaning schedule is affected, and the processing unit 100 needs to adjust the set schedule.

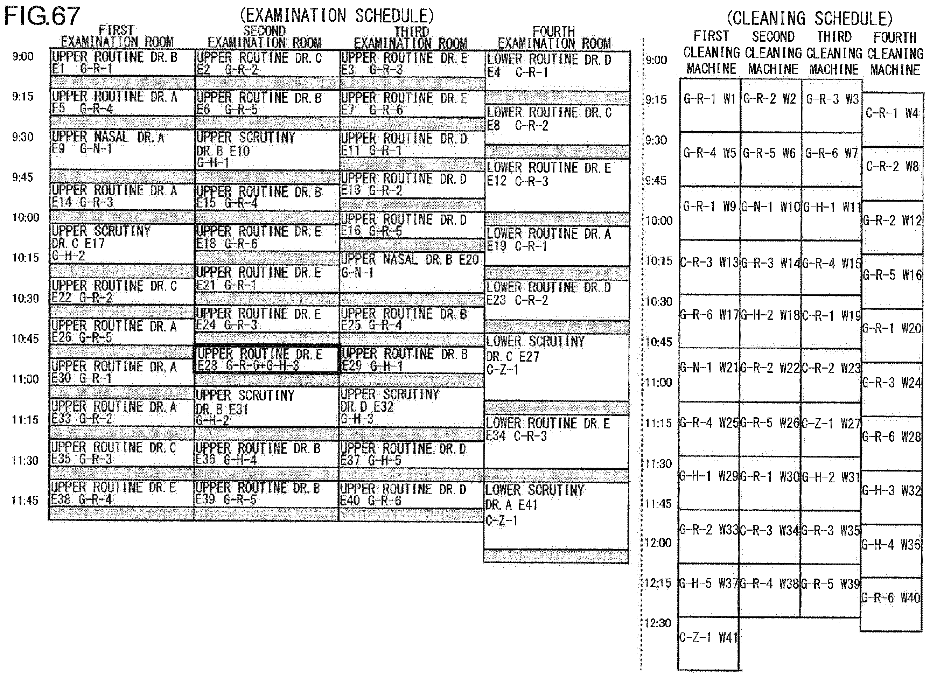

Fig. 67 illustrates an example in which an additional endoscope is used in addition to the scheduled endoscope in the actual examination. In the examination E28 being performed in the second examination room 20b, the assigned endoscope G-R-6 is first used, but it is removed from the endoscopic observation device 22b, and the endoscope G-H-3 is connected instead. It is assumed that the examination E28 was started at 10:50 as scheduled and the endoscope G-H-3 was connected to the endoscopic observation device 22b at 10:55. Hereinafter, rescheduling processing in this case will be described.

A nurse or the like brings into the second examination room 20b the endoscope G-R-6 during a preparation period (10:45 to 10:50) of the examination E28, and connects to the endoscopic observation device 22b. When the endoscope G-R-6 is connected, the endoscopic observation device 22b transmits the endoscope ID, the examination ID, and the observation device ID to the information management device 10. In the information management device 10, the situation information acquisition unit 170 acquires the endoscope ID, the examination ID, and the observation device ID as the situation information on the situation of the endoscope. The situation information acquisition unit 170 provides the acquired situation information to the rescheduling processing unit 172.

When the scheduled start time (10:50) of the examination is reached, the examination start button is operated, and an examination start notice is transmitted to the information management device 10 along with the examination ID and the observation device ID. The situation information acquisition unit 170 acquires the examination start notice information, the examination ID, and the observation device ID as the situation information on the implementation situation of the examination. The situation information acquisition unit 170 provides the acquired situation information to the rescheduling processing unit 172.

Alternatively, the endoscopic observation device 22 may transmit the endoscope ID to the information management device 10 along with the examination start notice information, the examination ID, and the observation device ID when the examination start button is operated, as described in Example 9. In this case, the situation information acquisition unit 170 acquires the endoscope ID and the examination start notice information at the same timing.

When receiving the examination start notice information from the situation information acquisition unit 170, the rescheduling processing unit 172 determines whether the endoscope ID of the endoscope 30 actually used in the examination E28 coincides with the endoscope ID assigned to the examination E28 in the examination schedule. At this time, the endoscope ID acquired by the situation information acquisition unit 170 is "G-R-6", the endoscope ID assigned to the examination E28 is "G-R-6." Therefore, the rescheduling processing unit 172 detects that the endoscope in use is the same as the scheduled endoscope, and determines that it is not necessary to change the examination schedule and/or the cleaning schedule.

Herein, the endoscope G-R-6 is removed from the endoscopic observation device 22b at 10: 54 when the examination is being performed, and the endoscope G-H-3 is newly connected to the endoscopic observation device 22b. When the endoscope G-R-3 is connected, the endoscopic observation device 22b transmits the endoscope ID, the examination ID, and the observation device ID to the information management device 10. In the information management device 10, the situation information acquisition unit 170 acquires the endoscope ID, the examination ID, and the observation device ID as the situation information indicating that the endoscope is being used. The situation information acquisition unit 170 provides the acquired situation information to the rescheduling processing unit 172.

When receiving the endoscope ID from the situation information acquisition unit 170, the rescheduling processing unit 172 determines whether the endoscope ID of the endoscope 30 newly connected while the examination E28 is being performed coincides with the endoscope ID assigned to the examination E28 in the examination schedule. At this time, the endoscope ID acquired by the situation information acquisition unit 170 is "G-R-3", while the endoscope ID assigned to the examination E28 is "G-R-6." Therefore, the rescheduling processing unit 172 detects that the endoscope in use is different from the scheduled endoscope, and determines that it is necessary to change the examination schedule and/or the cleaning schedule.

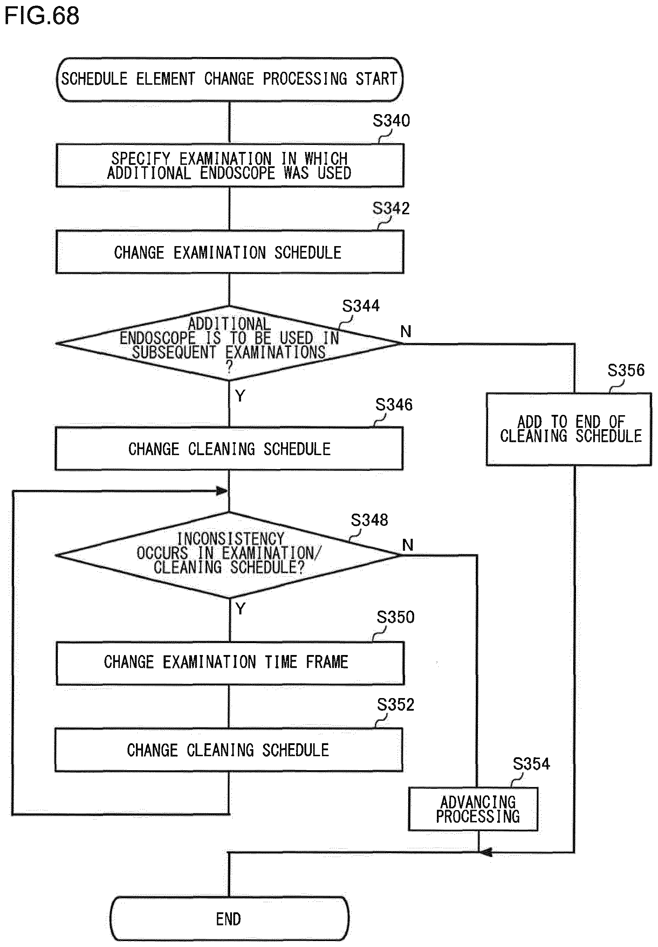

Fig. 68 illustrates a flowchart of the schedule element change processing in Example 10. Hereinafter, in the examination in which the endoscope 30 is additionally used, the endoscope additionally used in the examination is referred to as an "additional endoscope." When the endoscope G-H-3 is used in the examination E28 in addition to the scheduled endoscope G-R-6, as described above, the endoscope G-H-3 is an additional endoscope.

The rescheduling processing unit 172 compares, after the start of the examination, the endoscope ID acquired by the situation information acquisition unit 170 with the ID of the endoscope 30 scheduled to be used in the examination schedule, and when both the endoscope IDs are different from each other, the rescheduling processing unit 172 specifies the examination in which the additional endoscope was used (S340), and determines that it is necessary to change the examination schedule and/or the cleaning schedule. Herein, when the endoscope G-H-3 is used in the examination E28, the rescheduling processing unit 172 recognizes that the additional endoscope G-H-3 is used in addition to the scheduled endoscope G-R-6, and instructs at least one of the examination schedule management unit 110 and/or the cleaning schedule management unit 130 to change an element included in the examination schedule and/or the cleaning schedule.

Specifically, the rescheduling processing unit 172 instructs the examination schedule management unit 110 to add the endoscope ID of the additional endoscope as the endoscope ID assigned to the examination E28. This addition instruction includes the examination ID and the used endoscope ID, and therefore the examination schedule management unit 110 changes the endoscope ID of the examination E28 to "G-R-6" and "G-H-3" (S342). That is, the examination schedule management unit 110 registers "G-H-3" into the examination schedule, in addition to "G-R-6" set in advance.

Herein, the examination schedule management unit 110 investigates whether the additional endoscope G-H-3 is scheduled to be used in an examination scheduled after the examination E28 (S344). Herein, when the additional endoscope G-H-3 is not scheduled to be used after the examination E28 (S344/N), the examination schedule management unit 110 confirms that there is no change in the examination schedule due to the use of the endoscope G-H-3 in the examination E28. At this time, it is not necessary to hurry the cleaning of the endoscope G-H-3, and hence the second assignment processing unit 140 incorporates the cleaning processing of the endoscope G-H-3 into the end of the cleaning schedule such that the cleaning schedule is not affected (S356), whereby the cleaning thereof can be performed at the end of the cleaning schedule, and thereafter this flow is ended.

On the other hand, when the additional endoscope G-H-3 is scheduled to be used after the examination E28 (S344/Y), the cleaning schedule management unit 130 schedules the cleaning of the endoscope G-H-3 after the cleaning W28, and adjusts the subsequent cleaning so as to be shifted backward one by one (S346).

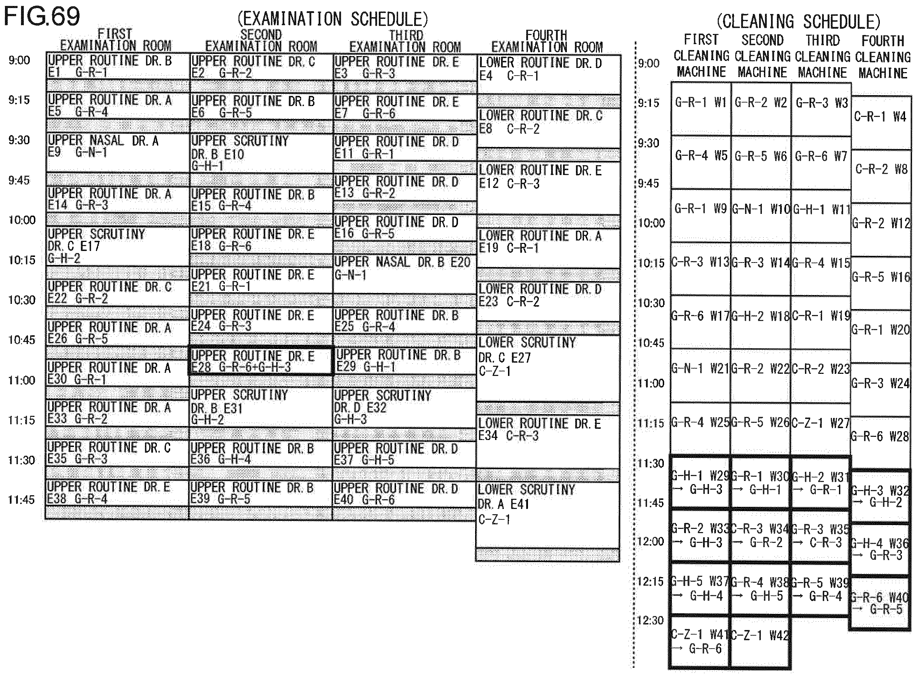

Fig. 69 illustrates an example in which the cleaning processing of the endoscope G-H-3 is inserted in the cleaning schedule. The cleaning of the endoscope G-H-3 is scheduled in the cleaning W 29 by the cleaning schedule change processing of S346, and the cleaning of an endoscope, which is assigned to cleaning after the cleaning W29 before changed, is changed to cleaning processing whose cleaning No. is incremented by one.

Subsequently, the examination schedule management unit 110 and the cleaning schedule management unit 130 verify the validity of the examination schedule and the cleaning schedule (S348). Herein, the examination schedule management unit 110 verifies, one by one, the validity of examinations included in the examination schedule, and if there is an invalid examination, the examination schedule management unit 110 performs processing for changing the time frame of the examination (S350). Subsequently, the cleaning schedule management unit 130 also verifies, one by one, the validity of the cleaning schedules included in the cleaning schedule, and if there is an invalid cleaning schedule, the cleaning schedule management unit 130 performs processing for changing the cleaning schedule (S 352).

The examination schedule management unit 110 first determines whether an inconsistency occurs in the examination schedule (S348). Herein, the case where an inconsistency occurs in the examination schedule means, for example: the case where the cleaning of an endoscope 30 assignd to an examination is not yet completed at the scheduled start time of the examination in the cleaning schedule; or the case where an endoscope 30 or a doctor is assigned to a plurality of examinations at the same time.

The examination schedule management unit 110 determines, one by one, whether an inconsistency occurs in an examinatios that will be started after the examination E28. At this time, it is detected that an inconsistency does not occur in the examinations E29 to E31 but occurs in the examination E32 (S348/Y).

The endoscope G-H-3 assigned to the examination E32 is one additionally used in the examination E28. The cleaning processing of the endoscope G-H-3 used in the examination E28 is set as the cleaning W29 in the cleaning schedule changed in S346, and therefore the examination schedule management unit 110 determines that the cleaning W29 is not completed at the scheduled start time of the examination E32.

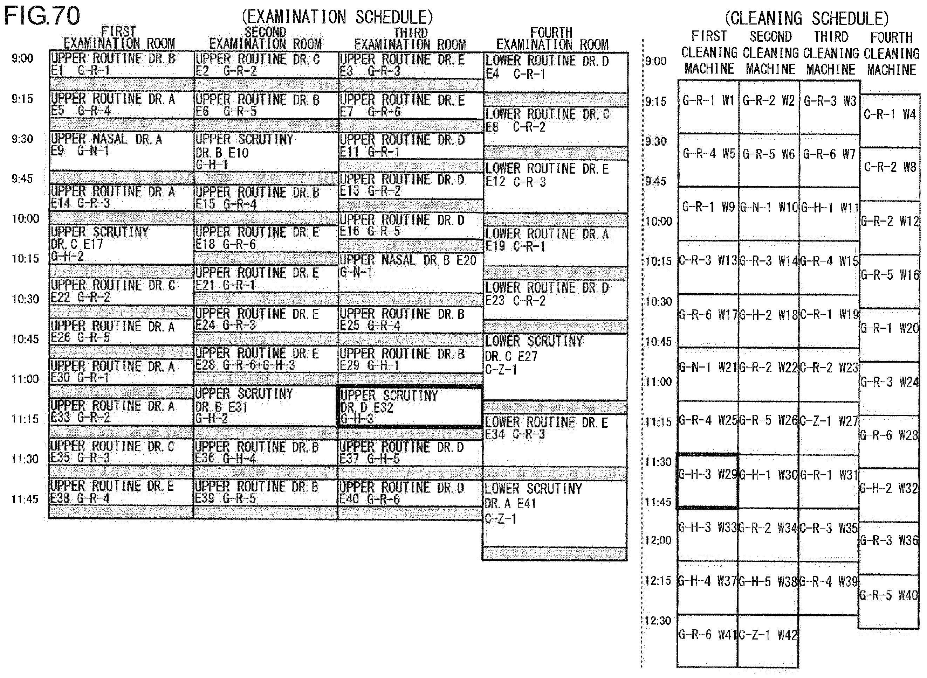

Fig. 70 illustrates the relationship between the examination E32 and the cleaning W29. The scheduled end time of the cleaning W29 is 11:50, and therefore the examination schedule management unit 110 sets the scheduled start time of the examination E32 in the third examination room 20c to be 11:50 (S350).

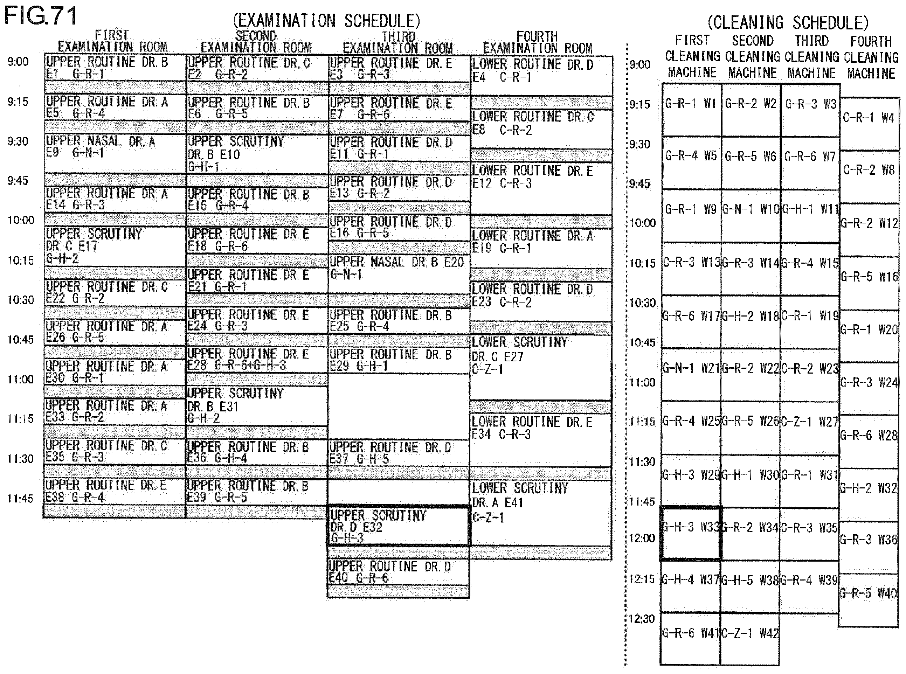

Fig. 71 illustrates a state where the time frame of the examination E32 is changed. The examination schedule management unit 110 may change the examinations E32, E37, and E40 so as to be shifted backward while this order is being kept, but it is known that when the scheduled start time of the examination E32 is set to be 11:50, the examination E37 can be performed as scheduled without shifting the scheduled time thereof backward. Therefore, the examination schedule management unit 110 moves the time frames of the examinations E32 and E40 backward, without moving the time frame of the examination E37.

Next, the cleaning schedule management unit 130 determines whether an inconsistency occurs in the cleaning schedule (S348). The case where an inconsistency occurs in the cleaning schedule means the case where the examination, in which the endoscope 30 is used, is not yet completed in the examination schedule at the scheduled cleaning start time of the endoscope 30. Herein, the cleaning schedule management unit 130 determines, one by one, whether an inconsistency occurs in the cleaning after the cleaning W29, the schedule of which was changed in S344. At this time, it is detected that an inconsistency does not occur in the cleaning W29 to W32 but occurs in the cleaning W33 (S348/Y).

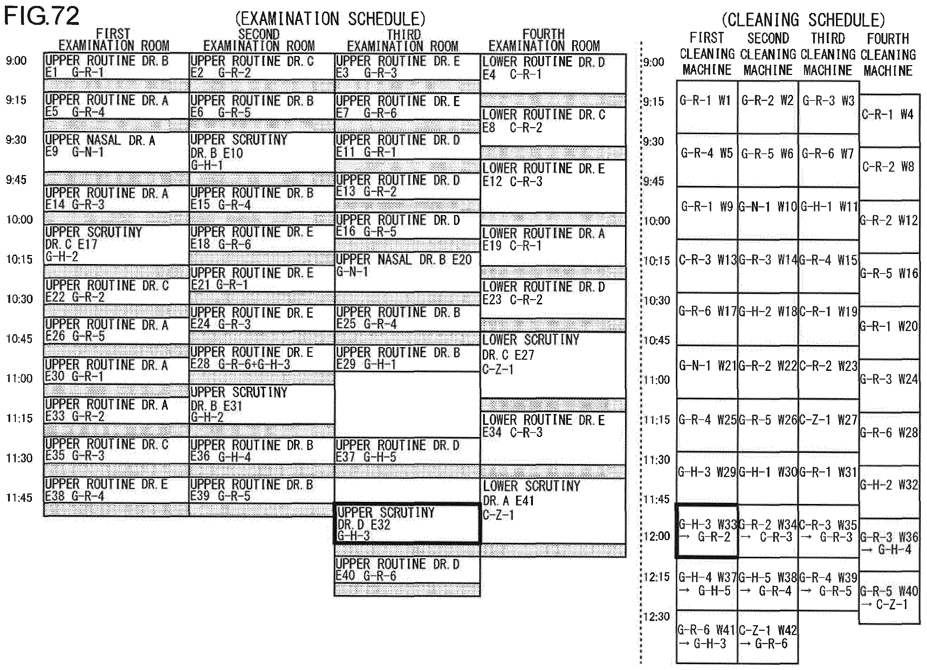

The endoscope G-H-3 scheduled to be cleaned in the cleaning W33 is an endoscope to be used in the examination E32, the time frame of which was shifted backward in time in S350. Therefore, with the scheduled start of the examination E32 delayed, it is necessary to also shift the scheduled cleaning of the endoscope G-H-3 backward in time. When detecting that an inconsistency occurs in the cleaning W33 in the cleaning schedule, the cleaning schedule management unit 130 instructs the cleaning machine assignment unit 144 in the second assignment processing unit 140 to perform the rescheduling processing of the cleaning processing after the cleaning W33, whereby the cleaning schedule change processing is executed (S352). Fig. 72 illustates the result of rescheduling the cleaning schedule in accordance with the examination schedule.

The steps of S348 to S352 are executed until all inconsistencies in the examination schedule and the cleaning schedule are resolved. Herein, when all the inconsistencies are resolved (S348/N), the examination schedule management unit 110 may perform advancing processing on the examination E40 (S354).

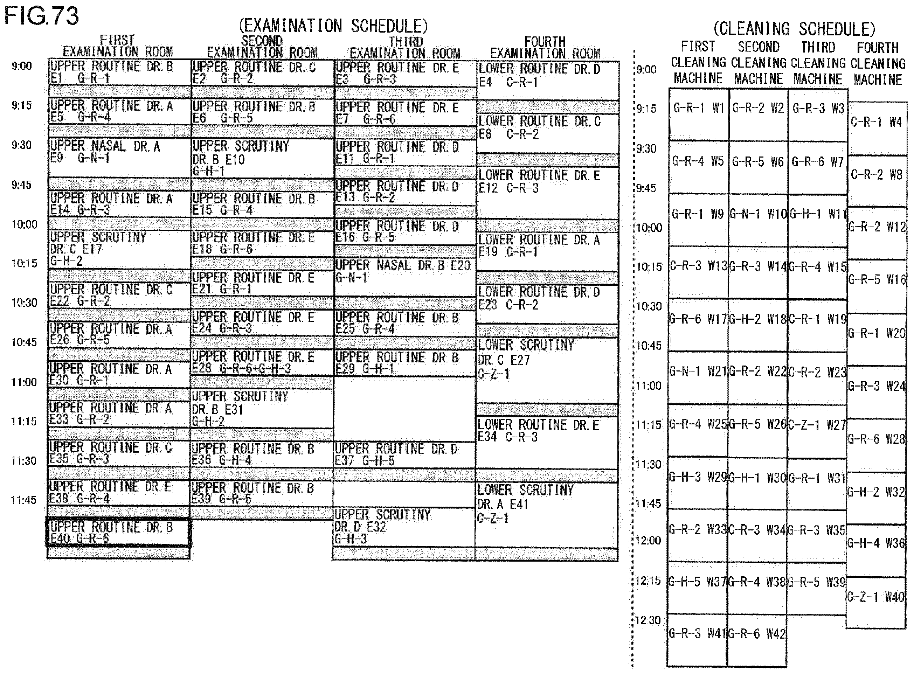

Fig. 73 illustrates an example in which the examination E40 is moved to the end of the examination schedule in the first examination room 20a. When the entire work time is shortened by assigning the examination E40 in the third examination room 20c to another examination room, as described above, the examination schedule management unit 110 may move the examination E40 to another examination room.

Actually, after the advancing processing is performed on the examination E40 in S354, the step of S348 is re-executed. When it is determined that the primary doctor D of the examination E40 overlaps the primary doctor of the examination E32 as a result of performing the advancing processing on the examination E40, the examination schedule management unit 110 instructs the doctor assignment unit 129 in the first assignment processing unit 120 to perform reassignment processing on the doctor of the advanced examination E40. As a result, the primary doctor of the examination E40 is changed to the doctor B, whereby the rescheduling processinf is completed.

Example 10 has described the case where one endoscope G-H-3 is additionally used in the examination E28; however, even when two or more endoscopes are additionally used, the examination schedules and the cleaning schedules can be rescheduled by repeating the flow illustrated in Fig. 68.

In the flowchart illustrated in Fig. 68, when the additional endoscope G-H-3 is not scheduled to be used (S344/N), the second assignment processing unit 140 performs processing for incorporating the cleaning processing of the endoscope G-H-3 into the end of the cleaning schedule (S356), so that a change of the examination schedule, other than the additional registration of "G-H-3" into the endoscopes IDs of the examination E28, is not performed. Herein, in a medical facility where cleaning steps are executed based on the policy that used endoscopes 30 should be promptly cleaned, the cleaning schedule change processing of S346 may be executed, regardless of whether the additional endoscope G-H-3 is scheduled to be used in the subsequent examinations. In this case, the step of S344 is omitted, and the cleaning schedule management unit 130 reschedules the cleanig of the endoscope G-H-3 after the cleaning W28 and adjusts the subsequent cleaning so as to be shfted backward one by one (S346), and thereafter the steps of S348 to S352 may be executed.

### <Example 11>

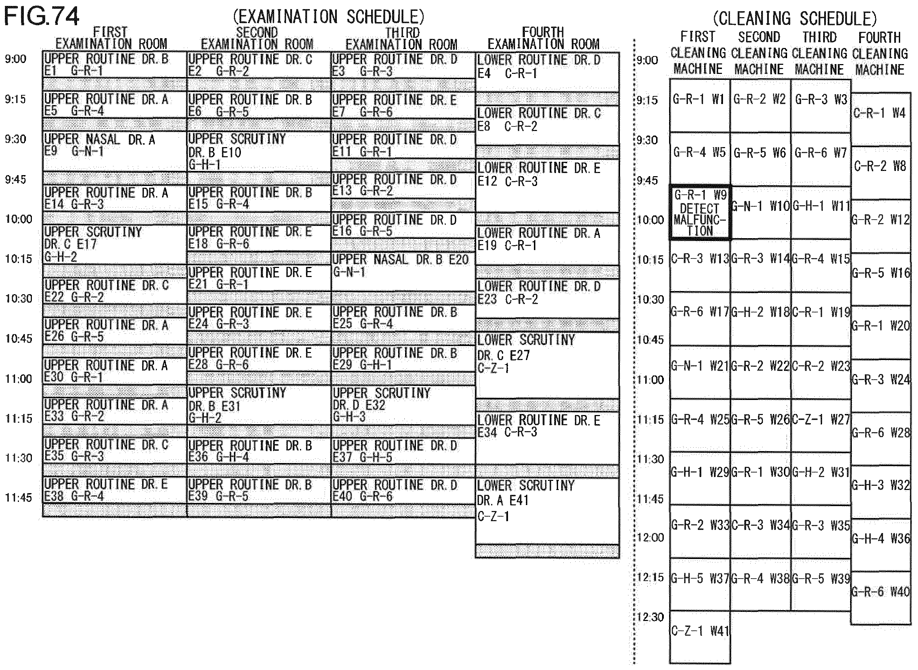

In Example 11, a case is assumed where the endoscope 30 assigned to an examination in the examination schedule cannot be used. Fig. 74 illustrates an example in which a malfunction of the endoscope G-R-1 is detected during the cleaning thereof. The situation information acquisition unit 170 includes a communication unit that communicates with the cleaning machine 50, and may have the function of acquiring the information transmitted from the cleaning machine 50. A means for reading the endoscope ID of an endoscope 30 to be cleaned is provided in the cleaning machine 50, and a person-in-charge of cleaning causes the reading means to read the endoscope ID of an endoscope 30 before the start of cleaning. When the cleaning start button is operated, the cleaning machine 50 transmits the cleaning start notice information to the information management device 10 via the network 2, along with the endoscope ID and information for identifying the cleaning machine 50 (cleaning machine ID). When cleaning ends, the cleaning machine 50 transmits the cleaning end notice information to the information management device 10 via the network 2, along with the endoscope ID and the cleaning machine ID. The situation information acquisition unit 170 monitors the information transmitted from the cleaning machine 50, and acquires the cleaning start notice information and the cleaning end notice information as the situation information indicating the cleaning situation of an endoscope. The situation information acquisition unit 170 immediately (in real time) provides the acquired situation information to the rescheduling processing unit 172 along with the acquired time information, and the rescheduling processing unit 172 notifies the cleaning schedule management unit 130 of the situation information and the time information.

When water leakage is detected in a cleaning step, the cleaning machine 50 transmits water leakage detection information to the information management device 10, along with the detected time information, endoscope ID, and cleaning machine ID. It may be configured such that when water leakage of an endoscope 30 is detected by, for example, a water leakage tester in the cleaning machine 50, the water leakage detection information is automatically transmitted to the information management device 10. Alternatively, the water leakage detection information may be input to the cleaning machine 50 by a user and transmitted to the information management device 10.

The situation information acquisition unit 170 acquires the water leakage detection information as the situation information on the situation of the endoscope 30. The water leakage detection information is one indicating that an endoscope is in a state of not being able to be used, and the situation information acquisition unit 170 provides the acquired situation information to the rescheduling processing unit 172.

The rescheduling processing unit 172 determines based on the situation information whether it is necessary to change an element included in the examination schedule and/or the cleaning schedule illustrated in Fig. 60. When the water leakage detection time of the endoscope G-R-1 is provided from the situation information acquisition unit 170, the rescheduling processing unit 172 determines that the endoscope G-R-1 cannot be used in the subsequent examination schedule.

When determining based on the situation information that it is necessary to change the examination schedule and/or the cleaning schedule, the rescheduling processing unit 172 instructs at least one of the examination schedule management unit 110 and the cleaning schedule management unit 130 to change an element included in the examination schedule and/or the cleaning schedule. The change instruction includes an endoscope ID that cannot be used and time information (water leakage detection time information) on a time when it cannot be used.

When receiving the change instruction from the rescheduling processing unit 172, the examination schedule management unit 110 searches for examinations in which the endoscope G-R-1 is scheduled to be used. Herein, the examination, in which the endoscope G-R-1 is scheduled to be used for the first time after the detection of a malfunction of the endoscope G-R-1, is the examination E21, and the examination schedule management unit 110 deletes the information on the endoscope 30 that was assigned to an examination whose scheduled start time is later than that of the examination E21.

In response to the change instruction from the rescheduling processing unit 172, the cleaning schedule management unit 130 deletes, from the cleaning schedule, cleaning processing whose scheduled cleaning start time is later than the cleaning end time of the cleaning W9.

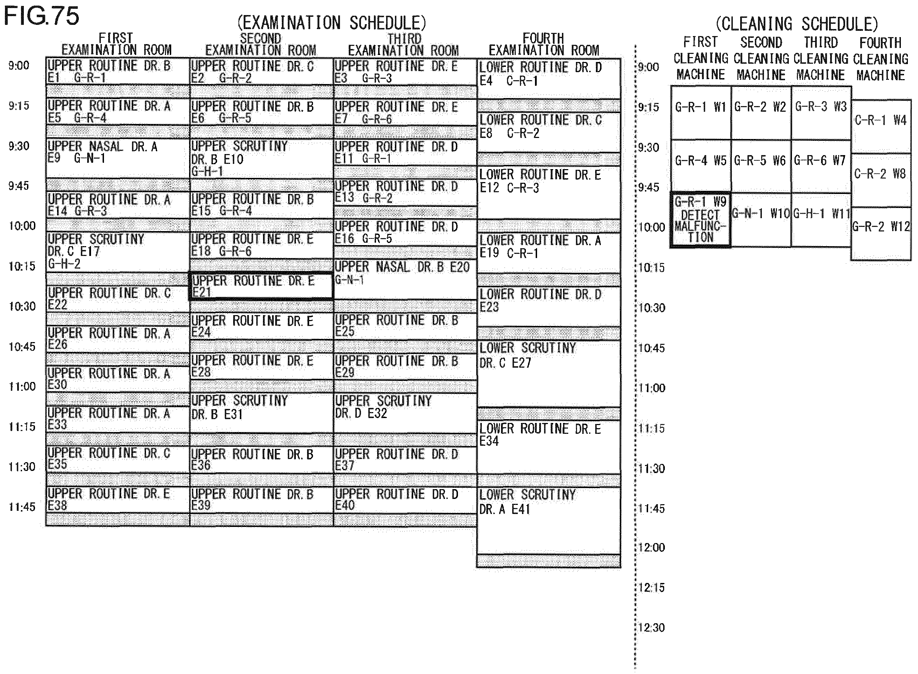

Fig. 75 illustrates a state where the endoscope assignment information in the examination schedule and the cleaning schedule are deleted. As described in the embodiment and Examples 1 to 5, the processing unit 100 executes the endoscope assignment processing and the cleaning machine assignment processing from the scheduled state of Fig. 75 to reconstruct the examination schedule and the cleaning schedule. Thereby, even when the endoscope 30 cannot be used during the examination work, the examination schedule and the cleaning schedule can be promptly reconstructed.

When it is found before the start of the examination work that the endoscope 30 cannot be used, the scheduling processing may be re-executed, as described in the embodiment and Examples 1 to 5. Example 11 has described the case where it is found during the cleaning that the endoscope 30 cannot be used; however, even when it is found at the start of an examination or during an examination, the rescheduling processing can be performed similarly.

The rescheduling processing of the present invention has been described above based on Examples 9 to 11. These examples are illustrative in nature, and it should be appreciated by a person skilled in the art that various modifications can be made to the combinations of the components and the processing processes and such modifications also fall within the scope of the invention.

The above embodiment and examples have described, for example, the doctor assignment unit 129 that assigns a doctor to an examination and the person-in-charge assignment unit 149 that assigns a person-in-charge of cleaning work. For example, an auxiliary work practitioner assignment unit, which assigns an auxiliary work practitioner who assists an examination to an examination, may be further provided in the information management device 10.

An examination, in which an assigned element cannot be changed, also exists in the examination schedule. If there is a circumstance in which an examination can be performed only in a certain time zone due to, for example, the convenience of a patient or a doctor, the scheduled examination start time of the examination cannot be moved. In addition, if an examination requires a special skill, only a doctor having the skill can take charge of the examination, and hence at least the primary doctor of the examination cannot be changed. Even in the endoscopes 30 of the same type, there are various models ranging from the latest one to an old-fashioned one, but for example, in a special examination, only the latest endoscope 30 may be used.

Therefore, in a variation, the change-disallowed examination specification unit 174 specifies an endoscopic examination in which the change of at least one element, of a plurality of elements assigned in advance to an endoscopic examination in the examination schedule, is made disallowed. An input interface, such as a keyboard or a mouse, is connected to the terminal device 12, and the input receiving unit 176 receives an input operation from a user via the terminal device 12. For example, when an examination schedule is generated and when a user designates an examination in which the change of an element is made disallowed, the input receiving unit 176 receives the designating operation from the user, and the change-disallowed examination specification unit 174 specifies an endoscopic examination in which the change of an element is made disallowed. Alternatively, it may be configured that a user can designate, of a plurality of elements that form the schedule of an endoscopic examination, an element, the change of which is made disallowed.

Herein, the elements that form the examination schedule include at least an assigned examination room, information on scheduled examination start time, that on scheduled examination end time, primary doctor, and endoscope ID. Of these elements, a user can designate that the change, for example, of the endoscope ID is prohibited. When such designation is made, the examination room, the scheduled examination times, and the primary doctor can be changed, and the change-disallowed examination specification unit 174 specifies an examination element, the change of which is made disallowed, so that such an examination element is not changed when the rescheduling processing is performed.

In the rescheduling processing described in Examples 9 to 11, the change-disallowed examination specification unit 174 specifies an examination in which the change of at least one element, of the elements assigned in advance to the examination, is made disallowed. The rescheduling processing unit 172 may notify the examination schedule management unit 110 of an element of the examination specified by the change-disallowed examination specification unit 174, the change of which is made disallowed, so that the change of the element is prohibited. Thereby, the examination schedule management unit 110 does not change the notified element of the examination schedule, but performs the rescheduling processing on other schedule elements.

Further, it has been described that an examination, in which the change of a schedule element is made disallowed, is specified by the change-disallowed examination specification unit 174, and that the rescheduling processing unit 172 notifies the examination schedule management unit 110 of the schedule element, the change of which is made disallowed; however, with this processing, there is the possibility that when the examination schedule management unit 110 changes an element of the examination schedule, the change of which is not disallowed, the endoscopes 30 to be assigned may run short, so that a situation where the schedule is greatly delayed, or the like may occur.

Therefore, the examination schedule management unit 110 holds a predetermined rescheduling condition with respect to the rescheduling, and notifies a user that the examination schedule cannot be changed when this rescheduling condition is not satisfied. The rescheduling condition may be set such that a delay of the start of an examination falls within a predetermined time (e.g., one hour). When there is a schedule element, the change of which is made disallowed by the change-disallowed examination specification unit 174, and when the first assignment processing unit 120 assigns an endoscope 30 and a doctor, a restriction is also placed on the flexibility of the assignment, because there is a schedule element, the change of which is made disallowed. Therefore, when the examination schedule management unit 110 causes the first assignment processing unit 120 to execute the schedule element reassignment processing in order to change an element of the examination schedule, it is preferable to notify a user that the examination schedule cannot be changed when the predetermined rescheduling condition is not satisfied.

Even when the rescheduling condition is satisfied and the examination schedule can be changed, but when the time frame of an examination is adjusted, it is preferable for the examination schedule management unit 110 to notify a user of the fact. As a result, a user, such as a doctor, can recognize that the end of the examination will be delayed than scheduled, and it becomes possible to take action, such as to review the schedule of himself/herself after the end of the examination.

Although various scheduling and rescheduling algorithm have been proposed in the present application, it is also possible to generate a plurality of types of examination schedules and cleaning schedules by utilizing some of these scheduling and rescheduling algorithms. Therefore, the display processing unit 150 has the function of displaying the changed examination schedule and/or cleaning schedule on the display of the terminal device 12, and when there are a plurality of candidates for the examination schedule and/or the cleaning schedule, each candidate may be displayed on the display such that a user can select any one of the candidates. When a user selects any one of the candidates, the examination schedule management unit 110 and the cleaning schedule management unit 130 fix the changes of the examination schedule and/or the cleaning schedule.

### <Rescheduling Processing 3>

With the processing described with respect to the embodiment and Examples 1 to 5, an examination schedule and a cleaning schedule are generated before the start of endoscopic examination work for one day. In a medical facility, it is ideal that the examination work proceeds according to the generated examination schedule and cleaning schedule, however, at a work site, the work may not proceed as scheduled in the examination schedule and/or the cleaning schedule due to various factors.

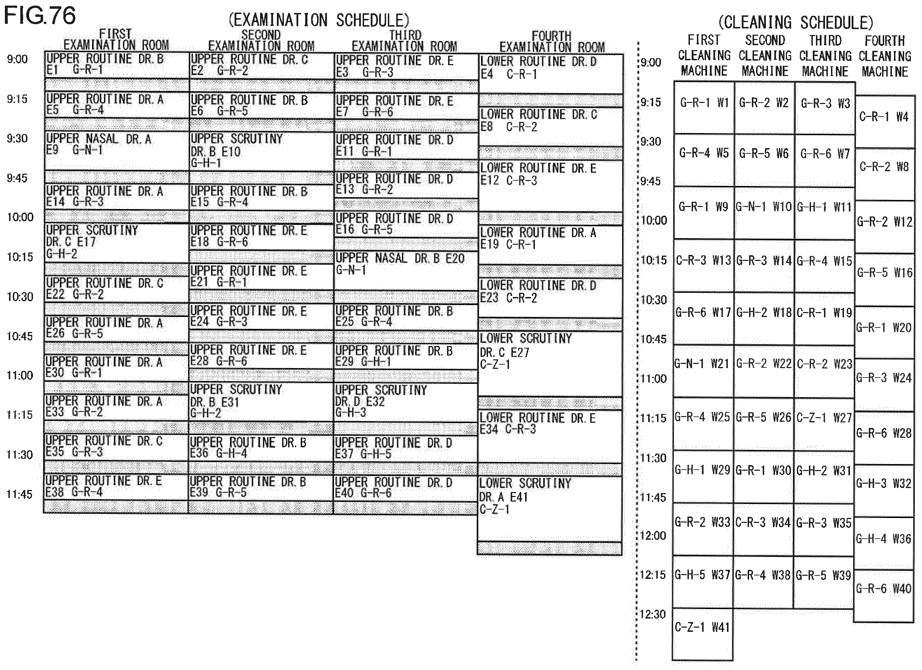

Fig. 76 illustrates examples of an examination schedule generated by the examination schedule management unit 110 and a cleaning schedule generated by the cleaning schedule management unit 130. The examination schedule illustrated in Fig. 76 is generated by assigning an endoscope to each examination by the endoscope assignment processing described with respect to Example 1 and according to the endoscope order table. The cleaning schedule illustrated in Fig. 76 is generated by the cleaning machine assignment processing described with respect to the embodiment. In the rescheduling processing described in the following Examples 12 to 14, the elements included in the examination schedule and cleaning schedule illustrated in Fig. 76 are subjected to the rescheduling processing, but this is only one example and the elements included in the examination schedule and cleaning schedule illustrated in Fig. 20 may be subjected to. In either case, it is premised in the rescheduling processing that an examination schedule and a cleaning schedule are generated in advance. For convenience of description, cleaning Nos. of W1 to W41 are attached in the cleaning schedule in order to specify respective cleaning processing.

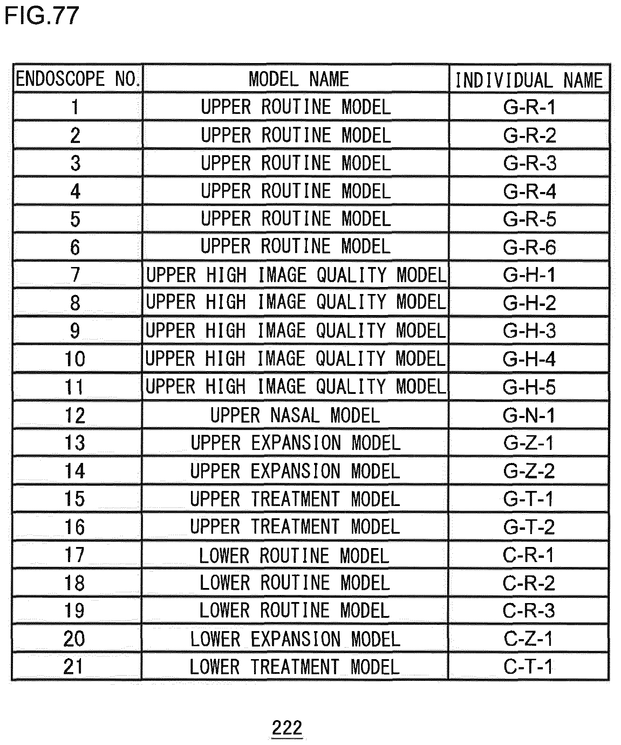

Also, in Fig. 76, it is premised that a medical facility possesses five upper high image quality models. Fig. 77 illustrates one example of the possessed endoscope master table 222. The possessed endoscope master table 222 is different from that illustrated in Fig. 6 in that a medical facility possesses five upper high image quality models. That is, the possessed endoscope master table 222 illustrated in Fig. 77 is different from that illustrated in Fig. 6 in that two upper high image quality models G-H-4 and G-H-5 are added, and in the examination schedule in Fig. 76, the endoscope G-H-4 is assigned to the examination E36 and the endoscope G-H-5 to the examination E37, respectively.

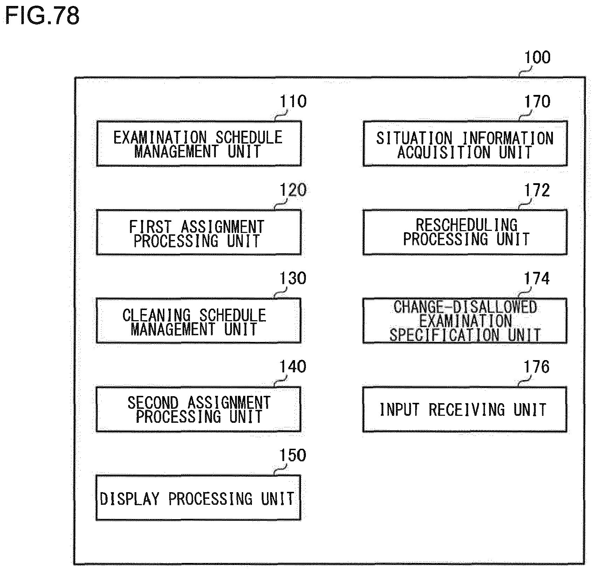

Fig. 78 illustrates, of the configuration of the information management device 10, the configuration of the processing unit 100 having the function of executing rescheduling processing. The processing unit 100 includes the examination schedule management unit 110, the first assignment processing unit 120, the cleaning schedule management unit 130, the second assignment processing unit 140, the display processing unit 150, the situation information acquisition unit 170, the rescheduling processing unit 172, the change-disallowed examination specification unit 174, and the input receiving unit 176. Although not illustrated in Fig. 78, the processing unit 100 is configured to also include the display content derivation unit 152, the period designation unit 154, and the usage condition monitoring unit 160, as illustrated in Fig. 3; the first assignment processing unit 120 is configured to include the examination extraction unit 122, the endoscope specification unit 124, the endoscope assignment unit 126, the endoscope assignment availability confirmation unit 128, and the doctor assignment unit 129; and the second assignment processing unit 140 is configured to include the cleaning machine specification unit 142, the cleaning machine assignment unit 144, the end time determination unit 146, the cleaning machine assignment availability confirmation unit 148, and the person-in-charge assignment unit 149.

Each component of the processing unit 100 can be realized by a CPU, memory, or other LSIs of an arbitrary computer in terms of hardware, and realized by a program or the like loaded in a memory in terms of software, but herein functional blocks realized by the cooperation of hardware and software are depicted. Therefore, it is to be understood by those skilled in the art that these functional blocks can be realized in various forms, namely, solely in hardware, solely in software, or through a combination of hardware and software.

### <Example 12>

In Example 12, a case will be described in which a delay occurs in the cleaning schedule due to occurrence of a trouble in the cleaning processing by the cleaning machine 50. For example, in the cleaning machine 50, the cleaning processing may be stopped halfway, because a medicinal solution runs short after the start of the cleaning and an error occurs. In addition, the cleaning processing may be forcibly ended halfway by a person-in-charge of cleaning pushing a stop button of the cleaning machine 50 because occurrence of an unusual odor, or the like. In such a case, the person-in-charge of cleaning makes the cleaning machine 50 available by refilling the cleaning machine 50 with a medicinal solution, exchanging filter, or the like, and then restarts the cleaning processing of an endoscope. Therefore, the cleaning processing is delayed more than scheduled, and the processing unit 100 needs to reconstructe the cleaning schedule and/or the examination schedule.

The situation information acquisition unit 170 acquires situation information on the situation of a cleaning machine 50. The situation information acquisition unit 170 includes a communication unit that communicates with the cleaning machine 50, and may have the function of acquiring the information transmitted from the cleaning machine 50. A means for reading the endoscope ID of an endoscope 30 to be cleaned is provided in the cleaning machine 50, and a person-in-charge of cleaning causes the reading means to read the endoscope ID of an endoscope 30 before the start of cleaning. When the cleaning start button is operated, the cleaning machine 50 transmits the cleaning start notice information to the information management device 10 via the network 2, along with the endoscope ID and information for identifying and the cleaning machine 50 (cleaning machine ID). When cleaning ends, the cleaning machine 50 transmits the cleaning end notice information to the information management device 10 via the network 2, along with the endoscope ID and the cleaning machine ID. The situation information acquisition unit 170 monitors the information transmitted from the cleaning machine 50, and acquires the cleaning start notice information and the cleaning end notice information as the situation information indicating the usage condition of the cleaning machine 50. The situation information acquisition unit 170 immediately (in real time) provides the acquired situation information to the rescheduling processing unit 172 along with the acquired time information, and the rescheduling processing unit 172 notifies the cleaning schedule management unit 130 of the situation information and the time information. Alternatively, the situation information acquisition unit 170 may periodically provide the acquired situation information to the rescheduling processing unit 172 along with the acquired time information.

When receiving the cleaning start notice information and the cleaning end notice information at the time scheduled in the cleaning schedule, the rescheduling processing unit 172 determines that it is not necessary to change the cleaning schedule and/or the examination schedule. On the other hand, when receiving the cleaning start notice information or the cleaning end notice information at a time different from the time scheduled in the cleaning schedule, the rescheduling processing unit 172 determines that it is necessary to change the cleaning schedule and/or the examination schedule.

Alternatively, the cleaning start notice information and the cleaning end notice information may be input by a user and received by the input receiving unit 176 along with the time information, and the input receiving unit 176 may transfer the cleaning start notice information and the cleaning end notice information to the situation information acquisition unit 170 as the situation information. Alternatively, the situation information acquisition unit 170 has the function of communicating with a terminal device other than cleaning machine 50, and may acquire the cleaning start notice information and the cleaning end notice information from the terminal device. The situation information acquisition unit 170 only has to be able to acquire the situation information indicating the usage condition of the cleaning machine 50 by some means, but in order to execute efficient rescheduling processing, it is preferable to acquire the situation information in real time and to provide it to the rescheduling processing unit 172 along with the acquired time information.

The rescheduling processing unit 172 determines based on the situation information whether it is necessary to change an element included in the cleaning schedule and/or the examination schedule illustrated in Fig. 76. For example, when the time, at which the situation information acquisition unit 170 acquires the cleaning start notice information or the cleaning end notice information, is later than the scheduled cleaning start time or scheduled cleaning end time of the cleaning processing by a predetermined time (e.g., three minutes) or more, the rescheduling processing unit 172 determines that it is necessary to cange the cleaning schedule and/or the examination schedule.

When determining based on the situation information that it is necessary to change the examination schedule and/or the cleaning schedule, the reschedule processing unit 172 instructs at least one of the cleaning schedule management unit 130 and the examination schedule management unit 110 to change an element included in the cleaning schedule or the examination schedule. Herein, the element included in the examination schedule is an examination specified by an examination ID, an examination room assigned to an examination, information on scheduled examination start time, that on scheduled examination end time, a primary doctor, or an endoscope 30; while the element included in the cleaning schedule is a cleaning machine assigned to an endoscope 30, information on scheduled cleaning start time, or that on scheduled cleaning end time: When a person-in-charge is assigned to cleaning processing in the cleaning schedule, as illustrated in Example 5 (Fig. 38), person-in-charge information is also included in the element included in the cleaning schedule.

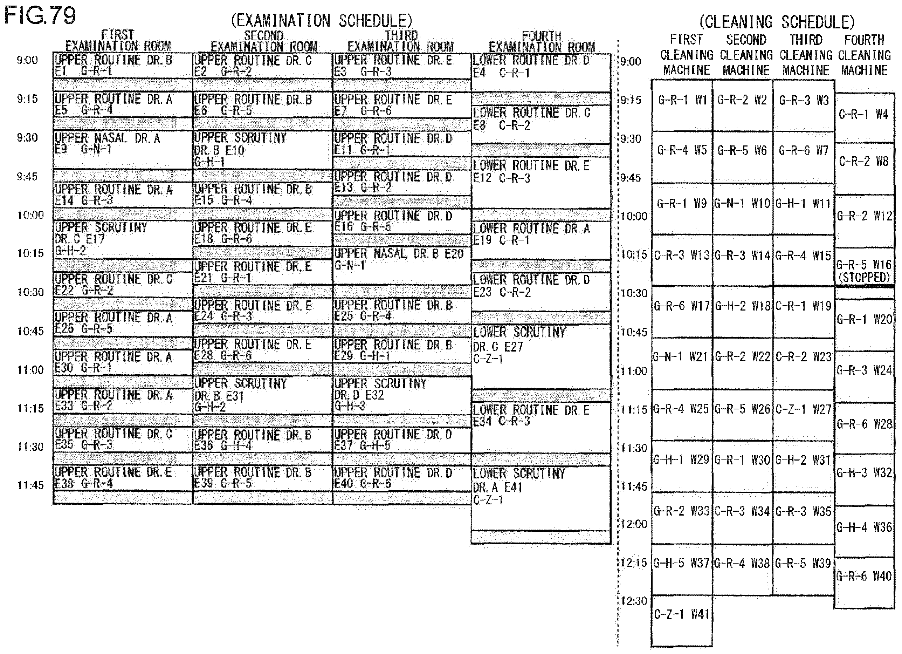

Hereinafter, specific rescheduling processing will be described with reference to Figs. 79 to 81. Fig. 79 illustrates an example in which the cleaning processing is stopped halfway due to occurrence of a trouble in the processing. The scheduled cleaning start time of the cleaning W16 of the endoscope G-R-5 is 10:15, and the scheduled cleaning end time is 10:35, and although the cleaning W16 was started just at the scheduled time, but it is stopped halfway. When the fourth cleaning machine 50d is automatically and forcibly stopped during the cleaning processing of the cleaning W16 due to, for example, a shortage of a medical solution, the fourth cleaning machine 50d transmits cleaning end notice information indicating that the processing was forcibly stopped to the information management device 10, along with the endoscope ID and the cleaning machine ID. When acquiring the cleaning end notice information at 10:22, the situation information acquisition unit 170 immediately notifies the rescheduling processing unit 172 of the acquisition of the cleaning end notice information on the cleaning W16 at 10:22.

In response to the notice from the situation information acquisition unit 170, the rescheduling processing unit 172 detects that the cleaning W16 is not performed until the end, and at this time the rescheduling processing unit 172 can determine that it is necessary to change the cleaning schedule and/or the examination schedule. Although the rescheduling processing unit 172 may notify the cleaning schedule management unit 130 of this determination result, this notice may be caused to wait until the start time of the recleaning of the endoscope G-R-5 is fixed.

A person-in-charge of cleaning solves the trouble occurring in the fourth cleaning machine 50d, and resumes the cleaning W16 of the endoscope G-R-5. If the trouble is caused, for example, by a shortage of a medical solution, the person-in-charge of cleaning restores the fourth cleaning machine 50d by refilling with the medical solution. Herein, it is assumed that the person-in-charge of cleaning resumes the cleaning W16 at 10:30.

When the cleaning start button is operated at 10:30, the fourth cleaning machine 50d transmits cleaning start notice information to the information management device 10, along with the endoscope ID and the cleaning machine ID. When acquiring the cleaning start notice information, the situation information acquisition unit 170 immediately notifies the rescheduling processing unit 172 of the acquisition of the cleaning start notice information on the cleaning W16 at 10:30.

In response to the notice from the situation information acquisition unit 170, the rescheduling processing unit 172 detects that the cleaning W16 was resumed at 10:30, and determines that it is necessary to change the cleaning schedule and/or the examination schedule. When determining that it is necessary to change the schedule, the rescheduling processing unit 172 instructs at least one of the cleaning schedule management unit 130 and the examination schedule management unit 110 to change an element included in the schedule. In this Example 12, the rescheduling processing unit 172 instructs the cleaning schedule management unit 130 to change an element included in the cleaning schedule, and the cleaning schedule management unit 130 performs processing for moving the time frames of the cleaning designated by the information on scheduled cleaning start time and that on scheduled cleaning end time as the elements of the cleaning schedule.

At this time, the rescheduling processing unit 172 may specify, of the elements included in the cleaning schedule, information on scheduled end time of the cleaning W16 as an element to be changed, and may instruct the cleaning schedule management unit 130 to change the specified element. The rescheduling processing unit 172 may specify based on the situation information an element to be changed in this way, but the cleaning schedule management unit 130 may specify an element to be changed by being notified of the start time of the cleaning W16 from the rescheduling processing unit 172.

The cleaning schedule management unit 130 specifies a cleaning room by which it becomes necessary to change the cleaning schedule, based on the schedule element change instruction provided from the rescheduling processing unit 172, and changes the information on scheduled start time and that on scheduled end time of cleaning processing in the specified cleaning room. Specifically, the schedule element change instruction includes the start time of the cleaning W16, and in response to this change instruction, the cleaning schedule management unit 130 specifies the fourth cleaning machine 50d by which the cleaning W16 is performed, and resets the information on scheduled cleaning start time and that on scheduled cleaning end time of each of the cleaning W16 and the cleaning W20, W24, W28, W32, W36, and W40, which are scheduled in the fourth cleaning machine 50d and the cleaning W20 to W40 are schedulded after the cleaning W16.

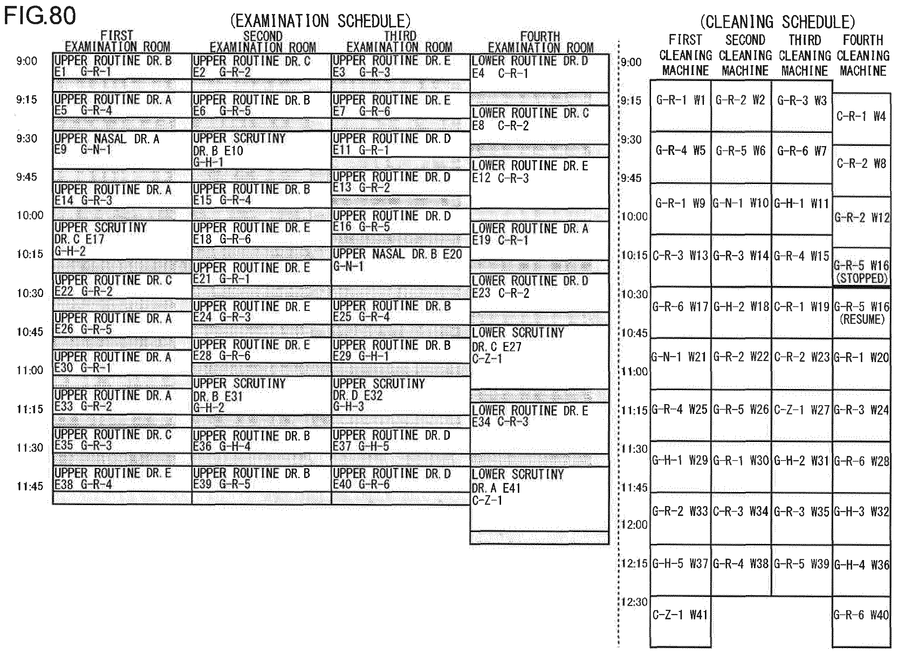

Fig. 80 illustrates a state where the scheduled cleaning start time and scheduled cleaning end time of each of the cleaning W16, W20, W24, W28, W32, W36, and W40 is delayed by 15 minutes, respectively. When the cleaning processing in the fourth cleaning machine 50d after the cleaning W16 are delayed, there is the possibility that an examination, to which a cleaned endoscope 30 is assigned, may be affected. Therefore, in Example 12, the examination schedule management unit 110 first investigate, by paying attention to the time frames of the examinations, whether an inconsistency occurs in the endoscope 30, which is assigned to the time frame of an examination whose scheduled start times is later than the scheduled end time (10:35) of the cleaning W16 before changed. Herein, the case where an inconsistency occurs means the case where an endoscope 30, the cleaning of which is not yet completed, is assigned to an examination.

Specifically, the examination schedule management unit 110 investigates whether there is any endoscope 30 that assigned to an examination before the cleaning thereof is completed, as a result of delaying the cleaning processing after the cleaning W16 in the fourth cleaning machine 50d by 15 minutes, respectively, as illustrated in Fig. 80. Herein, the examination schedule management unit 110 specifies that the endoscope G-R-5 in the examination E26, the endoscope G-R-1 in the examination E30, the endoscope G-R-3 in the examination E35, these three examinations being scheduled to be performed in the first examination room 20a, and the endoscope G-R-6 in the examination E40 in the third examination room 20c are scheduled to be used before the scheduled cleaning end times of them.

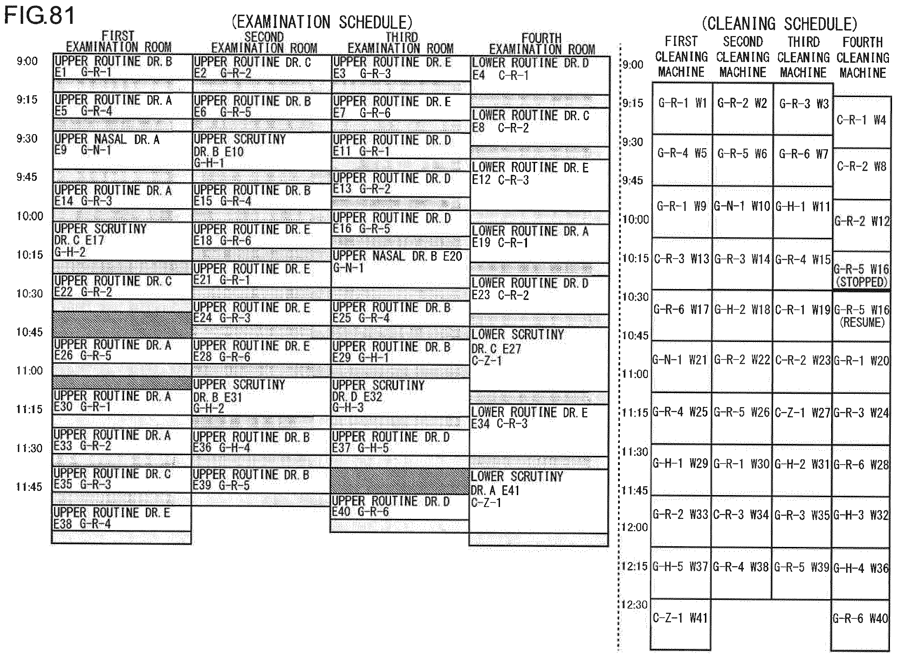

Therefore, the examination schedule management unit 110 performs processing for changing the time frames of these examinations so as to be shifted backward such that the scheduled use start times of the respective endoscopes are later than the scheduled cleaning end times of them. Fig. 81 illustrates a state where the time frames of the examinations are reset. By respectively resetting the information on scheduled examination start times and that on scheduled examination end times of the examinations E26, E30, E35, and E40 in this way, the scheduled start time of each examination becomes later than the scheduled cleaning end time of the endoscope to be used.

Next, the examination schedule management unit 110 investigates whether an inconsistency occurs in a primary doctor of an examination in the changed examination schedule. Herein, the case where an inconsistency occurs means the case where one doctor is assigned to a plurality of examinations at the same time.

An inconsistency does not occur in a primary doctor in the examination schedule illustrated in Fig. 81. Therefore, the examination schedule management unit 110 temporarily sets this examination schedule. When a primary doctor is assigned to a plurality of examinations, the examination schedule management unit 110 reschedules the examination schedule so as to resolve the inconsistency by shifting an examination, the scheduled start time of which is later, backward in time.

Subsequently, the cleaning schedule management unit 130 investigates whether an inconsistency occurs between the temporarily set examination schedule and the cleaning schedule. Herein, the case where an inconsistency occurs means the case where the cleaning of an endoscope to be used in an examination is started before the scheduled end time of the examination, this inconsistency occurring because an examination is shifted backward in time in the examination schedule.

In the cleaning schedule illustrated in Fig. 81, there is no cleaning processing in which the cleaning of an endoscope to be used in an examination is started before the scheduled end time of the examination. Therefore, the cleaning schedule management unit 130 registers the examination schedule, and the examination schedule management unit 110 also registers the temporarily set examination schedule. Herein, the registration means: that the elements of an examination schedule are fixed and the examination schedule is recorded in the examination schedule holding unit 206; and that the elements of a cleaning schedule are fixed and the cleaning schedule is recorded in the cleaning schedule holding unit 208. Thereby, the rescheduling processing is completed.

When an inconsistency occurs in the cleaning schedule and when the cleaning schedule management unit 130 changes the cleaning schedule, the examination schedule management unit 110 investigates whether with this change, the examination schedule is affected, that is, whether an inconsistency occurs in the examination schedule. As described above, when an inconsistency occurs in the examination schedule, the examination schedule management unit 110 adjusts, and subsequently when an inconsistency occurs in the cleaning schedule, the cleaning schedule management unit 130 adjusts; and when there is no inconsistency in the cleaning schedule with respect to the adjusted examination schedule by repeating the above adjustment, the schedule elements are fixed and the rescheduling processing is completed.

As described above, the processing unit 100 executes the rescheduling processing on the cleaning schedule and the examination schedule, depending on the usage condition of a cleaning machine 50. Example 12 has described the case where the cleaning processing is once stopped and then resumed, however, when the actual cleaning start time or cleaning end time is advanced than scheduled, or even when it is delayed, the rescheduling processing of Example 12 can be applied. In addition, even when the interrupt cleaning of an unscheduled endoscope is performed because the unscheduled endoscope was used in an examination, or even when the scheduled cleaning processing is canceled, the rescheduling processing of Example 12 can also be applied.

Example 12 has described the case where a trouble occurs in the cleaning processing, but even when a cleaning machine 50 breaks down and cannot be used thereafter, the rescheduling processing of Example 12 can be applied.

As described above, even when a cleaning machine 50 indicates an unscheduled usage condition, the cleaning schedule and the examination schedule can be properly reconstructed according to the rescheduling processing of Example 12,

In the following Examples 13 and 14, a case will be described in which a primary doctor assigned to an examination cannot perform the examination due to a sudden reason. Before the start of examination work for one day, if it is found that, for example, the doctor C cannot perform all the examininations for the day, the doctor assignment unit 129 in the first assignment processing unit 120 only has to reconstruct the examination schedule by assigning a doctor other than the doctor C to an examination in each examination room 20. However, when a doctor assigned to an examination cannot perform the examination due to an unscheduled circumstance after the start of the examination work for the day, it is necessary at the time to assign another doctor to each examination by the processing unit 100 performing the rescheduling processing.

There are individual differences among the skills of doctors. In general, there is the tendency that veteran doctors have high skills and can deal with various types of examinations but the types of examinations that can be performed by inexperienced young doctors are limited. Therefore, when the doctor assignment unit 129 assigns a doctor to an examination, it is desirable to compare an examination type with the skill of a doctor. To put it simply, it is necessary to assign a veteran doctor (a skillful doctor) to a highly difficult examination, instead of a young doctor.

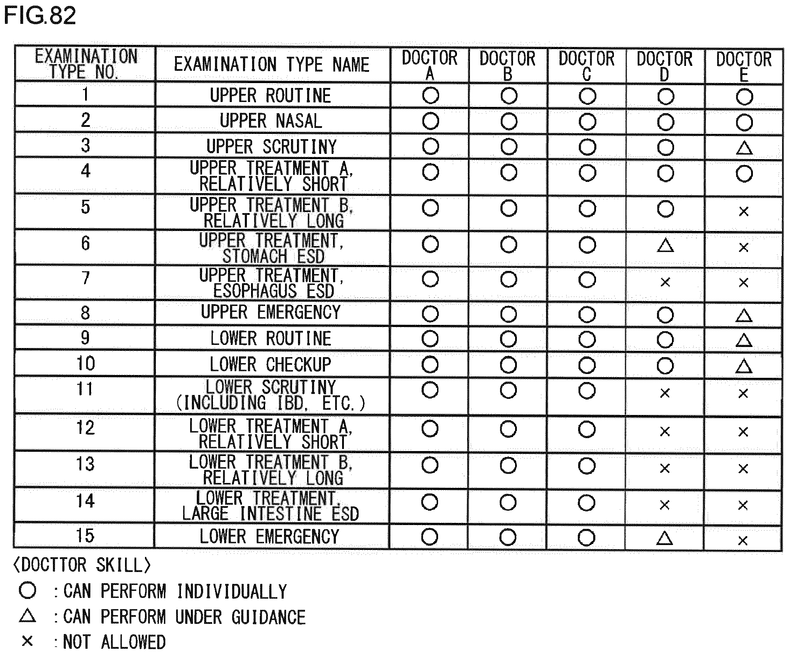

From such a viewpoint, the storage unit 200 illustrated in Fig. 3 may further include a doctor skill table in which the types of examinations that can be dealt with by doctors are recorded. Fig. 82 illustrates one example of the doctor skill table. The doctor skill table is stored in the storage unit 200, and is referred to when the doctor assignment unit 129 assigns a doctor to an examination.

The doctor skill table records the relationships between doctors and the types of examinations that the doctors can deal with. The doctor skill table is updated in accordance with the skill levels of doctors, that is, when a doctor accumulates experiences and the skill thereof is improved, the number of the types of examinations that can be dealt with is increased. In this example, the doctors A to C are veteran doctors, and it is said that each of them can individually perform all the examinations with examination type Nos. 1 to 15. The doctor E is a young doctor, and it is said that he/she can individually perform the examinations with examination type Nos. 1, 2, and 4 and can perform the examinations with examination type Nos. 3 and 8 to 10 under the guidance of a supervising doctor; however, it is not allowed to perform the examinations with other examination type Nos. because he/she is not experienced. The doctor D is a mid-level doctor between young and veteran doctors and the number of examinations that can be dealt with is slightlly larger than a yound doctor, but there are still examinations that are not allowed to be performed.

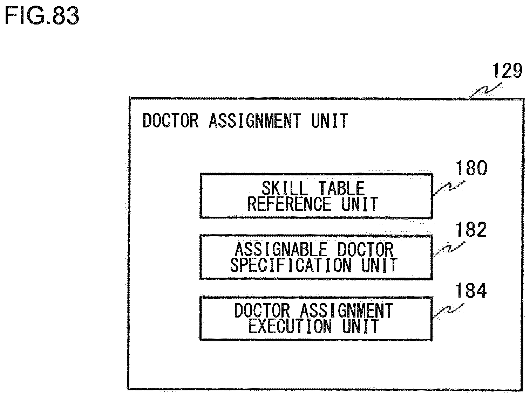

Fig. 83 illustrates the configuration of the doctor assignment unit 129. The doctor assignment unit 129 is configured to include a skill table reference unit 180, an assignable doctor specification unit 182, and a doctor assignment execution unit 184. The skill table reference unit 180 notifies the assignable doctor specification unit 182 of doctors who can perform an examination by referring to a doctor skill corresponding to an examination type from the doctor skill table stored in the storage unit 200. The assignable doctor specification unit 182 has the function of specifying doctors who can be assigned to the examination from the notified doctors. The doctor assignment execution unit 184 determines a doctor who is assigned to the examination from the doctors specified by the assignable doctor specification unit 182. In the following Examples 13 and 14, rescheduling processing, which is performed in the case where the doctor C has urgent business after the start of examination work, so he/she cannot take charge of an examination during the time zone, will be described. In Examples 13 and 14, it is assumed that the doctor C cannot perform an examination between 10:25 and 11:00.

### <Example 13>

An input interface, such as a keyboard or a mouse, is connected to the terminal device 12, and the input receiving unit 176 receives an input operation from a user via the terminal device 12. When a user, such as the doctor C or a nurse, inputs a time zone when the doctor C cannot perform an examination from the input interface, the input receiving unit 176 receives the examination impossible time zone (10:25 to 11:00) of the doctor C, and provides it to the situation information acquisition unit 170. The situation information acquisition unit 170 acquires the examination impossible time zone of the doctor C as situation information indicating the situation of a doctor, and provides it to the rescheduling processing unit 172.

The reschedule processing unit 172 determines based on the situation information whether it is necessary to change an element included in the examination schedule and/or the cleaning schedule illustrated in Fig. 76. Specifically, when receiving the examination impossible time zone (10:25 to 11:00) of the doctor C as the situation information, the rescheduling processing unit 172 reads the examination schedule information from the examination schedule holding unit 206 to investigate whether the examination, to which the doctor C is assigned, is included in the examination impossible time zone.

With reference to the examination schedule in Fig. 76, the examinations, which the docotr C takes charge of during 10:25 to 11:00, includes the examination E22 (10:25 to 10:35) and the examination E27 (10:45 to 11:10). Therefore, the rescheduling processing unit 172 determines that it is necessary to change the examination schedule and/or the cleaning schedule. At this time, the rescheduling processing unit 172 determines that it is necessary to change at least the examination schedule.

When determining based on the situation information that it is necessary to change the cleaning schedule and/or the examination schedule, the reschedule processing unit 172 instructs at least one of the examination schedule management unit 110 and the cleaning schedule management unit 130 to change an element included in the examination schedule and/or the cleaning schedule. Herein, the element included in the examination schedule is an examination specified by an examination ID, an examination room assigned to an examination, information on scheduled examination start time, that on scheduled examination end time, a primary doctor, or an endoscope 30; while the element included in the cleaning schedule is a cleaning machine assigned to an endoscope 30, information on scheduled cleaning start time, or that on scheduled cleaning end time. When a person-in-charge is assigned to cleaning processing in the cleaning schedule, as illustrated in Example 5 (Fig. 38), person-in-charge information is also included in the element included in the cleaning schedule.

Herein, the rescheduling processing unit 172 notifies the examination schedule management unit 110 that the doctor C cannot take charge of the examinations E22 and E27, and instructs to change an element of the examination schedule. At this time, the rescheduling processing unit 172 also notifies the examination impossible time zone of the doctor C. Thereby, the examination schedule management unit 110 deletes the primary doctor C assigned to the examinations E22 and E27, and instructs the doctor assignment unit 129 to assign another doctor other than the doctor C to the examinations E22 and E27.

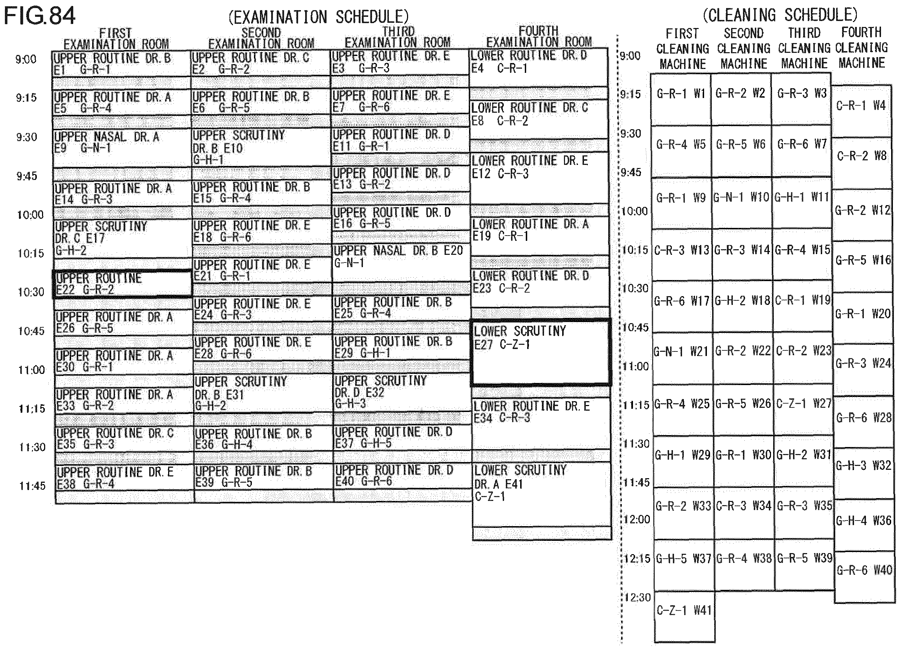

Fig. 84 illustrates a state where the doctor C assigned to the examinations E22 and E27 is deleted. The doctor assignment unit 129 executes processing for assigning a doctor, who is not yet be assigned, to the examinations E22 and E27. When a schedule element is changed, the subsequent schedule elements may be affected. Therefore, the doctor assignment unit 129 executes the doctor assignment processing on, of the examinations E22 and E27, the previously scheduled examination E22, and thereafter executes the doctor assignment processing on the examination E27.

In the doctor assignment unit 129 illustrated in Fig. 83, the skill table reference unit 180 notifies the assignable doctor specification unit 182 of doctors who can perform the examination E22 by referring to the record of the type (upper routine) of the examination E22 in the doctor skill table. In this record, the doctors A, B, C, D, and E are recorded as a doctor who can individually perform an upper routine examination, and therefore the skill table reference unit 180 notifies the assignable doctor specification unit 182 that all the doctors A to E can perform the examination E22.

The assignable doctor specification unit 182 excludes the doctor C from the notified doctors according to the instruction from the examination schedule management unit 110. Thereby, the assignable doctor specification unit 182 specifies the doctors A, B, D, and E as assignable doctors, and notifies the doctor assignment execution unit 184.

In Example 13, the doctor assignment execution unit 184 assigns a doctor to an examination such that the start time of the examination is as scheduled or delayed least. The doctors A, B, D, and E are specified as assignable doctors to the examination E22, and the doctor assignment execution unit 184 derives a scheduled start time when each assignable doctor is assigned to the examination E22, and determines a doctor whose derived scheduled start time is earliest. It is assumed that 5 minutes after the end of the previous examination, a doctor can take charge of the next examination.

When the doctor A is assigned to the examination E22, the scheduled end time of the previous examination E19 by the doctor A is 10:20, and hence the doctor A can start the examination E22 from the scheduled start time (10:25) thereof. When the doctor B is assigned to the examination E22, the scheduled end time of the previous examination E20 by the doctor B is 10:30, and hence the doctor B can start the examination E22 with a delay of 10 minutes from the scheduled start time (10:25) thereof. When the doctor D is assigned to the examination E22, the scheduled end time of the previous examination E23 by the doctor D is 10:40, and hence the doctor D can start the examination E22 with a delay of 20 minutes from the scheduled start time (10:25) thereof. When the doctor E is assigned to the examination E22, the scheduled end time of the previous examination E21 by the doctor E is 10:30, and hence the doctor E can start the examination E22 with a delay of 10 minutes from the scheduled start time (10:25) thereof.

As a result of the above verification, it is determined that the examination E22 can be started without a delay when the doctor A is assigned to the examination E22. Therefore, the doctor assignment execution unit 184 assigns the doctor A to the examination E22.

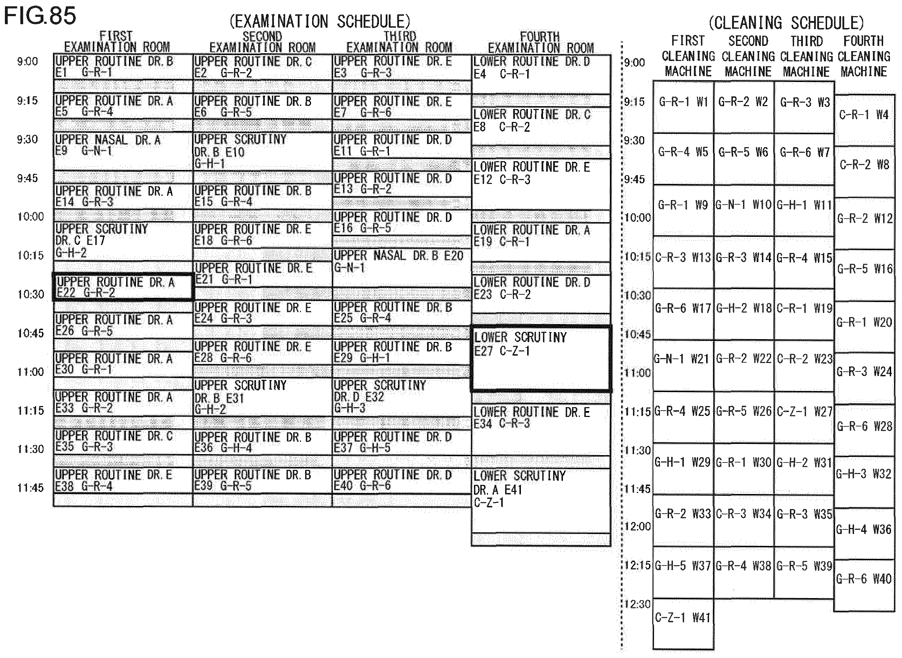

Fig. 85 illustrates a state where the doctor A is assigned to the examination E22. After the doctor assignment execution unit 184 assigns the doctor A to the examination E 22, the examination schedule management unit 110 investigates whether an inconsistency occurs between examination schedules between examination rooms when the doctor A is assigned to the examination E22. Herein, the case where an inconsistency occurs means: with respect to the doctor information, the case where the same doctor is assigned to a plurality of examinations overlapping each other in time; and with respect to the endoscope 30, the case where one endoscope 30 is assigned to a plurality of examinations overlapping each other in time, or the case where an endoscope 30, the cleaning of which is not yet completed, is assigned to an examination.

In Example 13, when the same doctor is assigned to a plurality of examinations overlapping each other in time, or when one endoscope 30 is assigned to a plurality of examinations overlapping each other in time, the examination schedule management unit 110 reschedules the examination schedule so as to resolve the inconsistency by adjusting an examination, the scheduled start time of which is later, to be shifted backward. In addition, when an endoscope 30, the cleaning of which is not yet completed, is assigned to an examination, the examination schedule management unit 110 reschedules the examination schedule so as to resolve the inconsistency by adjusting the examination such that the start time thereof is later than the scheduled cleaning end time of the endoscope 30.

The cleaning schedule management unit 130 investigates whether an inconsistency occurs between the changed examination schedule and the cleaning schedule. Herein, the case where an inconsistency occurs means the case where an endoscope 30, the examination of which is not yet ended, is assigned to cleaning in the cleaning schedule.

When an endoscope 30, the examination of which is not yet ended, is assigned to cleaning in the cleaning schedule, the cleaning schedule management unit 130 reschedules the cleaning schedule so as to resolve the inconsistency by adjusting the cleaning processing such that the scheduled cleaning start time is later than the scheduled ecxamination end time.

The examination schedule management unit 110 and the cleaning schedule management unit 130 reschedule the schedules by adjusting so as to alternately shift schedules backward such that inconsistencies are resolved, respectively, whereby schedules, in which an inconsistency is finally eliminated, can be reset.

Herein, as a result of assigning the doctor A to the examination E22, the examination schedule management unit 110 confirms that there is no inconsistency in the examination schedule, and the cleaning schedule management unit 130 confirms that there is no inconsistency in the cleaning schedule. Thereby, the assignment of the doctor A to the examination E22 is fixed.

Next, the skill table reference unit 180 notifies the assignable doctor specification unit 182 of doctors who can perform the examination E27 by referring to the record of the type (lower scrutiny) of the examination E27 in the doctor skill table. In this record, the doctors A, B, and C are recorded as a doctor who can individually perform an lower scrutiny examination, and therefore the skill table reference unit 180 notifies the assignable doctor specification unit 182 that the doctors A, B, and C can perform the examination E27.

The assignable doctor specification unit 182 excludes the doctor C from the notified doctors according to the instruction from the examination schedule management unit 110. Thereby, the assignable doctor specification unit 182 specifies the doctors A and B as assignable doctors, and notifies the doctor assignment execution unit 184.

The doctor assignment execution unit 184 derives a scheduled start time when each assignable doctor is assigned to the examination E27, and determines a doctor whose derived scheduled start time is earliest. It is assumed that 5 minutes after the end of the previous examination, a doctor can take charge of the next examination.

When the doctor A is assigned to the examination E27, the scheduled end time of the previous examination E26 by the doctor A is 10:50, and hence the doctor A can start the examination E27 with a delay of 10 minutes from the scheduled start time (10:45) thereof. When the doctor B is assigned to the examination E27, the scheduled end time of the previous examination E25 by the doctor B is 10:45, and hence the doctor B can start the examination E27 with a delay of 5 minutes from the scheduled start time (10:45) thereof.

As a result of the above verification, it is determined that the examination E27 can be started with a minimum delay time when the doctor B is assigned to the examination E27. Therefore, the doctor assignment execution unit 184 assigns the doctor B to the examination E27.

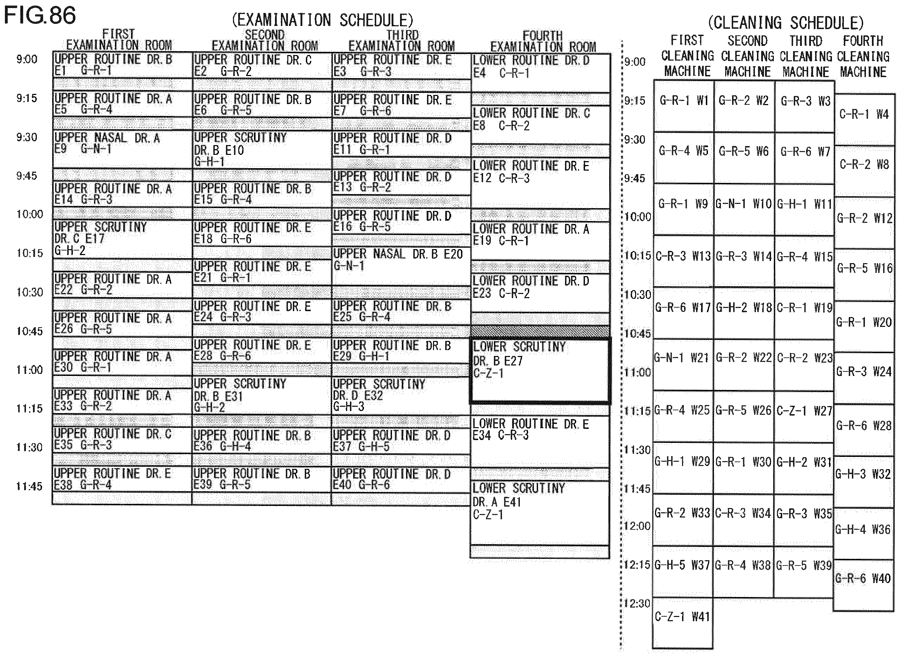

Fig. 86 illustrates a state where the doctor B is assigned to the examination E27. After the doctor assignment execution unit 184 assigns the doctor B to the examination E27, the examination schedule management unit 110 investigates whether an inconsistency occurs between examination schedules between examination rooms when the doctor B is assigned to the examination E27. When the same doctor is assigned to a plurality of examinations overlapping each other in time, or when one endoscope 30 is assigned to a plurality of examinations overlapping each other in time, the examination schedule management unit 110 reschedules the examination schedule so as to resolve the inconsistency by changing an examination, the scheduled start time of which is later, so as to be shifted backward in time. In addition, when an endoscope 30, the cleaning of which is not yet completed, is assigned to an examination, the examination schedule management unit 110 reschedules the examination schedule so as to resolve the inconsistency by adjusting the examination such that the start time thereof is later than the scheduled cleaning end time of the endoscope 30.

Herein, the doctor B assigned to the examination E 27 is a primary doctor of the examinations E29 and E31, the schedulesd examination times of which overlap each other, and the examination schedule management unit 110 determines that an inconsistency occurs in the examination schedule. Therefore, the examination schedule management unit 110 changes the time frames of both the examinations after the examination B31 in the second examination room 20b and the examinations after the examination E29 in the third examination room 20c to be shifted backward to the point where the inconsistency is resolved.

Next, the cleaning schedule management unit 130 investigates whether an inconsistency occurs between the changed examination schedule and the cleaning schedule. When an endoscope 30, the examination of which is not yet completed, is assigned to cleaning in the cleaning schedule, the cleaning schedule management unit 130 reschedules the cleaning schedule so as to resolve the inconsistency by adjusting the time of the cleaning processing such that the scheduled cleaning start time is later than the scheduled ecxamination end time.

The examination schedule management unit 110 and the cleaning schedule management unit 130 reschedule the schedules by adjusting so as to alternately shift schedules backward such that inconsistencies are resolved, respectively, whereby schedules, in which an inconsistency is finally eliminated, can be reset. Specifically, when it is determined that with respect to one of the examination schedule and the cleaning schedule that is changed such that there is no inconsistency, there is no inconsistency in the other of both the schedules, the examination schedule and the cleaning schedule are fixed.

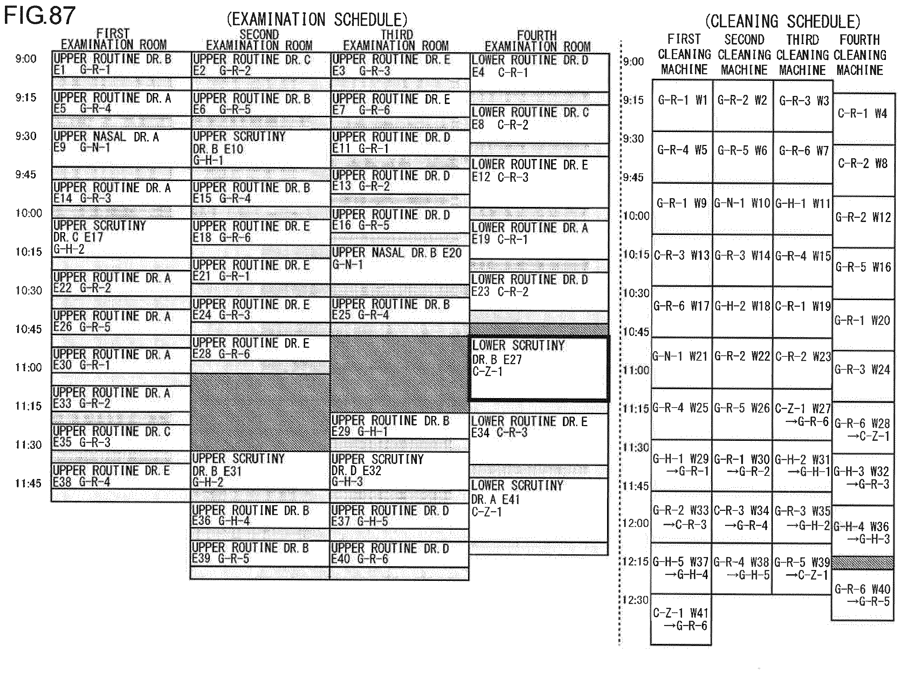

Fig. 87 illustrates the result of rescheduling the examination schedule and the cleaning schedule. In Example 13, rescheduling is executed so as not to change: the order of examinations in an examination room; the assigned endoscopes 30; and primary doctors of examinaions other than the examinations E22 and E27. Doctors, etc., confirm the schedule information before the start of examination work for one day, and often put into their mind what types of examinations are to be performed in which examination rooms and at which times. Therefore, it is meaningful for work efficiency to maintain as much as possible the schedule information set in advance before the start of the work, as described in Example 13.

### <Example 14>

Example 13 has described an example in which the doctor assignment unit 129 reassigns a doctor to the examinations E22 and E27 to which the doctor assignment was canceled due to convenience of the doctor C. As a result, the schedule information before the start of the work are almost maintained, but as illustrated in Fig. 87, there is a slight delay in each of the examination schedule and the cleaning schedule. Therefore, in Example 14, rescheduling processing for eliminating or reducing as much as possible a delay in the schedules will be described.

The input receiving unit 176 receives an input operation from a user via the terminal device 12, similarly to Example 13. When a user, such as a doctor C or a nurse, inputs a time zone when the doctor C cannot take charge of an examination from the input interface, the input receiving unit 176 receives the examination impossible time zone (10:25 to 11:00) of the doctor C, and provides it to the situation information acquisition unit 170. The situation information acquisition unit 170 acquires the examination impossible time zone of the doctor C as situation information indicating the situation of a doctor, and provides it to the rescheduling processing unit 172.

The reschedule processing unit 172 determines based on the situation information whether it is necessary to change an element included in the examination schedule and/or the cleaning schedule illustrated in Fig. 76. Specifically, when receiving the examination impossible time zone (10:25 to 11:00) of the doctor C as the situation information, the rescheduling processing unit 172 reads the examination schedule information from the examination schedule holding unit 206 to determine whether there is an examination that the doctor C takes charge of in the examination impossible time zone.

With reference to the examination schedule in Fig. 76, the examinations, which the docotr C takes charge of during 10:25 to 11:00, includes the examination E22 (10:25 to 10:30) and the examination E27 (10:45 to 11:10). Therefore, the rescheduling processing unit 172 determines that it is necessary to change the examination schedule and/or the cleaning schedule.

When determining based on the situation information that it is necessary to change the cleaning schedule and/or the examination schedule, the reschedule processing unit 172 instructs at least one of the examination schedule management unit 110 and the cleaning schedule management unit 130 to change an element included in the examination schedule and/or the cleaning schedule. Herein, the rescheduling processing unit 172 notifies the examination schedule management unit 110 that the doctor C cannot take charge of the examinations E22 and E27, and instructs to change an element of the examination schedule. At this time, the rescheduling processing unit 172 also notifies the examination impossible time zone of the doctor C. In Example 14, the examination schedule management unit 110 extracts examinations in all the examination rooms, the scheduled examination start times of which are included in the examination impossible time zone, and deletes the endoscope information assigned to the examinations from the extracted examinations.

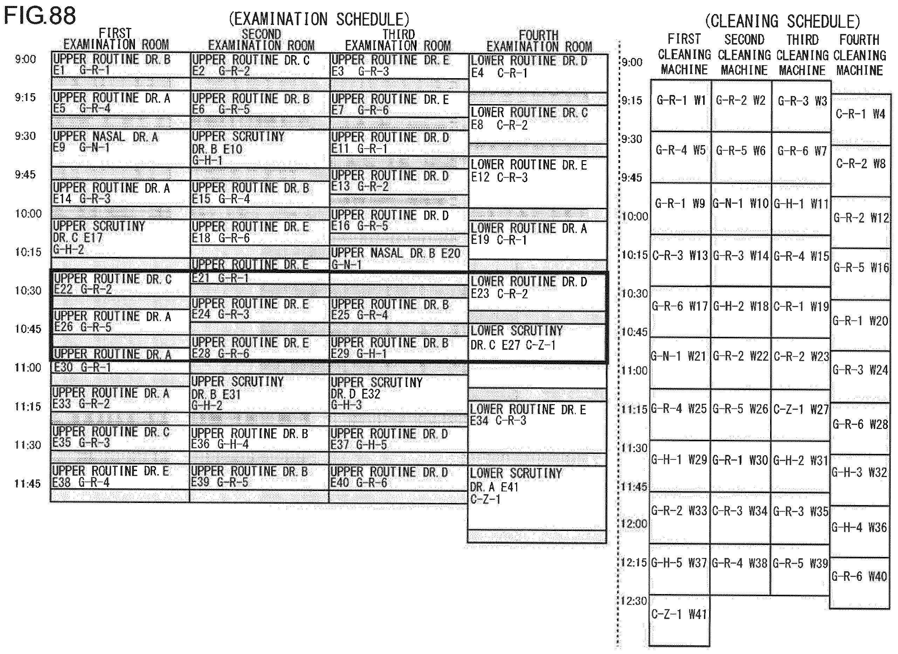

Fig. 88 illustrates the relationship between the examination schedule and the examination impossible time zone of the doctor C. In Fig. 88, the examination impossible time zone of the doctor C is surrounded by a black thick frame line. The examination schedule management unit 110 extracts examinations in all the examination rooms, which are scheduled to be started in the examination impossible time zone.

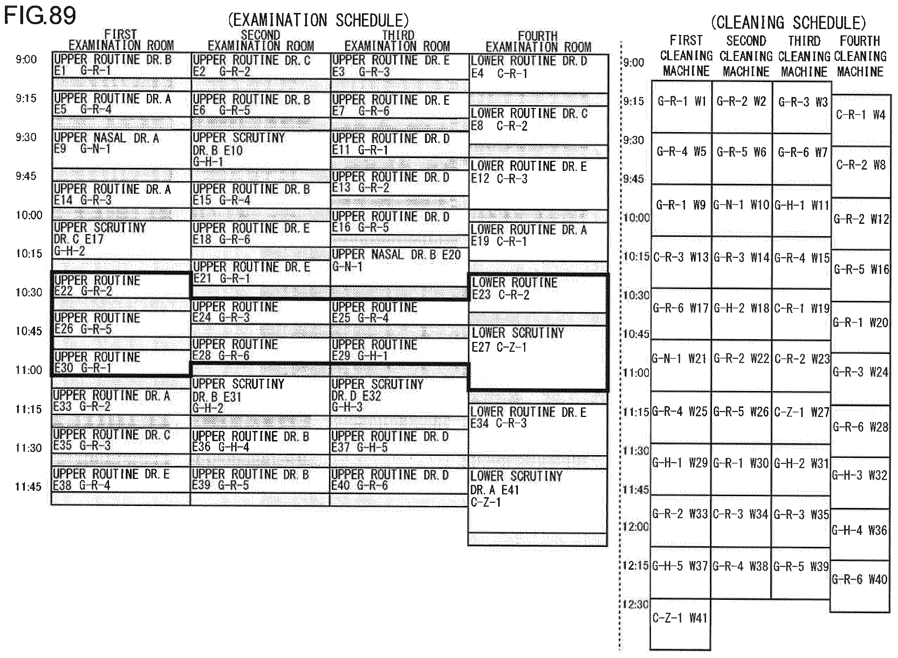

Fig. 89 illustrates a state where the endoscope information assigned to the examinations are deleted. The examination schedule management unit 110 extracts the examinations E22 to E30 as an examination whose scheduled examination start time is included in the examination impossible time zone of the doctor C, and deletes the assigned endoscope information. After deleting the assigned endoscope information, the examination schedule management unit 110 instructs the doctor assignment unit 129 to assign doctors different from the doctor C to the examinations E22 to E30.

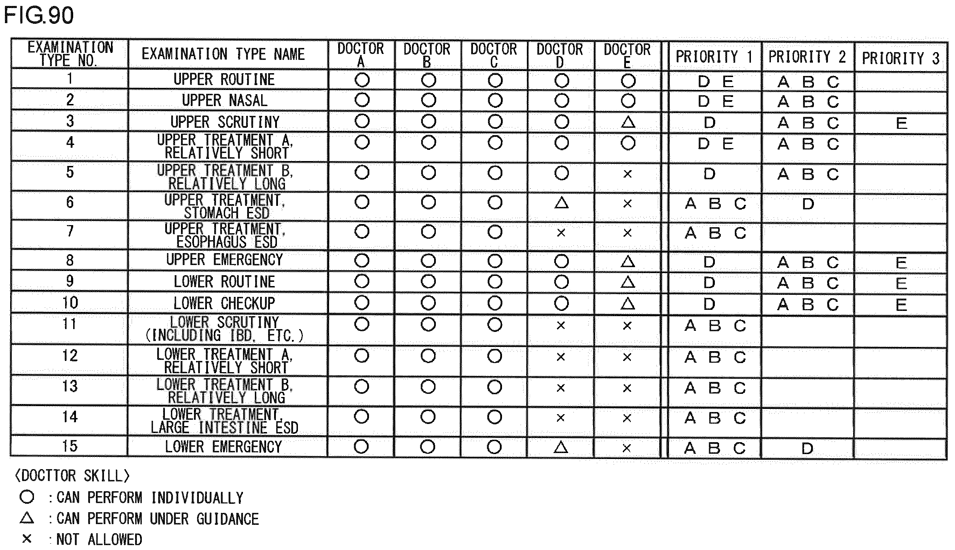

Fig. 90 illustrates another example of the doctor skill table. This doctor skill table records information indicating whether doctors can deal with examination types and priority orders indicating doctor to be preferentially assigned, by associating with each other. Herein, "Priority 1" is a doctor to be assigned with the highest priority, "Priority 2" is a doctor to be assigned with the second highest priority, and "Priority 3" is a doctor to be assigned with the third highest priority.

The priority order is defined in accordance with the skills of doctors who can deal with an examination type. For example, an upper routine examination can be dealt with by all the doctors A to E, but it is an examination requiring relatively little skill, and hence it is preferable to cause a doctor inexperienced as much as possible to take charge of the examination. Therefore, for an upper routine examination, the doctors D and E are set to be "Priority 1", and the doctors A to C are set to be "Priority 2."

In addition, for a lower scrutiny examination, the doctors A to C can deal with and the doctors D and E cannot, and hence the doctors A to C are set to be "Priority 1", and the orders of and after Priority 2 are not set. It can be said that the number of doctors who can deal with a highly difficult examination type is smaller than that of doctors who can deal with a less difficult examination type, as descrribed above.

Returning to Fig. 89, the doctor assignment unit 129 executes processing for assigning doctors, who are not yet be assigned, to the examinations E22 and E30. In Example 14, the doctor assignment unit 129 executes the doctor reassignment processing based on the assignment priority orders in the doctor skill table illustrated in Fig. 90. In this reassgnmment processing, a doctor is first assigned to a highly difficult examination. This is because the number of doctors who can be assigned to a highly difficult examination is small. The degree of difficulty of an examination is determined by the number of doctors who can deal with, and therefore of the examinations E22 to E30, the difficulty of a lower scrutiny examination of the examination E27 is the highest; and the difficulties of a lower routine examination of the examination E23 and upper routine examinations of the examinations E22, E24 to E26 and E28 to E30 are lower than that of the examinations E27 and these are the same as each other. As described above, the difficulty of an examination may be determined by the number of doctors who can deal with, but the degree of difficulty may be determined by referring to a prepared table in which the difficulties of examination types are defined in advance.

In this Example 14, a doctor is first assigned to the examination E27, and doctors are assigned to the remaining examinations in the order of earlier scheduled examination start times.

First, the doctor assignment unit 129 executes determination processing for assigning a doctor to the examination E27. In the doctor assignment unit 129 illustrated in Fig. 83, the skill table reference unit 180 refers to the priority order of the type (lower scrutiny) of the examination E 27 in the doctor skill table, and notifies the assignable doctor specification unit 182 of the doctors A to C whose priorities are set to be Priority 1. The assignable doctor specificatin unt 182 excludes the doctor C from the notifid doctos A to C according to the instruction from the examinatin schedue management unt 110. Thereby, the assignable doctor specification unit 182 specifies the doctors A and B as assignable doctors, and notifies the doctor assignment execution unit 184.

The doctor assignment execution unit 184 determines a doctor to be assigned to the examination E27 so as not to overlap, in an examination room different from the examination E27, both the doctor of an examination that is scheduled to be ended after the scheduled start time of the examination E27 and the doctor of an examination that is schedued to be started before the scheduled end time of the examination E27. Of the assignable doctors A and B, the doctor A is not assigned to an examination that overlaps in time, but the doctor B is assigned to the examination E31, and hence the doctor assignment execution unit 184 assigns the doctor A to the examination E27.

Next, the doctor assignment unit 129 executes determination processing for assigning a doctor to the examination E22. The skill table reference unit 180 refers to the priority order of the type (upper routine) of the examination E22 in the doctor skill table, and notifies the assignable doctor specification unit 182 of the doctors D and E whose priorities are set to be Priority 1. Thereby, the assignable doctor specification unit 182 specifies the doctors D and E as assignable doctors, and notifies the doctor assignment execution unit 184.

The doctor assignment execution unit 184 determines a doctor to be assigned to the examination E22 so as not to overlap, in an examination room different from the examination E22, both the doctor of an examination that is scheduled to be ended after the scheduled start time of the examination E22 and the doctor of an examination that is schedued to be started before the scheduled end time of the examination E22. Of the assignable doctors D and E, the doctor D is not assigned to an examination that overlaps in time, but the doctor E is assigned to the examination E21, and hence the doctor assignment execution unit 184 assigns the doctor D to the examination E22.

Next, the doctor assignment unit 129 executes determination processing for assigning a doctor to the examination E23. The skill table reference unit 180 refers to the priority order of the type (lower routine) of the examination E23 in the doctor skill table, and notifies the assignable doctor specification unit 182 of the doctor D whose priority is set to be Priority 1. Thereby, the assignable doctor specification unit 182 specifies the doctor D as an assignable doctor, and notifies the doctor assignment execution unit 184.

The doctor assignment execution unit 184 determines a doctor to be assigned to the examination E23 so as not to overlap, in an examination room different from the examination E23, both the doctor of an examination that is scheduled to be ended after the scheduled start time of the examination E23 and the doctor of an examination that is schedued to be started before the scheduled end time of the examination E23. Herein, the doctor D is assigned to the examination E22, and hence the doctor assignment execution unit 184 determines that the doctor D cannot be assigned to the examination E22.

In response to this determination result, the skill table reference unit 180 refers to the priority order of the type (lower routine) of the examination E23 in the doctor skill table, and notifies the assignable doctor specification unit 182 of the doctors A to C whose priorities are set to be Priority 2. The assignable doctor specificatin unt 182 excludes the doctor C from the notifid doctos A to C according to the instruction from the examinatin schedue management unt 110. Thereby, the assignable doctor specification unit 182 specifies the doctors A and B as assignablea doctors, and notifies the doctor assignment execution unit 184.

The doctor assignment execution unit 184 determines a doctor to be assigned to the examination E23 so as not to overlap, in an examination room different from the examination E23, both the doctor of an examination that is scheduled to be ended after the scheduled start time of the examination E23 and the doctor of an examination that is schedued to be started before the scheduled end time of the examination E23. Of the assignable doctors A and B, the doctor A is not assigned to an examination that overlaps in time, but the doctor B is assigned to the examination E20, and hence the doctor assignment execution unit 184 assigns the doctor A to the examination E23.

The doctor assignment unit 129 also executes the above processing on the remaining examinations E24 to E26 and E28 to E30. Thereby, doctors other than the doctor C are reassigned to the examinations E22 to E30. When the doctor assignment execution unit 184 cannot assign a doctor so as not to overlap in time, the doctor assignment execution unit 184 notifies the examination schedule management unit 110 of the fact, so that the examination schedule management unit 110 changes the examination schedule by adjusting so as to shift the time frame of an examination backward in time.

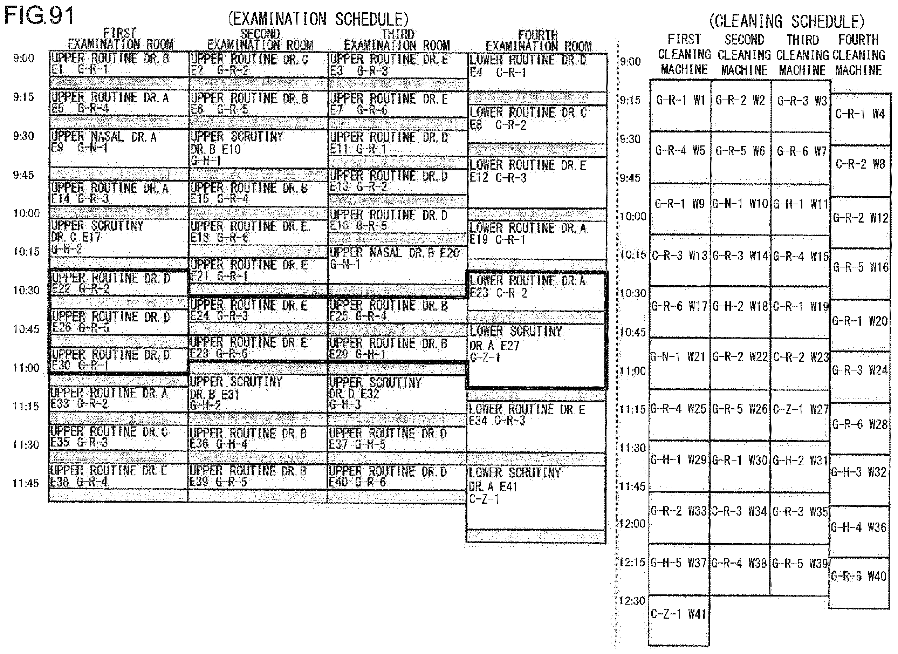

Fig. 91 illustrates the result of executing the doctor rescheduling processing. In this rescheduling result, the doctor A is assigned to the examinations E27 and E33 that are continuous in time, and the doctor D is also assigned to the examinations E30 and E32 that are continuous in time. When it is assumed that a doctor can take charge of the next examination after 5 minutes from the end of the previous examination, the respective time frames of the examinations E33 and E32 may be changed so as to be shifted backward by 5 minutes, respectively.

Examples 13 and 14 have described the case where when the doctor C becomes inconvenient after the start of examination work, a doctor is assigned to an examination, but also in the case where a person-in-charge of cleaning becomes inconvenient, which has been described in connection with Example 5, the rescheduling processing of Examples 13 and 14 can be applied.

The rescheduling processing of the present invention has been described above based on Examples 12 to 14. These Examples are illustrative in nature, and it should be appreciated by a person skilled in the art that various modifications can be made to the combinations of the components and the processing processes and such modifications also fall within the scope of the invention.

The above embodiment and Examples have described, for example, the doctor assignment unit 129 that assigns a doctor to an examination and the person-in-charge assignment unit 149 that assigns a person-in-charge of cleaning work. For example, an auxiliary work practitioner assignment unit, which assigns an auxiliary work practitioner who assists an examination to an examination, may be further provided in the information management device 10.

An examination, in which an assigned element cannot be changed, also exists in the examination schedule. If there is a circumstance in which an examination can be performed only in a certain time zone due to, for example, the convenience of a patient or a doctor, the scheduled examination start time of the examination cannot be moved. In addition, if an examination requires a special skill, only a doctor having the skill can take charge of the examination, and hence at least the primary doctor of the examination cannot be changed. Even with the endoscopes 30 of the same type, there are various models ranging from the latest one to an old-fashioned one, but for example, in a special examination, only the latest endoscope 30 may be used.

Therefore, in a variation, the change-disallowed examination specification unit 174 specifies an endoscopic examination in which the change of at least one element, of a plurality of elements assigned in advance to an endoscopic examination in the examination schedule, is made disallowed. An input interface, such as a keyboard or a mouse, is connected to the terminal device 12, and the input receiving unit 176 receives an input operation from a user via the terminal device 12. For example, when an examination schedule is generated and when a user designates an examination in which the change of an element is made disallowed, the input receiving unit 176 receives the designating operation from the user, and the change-disallowed examination specification unit 174 specifies an endoscopic examination in which the change of an element is made disallowed. Alternatively, it may be configured that a user can designate, of a plurality of elements that form the schedule of an endoscopic examination, an element, the change of which is made disallowed.

Herein, the elements that form the examination schedule include at least an assigned examination room, information on scheduled examination start time, that on scheduled examination end time, a primary doctor, and an endoscope ID. Of these elements, a user can designate that the change of, for example, the endoscope ID is prohibited. When such designation is made, the examination room, the scheduled examination times, and the primary doctor can be changed, and the change-disallowed examination specification unit 174 specifies an examination element, the change of which is made disallowed, so that such an examination element is not changed when the rescheduling processing is executed.

In the rescheduling processing described in Examples 12 to 14, the change-disallowed examination specification unit 174 specifies an examination in which the change of at least one element, of the elements assigned in advance to the examination, is made disallowed. The rescheduling processing unit 172 may notify the examination schedule management unit 110 of an element of the examination specified by the change-disallowed examination specification unit 174, the change of which is made disallowed, so that the change of the element is prohibited. Thereby, the examination schedule management unit 110 does not change the notified element of the examination schedule, but executes the rescheduling processing on other schedule elements.

Further, it has been described that an examination, in which the change of a schedule element is made disallowed, is specified by the change-disallowed examination specification unit 174, and that the rescheduling processing unit 172 notifies the examination schedule management unit 110 of the schedule element, the change of which is made disallowed; however, due to this processing, there is the possibility that when the examination schedule management unit 110 changes elements of the examination schedule, the changes of which are not disallowed, the endoscopes 30 to be assigned may run short, so that a situation where the schedule is greatly delayed, or the like may occur.

Therefore, the examination schedule management unit 110 holds a predetermined rescheduling condition with respect to the rescheduling, and notifies a user that the examination schedule cannot be changed when this rescheduling condition is not satisfied. The rescheduling condition may be set such that a delay of the start of an examination falls within a predetermined time (e. g. , one hour). When there is a schedule element, the change of which is made disallowed by the change-disallowed examination specification unit 174, and when the first assignment processing unit 120 assigns an endoscope 30 and a doctor, a restriction is also placed on the flexibility of the assignment, because there is a schedule element, the change of which is made disallowed. Therefore, when the examination schedule management unit 110 causes the first assignment processing unit 120 to execute schedule element reassignment processing in order to change an element of the examination schedule, it is preferable to notify a user that the examination schedule cannot be changed when the predetermined rescheduling condition is not satisfied.

Even when the rescheduling condition is satisfied and the examination schedule can be changed, but when the time frame of an examination is adjusted, it is preferable for the examination schedule management unit 110 to notify a user of the fact. As a result, a user, such as a doctor, can recognize that the end of the examination will be delayed than scheduled, and it becomes possible to take action, such as to review the schedule of himself/herself after the end of the examination.

Although various scheduling and rescheduling algorithm have been proposed in the present application, it is also possible to generate a plurality of types of examination schedules and cleaning schedules by utilizing some of these scheduling and rescheduling algorithms. Therefore, the display processing unit 150 has the function of displaying the changed examination schedule and/or the changed cleaning schedule on the display of the terminal device 12, and when there are a plurality of candidates for the examination schedule and/or the cleaning schedule, each candidate may be displayed on the display such that a user can select any one of the candidates. When a user selects any one of the candidates, the examination schedule management unit 110 and the cleaning schedule management unit 130 fix the changes of the examination schedule and/or the cleaning schedule.

## Claims

1. An endoscopic examination work support system comprising:
an examination schedule management unit that manages an examination schedule of a plurality of endoscopic examinations, including an examination room where an endoscopic examination is to be performed, information on scheduled examination start time, that on scheduled examination end time, and examination type information on an examination content of an endoscopic examination;
a first assignment processing unit that assigns, from a plurality of endoscopes, an endoscope to be used to each endoscopic examination managed by the examination schedule management unit;
a second assignment processing unit that assigns, from a plurality of cleaning machines, a cleaning machine for cleaning an endoscope to be used in the each endoscopic examination; and
a cleaning schedule management unit that manages a cleaning schedule of a plurality of endoscopes, including a cleaning machine, information on scheduled cleaning start time, and that on scheduled cleaning end time, wherein
the endoscopic examination work support system further comprises:
a situation information acquisition unit that acquires situation information on a situation of an examination; and
a rescheduling processing unit that determines based on the situation information that it is necessary to change an examination schedule and/or a cleaning schedule, wherein
the rescheduling processing unit instructs at least one of the examination schedule management unit and the cleaning schedule management unit to change an element included in the examination schedule and/or the cleaning schedule.

2. The endoscopic examination work support system according to claim 1, further comprising:
a change-disallowed examination specification unit that specifies an endoscopic examination in which, the change of at least one element, of the elements assigned in advance to an endoscopic examination in the examination schedule, is made disallowed, and wherein
the rescheduling processing unit notifies the examination schedule management unit of an element, of the endoscopic examination specified by the change-disallowed examination specification unit, the change of which is made disallowed, so that the change of the element is prohibited.

3. The endoscopic examination work support system according to claim 2, wherein
the change-disallowed examination specification unit specifies an endoscopic examination in which the changes of all the elements assigned in advance to an endoscopic examination are made disallowed, and wherein
the rescheduling processing unit instructs to change an element included in an examination schedule of an examination other than the endoscopic examination specified by the change-disallowed examination specification unit.

4. The endoscopic examination work support system according to claim 2, wherein
after the rescheduling processing unit notifies the examination schedule management unit of the element, the change of which is made disallowed, and when the examination schedule management unit changes an element of an examination schedule, the change of which is not made disallowed, the examination schedule management unit notifies a user that the examination schedule cannot be changed when a predetermined rescheduling condition is not satisfied.

5. The endoscopic examination work support system according to claim 1, further comprising:
a display processing unit that displays the changed examination schedule and/or cleaning schedule on a display, wherein
when there are a plurality of candidates for the examination schedule and/or the cleaning schedule, the display processing unit displays each candidate on the display, and wherein
when a user selects one of the candidates, the change of the examination schedule and/or the cleaning schedule is fixed.

6. The endoscopic examination work support system according to claim 1, wherein
the examination schedule management unit specifies, based on a schedule element change instruction, an examination room by which it becomes necessary to change the examination schedule, and changes the information on scheduled examination start time and that on scheduled examination end time of an endoscopic examination in the specified examination room.

7. The endoscopic examination work support system according to claim 6, wherein
the second assignment processing unit assigns a cleaning machine for cleaning an endoscope, which is assigned by first assignment processing unit to the endoscopic examination whose information on scheduled examination start time and that on scheduled examination end time were changed, so that a time after the scheduled examination end time of the endoscope becomes equal to a scheduled cleaning start time, and wherein
the first assignment processing unit assigns the endoscope to an endoscopic examination such that a time after the scheduled end time of cleaning by a cleaning machine assigned to the endoscope by the second assignment processing unit becomes equal to a scheduled examination start time.

8. The endoscopic examination work support system according to claim 1, wherein
when an examination reservation is canceled, the examination schedule management unit advances an examination of the same type, which is scheduled later than the canceled examination.

9. An endoscopic examination work support system comprising:
an examination schedule management unit that manages an examination schedule of a plurality of endoscopic examinations, including an examination room where an endoscopic examination is to be performed, information on scheduled examination start time, that on scheduled examination end time, and examination type information on an examination content of an endoscopic examination;
a first assignment processing unit that assigns, from a plurality of endoscopes, an endoscope to be used to each endoscopic examination managed by the examination schedule management unit;
a second assignment processing unit that assigns, from a plurality of cleaning machines, a cleaning machine for cleaning an endoscope to be used in the each endoscopic examination; and
a cleaning schedule management unit that manages a cleaning schedule of a plurality of endoscopes, including a cleaning machine, information on scheduled cleaning start time, and that on scheduled cleaning end time, wherein
the endoscopic examination work support system further comprises:
a situation information acquisition unit that acquires situation information on a situation of an endoscope; and
a rescheduling processing unit that determines based on the situation information that it is necessary to change the examination schedule and/or the cleaning schedule, and wherein
the rescheduling processing unit instructs at least one of the examination schedule management unit and the cleaning schedule management unit to change an element included in the examination schedule and/or the cleaning schedule.

10. The endoscopic examination work support system according to claim 9, wherein
the situation information acquisition unit acquires, as the situation information, identification information for specifying an endoscope to be used, and wherein
when the endoscope identification information acquired by the situation information acquisition unit is different from the endoscope identification information assigned by the first assignment processing unit to an endoscopic examination in the examination schedule, the rescheduling processing unit instructs at least one of the examination schedule management unit and the cleaning schedule management units to change an element included in the examination schedule and/or the cleaning schedule.

11. The endoscopic examination work support system according to claim 10, wherein
the examination schedule management unit changes the endoscope identification information assigned to an endoscopic examination in the examination schedule to the endoscope identification information acquired as the situation information, and the cleaning schedule management unit changes the cleaning schedule of an endoscope in the cleaning schedule to the cleaning schedule of an endoscope acquired as the situation information.

12. The endoscopic examination work support system according to claim 11, wherein
the examination schedule management unit determines whether the endoscope changed in the examination schedule can be used as scheduled in an examination scheduled thereafter.

13. The endoscopic examination work support system according to claim 10, wherein
when the situation information acquisition unit acquires identification information different from the assigned identification information in addition to the identification information on the endoscope assigned by the first assignment processing unit to an endoscopic examination in the examination schedule, the examination schedule management unit registers into the examination schedule the acquired different identification information in addition to the identification information on the endoscope assigned to an endoscopic examination in the examination schedule.

14. The endoscopic examination work support system according to claim 9, wherein
the situation information acquisition unit aquires, as the situation information, endoscope identification information along with information indicating that the endoscope is in a state where the use of it is disallowed, and wherein
the rescheduling processing unit notifies at least one of the examination schedule management unit and the cleaning schedule management units of the identification information on the endoscope the use of which is disallowed.

15. The endoscopic examination work support system according to claim 9, further comprising:
a change-disallowed examination specification unit that specifies an endoscopic examination in which the change of at least one element, of the elements assigned in advance to an endoscopic examination in the examination schedule, is made disallowed, wherein
the rescheduling processing unit notifies the examination schedule management unit of an element of the endoscopic examination specified by the change-disallowed examination specification unit, the change of which is made disallowed, so that the change of the element is prohibited.

16. The endoscopic examination work support system according to claim 15, wherein
after the rescheduling processing unit notifies the examination schedule management unit of the element, the change of which is made disallowed, and when the examination schedule management unit changes an element of the examination schedule, the change of which is not made disallowed, the examination schedule management unit notifies a user that the examination schedule cannot be changed when a predetermined rescheduling condition is not satisfied.

17. The endoscopic examination work support system according to claim 9, further comprising:
a display processing unit that displays the changed examination schedule and/or cleaning schedule on a display, wherein
when there are a plurality of candidates for the examination schedule and/or the cleaning schedule, the display processing unit displays each candidate on the display, and wherein
when a user selects one of the candidates, the change of the examination schedule and/or the cleaning schedule is fixed.

18. An endoscopic examination work support system comprising:
an examination schedule management unit that manages an examination schedule of a plurality of endoscopic examinations, including an examination room where an endoscopic examination is to be performed, information on scheduled examination start time, that on scheduled examination end time, and examination type information on an examination content of an endoscopic examination;
a first assignment processing unit that assigns, from a plurality of endoscopes, an endoscope to be used to each endoscopic examination managed by the examination schedule management unit;
a second assignment processing unit that assigns, from a plurality of cleaning machines, a cleaning machine for cleaning an endoscope to be used in the each endoscopic examination; and
a cleaning schedule management unit that manages a cleaning schedule of a plurality of endoscopes, including a cleaning machine, information on scheduled cleaning start time, and that on scheduled cleaning end time, wherein
the endoscopic examination work support system further comprises:
a situation information acquisition unit that acquires situation information on a situation of an cleaning machine; and
a rescheduling processing unit that determines based on the situation information that it is necessary to change the cleaning schedule and/or the examination schedule, and wherein
the rescheduling processing unit instructs at least one of the cleaning schedule management unit and the examination schedule management unit to change an element included in the examination schedule and/or the cleaning schedule.

19. The endoscopic examination work support system according to claim 18, wherein
the cleaning schedule management unit specifies, based on a schedule element change instruction, a cleaning machine by which it becomes necessary to change the cleaning schedule, and changes the information on scheduled cleaning start time and that on scheduled cleaning end time of the specified cleaning machine.

20. An endoscopic examination work support system comprising:
an examination schedule management unit that manages an examination schedule of a plurality of endoscopic examinations, including an examination room where an endoscopic examination is to be performed, information on scheduled examination start time, that on scheduled examination end time, and examination type information on an examination content of an endoscopic examination;
a first assignment processing unit that assigns, from a plurality of endoscopes, an endoscope to be used to each endoscopic examination managed by the examination schedule management unit;
a second assignment processing unit that assigns, from a plurality of cleaning machines, a cleaning machine for cleaning an endoscope to be used in the each endoscopic examination; and
a cleaning schedule management unit that manages a cleaning schedule of a plurality of endoscopes, including a cleaning machine, information on scheduled cleaning start time, and that on scheduled cleaning end time, wherein
the first assignment processing unit has a function of assigning a doctor to an endoscopic examination, wherein
the endoscopic examination work support system further comprises:
a situation information acquisition unit that acquires situation information on a situation of a doctor; and
a rescheduling processing unit that determines based on the situation information that it is necessary to change the examination schedule and/or the cleaning schedule, and wherein
the rescheduling processing unit instructs at least one of the examination schedule management unit and the cleaning schedule management unit to change an element included in the examination schedule and/or the cleaning schedule.

21. The endoscopic examination work support system according to claim 20, wherein
the situation information acquisition unit acquires a time zone when a doctor cannot perform an examination as the situation information, and wherein
when an examination to which the doctor is assigned is included in the time zone with reference to examination schedule information, the rescheduling processing unit determines that it is necessary to change the examination schedule.

22. The endoscopic examination work support system according to claim 21, wherein
when receiving an element change instruction from the rescheduling processing unit, the examination schedule management unit instructs the first assignment processing unit to assign another doctor to the examination that cannot be performed by the doctor.

23. The endoscopic examination work support system according to claim 21, wherein
the first assignment processing unit assigns a doctor different from the doctor to an examination in each examination room where an examination is scheduled to be started in the time zone.

24. The endoscopic examination work support system according to claim 20, wherein
the first assignment processing unit assigns a doctor to an examination based on a skill of a doctor associated with an examination type.

25. The endoscopic examination work support system according to claim 20, wherein
the first assignment processing unit assigns a doctor to an examination based on an assignment priority order of a doctor associated with an examination type.

26. The endoscopic examination work support system according to claim 18, further comprising:
a change-disallowed examination specification unit that specifies an endoscopic examination in which the change of at least one element, of the elements assigned in advance to an endoscopic examination in the examination schedule, is made disallowed, wherein
the rescheduling processing unit notifies the examination schedule management unit of an element of the endoscopic examination specified by the change-disallowed examination specification unit, the change of which is made disallowed, so that the change of the element is prohibited.

27. The endoscopic examination work support system according to claim 26, wherein
after the rescheduling processing unit notifies the examination schedule management unit of the element, the change of which is made disallowed, and when the examination schedule management unit changes an element of the examination schedule, the change of which is not made disallowed, the examination schedule management unit notifies a user that the examination schedule cannot be changed when a predetermined rescheduling condition is not satisfied.

28. The endoscopic examination work support system according to claim 18, further comprising:
a display processing unit that displays the changed examination schedule and/or cleaning schedule on a display, wherein
when there are a plurality of candidates for the examination schedule and/or the cleaning schedule, the display processing unit displays each candidate on the display, and wherein
when a user selects one of the candidates, the change of the examination schedule and/or the cleaning schedule is fixed.
